# EUROPEAN PATENT SPECIFICATION

(11) **EP 0 831 911 B1**
(45) Date of publication and mention of the grant of the patent: **17.04.2002**
(21) Application number: 96919173.3
(22) Date of filing: 05.06.1996
(51) Int. Cl.: A61K 45/06, A61K 31/585, A61K 31/41, A61P 9/00

(54) **SPIRONOLACTONE AND ANGIOTENSIN II ANTAGONIST COMBINATION THERAPY FOR TREATMENT OF CONGESTIVE HEART FAILURE**
KOMBINATIONSTHERAPIE ZUR BEHANDLUNG DES KONGESTIVEN HERZVERSAGENS MIT SPIRONOLACTON UND ANGIOTENSIN II-ANTAGONIST
THERAPIE MIXTE A BASE DE SPIRONOLACTONE ET D'UN ANTAGONISTE DE L'ANGIOTENSINE II POUR LE TRAITEMENT DE L'INSUFFISANCE CARDIAQUE GLOBALE

(30) Priority: 07.06.1995 US 486089
(43) Date of publication of application: 01.04.1998
(73) Proprietor: G.D. SEARLE & CO., Chicago, IL 60680-5110 (US)
(72) Inventor: MACLAUGHLAN, Todd, E., Grayslake, IL 60093 (US); SCHUH, Joseph, R., St. Louis, MO 63146 (US)
(74) Representative: Beil, Hans Christoph, Dr.
(86) International application number: US9609342
(87) International publication number: WO9640258

(56) References cited:
- EP-A- 0 481 448
- EP-A- 0 628 313
- WO-A-91/12001
- WO-A-91/15206
- WO-A-92/20662
- WO-A-94/09778
- WO-A-95/15166
- US-A- 3 257 390
- US-A- 4 129 564
- US-A- 4 789 668
- US-A- 5 049 565
- US-A- 5 264 447
- ANNALS OF INTERNAL MEDICINE, vol. 87, no. 2, 1987, pages 183-187, XP000610206 G.H. ANDERSON ET AL: "DIURETIC THERAPY AND RESPONSE OF ESSENTIAL HYPERTENSION TO SARALASIN"

## Description

### Field of the Invention

Combinations of spirolactone and an angiotensin II receptor antagonist are described for use in treatment of circulatory disorders, including cardiovascular diseases such as hypertension, congestive heart failure, cirrhosis and ascites.

### Background of the Invention

Myocardial (or cardiac) failure, whether a consequence of a previous myocardial infarction, heart disease associated with hypertension, or primary cardiomyopathy, is a major health problem of worldwide proportions. The incidence of symptomatic heart failure has risen steadily over the past several decades.

In clinical terms, decompensated cardiac failure consists of a constellation of signs and symptoms that arises from congested organs and hypoperfused tissues to form the congestive heart failure (CHF) syndrome. Congestion is caused largely by increased venous pressure and by inadequate sodium (Na⁺) excretion, relative to dietary Na⁺ intake, and is importantly related to circulating levels of aldosterone (ALDO). An abnormal retention of Na⁺ occurs via tubular epithelial cells throughout the nephron, including the later portion of the distal tubule and cortical collecting ducts, where ALDO receptor sites are present.

ALDO is the body's most potent mineralocorticoid hormone. As connoted by the term mineralocorticoid, this steroid hormone has mineral-regulating activity. It promotes Na⁺ reabsorption not only in the kidney, but also from the lower gastrointestinal tract and salivary and sweat glands, each of which represents classic ALDO-responsive tissues. ALDO regulates Na⁺ and water resorption at the expense of potassium (K⁺) and magnesium (Mg²⁺) excretion.

ALDO can also provoke responses in nonepithelial cells. Elicited by a chronic elevation in plasma ALDO level that is inappropriate relative to dietary Na⁺ intake, these responses can have adverse consequences on the structure of the cardiovascular system. Hence, ALDO can contribute to the progressive nature of myocardial failure for multiple reasons.

Multiple factors regulate ALDO synthesis and metabolism, many of which are operative in the patient with myocardial failure. These include renin as well as non-renin-dependent factors (such as K⁺, ACTH) that promote ALDO synthesis. Hepatic blood flow, by regulating the clearance of circulating ALDO, helps determine its plasma concentration, an important factor in heart failure characterized by reduction in cardiac output and hepatic blood flow.

The renin-angiotensin-aldosterone system (RAAS) is one of the hormonal mechanisms involved in regulating pressure/volume homeostasis and also in the development of hypertension. Activation of the renin-angiotensinaldosterone system begins with renin secretion from the juxtaglomerular cells in the kidney and culminates in the formation of angiotensin II, the primary active species of this system. This octapeptide, angiotensin II, is a potent vasoconstrictor and also produces other physiological effects such as stimulating aldosterone secretion, promoting sodium and fluid retention, inhibiting renin secretion, increasing sympathetic nervous system activity, stimulating vasopressin secretion, causing positive cardiac inotropic effect and modulating other hormonal systems.

Previous studies have shown that antagonizing angiotensin II binding at its receptors is a viable approach to inhibit the renin-angiotensin system, given the pivotal role of this octapeptide which mediates the actions of the renin-angiotensin system through interaction with various tissue receptors. There are several known angiotensin II antagonists, most of which are peptidic in nature. Such peptidic compounds are of limited use due to their lack of oral bioavailability or their short duration of action. Also, commercially-available peptidic angiotensin II antagonists (e.g., Saralasin) have a significant residual agonist activity which further limit their therapeutic application.

Non-peptidic compounds with angiotensin II antagonist properties are known. For example, early descriptions of such non-peptidic compounds include the sodium salt of 2-n-butyl-4-chloro-1-(2-chlorobenzyl)imidazole-5-acetic acid which has specific competitive angiotensin II antagonist activity as shown in a series of binding experiments, functional assays and in vivo tests [P. C. Wong et al, J. Pharmacol. Exp. Ther., 247(1), 1-7 (1988)]. Also, the sodium salt of 2-butyl-4-chloro-1-(2-nitrobenzyl)imidazole-5-acetic acid has specific competitive angiotensin II antagonist activity as shown in a series of binding experiments, functional assays and in vivo tests [A. T. Chiu et al, European J. Pharmacol., 157, 31-21 (1988)]. A family of l-benzylimidazole-5-acetate derivatives has been shown to have competitive angiotensin II antagonist properties [A. T. Chiu et al, J. Pharmacol. Exp. Ther., 250(3), 867-874 (1989)]. U.S. Patent No. 4,816,463 to Blankey et al describes a family of 4,5,6,7-tetrahydro-1H-imidazo(4,5-c)-tetrahydro-pyridine derivatives useful as antihypertensives, some of which are reported to antagonize the binding of labelled angiotensin II to rat adrenal receptor preparation and thus cause a significant decrease in mean arterial blood pressure in conscious hypertensive rats. Other families of non-peptidic angiotensin II antagonists have been characterized by molecules having a biphenylmethyl moiety attached to a heterocyclic moiety. For example, EP No. 253,310, published 20 January 1988, describes a series of aralkyl imidazole compounds, including in particular a family of biphenylmethyl substituted imidazoles, as antagonists to the angiotensin II receptor. EP No. 323,841 published 12 July 1989 describes four classes of angiotensin II antagonists, namely, biphenylmethylpyrroles, biphenylmethylpyrazoles, biphenylmethyl-1,2,3-triazoles and biphenylmethyl 4-substituted-4H-1,2,4-triazoles, including the compound 3,5-dibutyl-4-[(2'-carboxybiphenyl-4-yl)methyll-4H-1,2,4-triazole. U.S. Patent No. 4,880,804 to Carini et al describes a family of biphenylmethylbenzimidazole compounds as angiotensin II receptor blockers for use in treatment of hypertension and congestive heart failure.

Many aldosterone receptor blocking drugs are known. For example, spironolactone is a drug which acts at the mineralocorticoid receptor level by competitively inhibiting aldosterone binding. This steroidal compound has been used for blocking aldosterone-dependent sodium transport in the distal tubule of the kidney in order to reduce edema and to treat essential hypertension and primary hyperaldosteronism [F. Mantero et al, Clin. Sci. Mol. Med., 45 (Suppl 1), 219s-224s (1973)].
Spironolactone is also used commonly in the treatment of other hyperaldosterone-related diseases such as liver cirrhosis and congestive heart failure [F.J. Saunders et al, Aldactone; Spironolactone: A Comprehensive Review, Searle, New York (1978)]. Progressively-increasing doses of spironolactone from 1 mg to 400 mg per day [i.e., 1 mg/day, 5 mg/day, 20 mg/day] were administered to a spironolactone-intolerant patient to treat cirrhosis- related ascites [P.A. Greenberger et al, N. Eng. Reg. Allergy Proc., 7(4), 343-345 (Jul-Aug, 1986)]. It has been recognized that development of myocardial fibrosis is sensitive to circulating levels of both Angiotensin II and aldosterone, and that the aldosterone antagonist Spironolactone prevents myocardial fibrosis in animal models, thereby linking aldosterone to excessive collagen deposition [D. Klug et al, Am. J. Cardiol., 71 (3), 46A-54A (1993)]. Spironolactone has been shown to prevent fibrosis in animal models irrespective of the development of left ventricular hypertrophy and the presence of hypertension [C.G. Brilla et al, J. Mol. Cell. Cardiol., 25(5), 563-575 (1993)]. Spironolactone at a dosage ranging from 25 mg to 100 mg daily is used to treat diuretic-induced hypokalemia, when orally-administered potassium supplements or other potassium-sparing regimens are considered inappropriate [Physicians' Desk Reference, 46th Edn., p. 2153, Medical Economics Company Inc., Montvale, N.J. (1992)].

Previous studies have shown that inhibiting ACE inhibits the renin-angiotensin system by substantially complete blockade of the formation of angiotensin II.
Many ACE inhibitors have been used clinically to control hypertension. While ACE inhibitors may effectively control hypertension, side effects are common including chronic cough, skin rash, loss of taste sense, proteinuria and neutropenia.

Moreover, although ACE inhibitors effectively block the formation of angiotensin II, aldosterone levels are not well controlled in certain patients having cardiovascular diseases. For example, despite continued ACE inhibition in hypertensive patients receiving captopril, there has been observed a gradual return of plasma aldosterone to baseline levels [J. Staessen et al, J. Endocrinol., 91, 457-465 (1981)]. A similar effect has been observed for patients with myocardial infarction receiving zofenopril [C. Borghi et al, J. Clin. Pharmacol., 33, 40-45 (1993)]. This phenomenon has been termed "aldosterone escape".

Another series of steroidal-type aldosterone receptor antagonists is exemplified by epoxy-containing spironolactone derivatives. For example, U.S. Patent No. 4,559,332 issued to Grob et al describes 9α,11α-epoxy-containing spironolactone derivatives as aldosterone antagonists useful as diuretics. These 9α,11α-epoxy steroids have been evaluated for endocrine effects in comparison to spironolactone [M. de Gasparo et al, J. Pharm. Exp. Ther., 240(2), 650-656 (1987)].

Combinations of an aldosterone antagonist and an ACE inhibitor have been investigated for treatment of heart failure. It is known that mortality is higher in patients with elevated levels of plasma aldosterone and that aldosterone levels increase as CHF progresses from activation of the Renin-Angiontensin-Aldosterone System (RAAS). Routine use of a diuretic may further elevate aldosterone levels. ACE inhibitors consistently inhibit angiotensin II production but exert only a mild and transient antialdosterone effect.

Combining an ACE inhibitor and spironolactone has been suggested to provide substantial inhibition of the entire RAAS. For example, a combination of enalapril and spironolactone has been administered to ambulatory patients with monitoring of blood pressure [P. Poncelet et al, Am. J. Cardiol., 65(2), 33K-35K (1990)]. In a 90-patient study, a combination of captopril and spironolactone was administered and found effective to control refractory CHF without serious incidents of hyperkalemia [U. Dahlstrom et al, Am. J. Cardiol., 71, 29A-33A (21 Jan 1993)]. Spironolactone coadministered with an ACE inhibitor was reported to be highly effective in 13 of 16 patients afflicted with congestive heart failure [A.A. van Vliet et al, Am. J. Cardiol., 71, 21A-28A (21 Jan 1993)]. Clinical improvements have been reported for patients receiving a co-therapy of spironolactone and the ACE inhibitor enalapril, although this report mentions that controlled trials are needed to determine the lowest effective doses and to identify which patients would benefit most from combined therapy [F. Zannad, Am. J. Cardiol., 71(3), 34A-39A (1993)].

Combinations of an angiotensin II receptor antagonist and aldosterone receptor antagonist, are known. For example, PCT Application No. US91/09362 published 25 June 1992 describes treatment of hypertension using a combination of an imidazole- containing angiotensin II antagonist compound and a diuretic such as spironolactone.

WO-A-94 09778 US-A-5 264 447 EP-A-0 628 313 EP-A-0 481 448-WO-A-91 12001 US-A-5 049 565 WO-A-92 20662 US-A-4 129 564 US-A-4 789 668 US-A-3 257 390 disclose pharmaceutical compositions comprising angiotensin II antagonists e.g. phenyl- or biphenyl-substituted-heterocyclic compounds, optionally having an acidic function in combination with a diuretic, such as spironolactone for the treatment of hypertensin/cardiovascular disease.
The aldosterone antagonsists are usually used in a diuretic amount.

### Summary of the Invention

A combination for therapy comprising a therapeutically-effective amount of a determined angiotensin II receptor antagonist and a therapeutically-effective, non-diuretic amount of the epoxy- free spirolactone- type aldosteron receptor antagonist spironolactone is described as useful to treat circulatory disorders, including cardiovascular disorders such as hypertension, congestive heart failure, cirrhosis and ascites.

The angiotensin II receptor antagonist has the ability to interact with a receptor site located on various human body tissues, which site is a receptor having a relatively high affinity for angiotensin II and which receptor site is associated with mediating one or more biological functions or events such as vasoconstriction or vasorelaxation, kidney-mediated sodium and fluid retention, sympathetic nervous system activity, and in modulating secretion of various substances such as aldosterone, vasopressin and renin, to lower blood pressure in a subject susceptible to or afflicted with elevated blood pressure. Interactions of such angiotensin II receptor antagonist with this receptor site may be characterized as being either "competitive" (i.e., "surmountable") or as being "insurmountable". These terms, "competitive" and "insurmountable", characterize the relative rates, faster for the former term and slower for the latter term, at which the antagonist compound dissociates from binding with the receptor site.

The epoxy-free spirolactone-type aldosterone receptor antagonist" spironolactone binds to the aldosterone receptor as a competitive inhibitor of the action of aldosterone itself at the receptor site in the renal tubules, so as to modulate the receptor-mediated activity of aldosterone.
The present spirolactone- type compound used as aldosterone receptor antagonist is spironolactone.

The phrase "combination therapy", in defining use of the angiotensin II antagonist and the spirolactone, is intended to embrace administration of each antagonist in a sequential manner in a regimen that will provide beneficial effects of the drug combination, and is intended to embrace coadministration of the antagonist agents in a substantially simultaneous manner, such as in a single capsule having a fixed ratio of active ingredients or in multiple, separate capsules for each antagonist agent.

The phrase "cherapeutically-effective" is intended to qualify the amount of each antagonist agent for use in the combination therapy which will achieve the goal of reduction of hypertension with improvement in cardiac sufficiency by reducing or preventing, for example, hypertension and/or the progression of congestive heart failure.

The phrase "low-dose amount", in characterizing a therapeutically-effective amount of the aldosterone receptor antagonist agent in the combination therapy, is intended to define a quantity of such agent, or a range of quantity of such agent, that is capable of improving cardiac sufficiency while reducing or avoiding one or more aldosterone-antagonist-induced side effects, such as hyperkalemia. A dosage of spironolactone, which accomplishes the therapic goal of favorably enhancing cardiac sufficiency, while reducing or avoiding side effects, is a dosage that substantially avoids inducing diuresis, that is, a substantially non-diuresis-effective dosage or a non-diuretic-effective amount of an aldosterone receptor antagonist.

Another combination therapy of interest would consist essentially of three active agents, namely, the AII antagonist, the aldosterone receptor antagonist agent and a diuretic.

For a combination the AII antagonist agent and the ALDO antagonist agent, the agents would be used in combination in a weight ratio range from about 0.5-to-one to about twenty-to-one of the AII antagonist agent to the aldosterone receptor antagonist agent. A preferred range of these two agents (AII antagonist-to-ALDO antagonist) would be from about one-to-one to about fifteen-to-one, while a more preferred range would be from about one-to-one to about five-to-one, depending ultimately on the selection of the AII antagonist and ALDO antagonist. The diuretic agent may be present in a ratio range of 0.1-to-one to about ten to one (AII antagonist to diuretic).

### Detailed Description of the Invention

Angiotensin II (AII) antagonists used in the combination therapy are saralasin acetate, CGP-63170, EMD-66397, KT3-671, LR-B/081, valsartan, A-81282, BIBR-363, BIBS-222, BMS-184698, KW-3433, L-161177, LR-B/057, LY-235656, PD-150304, U-96849, U-97018, UP-275-22, WAY-126227, WK-1492.2K, YM-31472, E-4177, EMD-73495, HN-65021 (elisartan), L-159282, ME-3221, SL-91.0102, Tasosartan, Telmisartan, UP-269-6 (ripisartan), YM-358, CGP-49870, GA-0056, L-159689, L-162441, L-163007, PD-123177, A-81988, BMS-180560, CGP-38560A, CGP-48369, DA-2079, DE-3489, DuP-167, EXP-063, EXP-6155, EXP-6803, EXP-7711, EXP-9270, FK-739, HR-720, ICI-D7155, isoteoline, KRI-1177, L-158978, L-159874, LR B087, LY-285434, LY-302289, LY-315995, RG-13647, RWJ-38970, RWJ-46458, S-8307, S-8308, saprisartan, saralasin, Sarmesin, WK-1360, X-6803, ZD-7155, CI-996, EXP-929, L-163017, LY-301875, XH-148, XR-510, and PD-123319, and 5-[(3,5-dibutyl-1H-1,2,4-triazol-1-yl)methyl]-2-[2-(1H-tetrazol-5-yl)phenyl]pyridine.

A preferred group of AII antagonists of interest consists of the following compounds:
saprisartan, elisartan, ripisartan, 5-[(3,5-dibutyl-1H-1,2,4-triazol-1-yl)methyl]-2-[2-(1H-tetrazol-5-yl)phenyl]pyridine, tasosartan, telmisartan and valsartan. A preferred combination of the above kind consists of combinations comprising the said angiotensin II receptor antagonist and 5 to 20 mg of spironolactone in the same unit dosage form or in two different associated dosage forms of the combinations.

Another embodiment of the present invention is a combination comprising 5 to 20mg of sprironolactone and a therapeutically- effective amount of an angiotensin II receptor antagonist selceted from compounds of the group consisting of candesartan, losartan, irbesartan, zolasartan, valsartan, tasosartan, telmisartan, saprisartan, elisartan, ripisartan and 5-[(3,5-dibutyl-1H-1,2,4-triazol-1-yl)methyl)-2-[2-(1H-tetrazol-5-yl)phenyl]pyridine in the same unit dosage forms or in two different associated dosage forms of the combination.
Especially preferred is a combination of the above kind in which spironolactone is present at not less than 10mg in the unit dosage form.
The aldosterone antagonist spironolactone has the following structure and formal 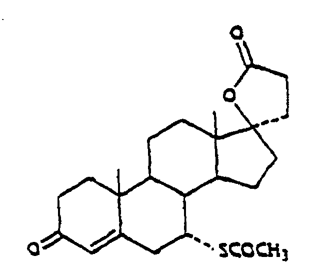 "spironolactone": 17-hydroxy-7α-mercapto-3-oxo-17α-pregn-4-ene-21-carboxylic acid, p-lactone acetate.

Spironolactone is sold by G.D. Searle & Co., Skokie, Illinois, under the trademark "ALDACTONE", in tablet dosage form at doses of 25 mg, 50 mg and 100 mg per tablet.

A diuretic agent may be used in the combination. Such diuretic agent may be selected from several known classes, such as thiazides and related sulfonamides, potassium-sparing diuretics, loop diuretics and organic mercurial diuretics.

Angiotensin II receptor antagonist compounds suitable for use in the combination therapy are described in Table II, below. preferred compounds for use in the combination therapy may be generally characterized structurally as having two portions. A first portion constitutes a mono-aryl-alkyl moiety, or a bi-aryl-alkyl moiety, or a mono-heteroaryl-alkyl moiety, or a bi-heteroaryl-alkyl moiety. A second portion constitutes a heterocyclic moiety or an open chain hetero-atom-containing moiety.
A preferred A II antagonist of the above kind is 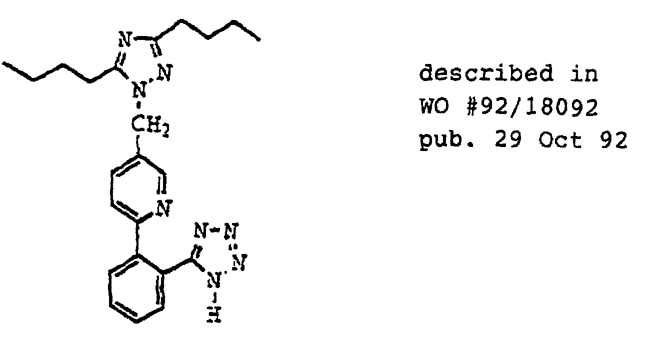 which is 5-[(3,5-dibutyl-1H-1,2,4-triazol-1-yl)methyl]-2-[2-(1H-tetrazol-5-yl)phenyl]pyridine.

The combination therapy of the invention would be useful in treating a variety of circulatory disorders, including cardiovascular disorders, such as hypertension, congestive heart failure, myocardial fibrosis and cardiac hypertrophy. The combination therapy would also be useful with adjunctive therapies. For example, the combination therapy may be used in combination with other drugs, such as a diuretic, to aid in treatment of hypertension.
Table II, below, contains description of other angiotensin II antagonist compounds of the above kind, many of which do not fall within the scope of the claims.

Associated with each compound listed in Table II is a published patent document describing the chemical preparation of the angiotensin II antagonist compound as well as the biological properties of such compound.

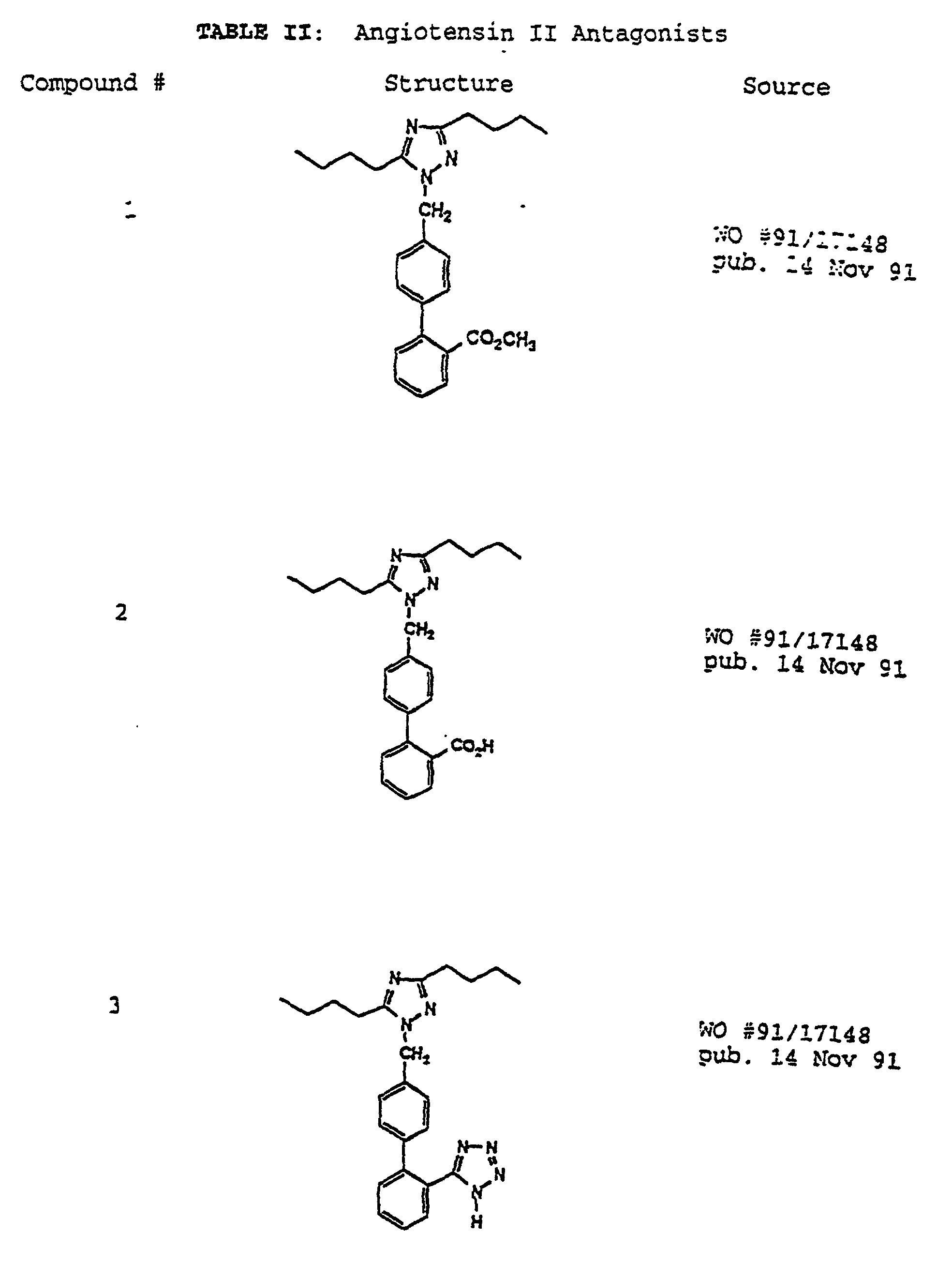

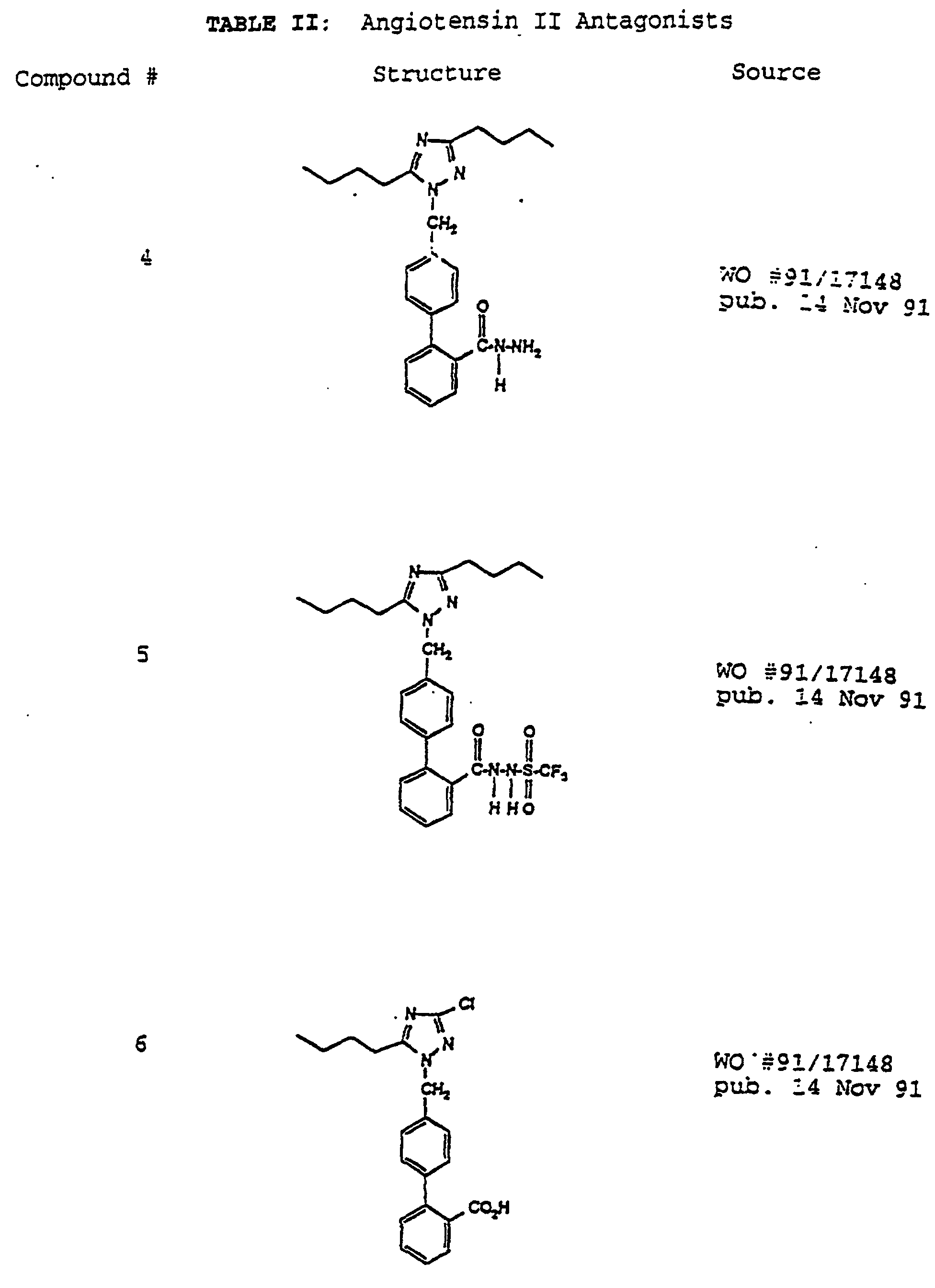

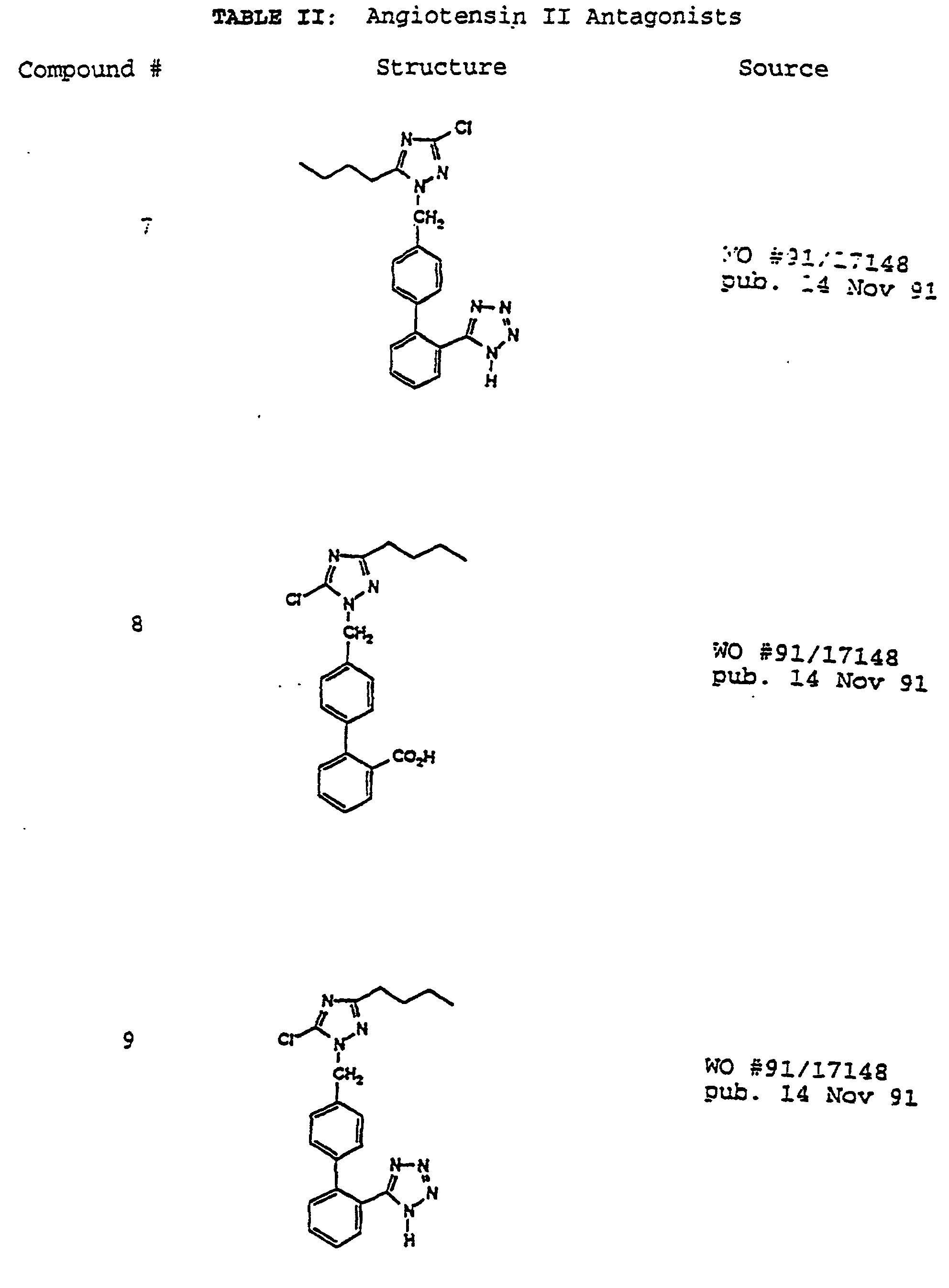

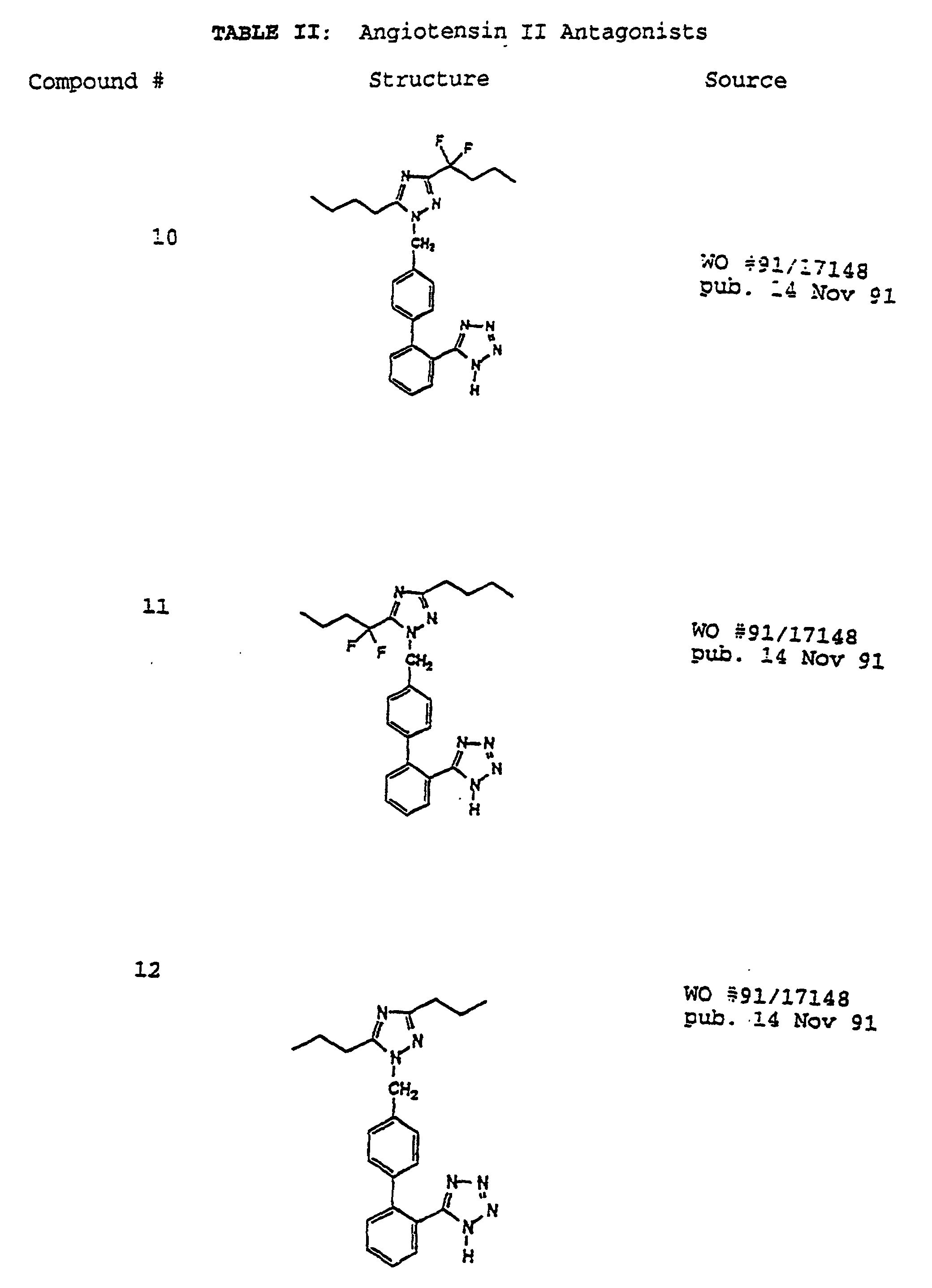

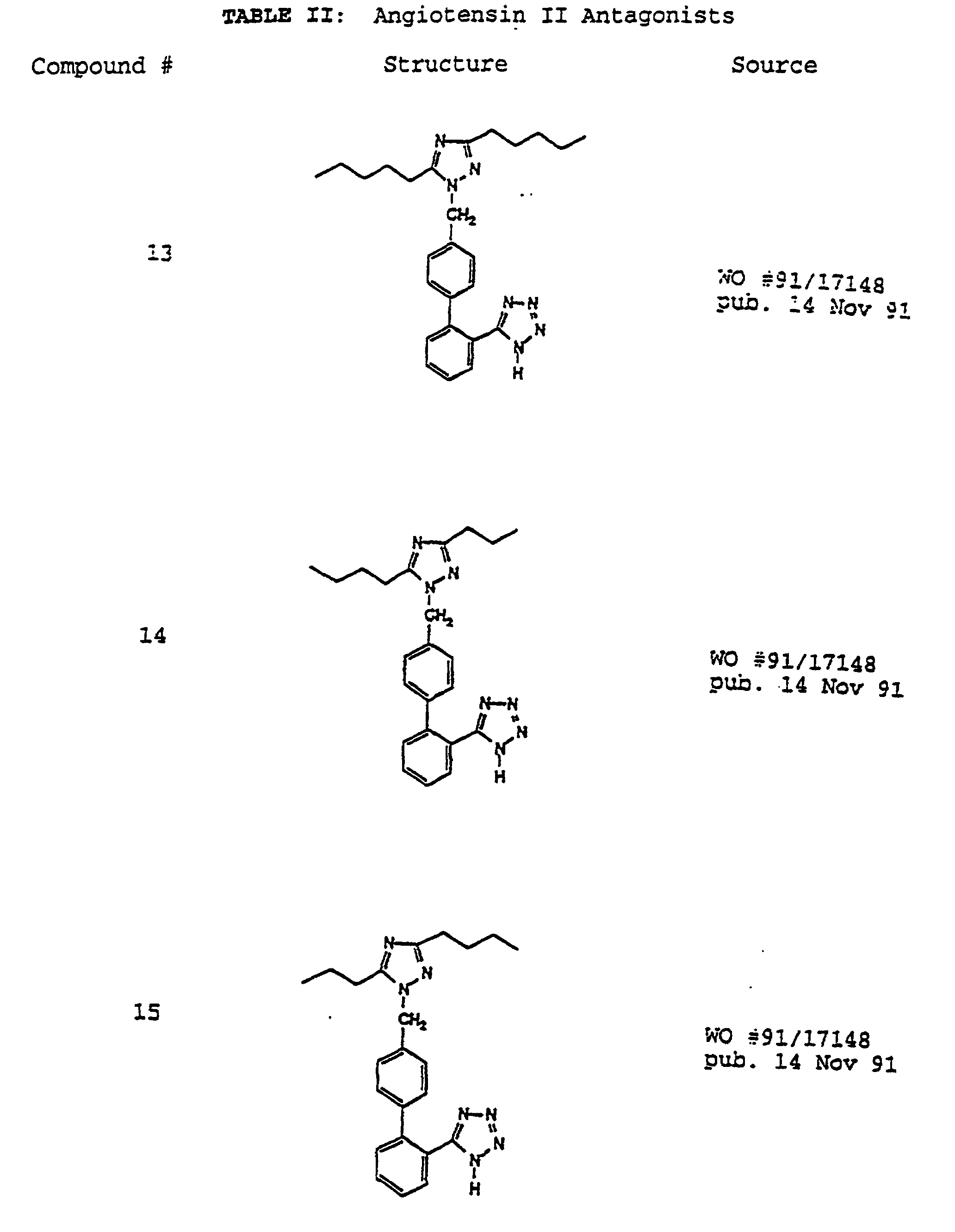

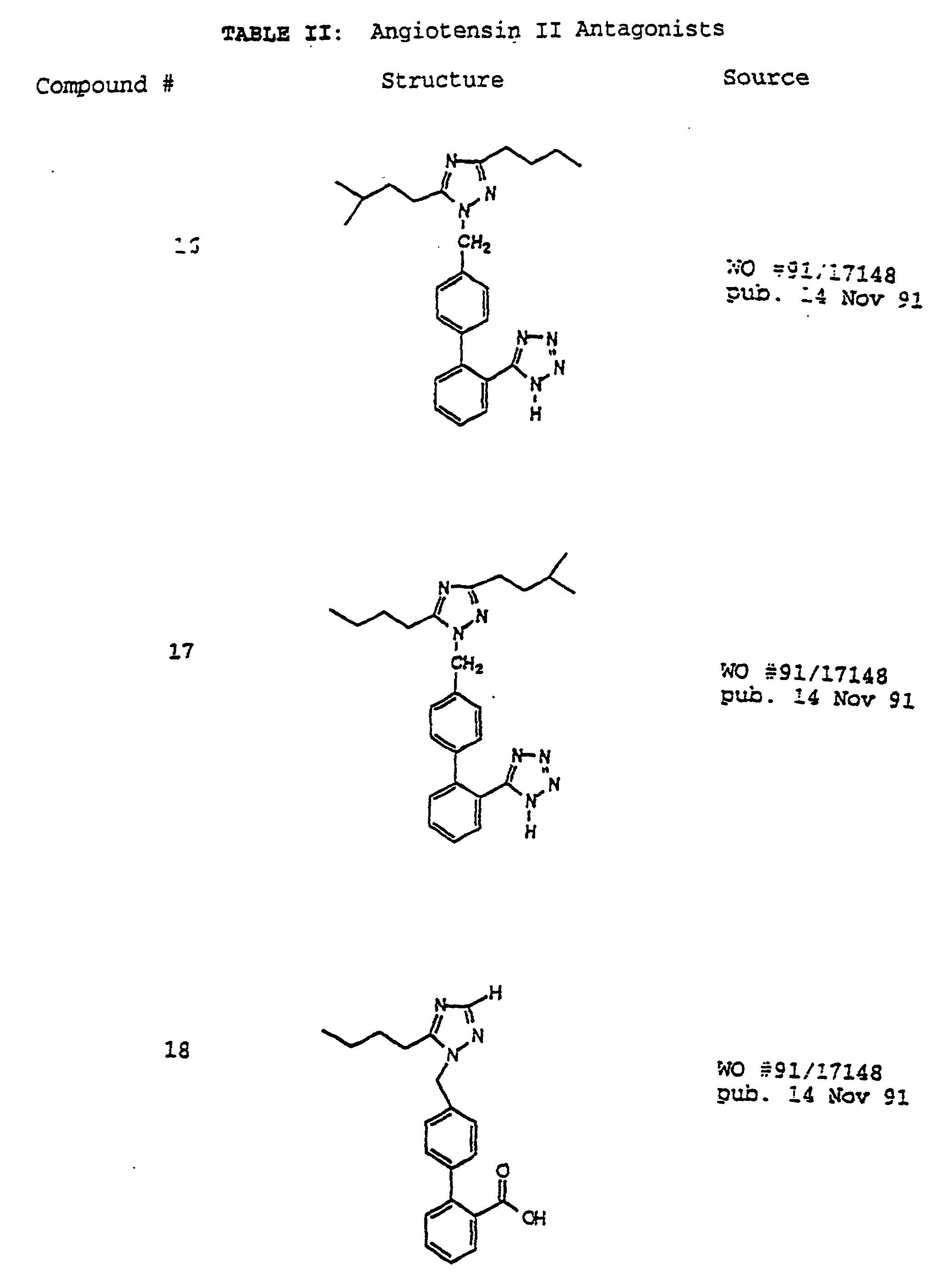

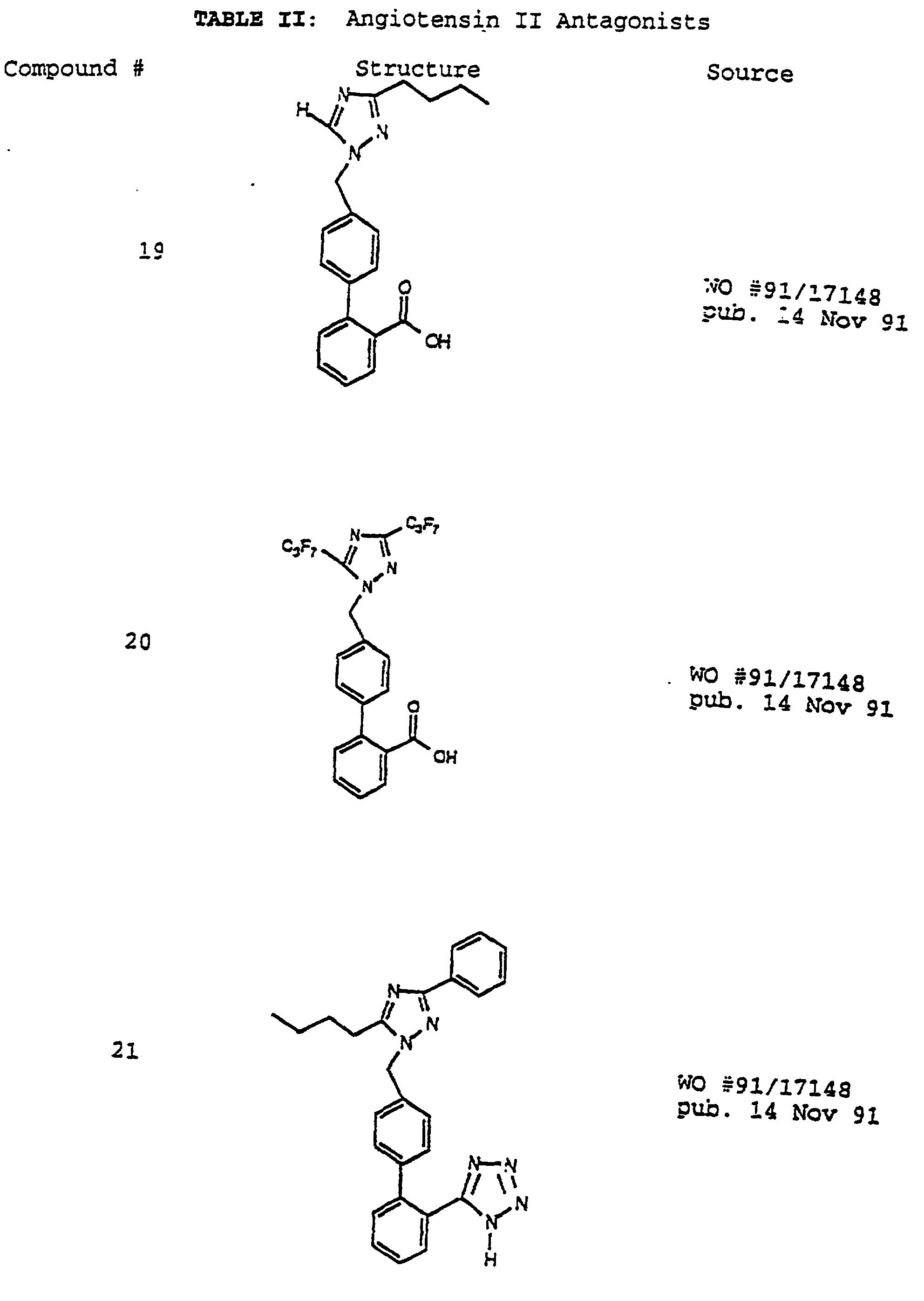

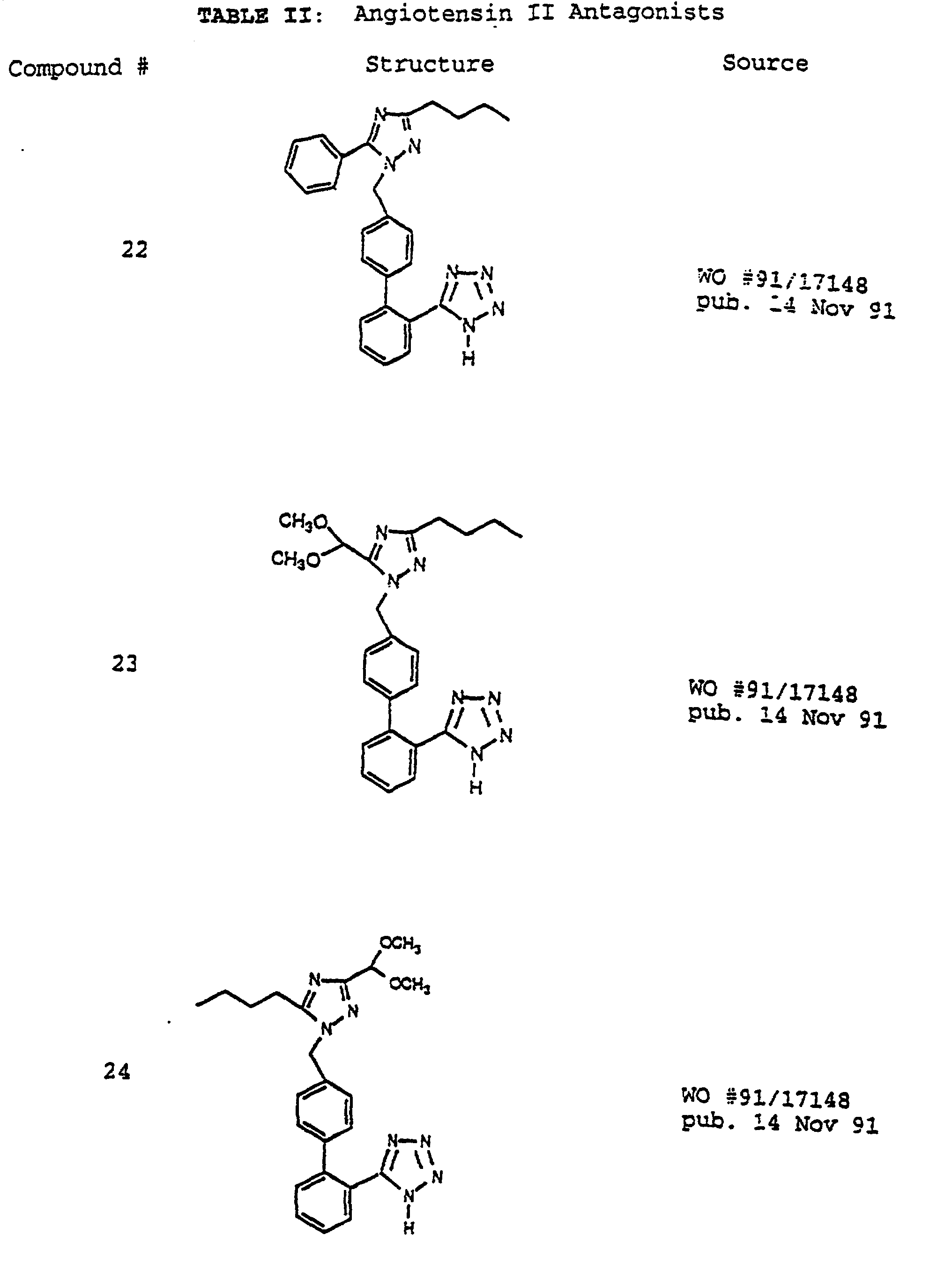

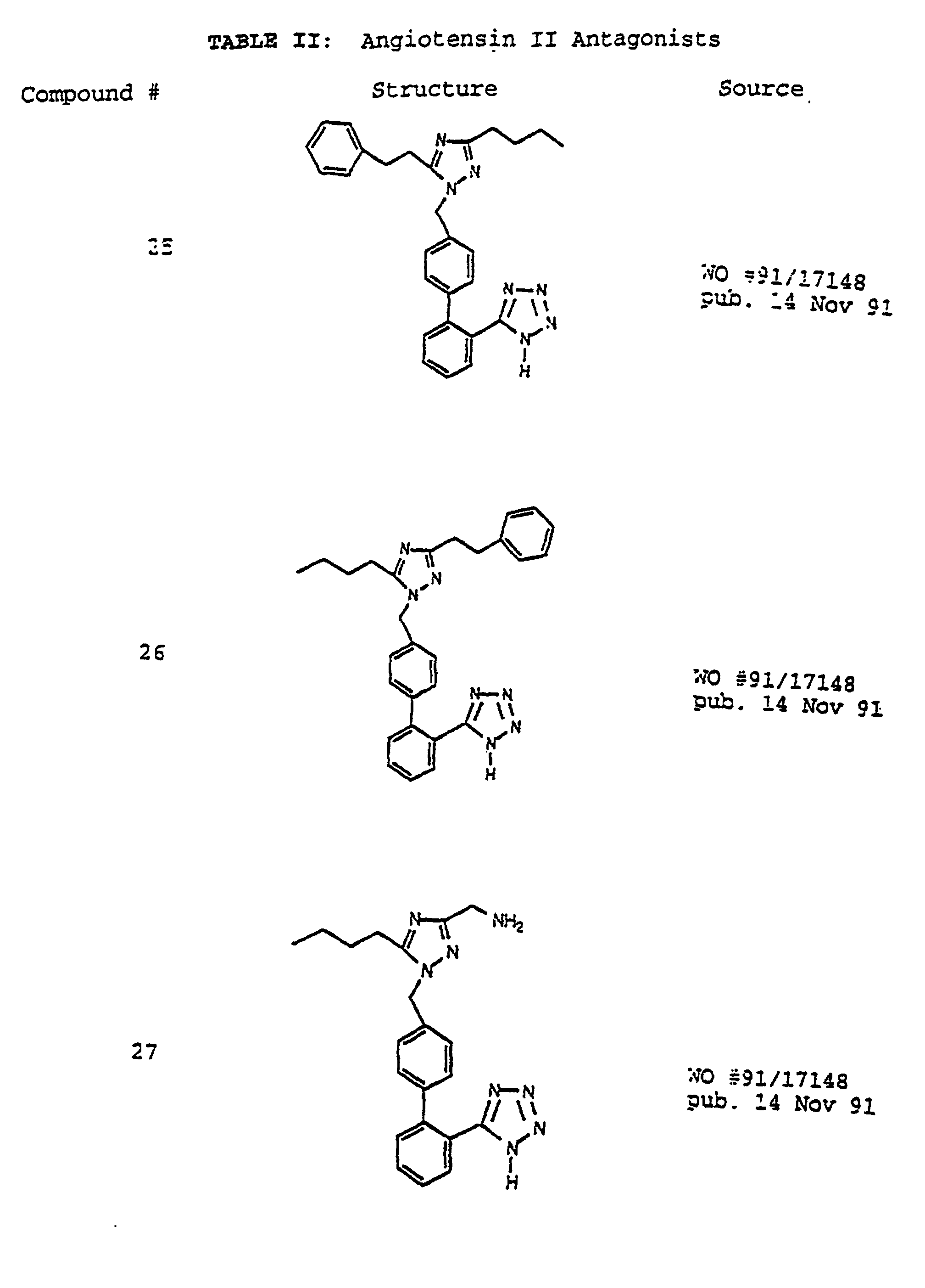

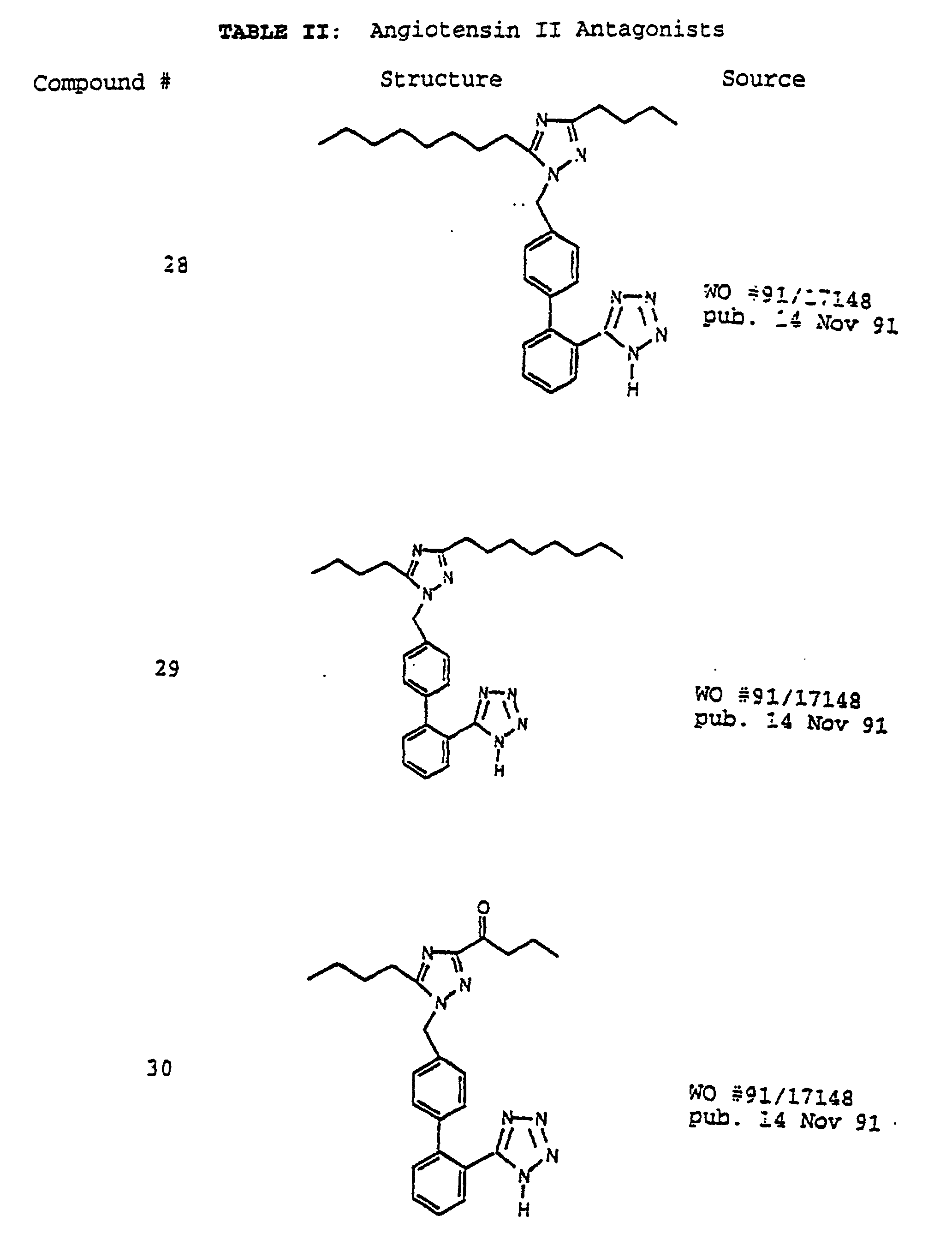

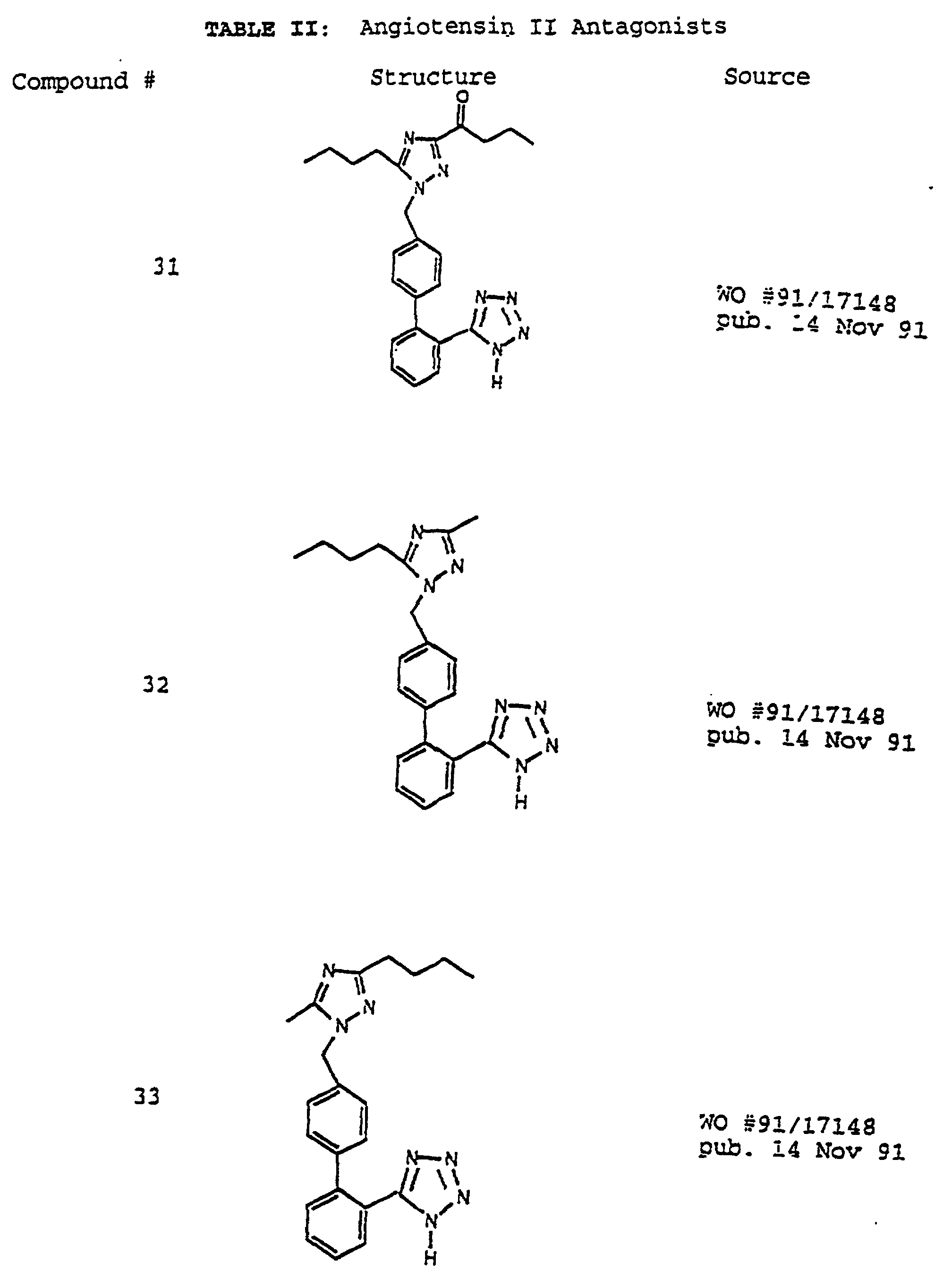

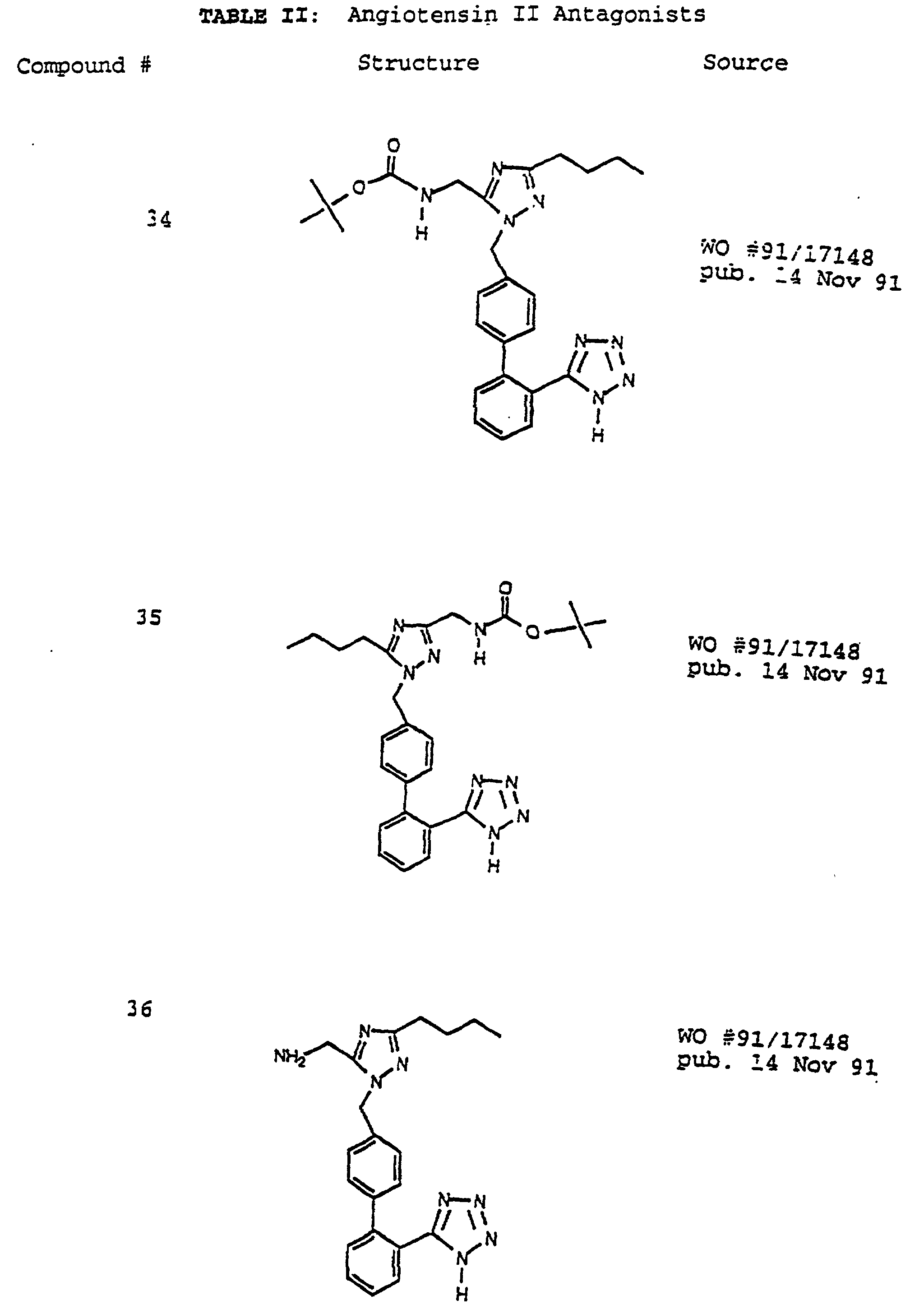

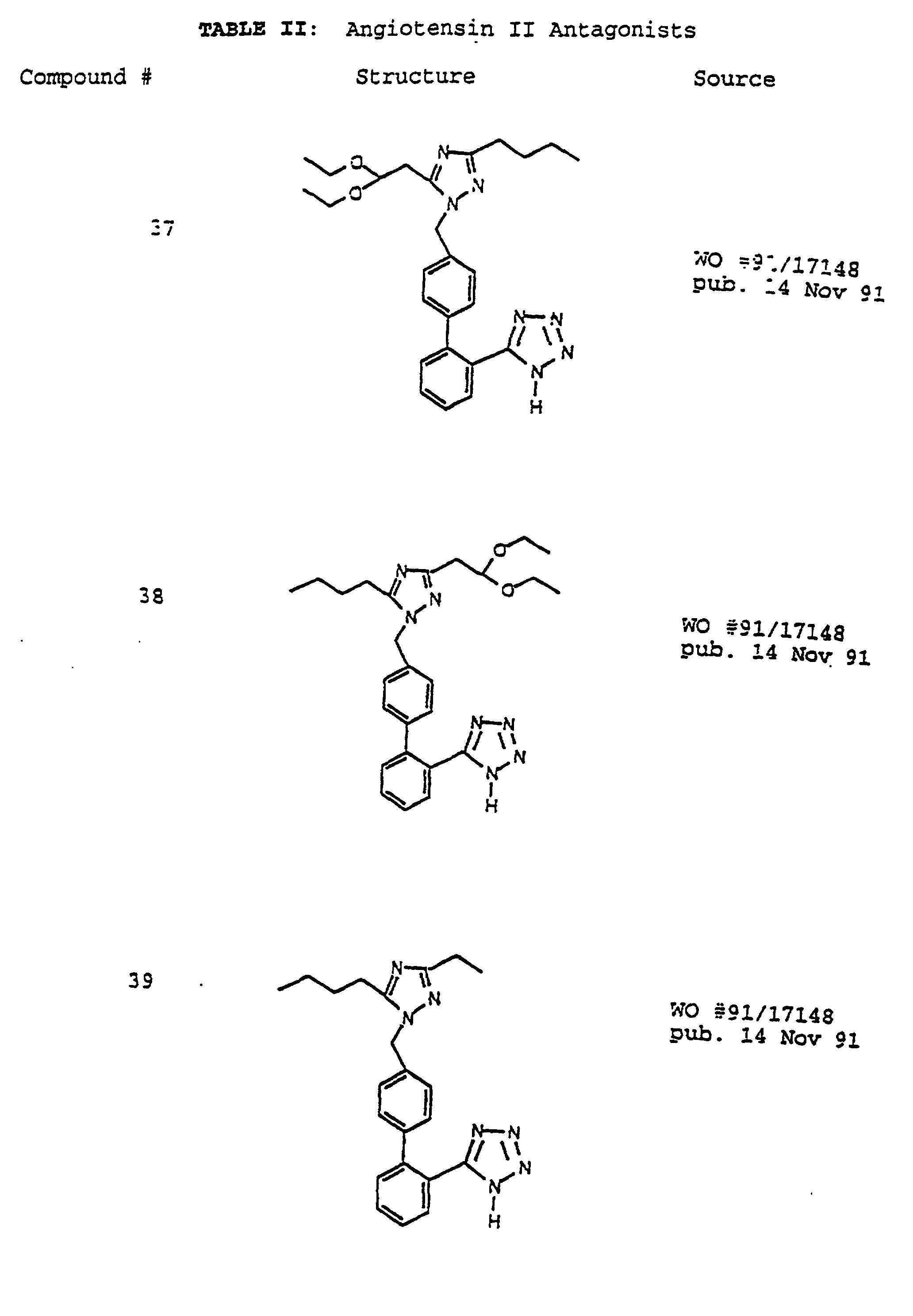

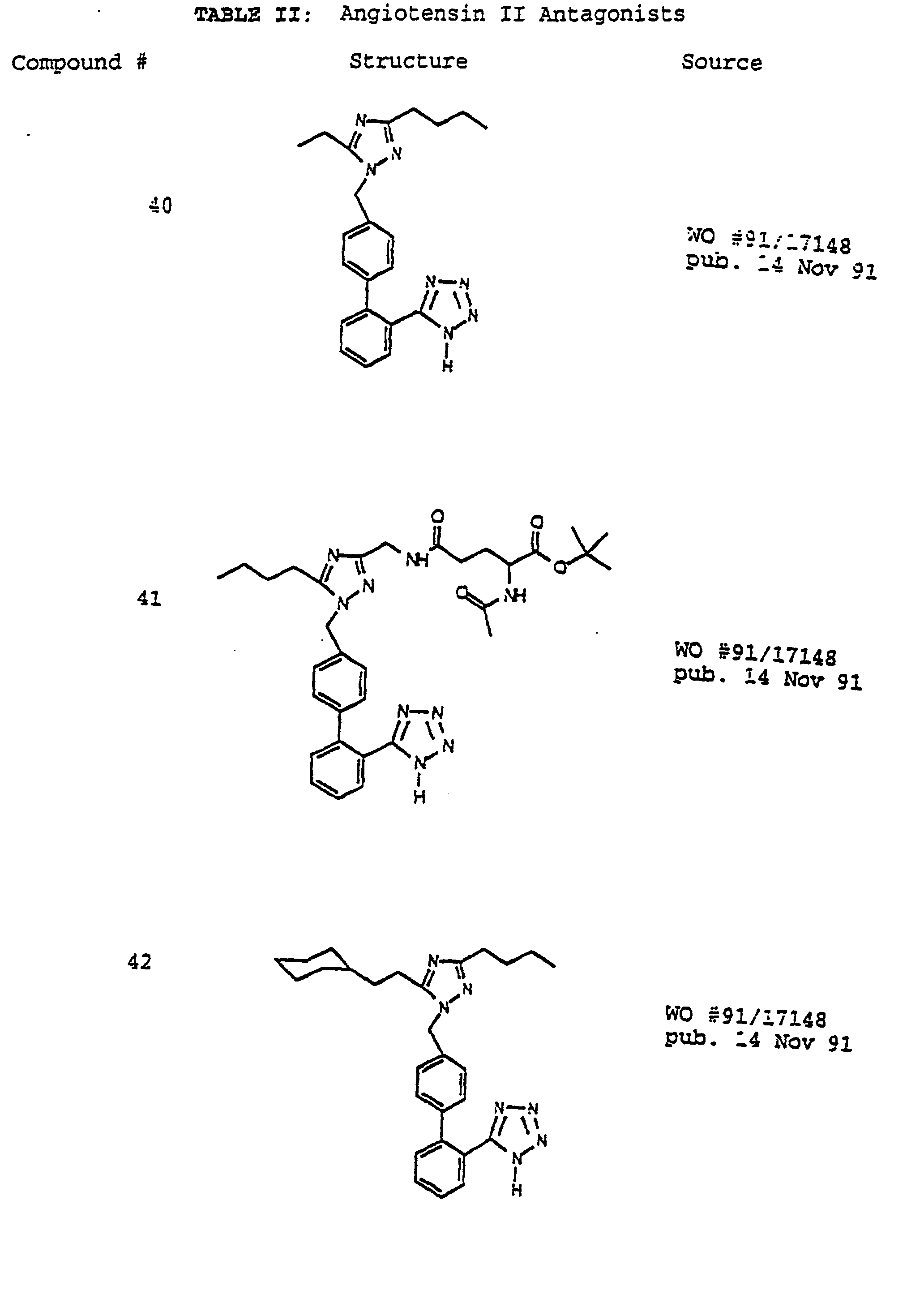

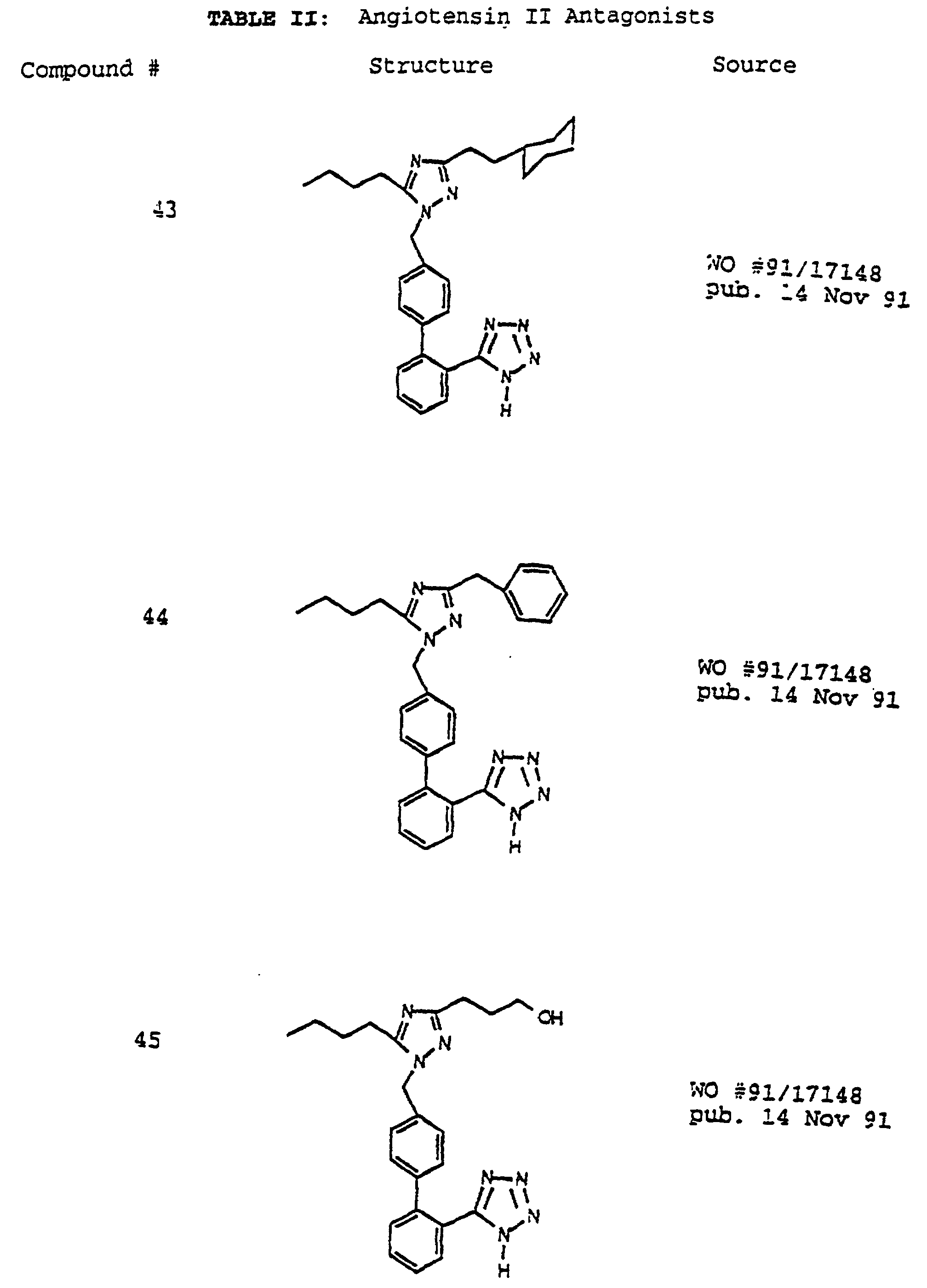

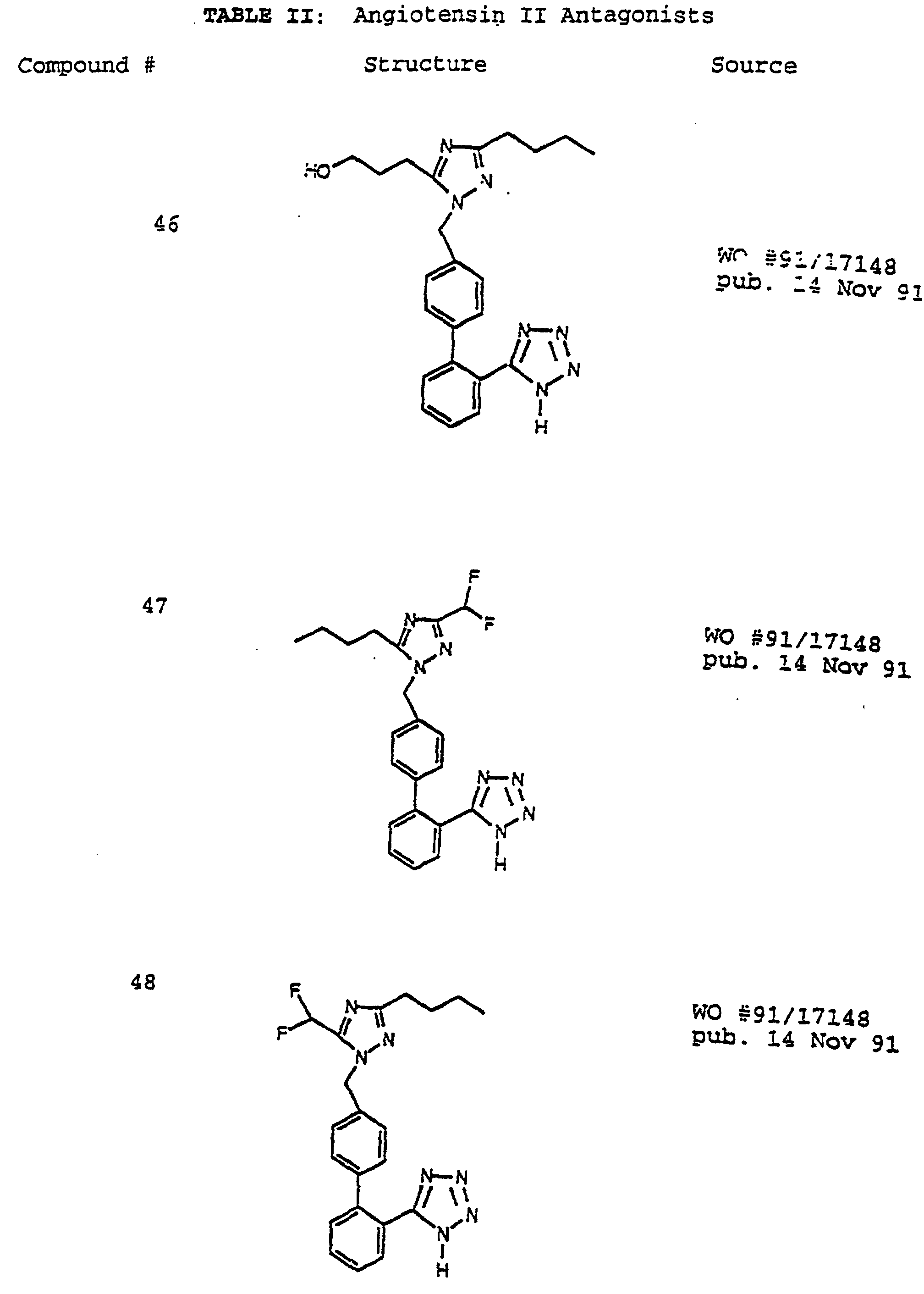

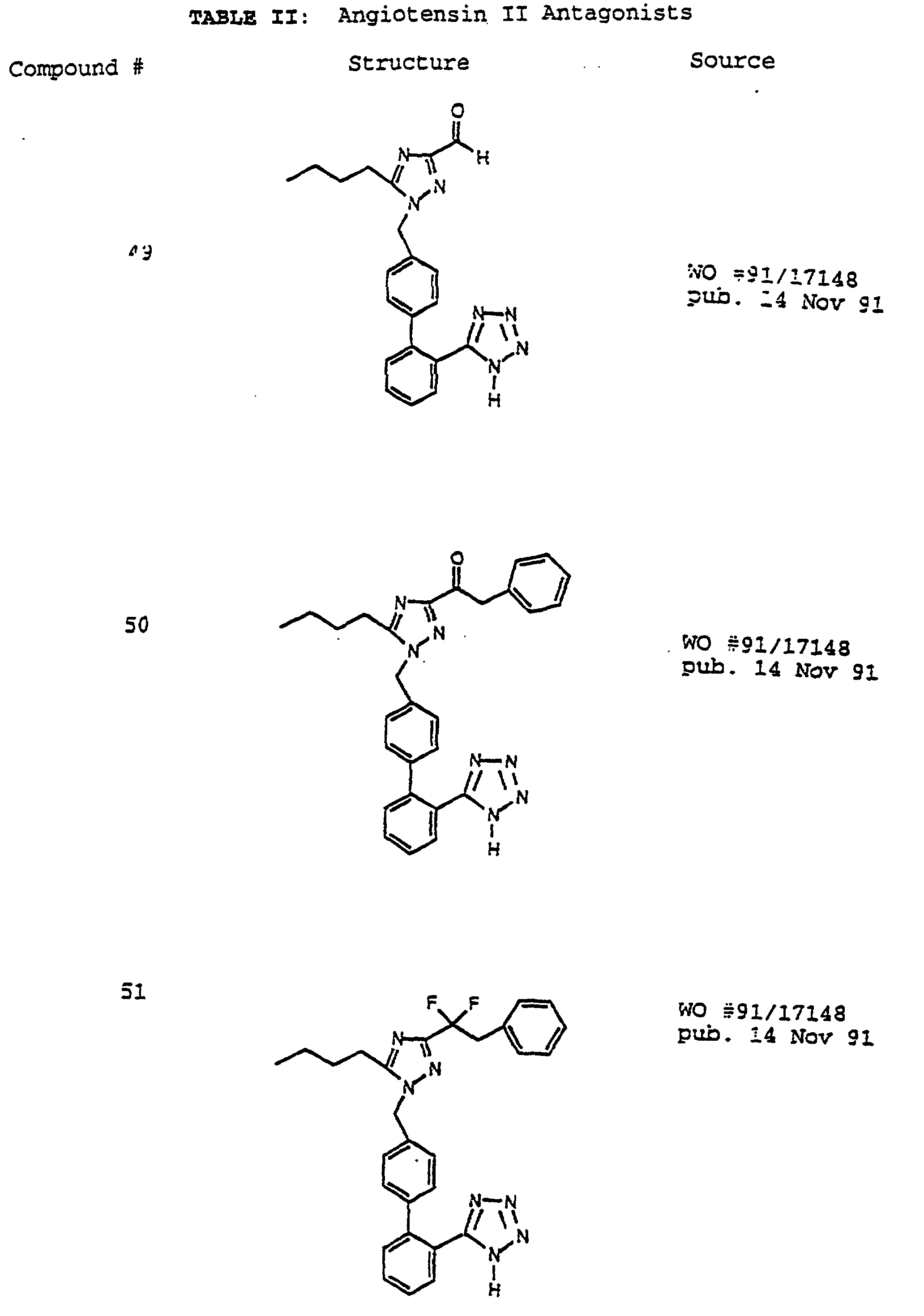

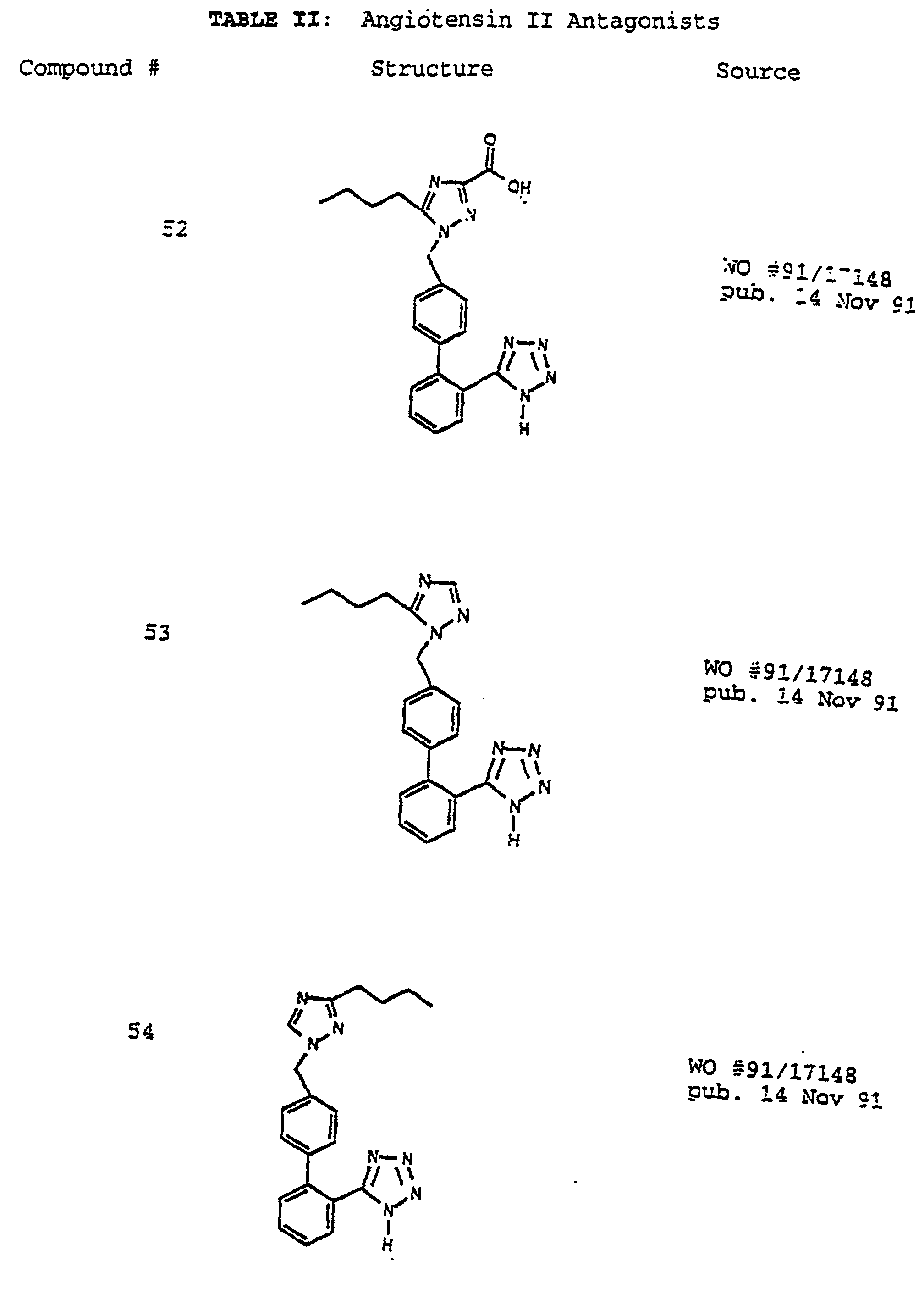

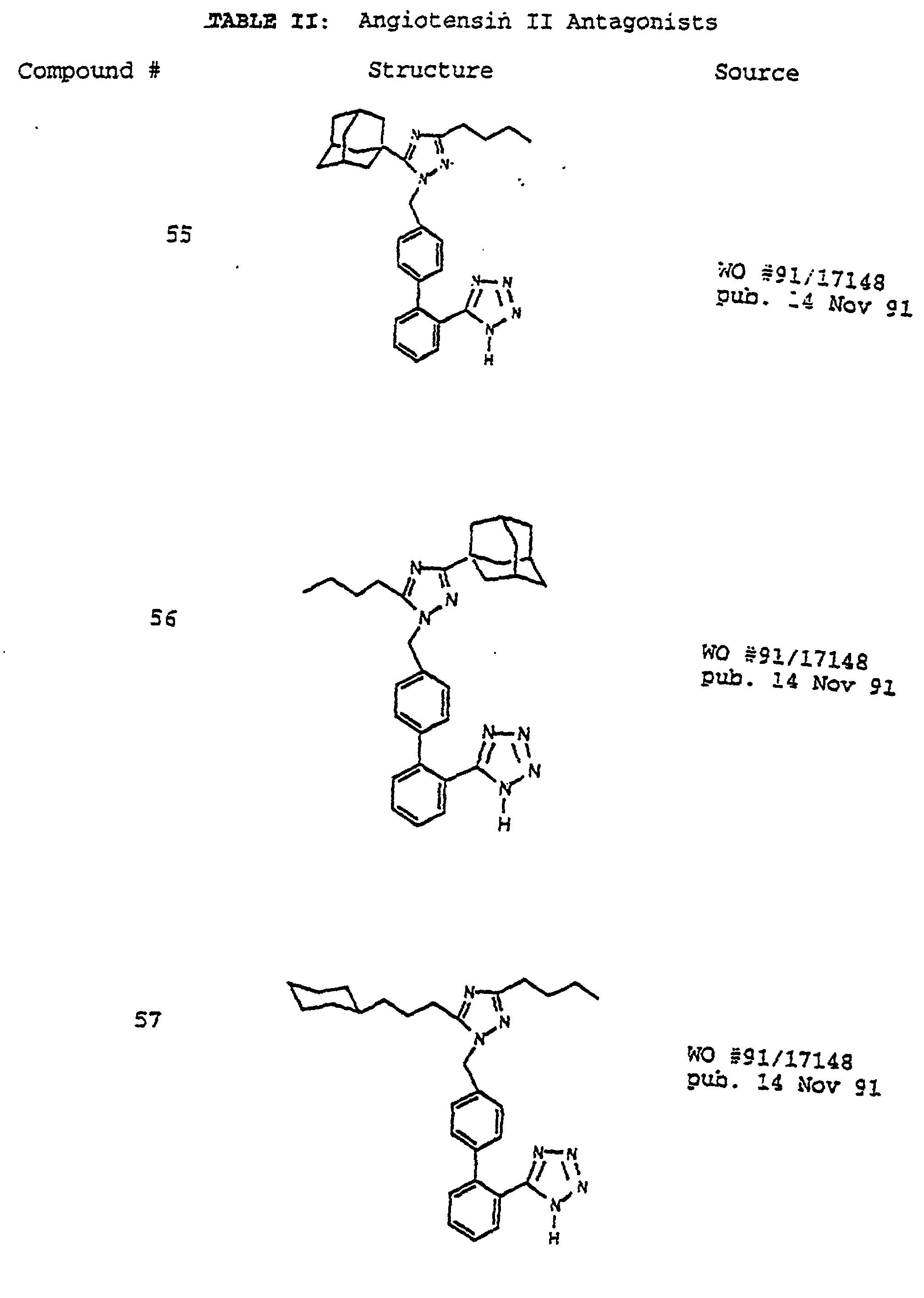

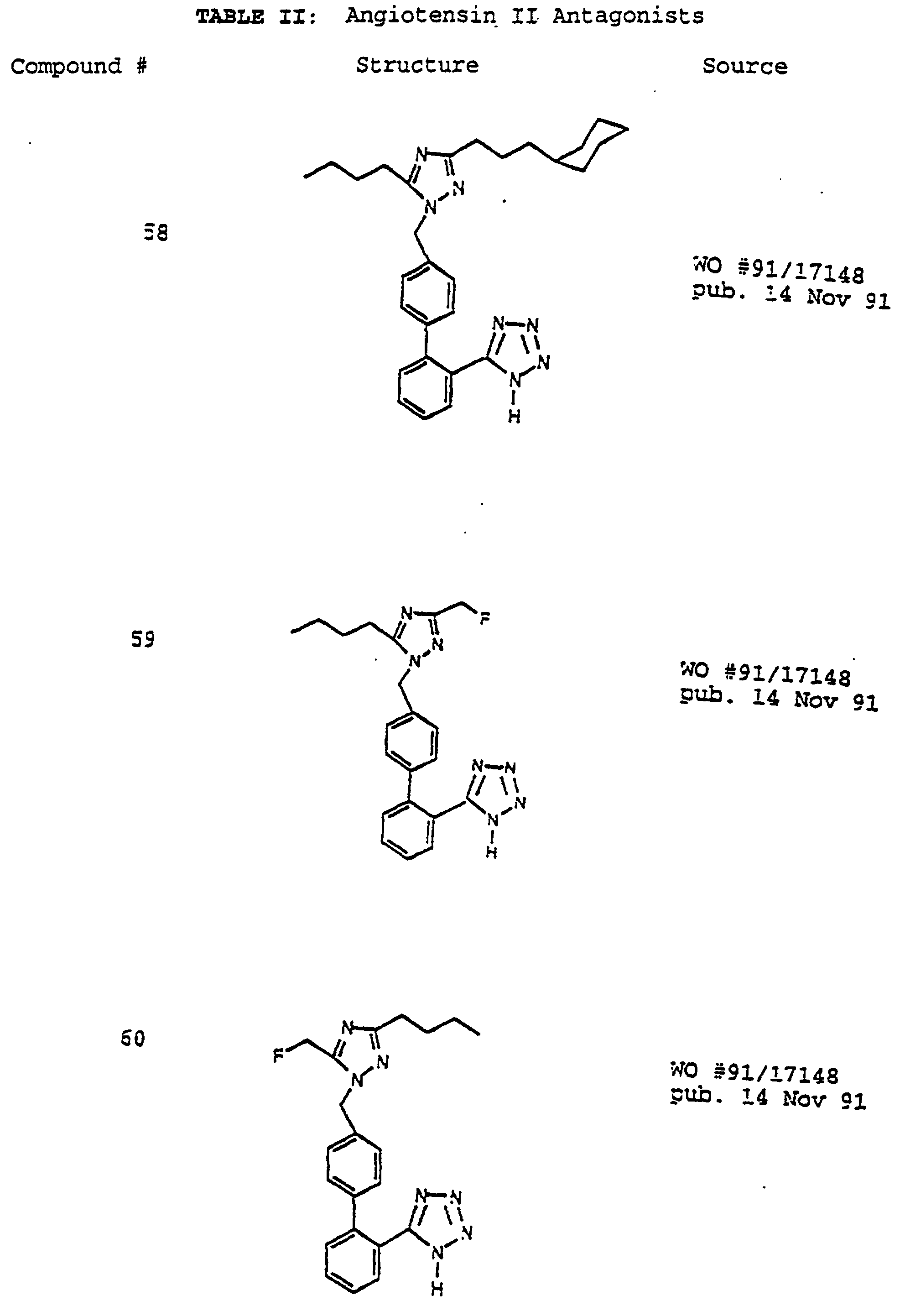

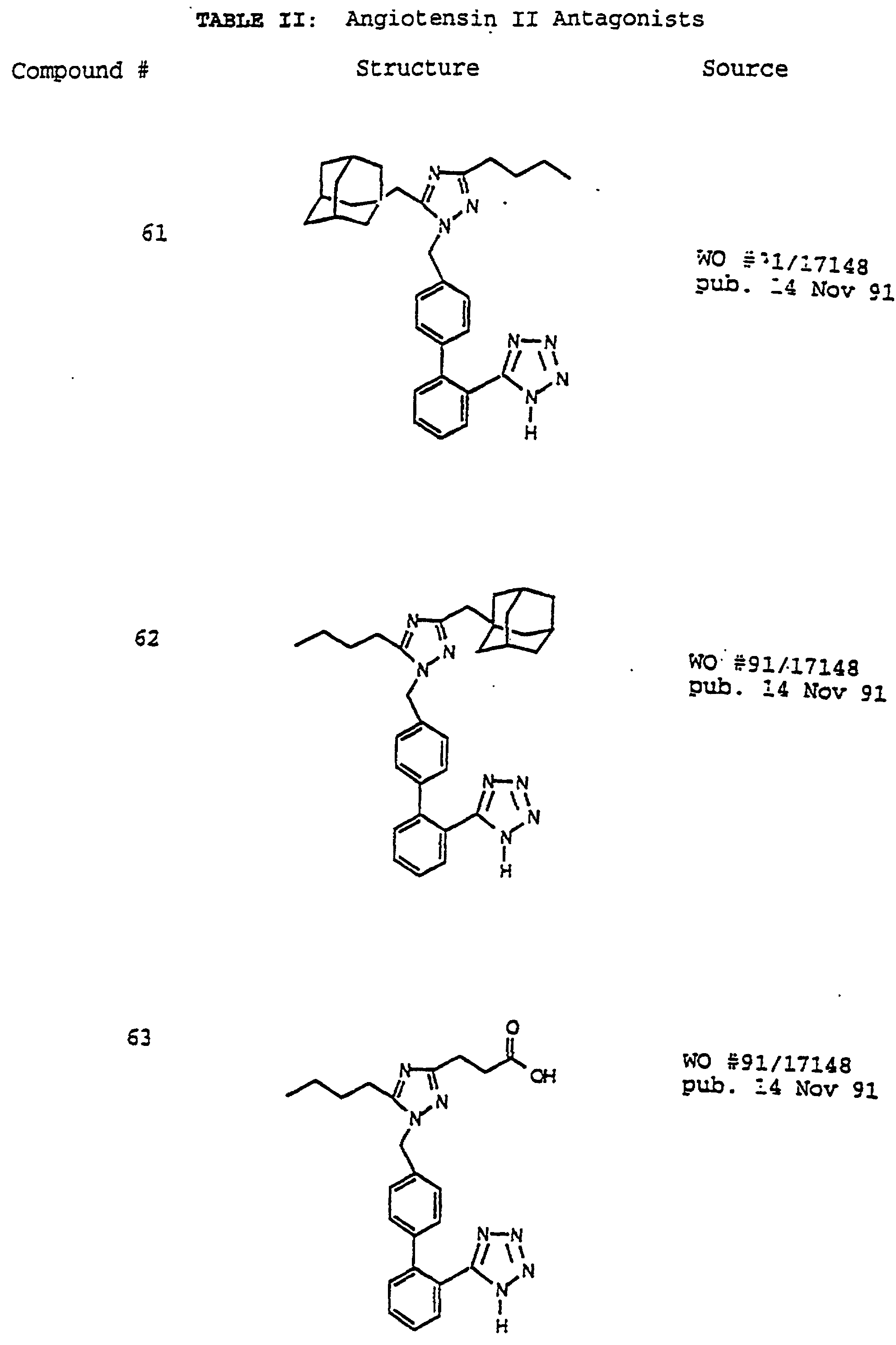

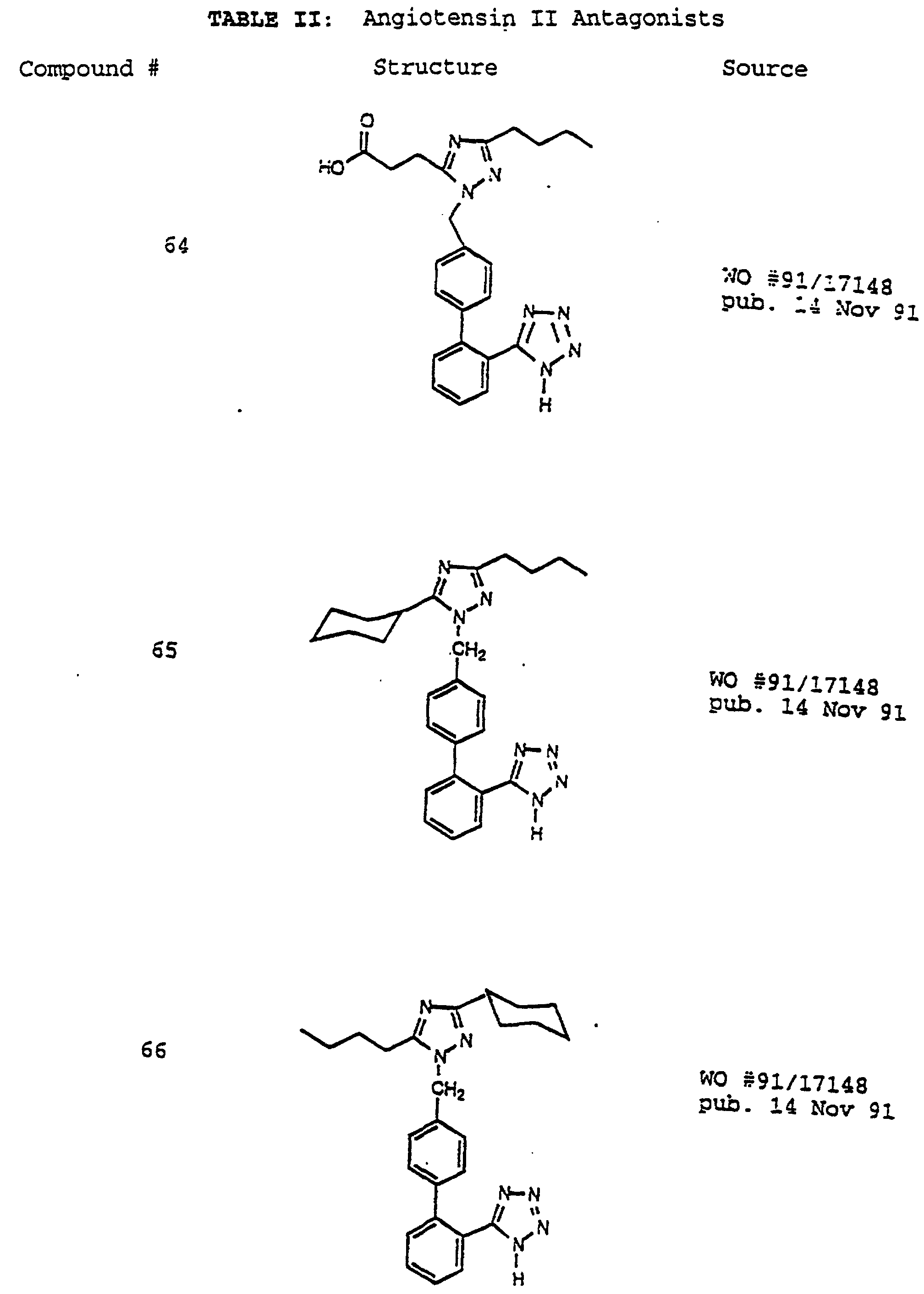

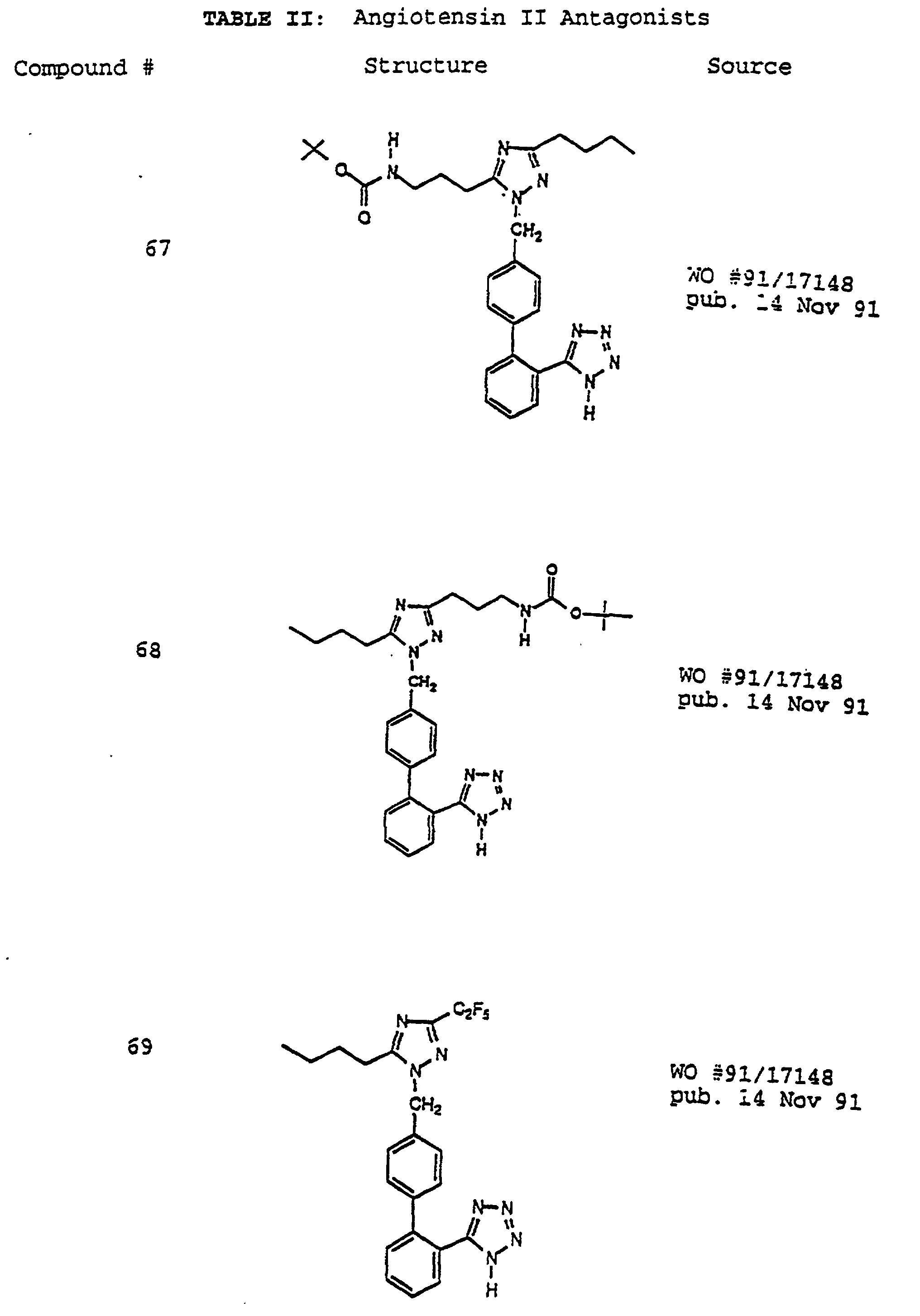

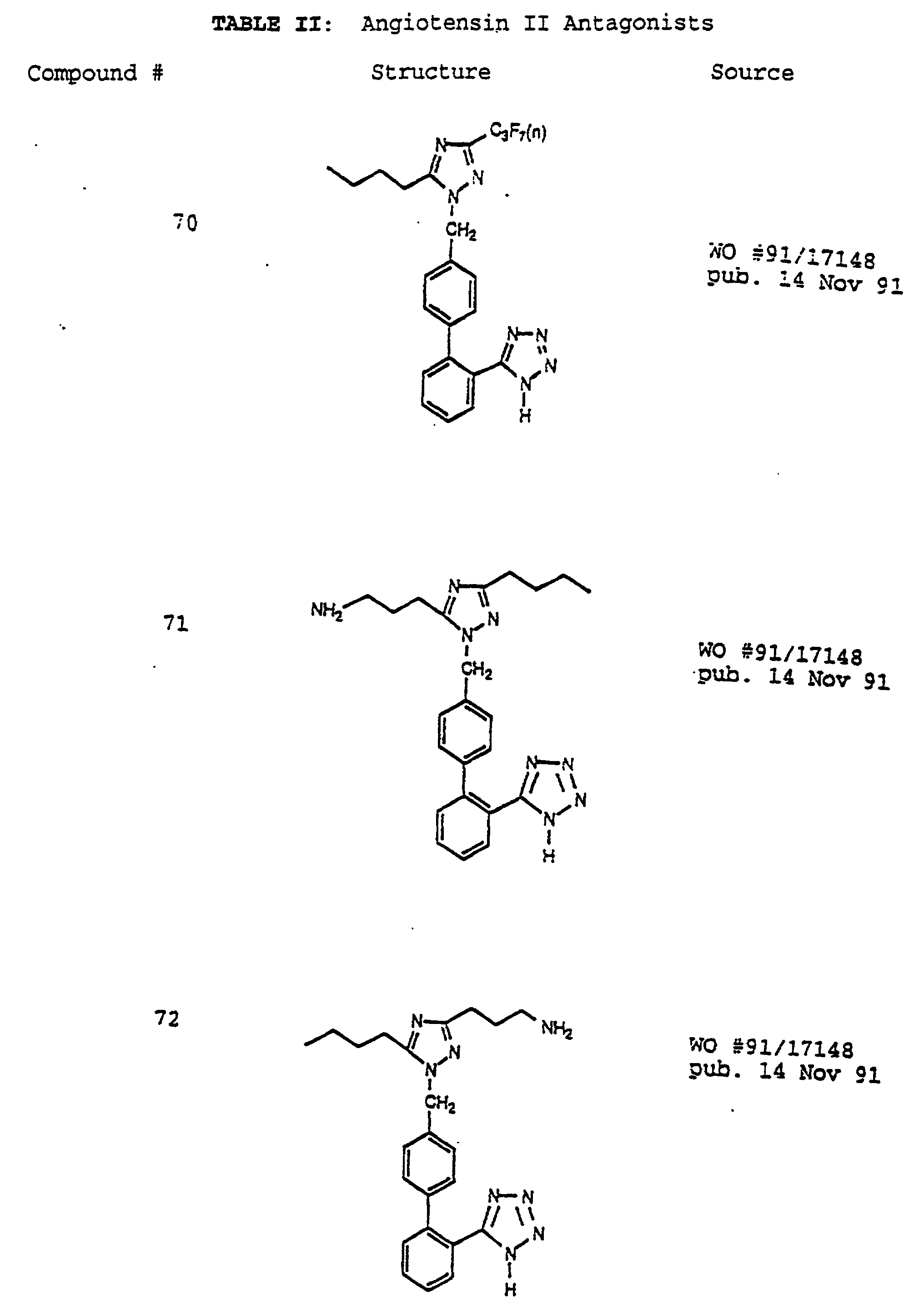

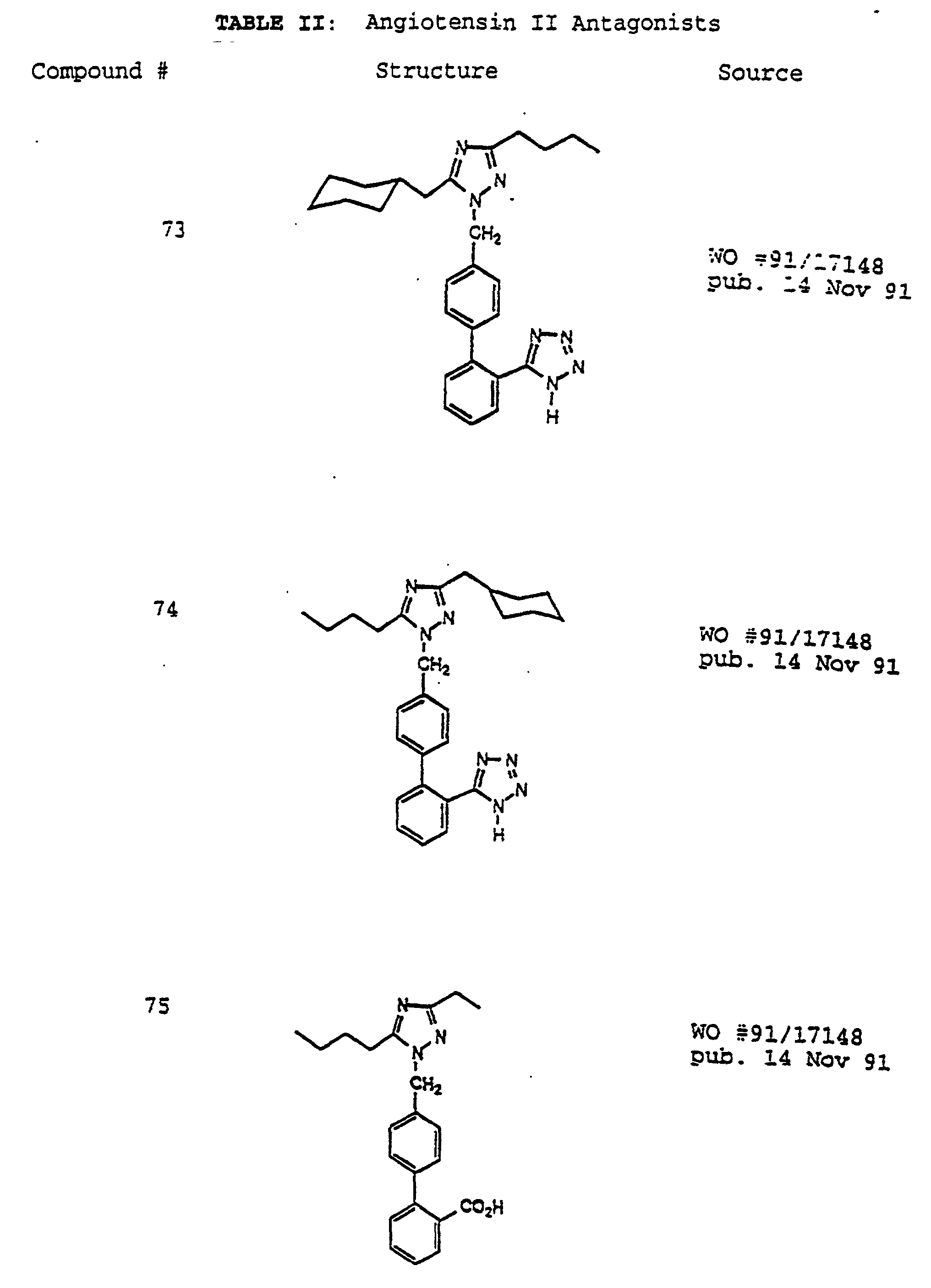

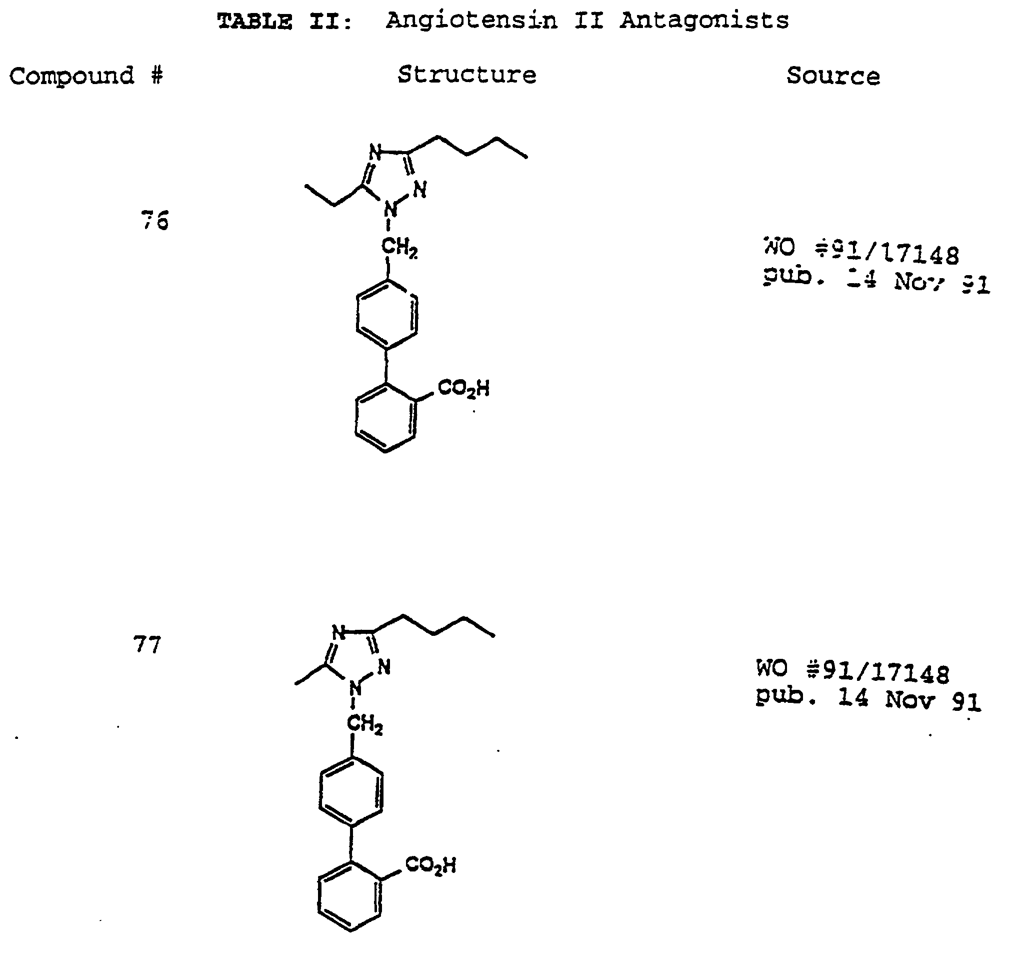

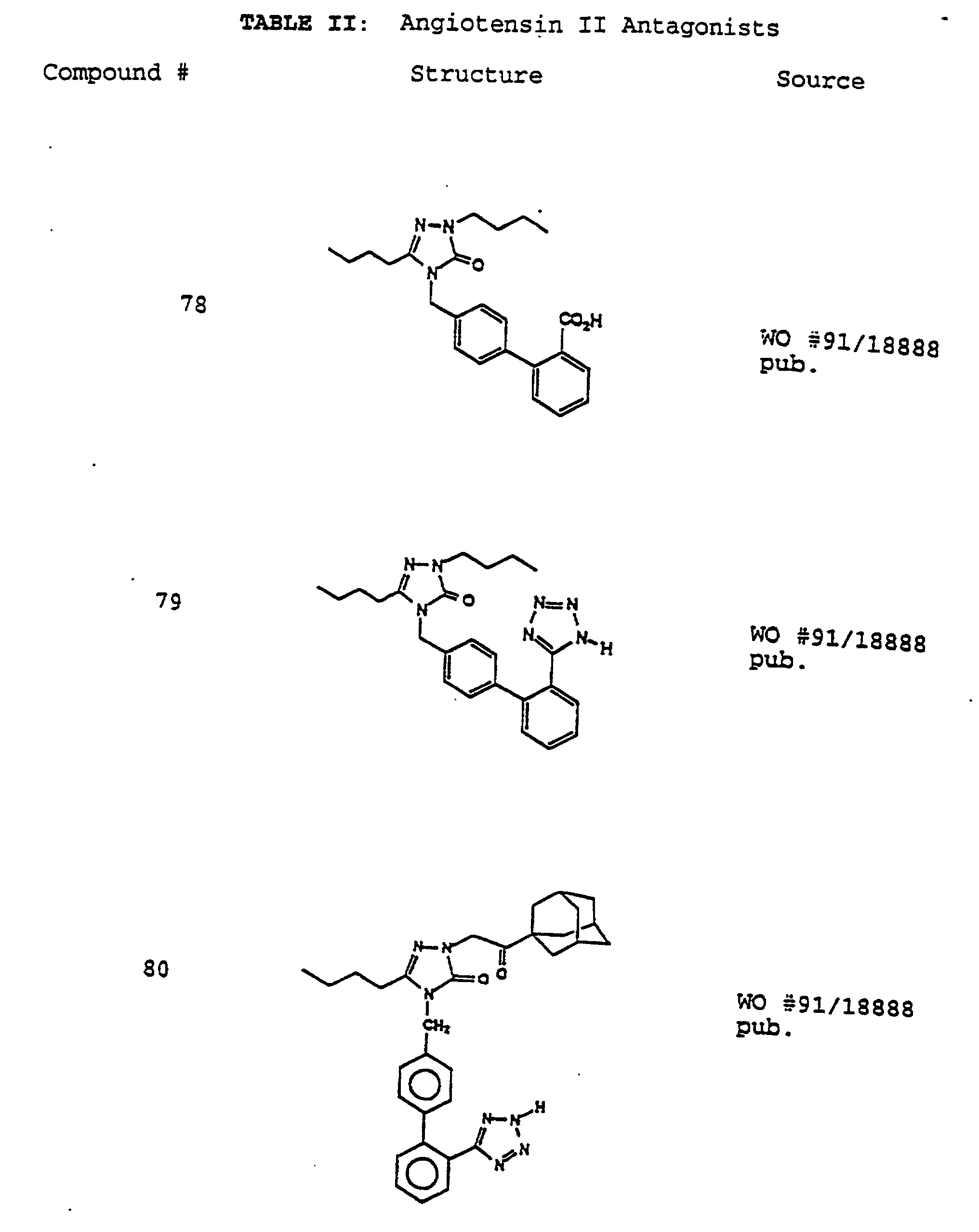

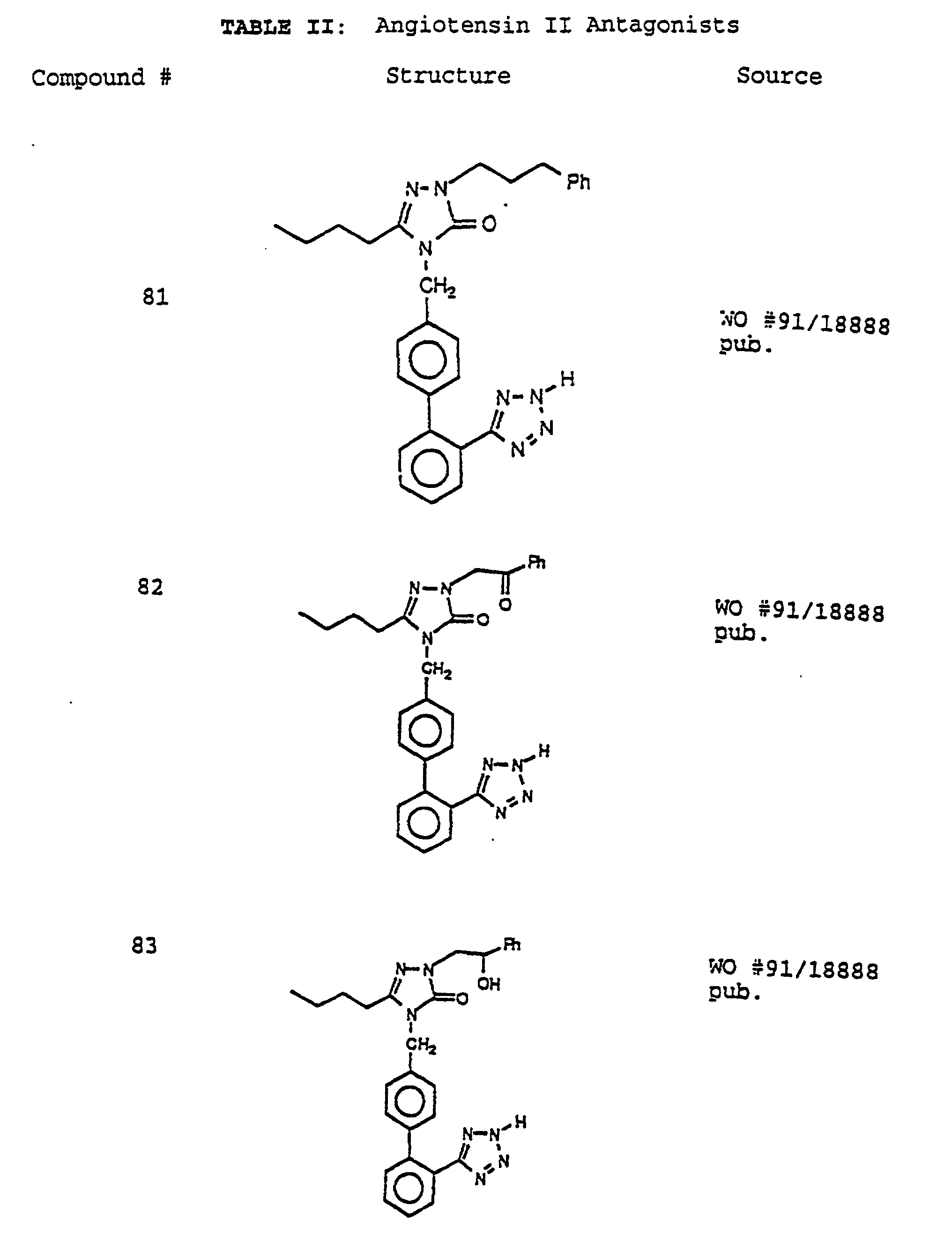

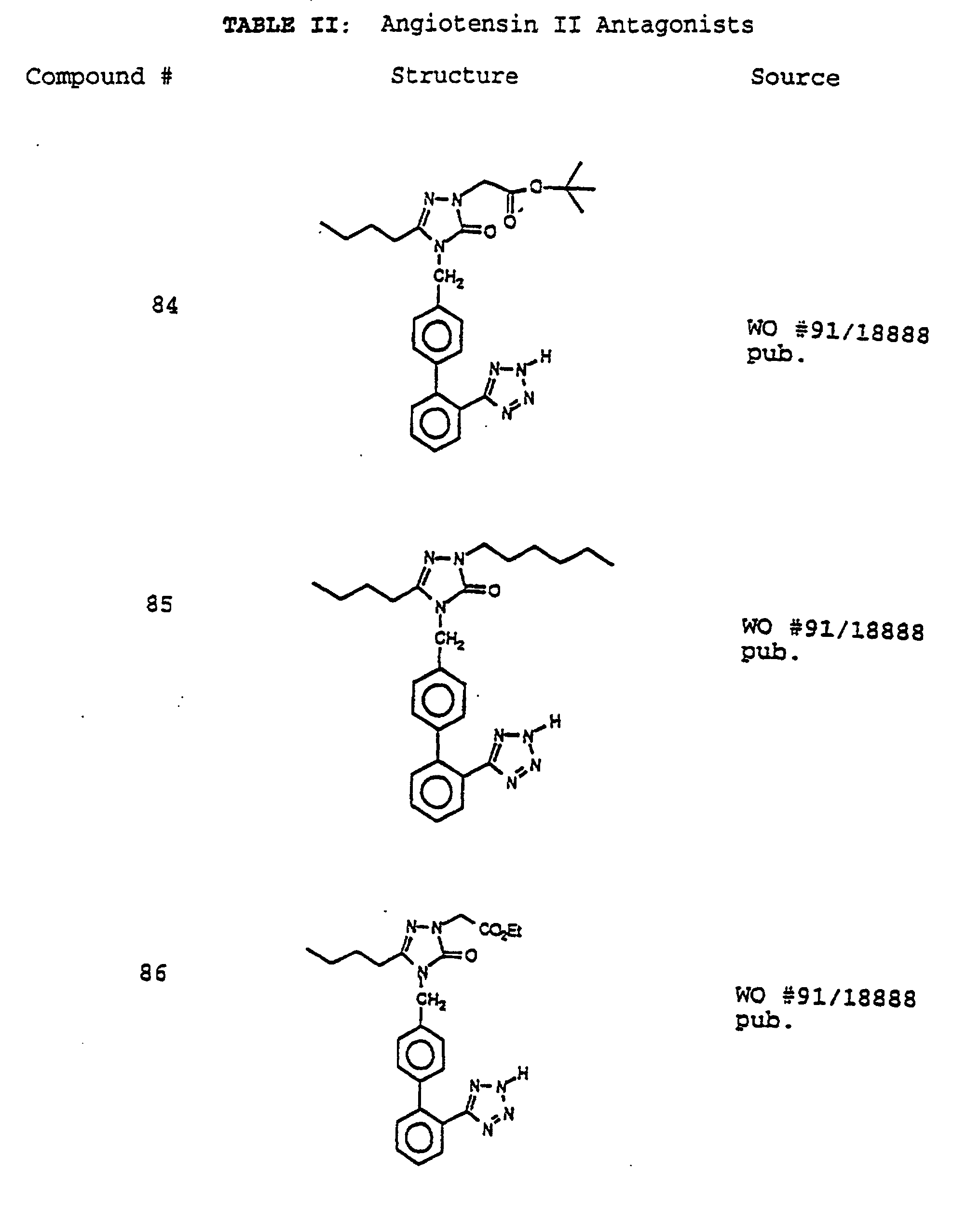

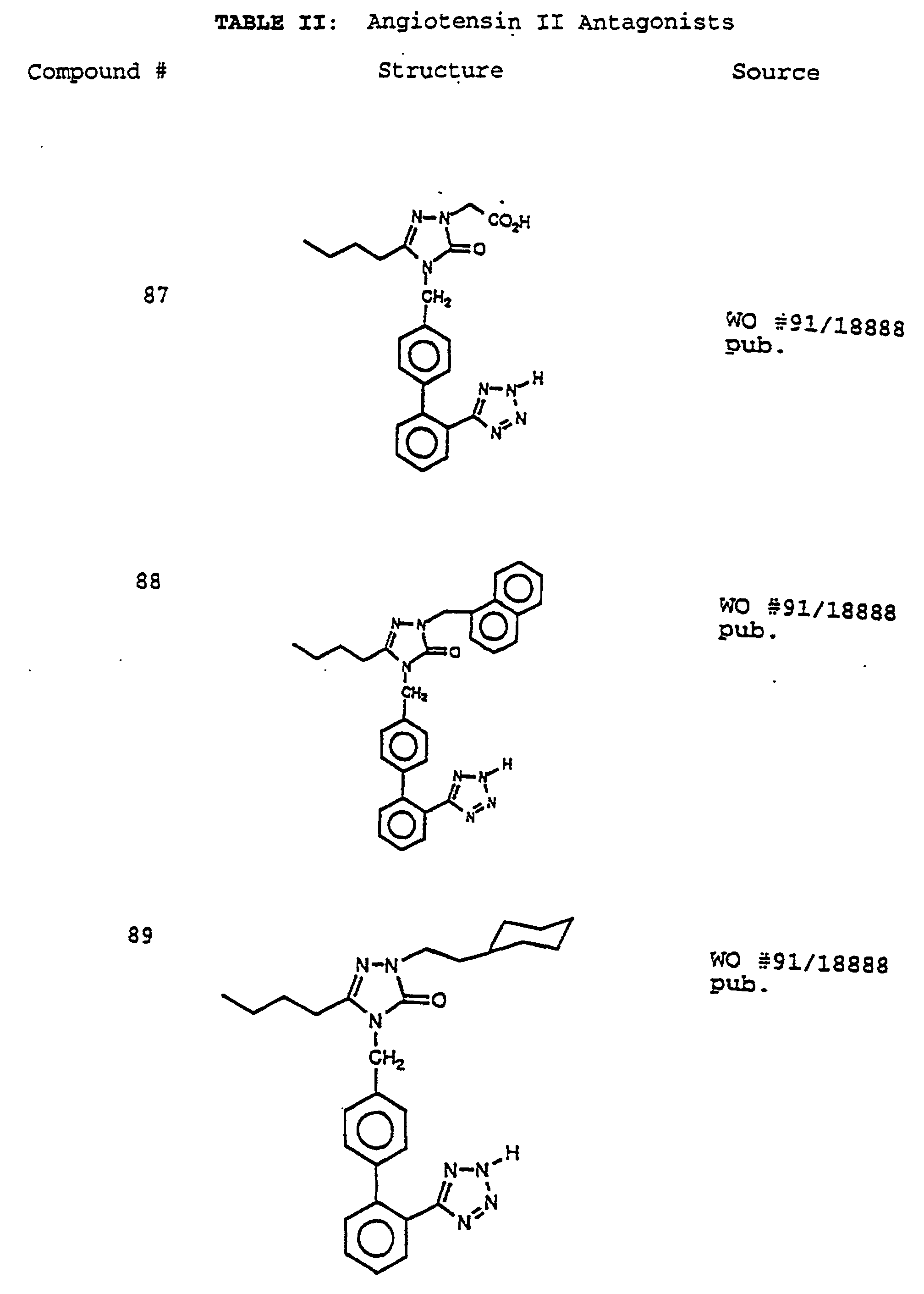

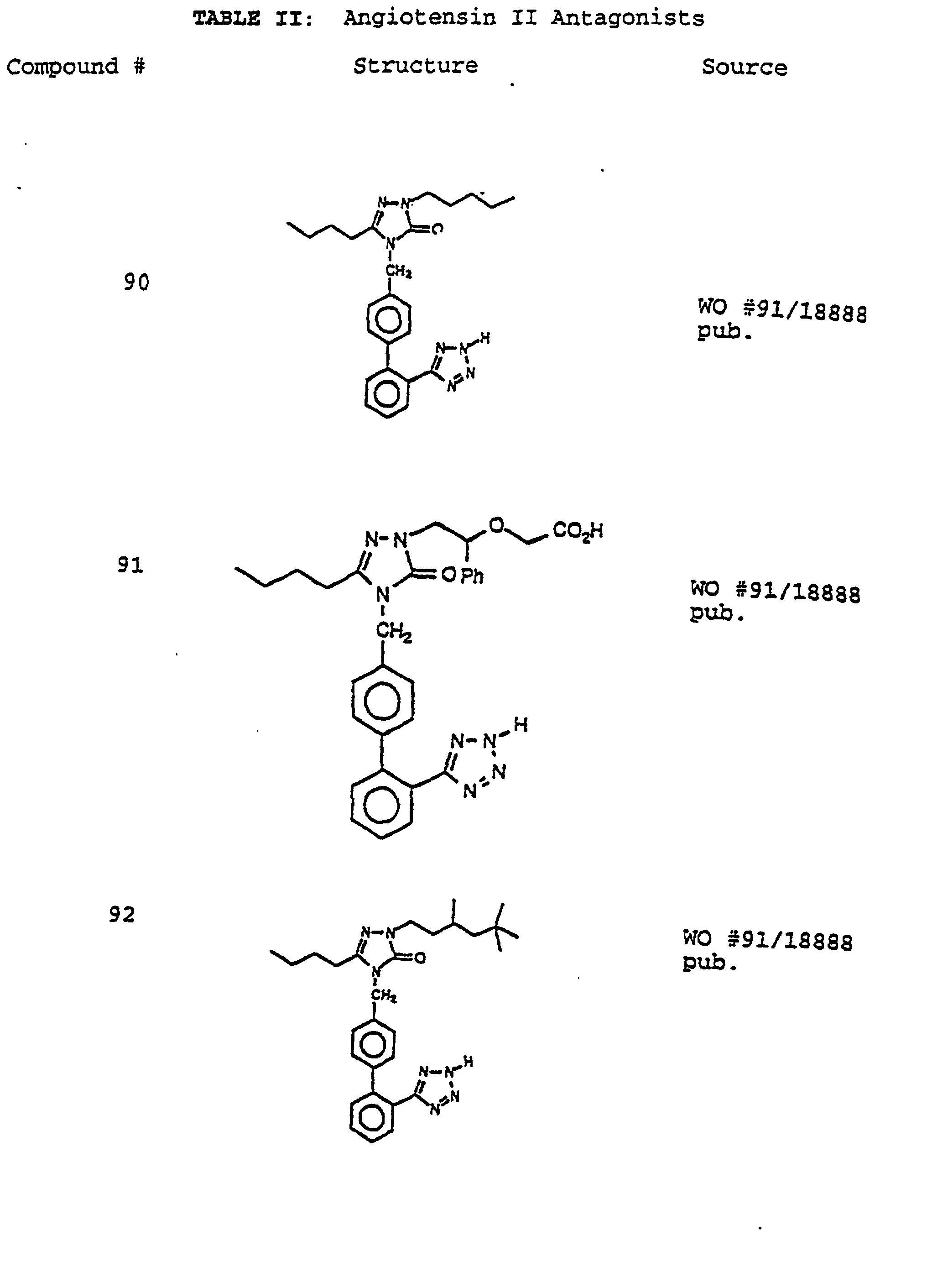

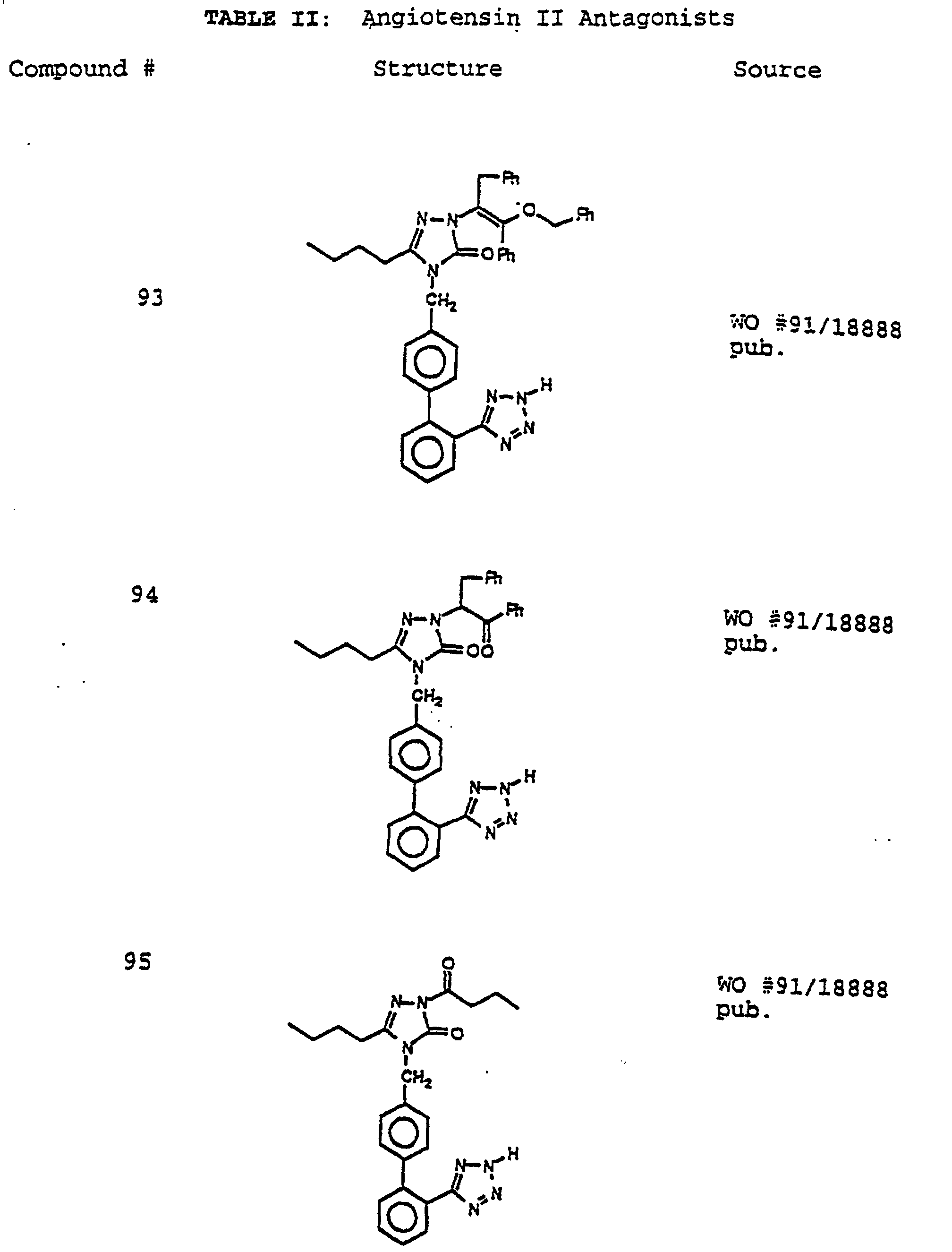

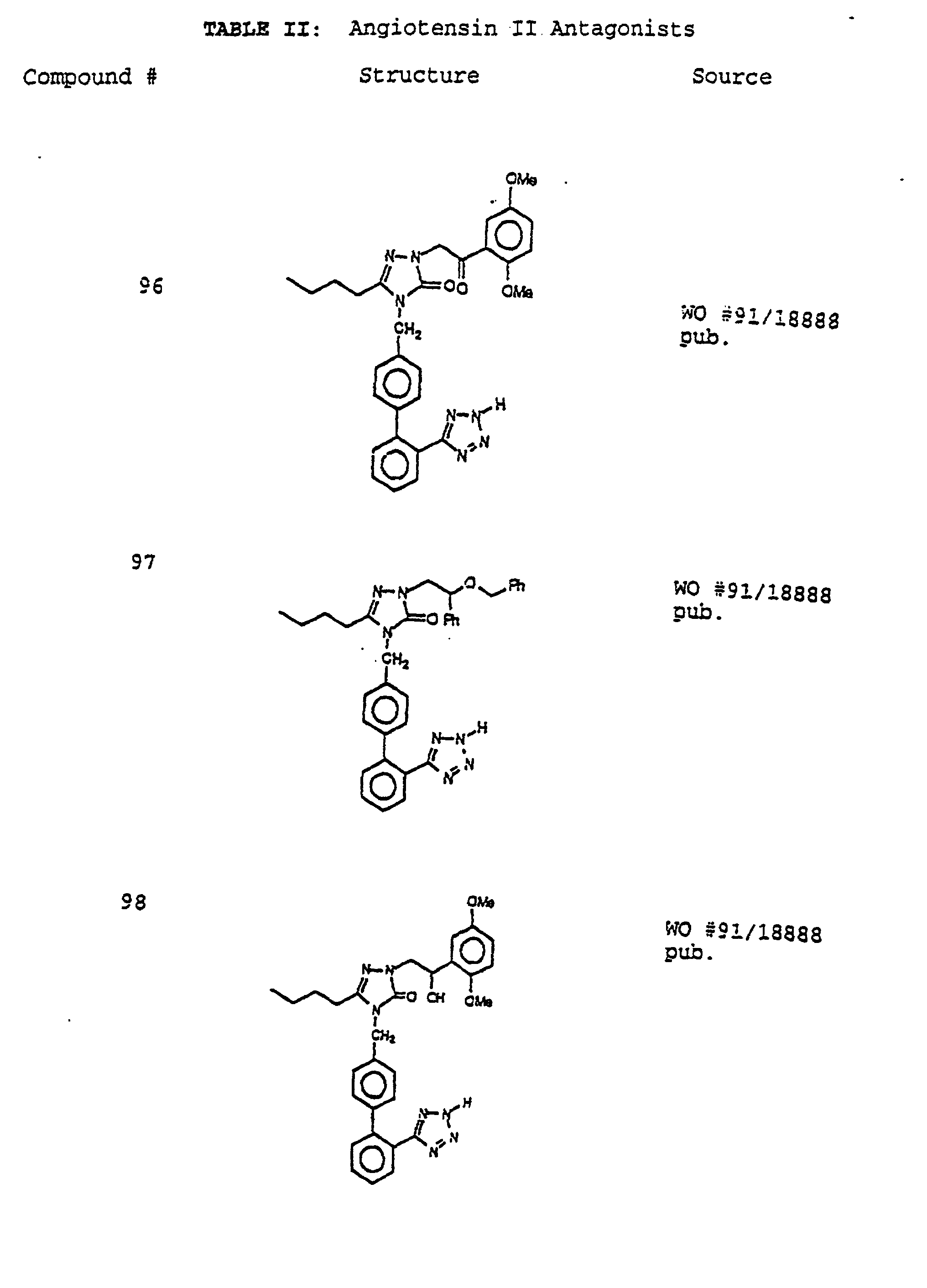

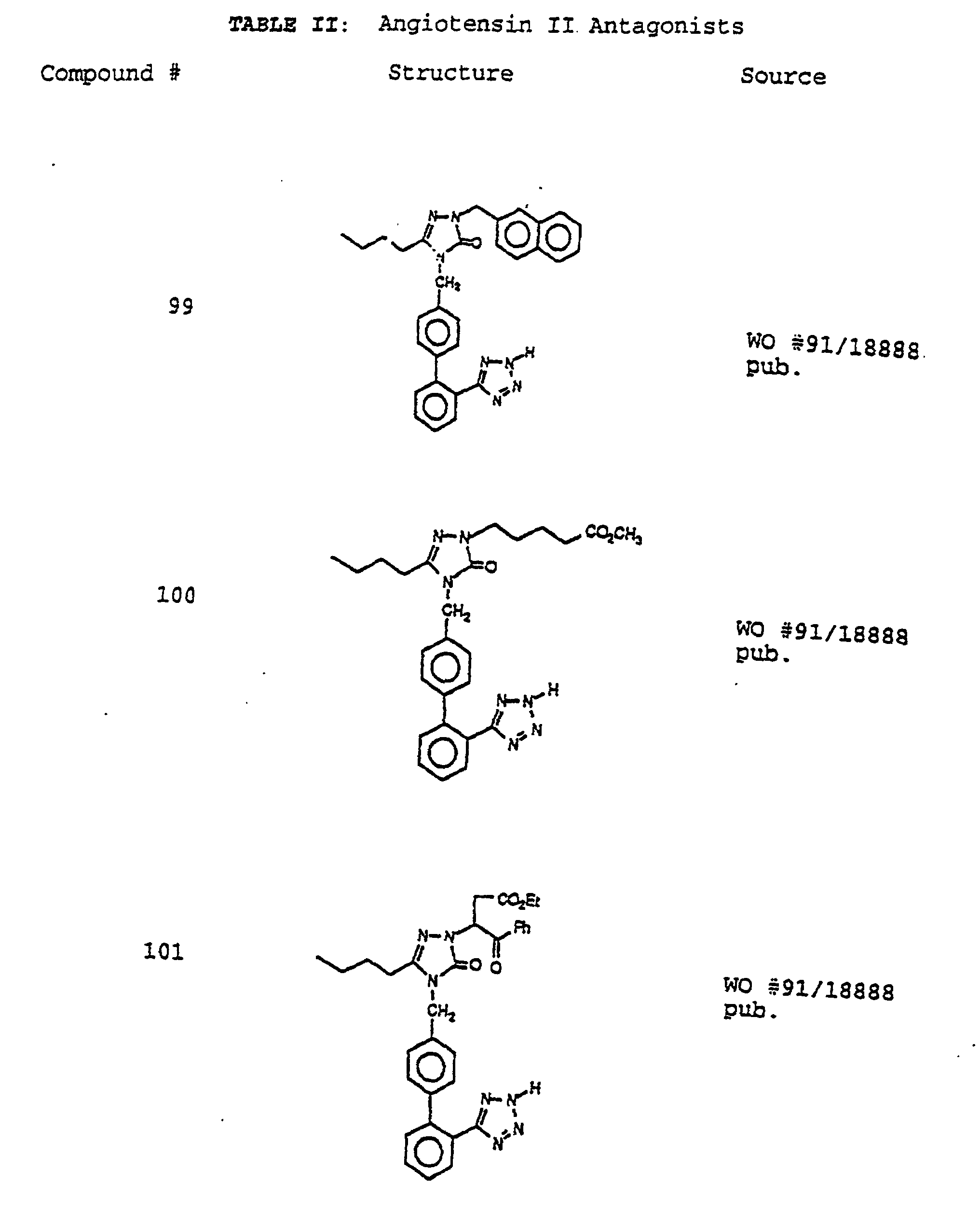

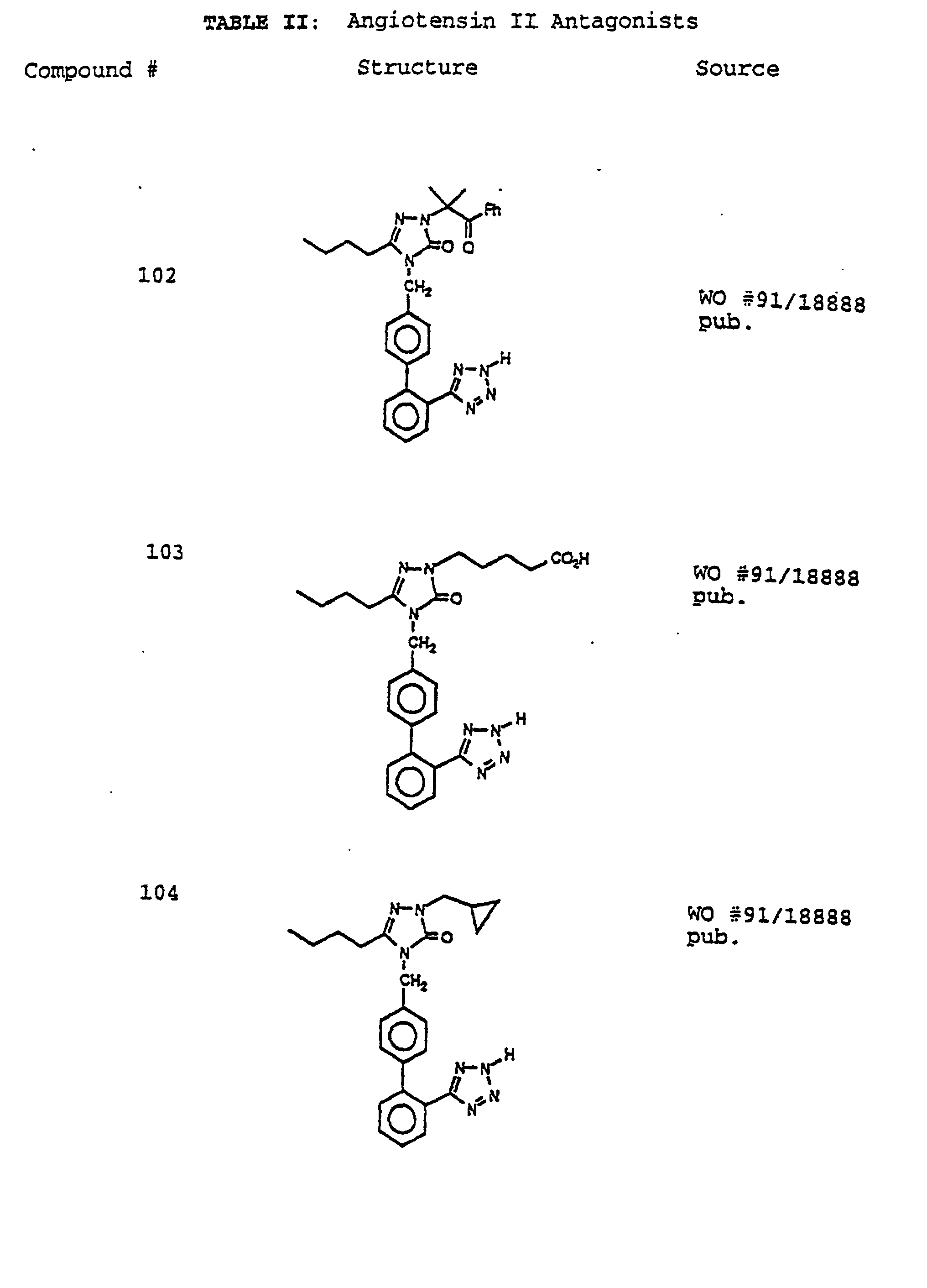

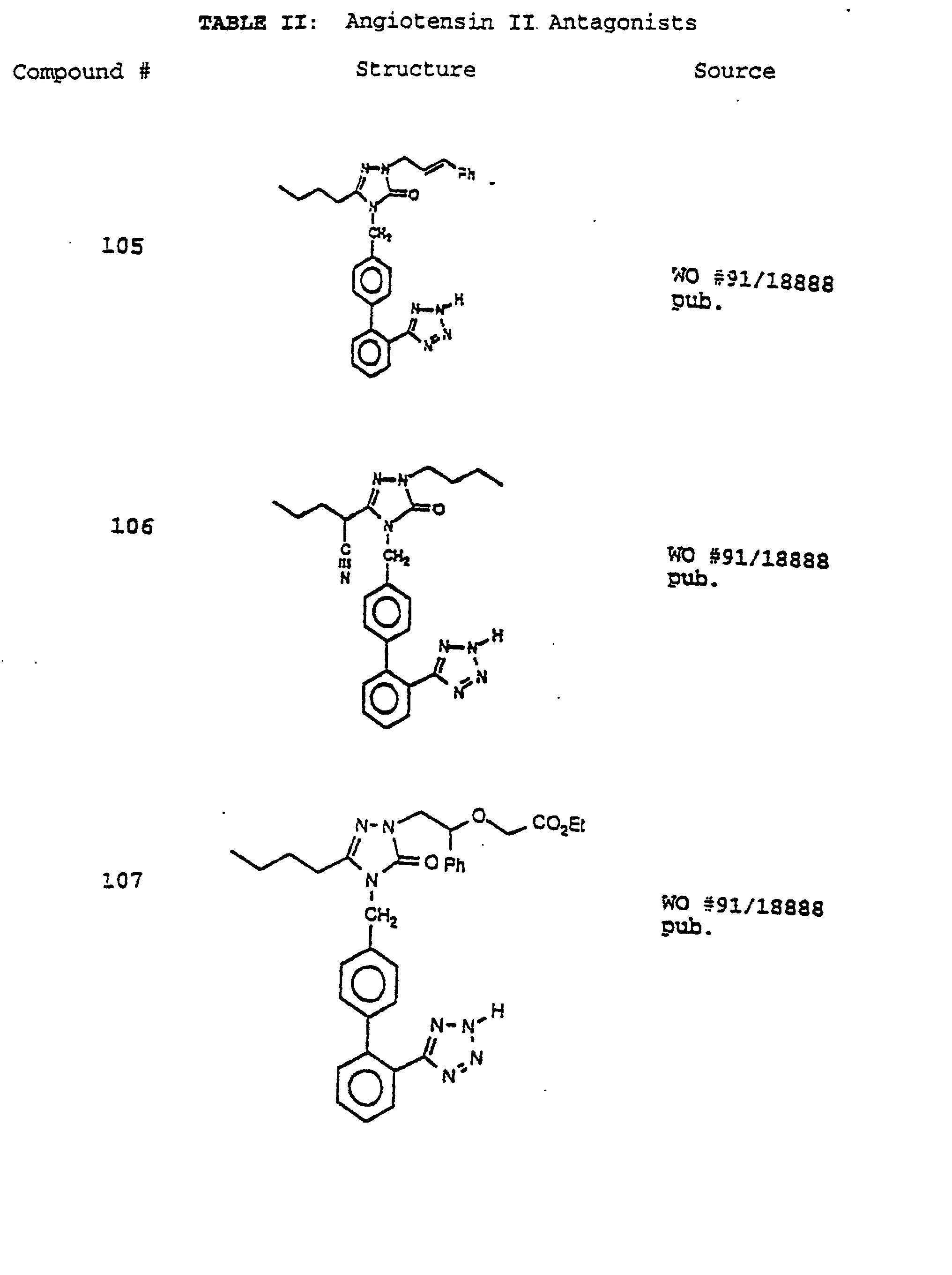

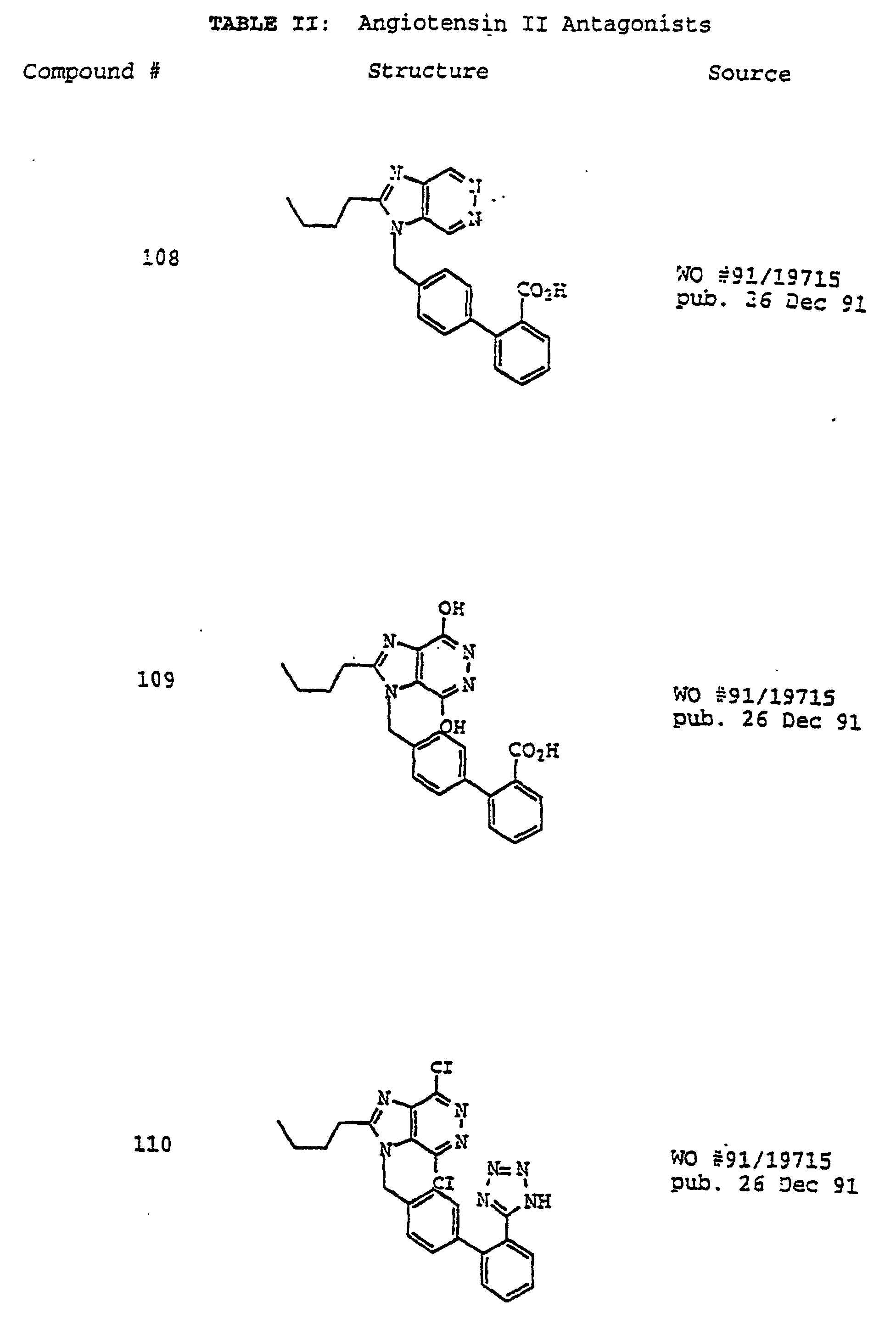

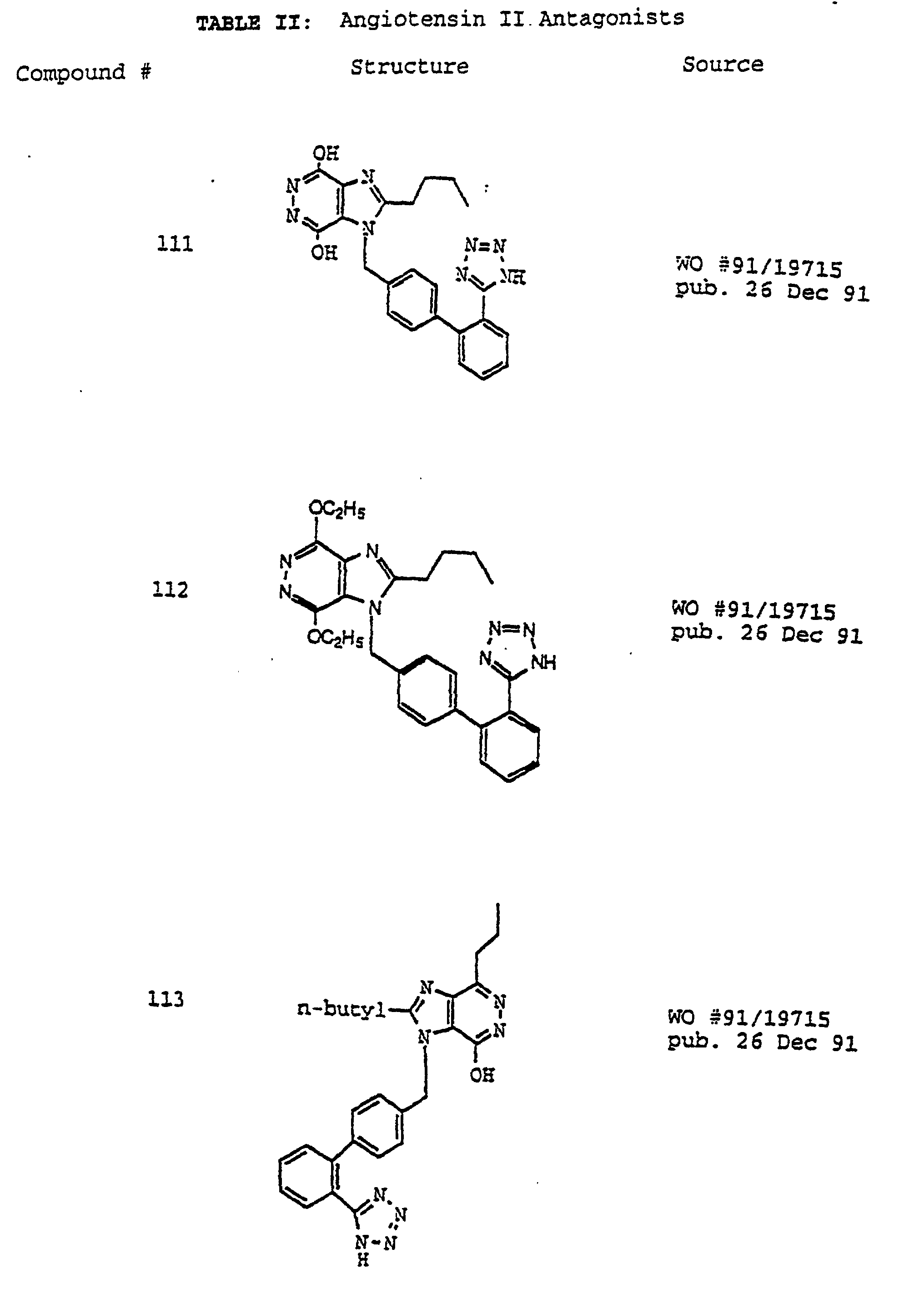

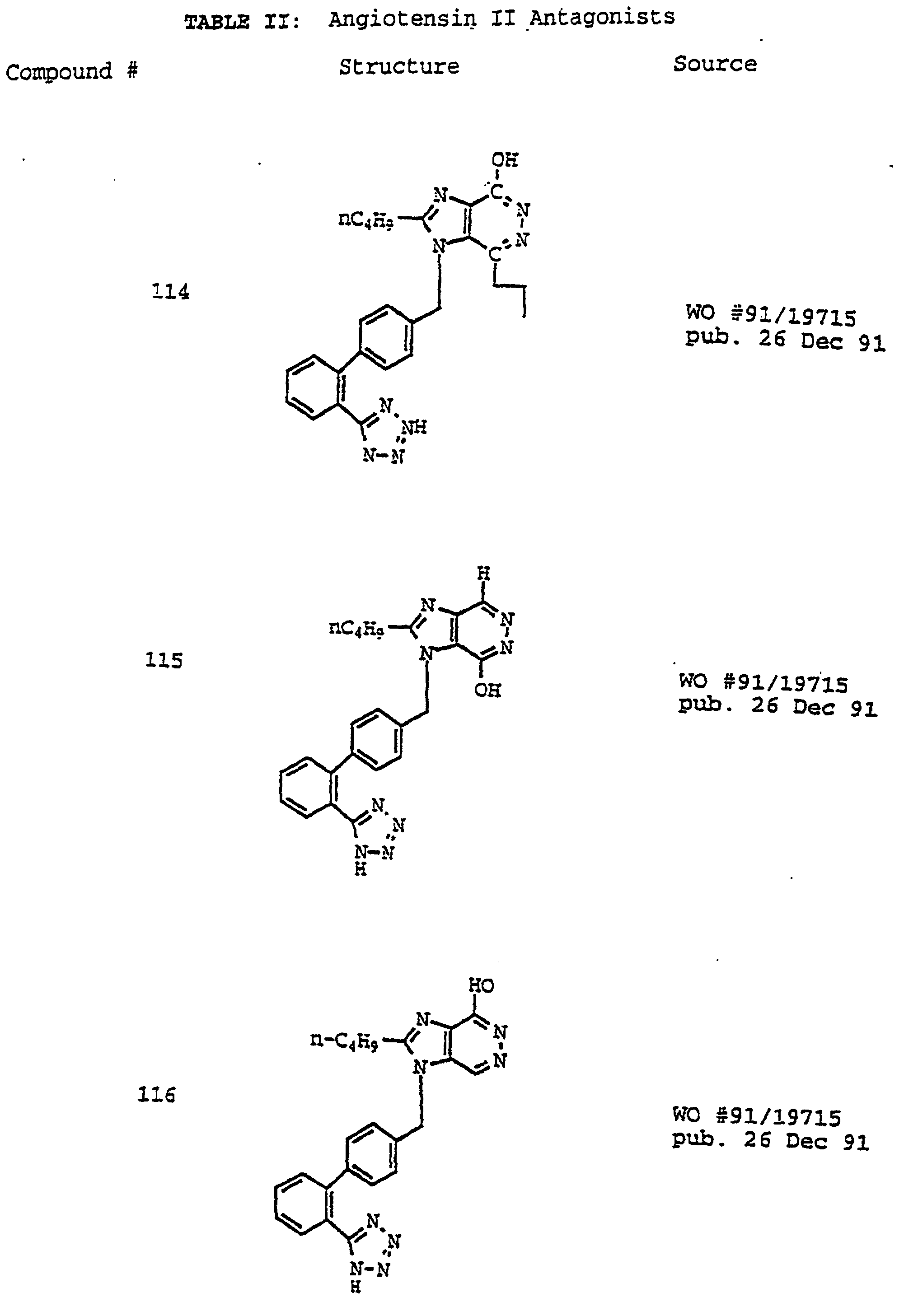

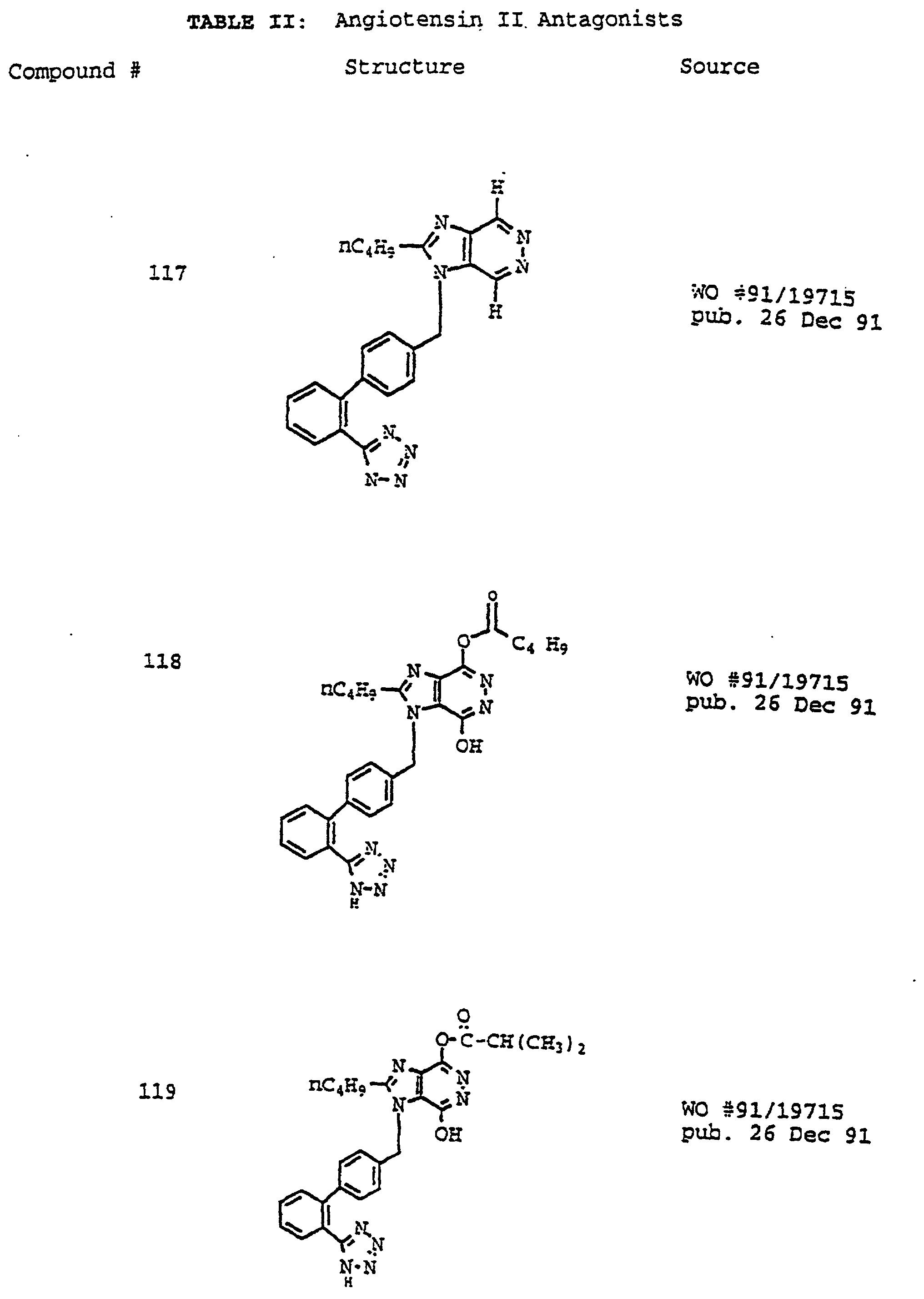

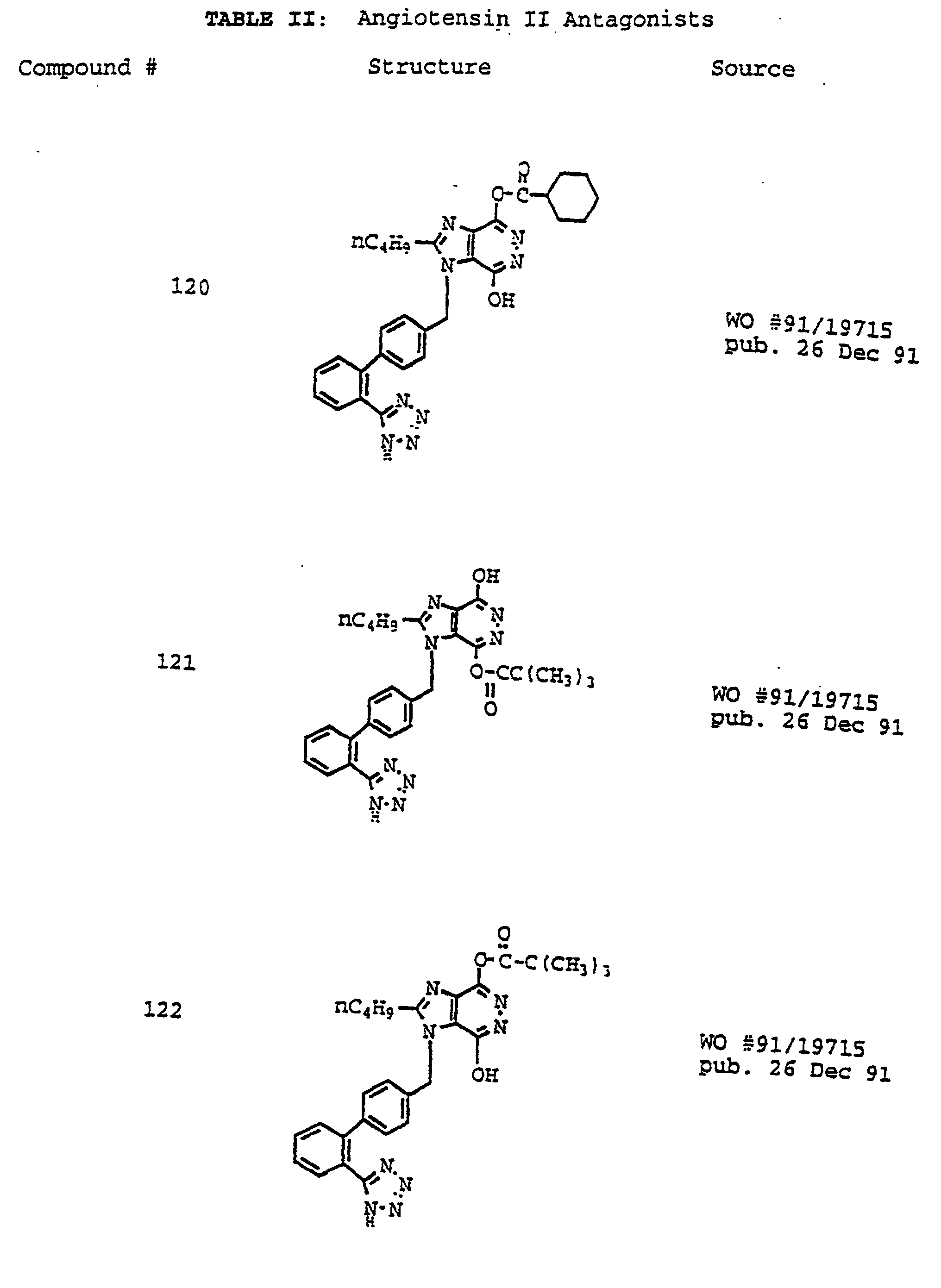

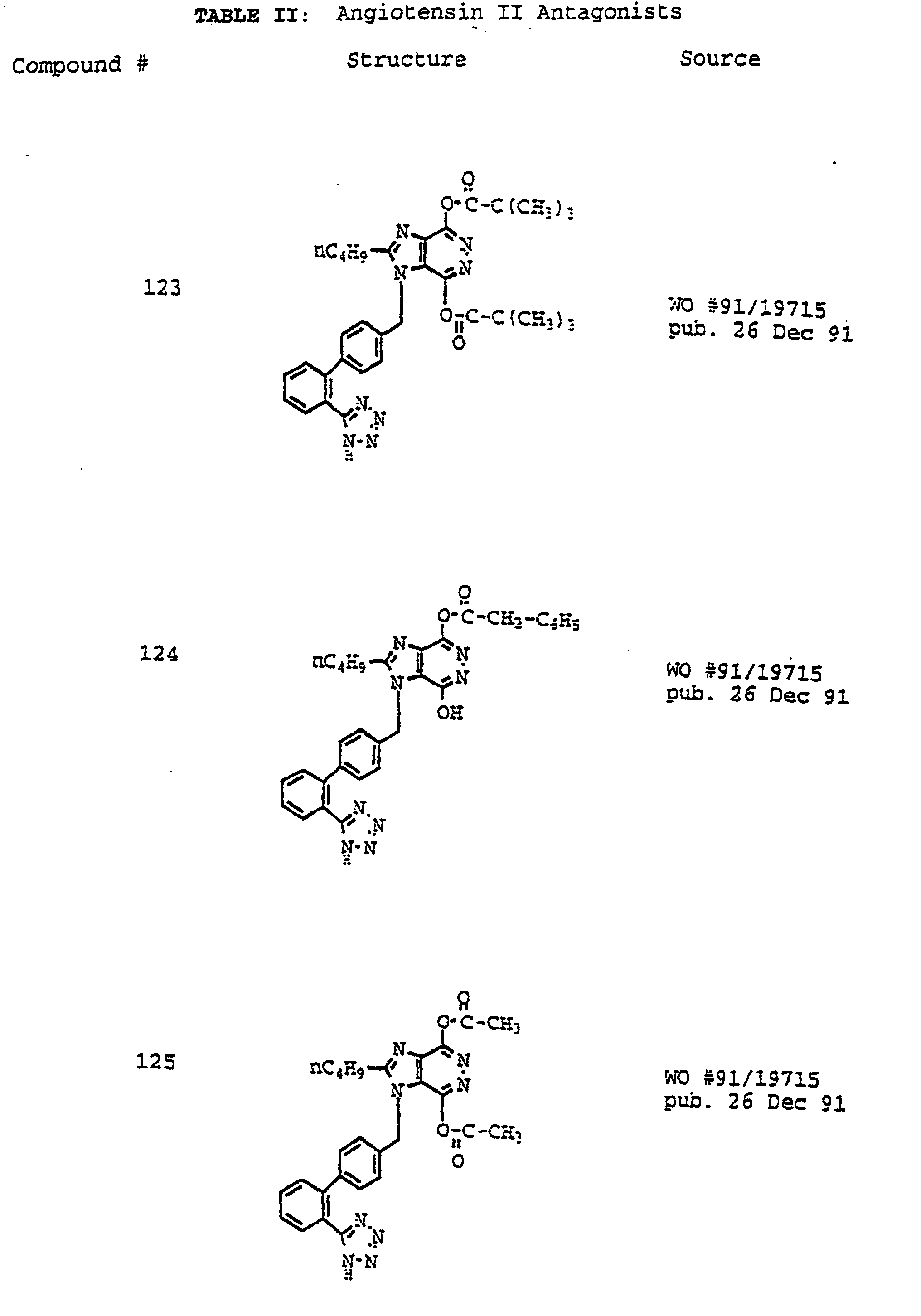

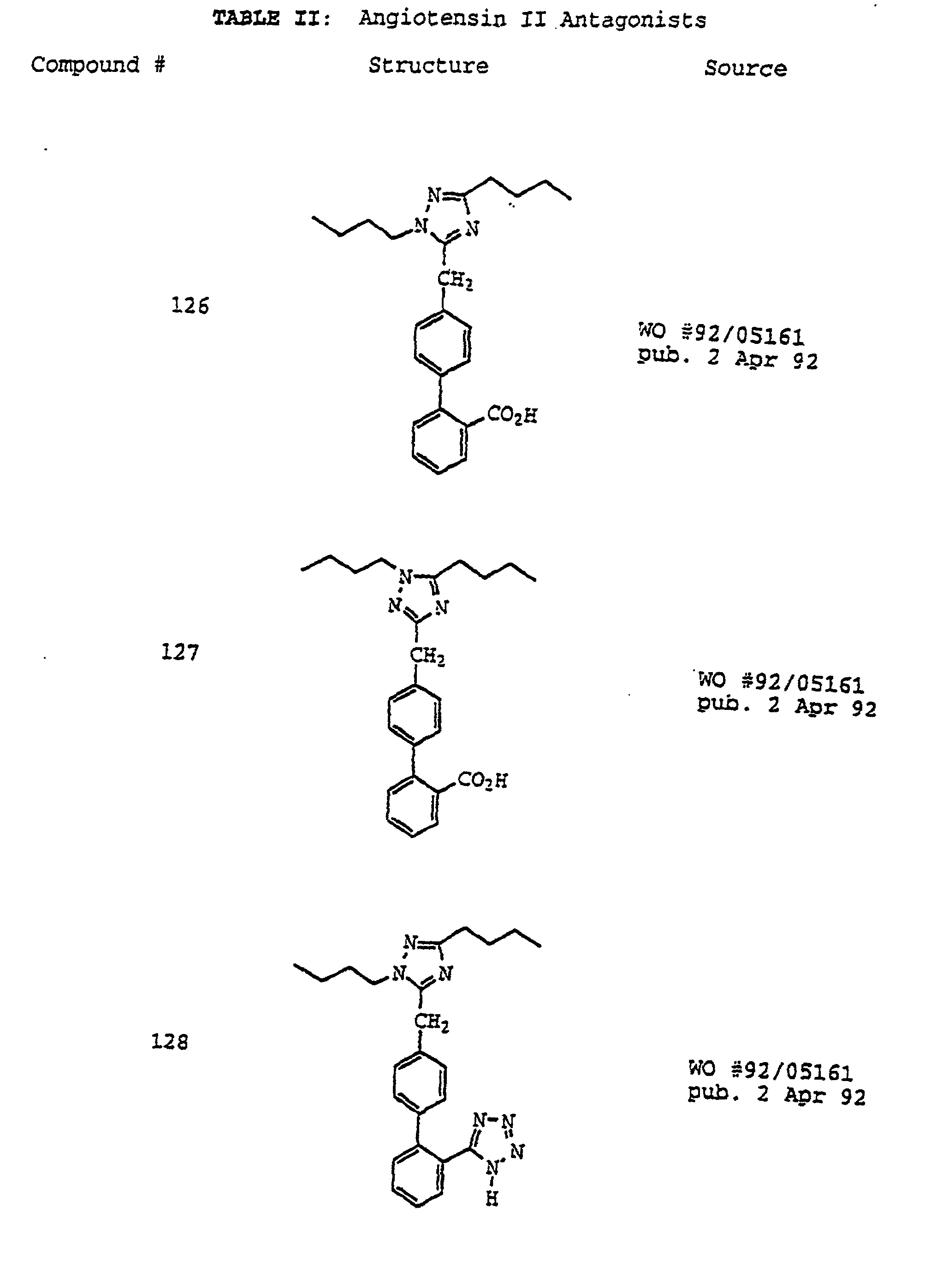

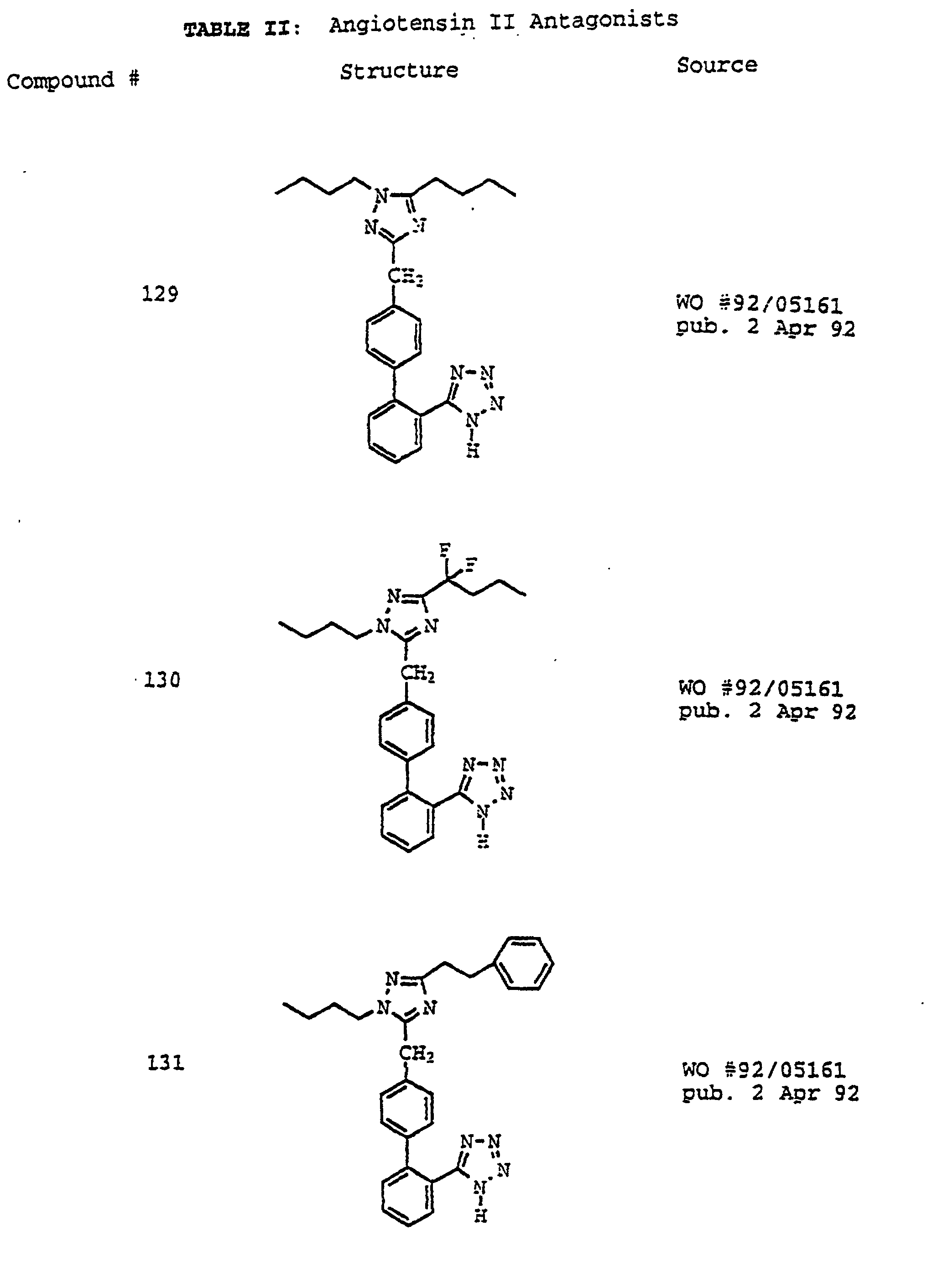

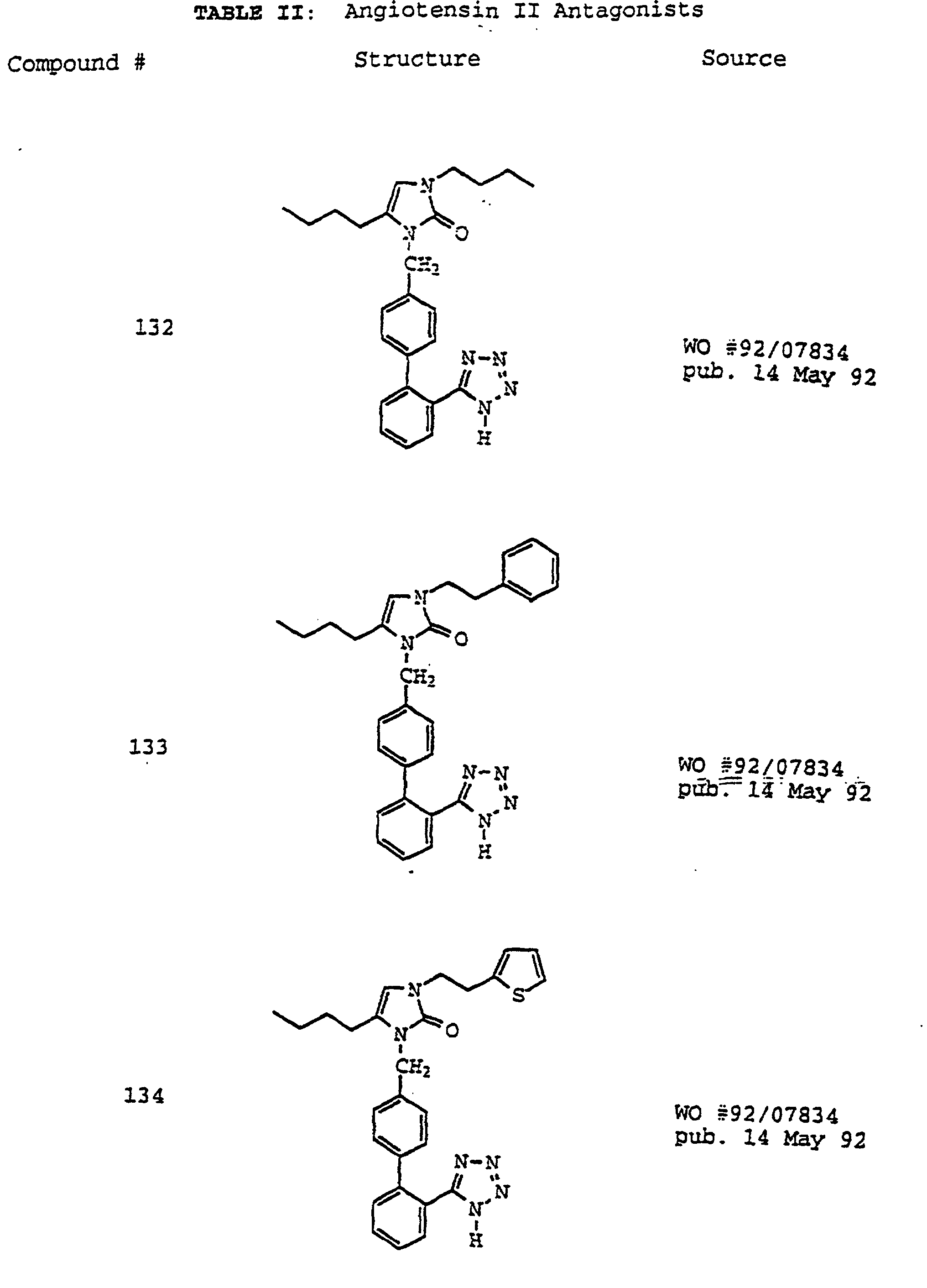

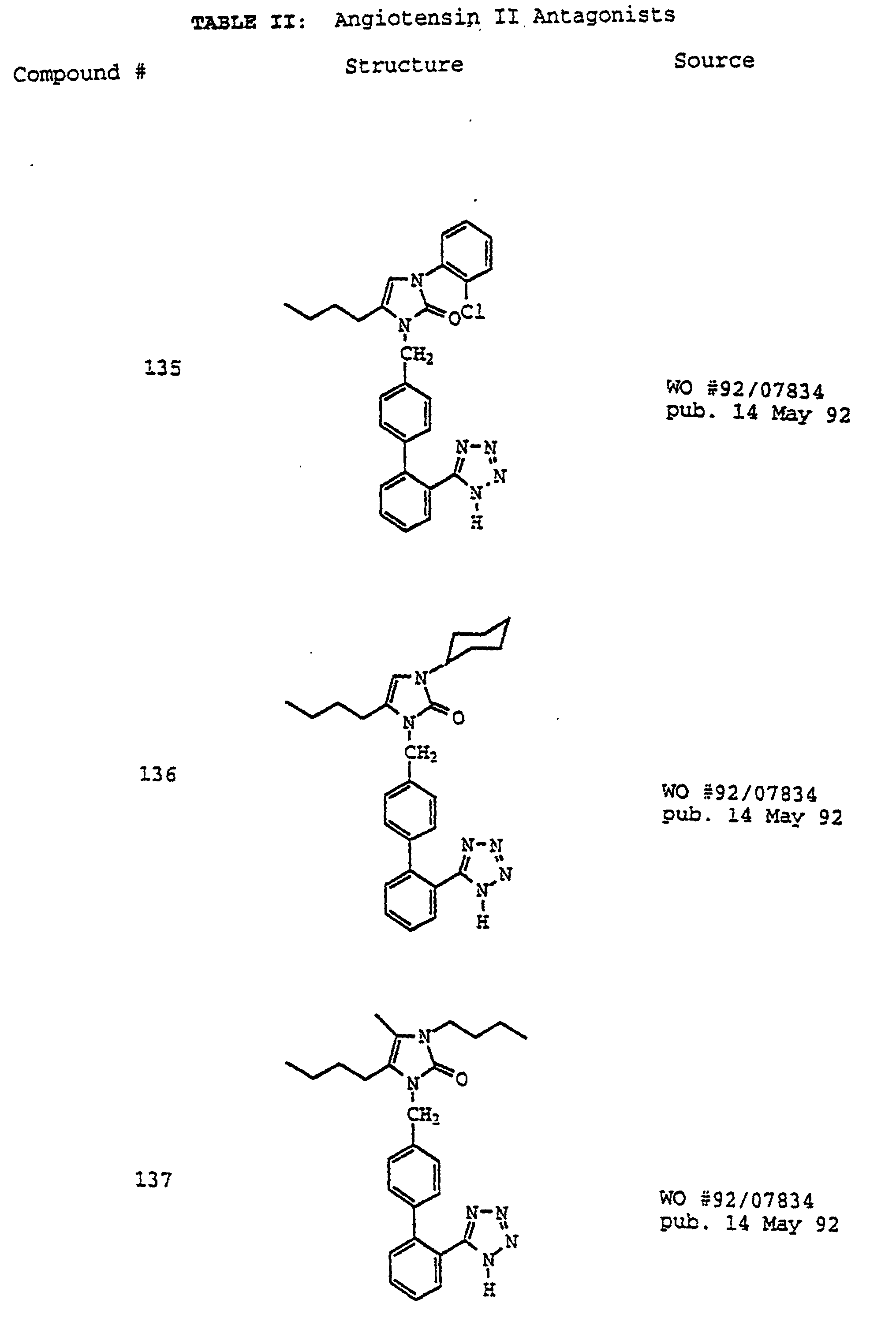

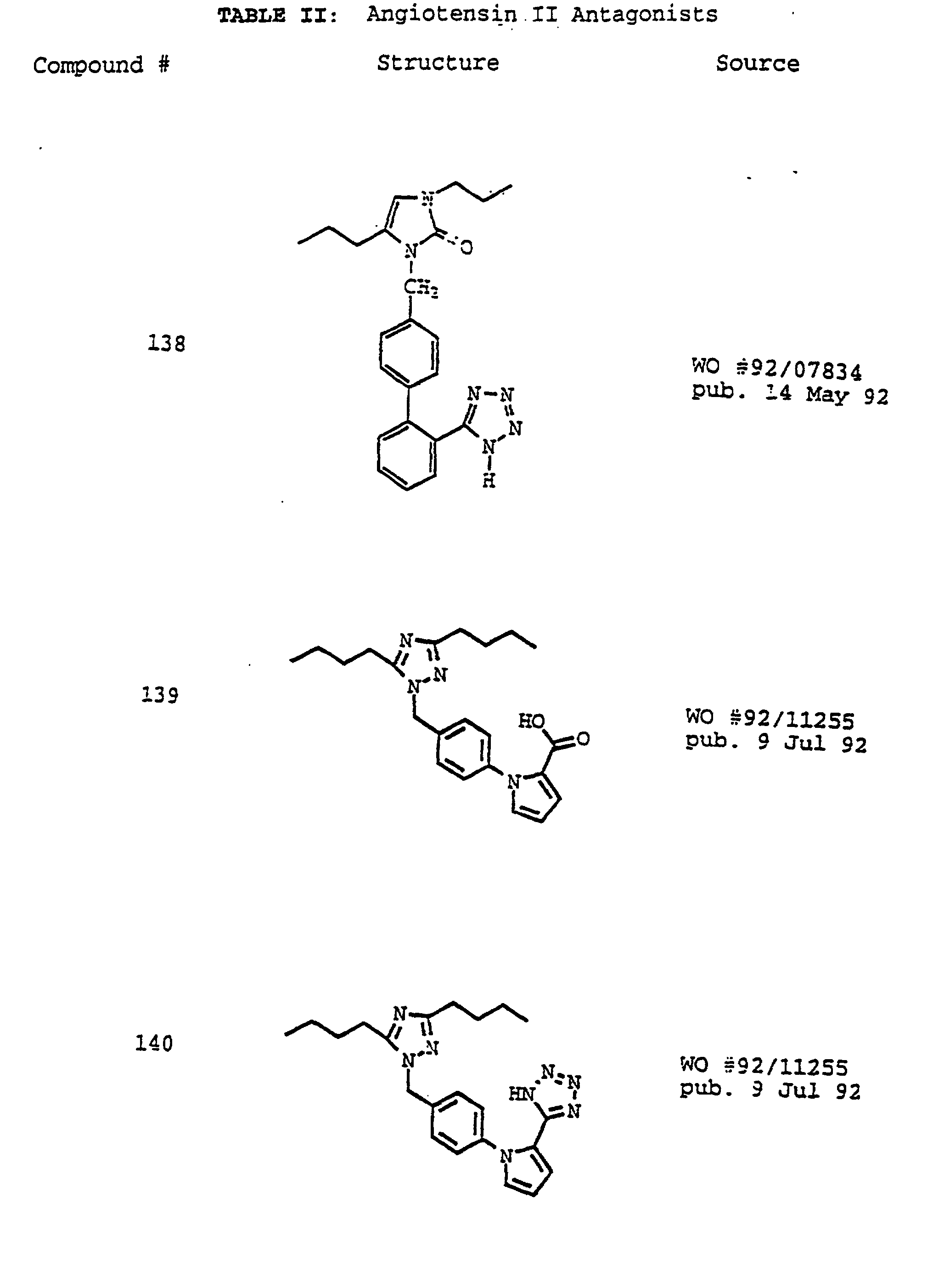

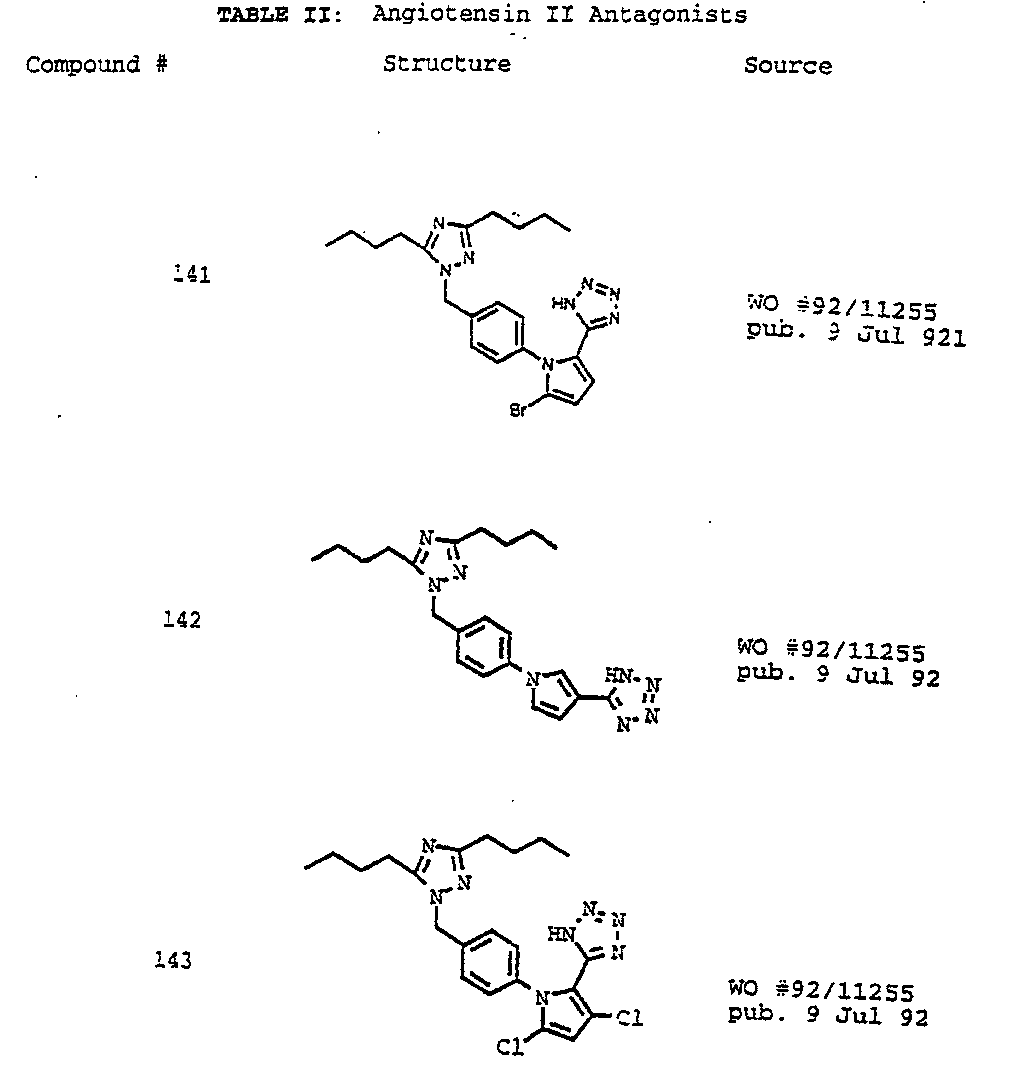

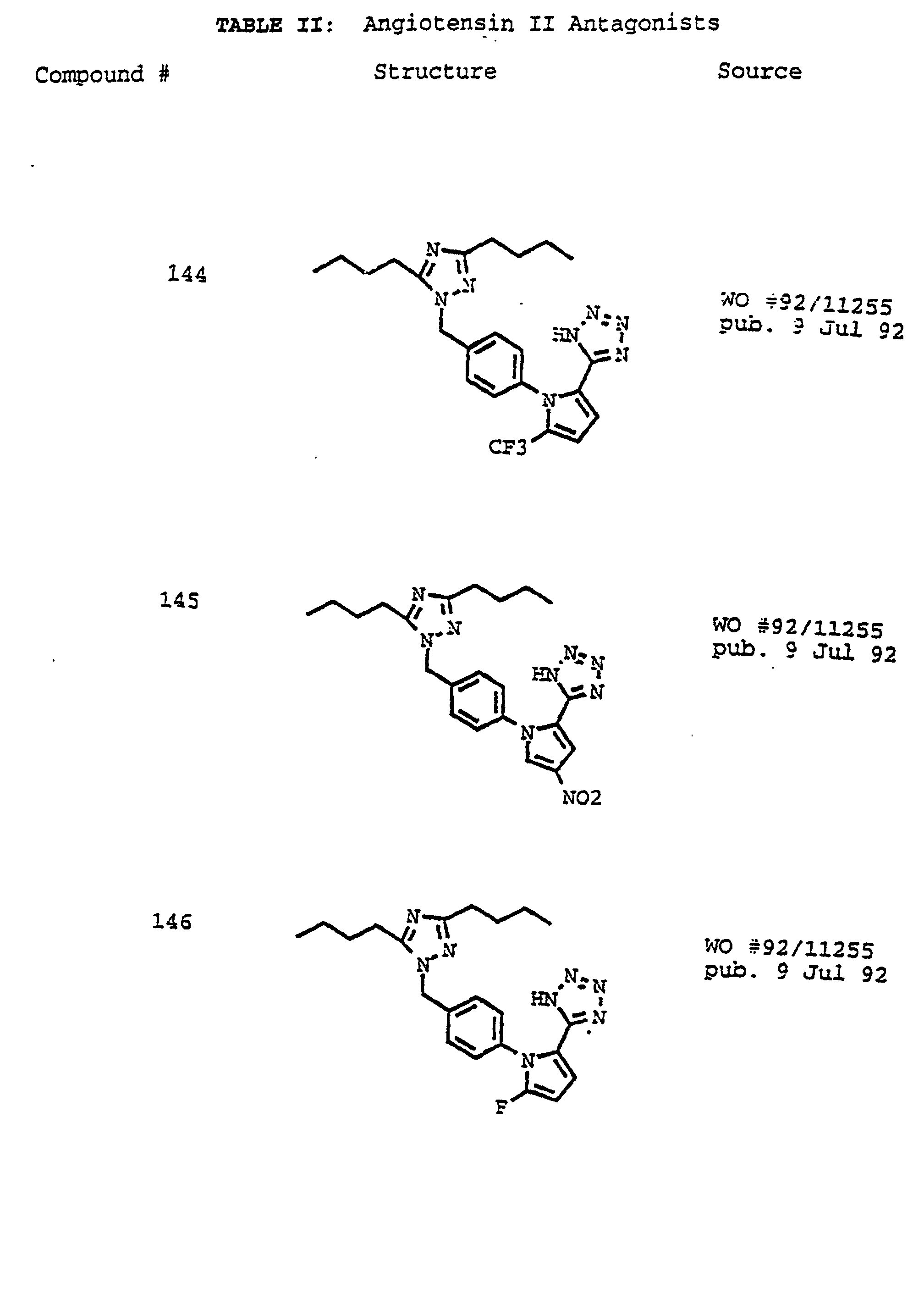

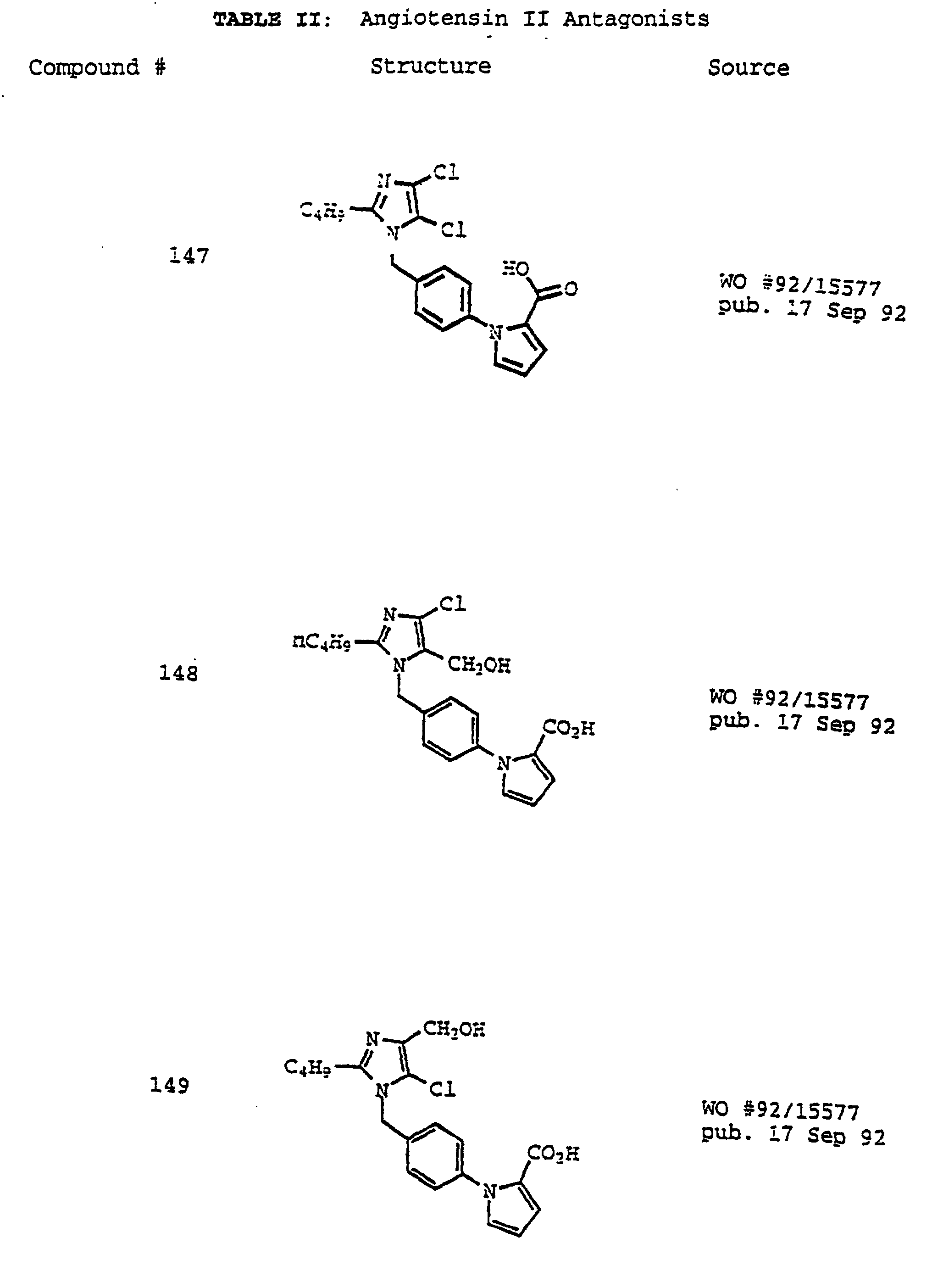

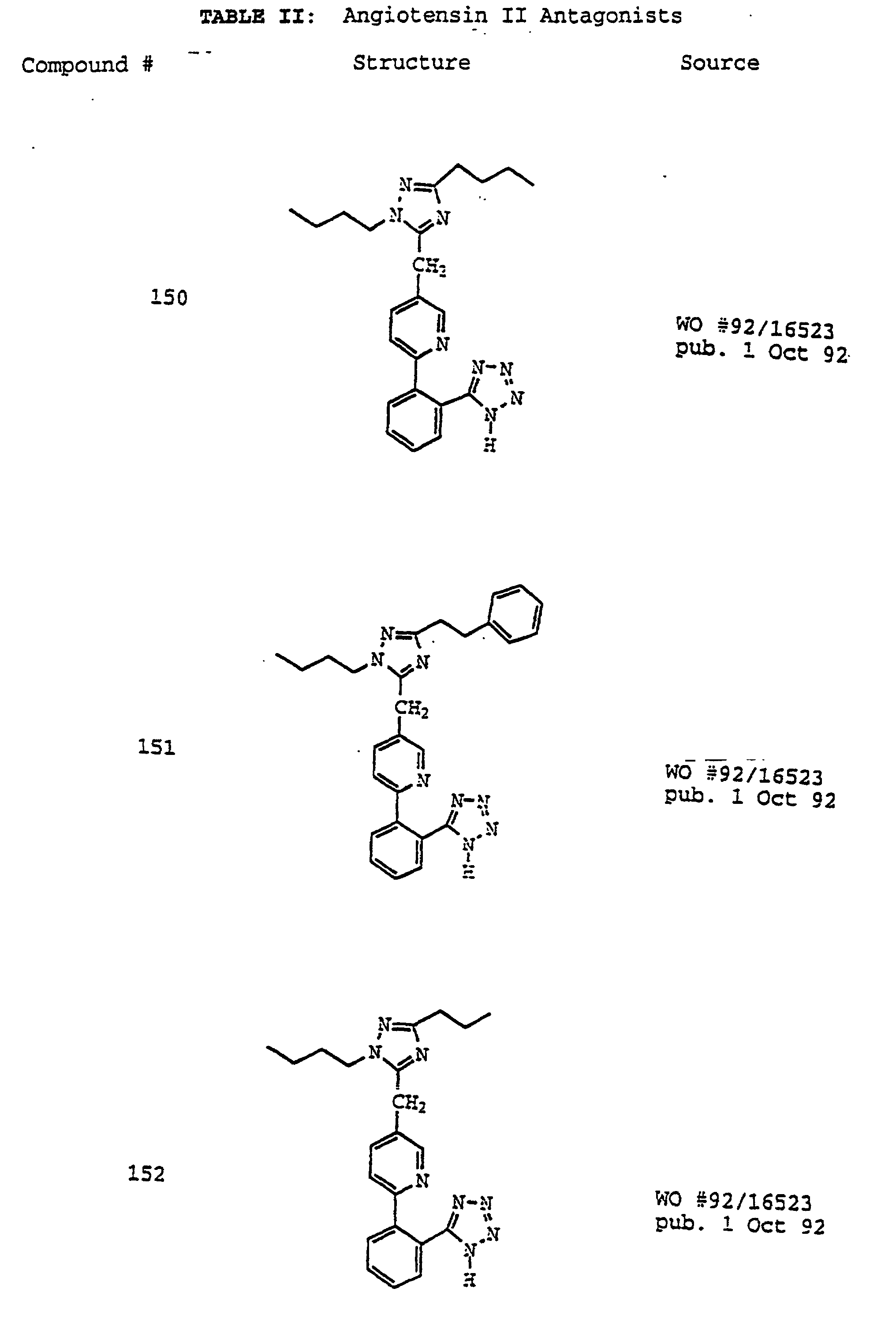

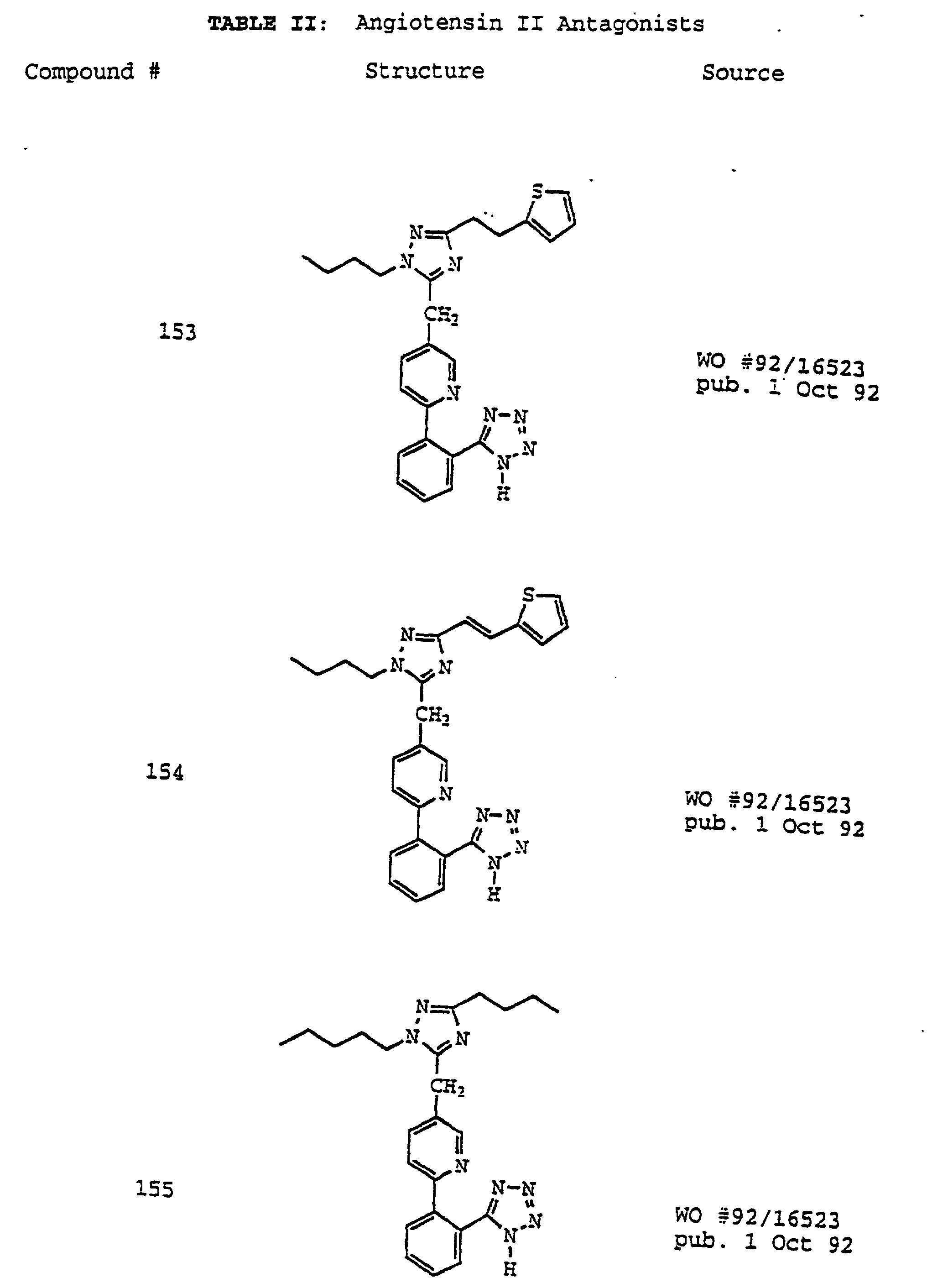

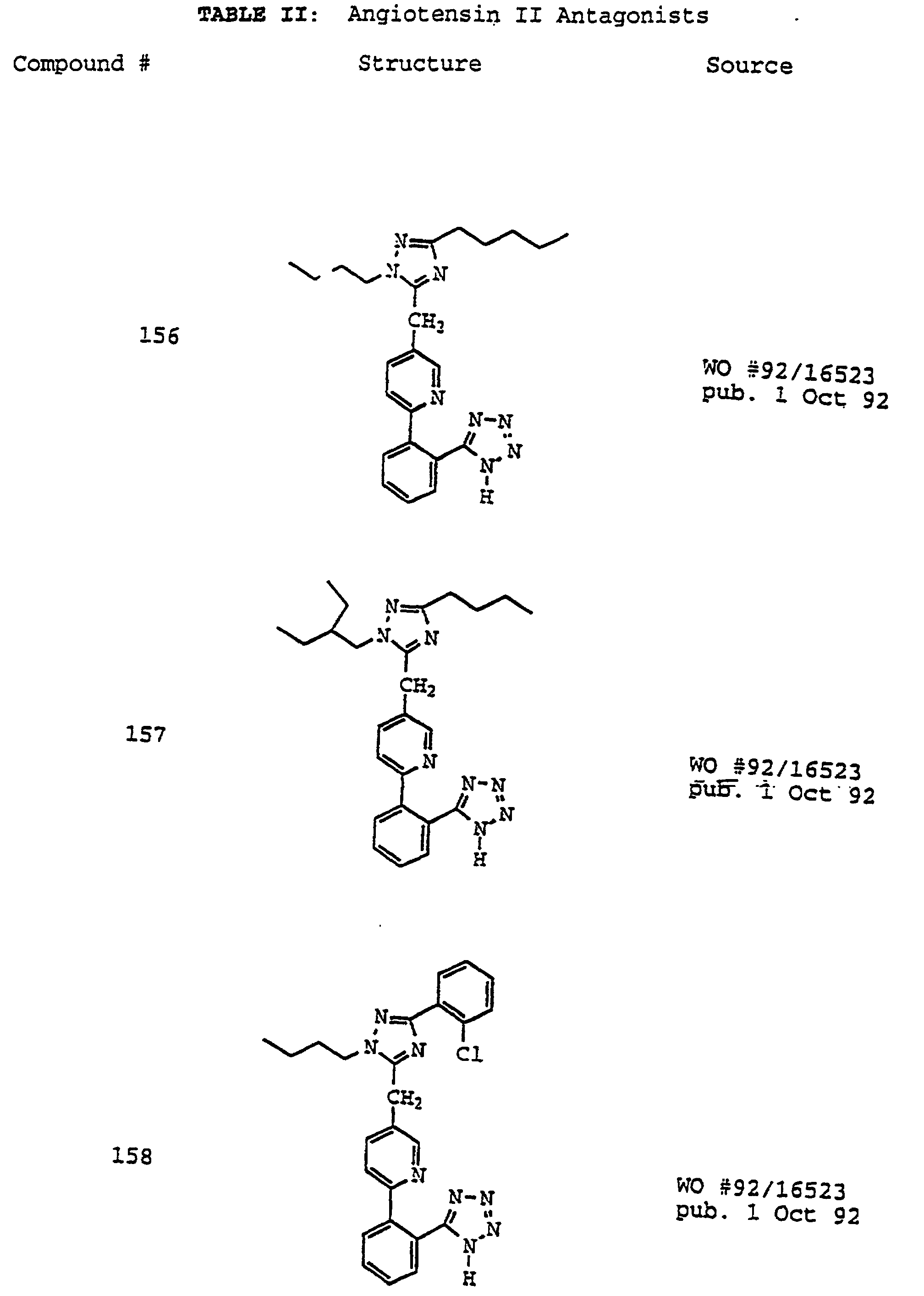

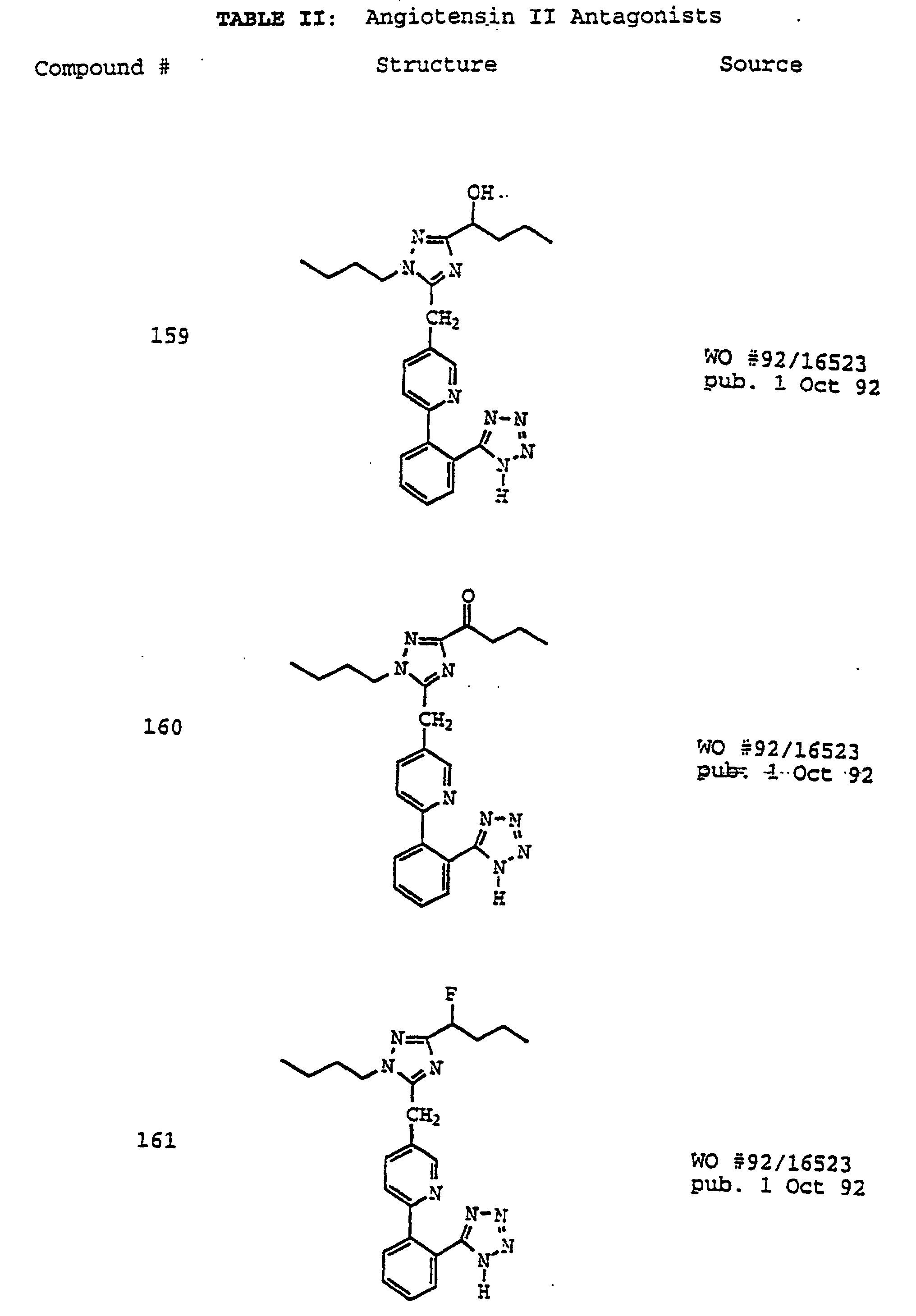

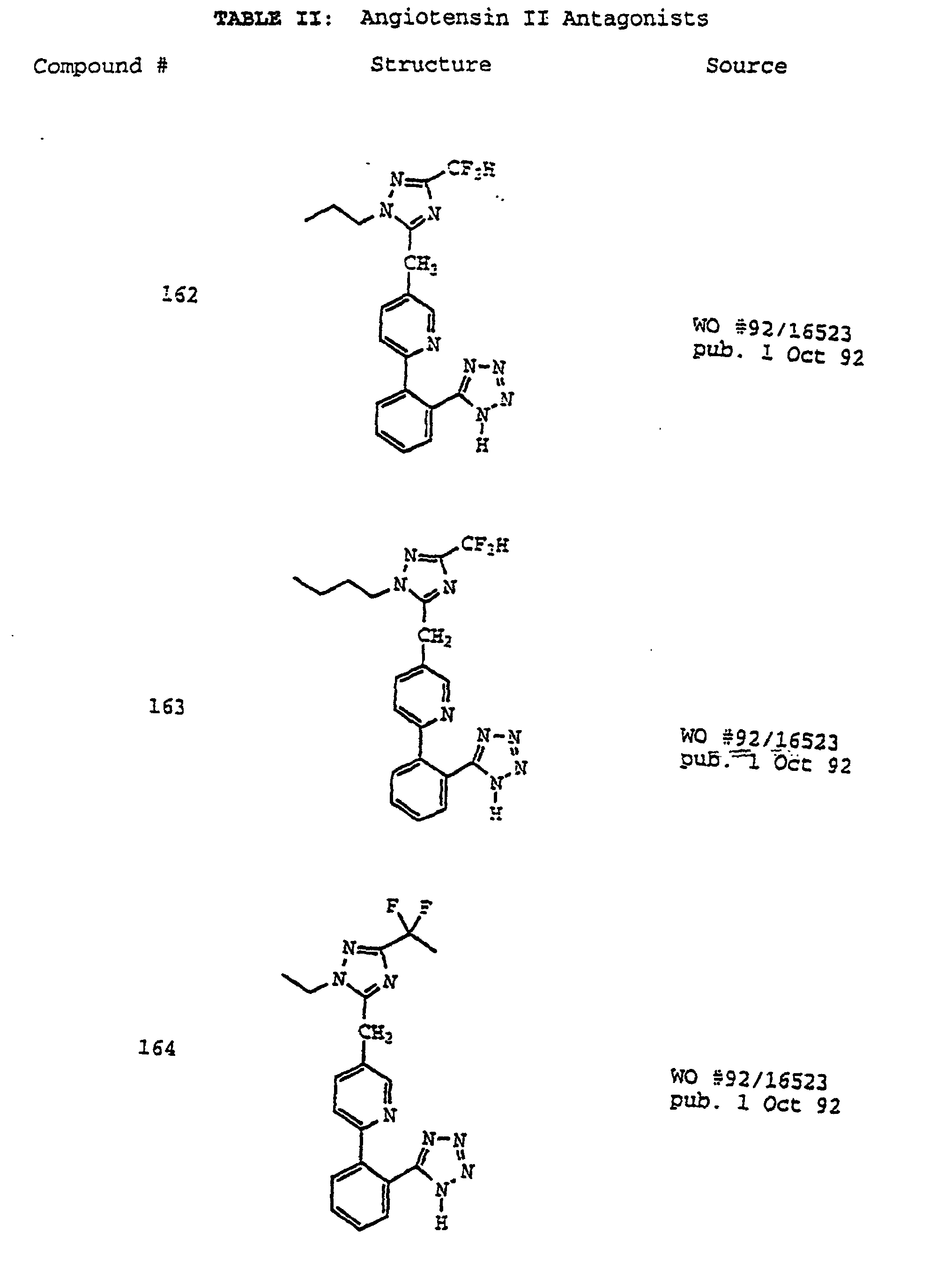

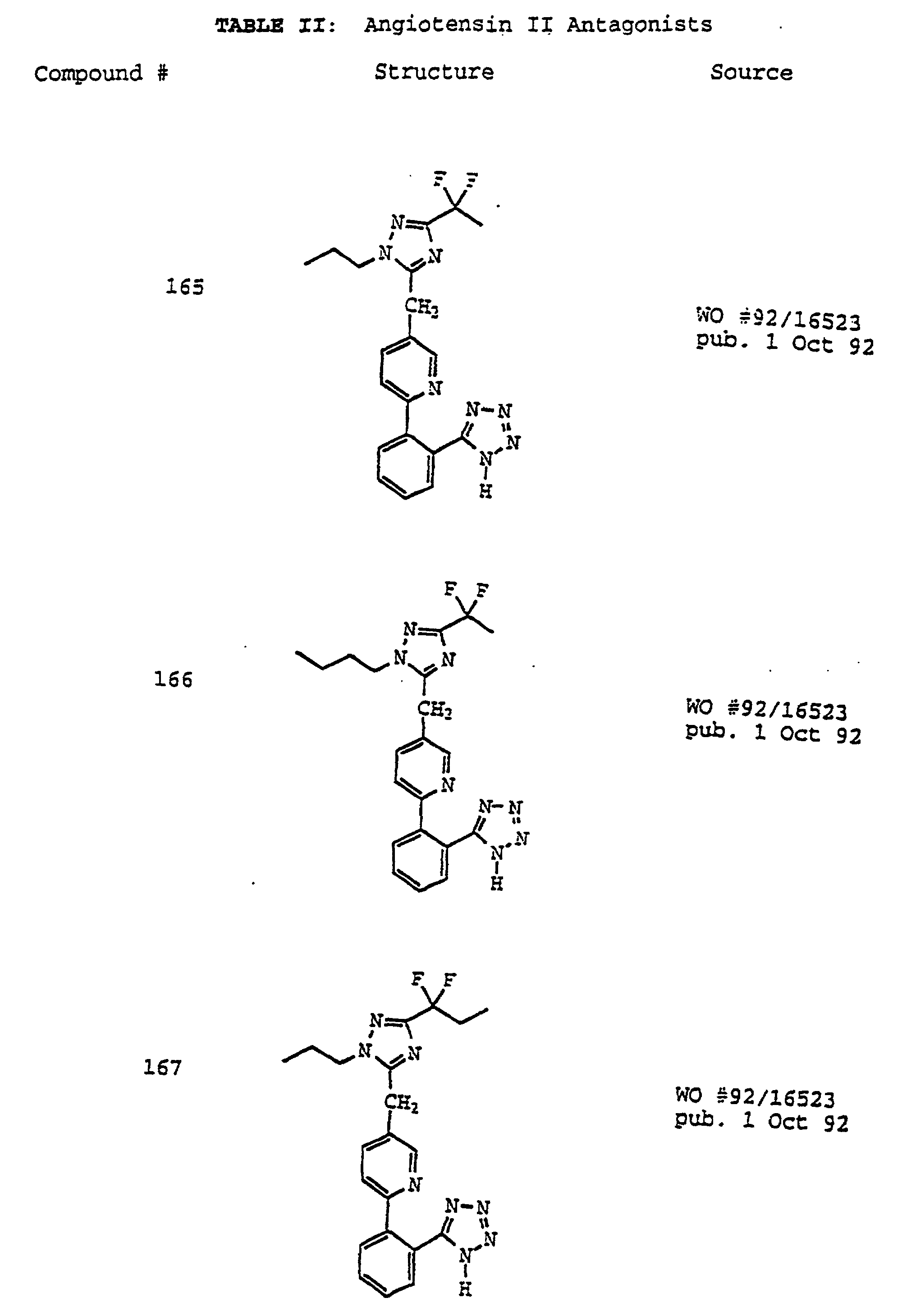

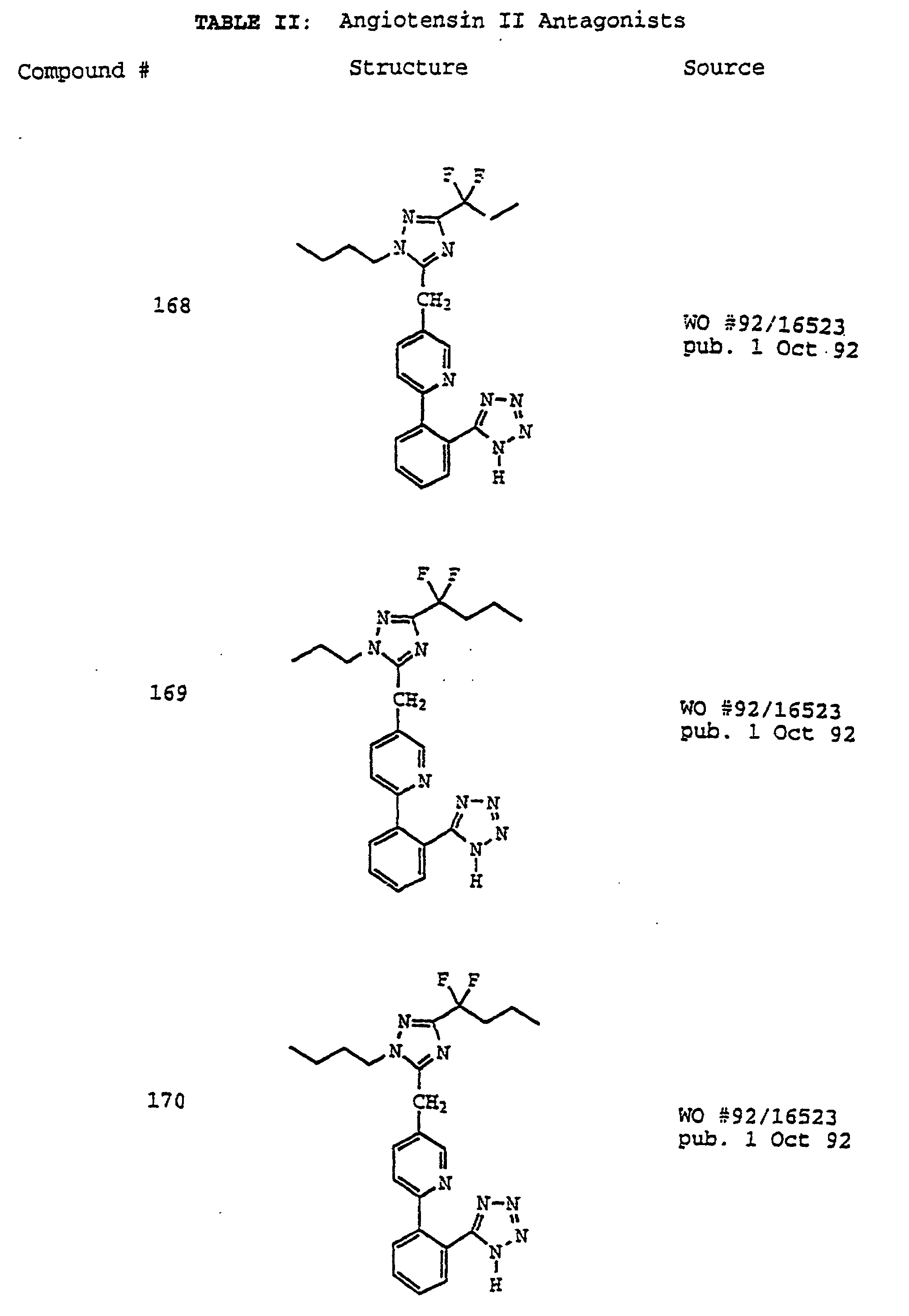

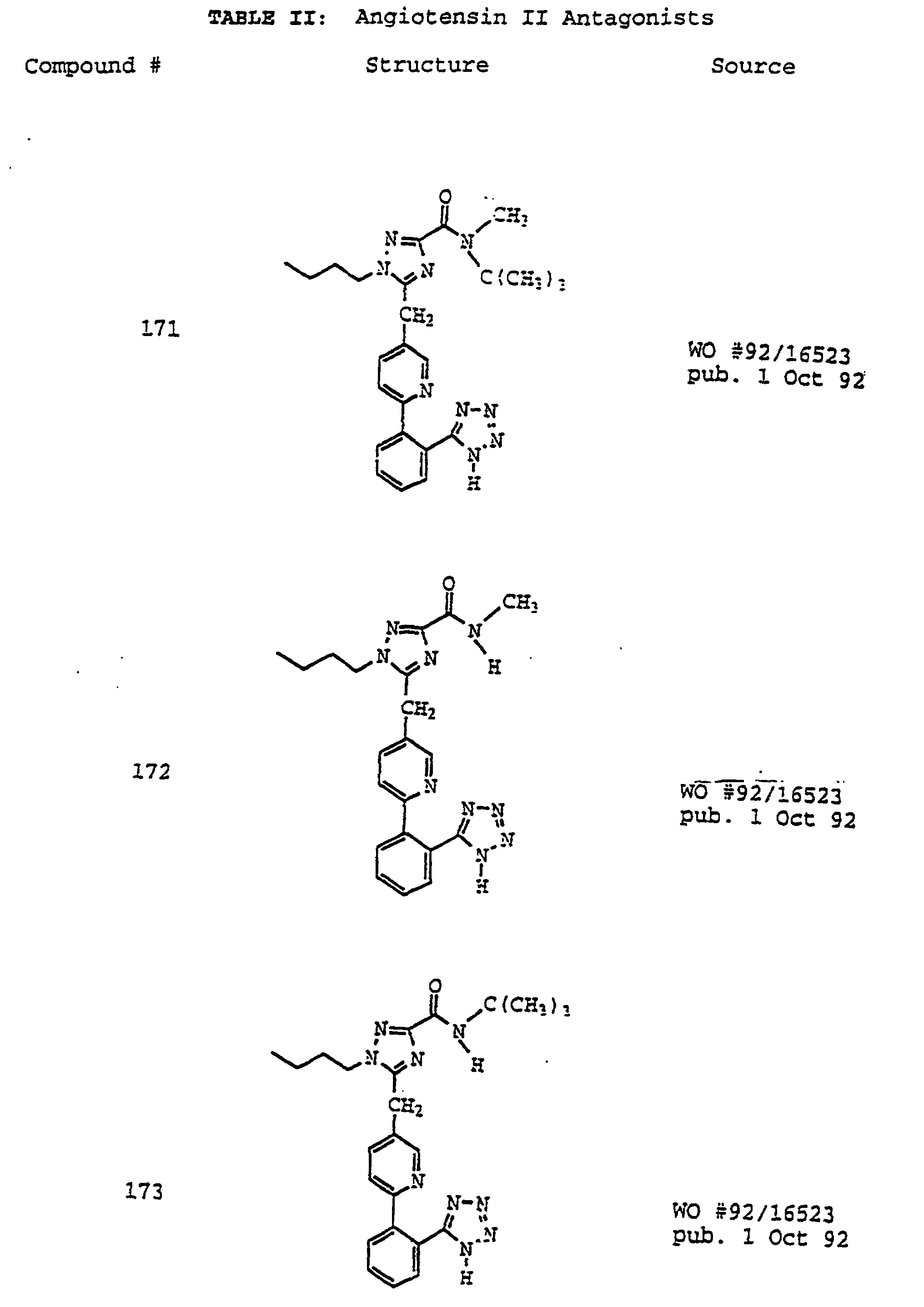

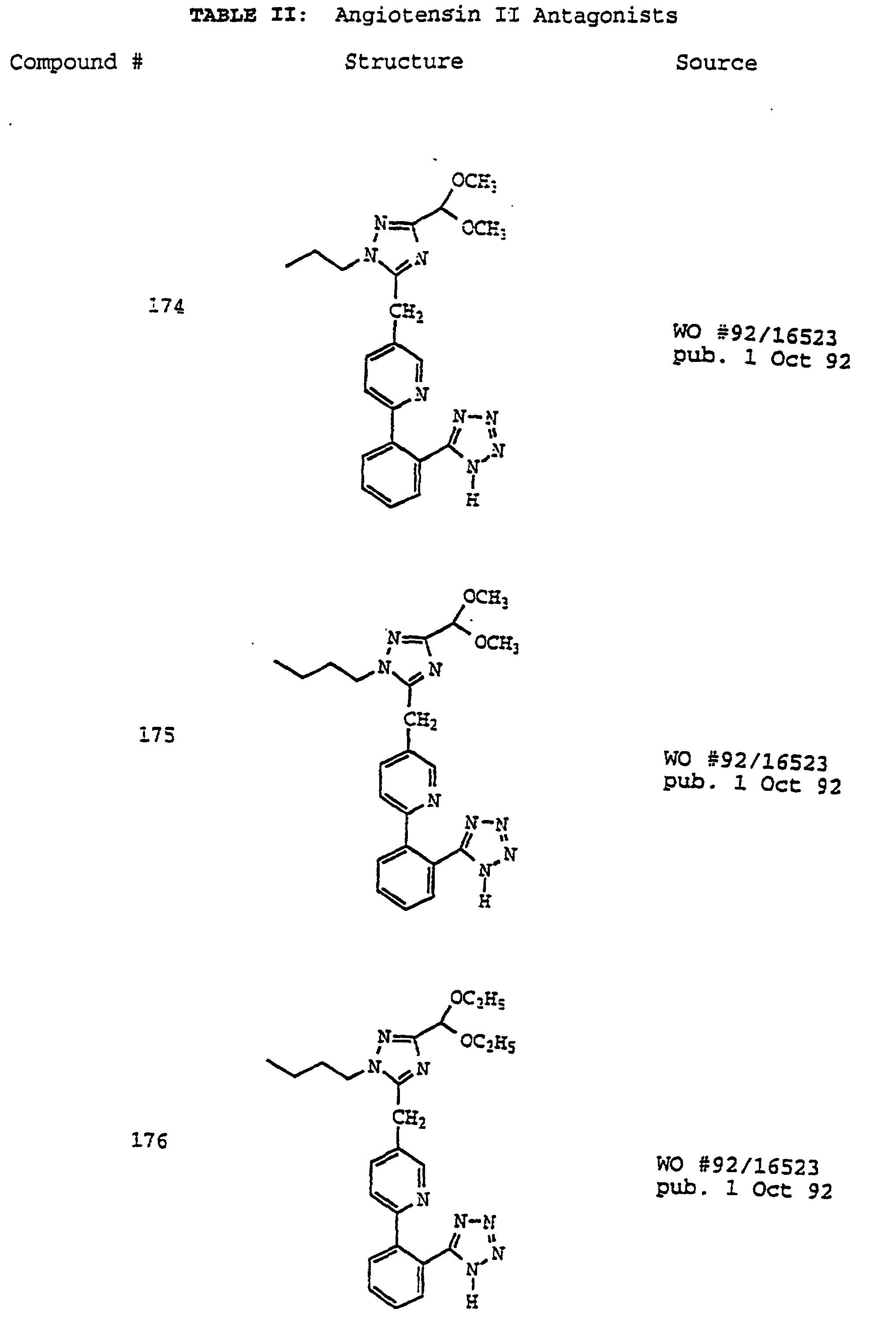

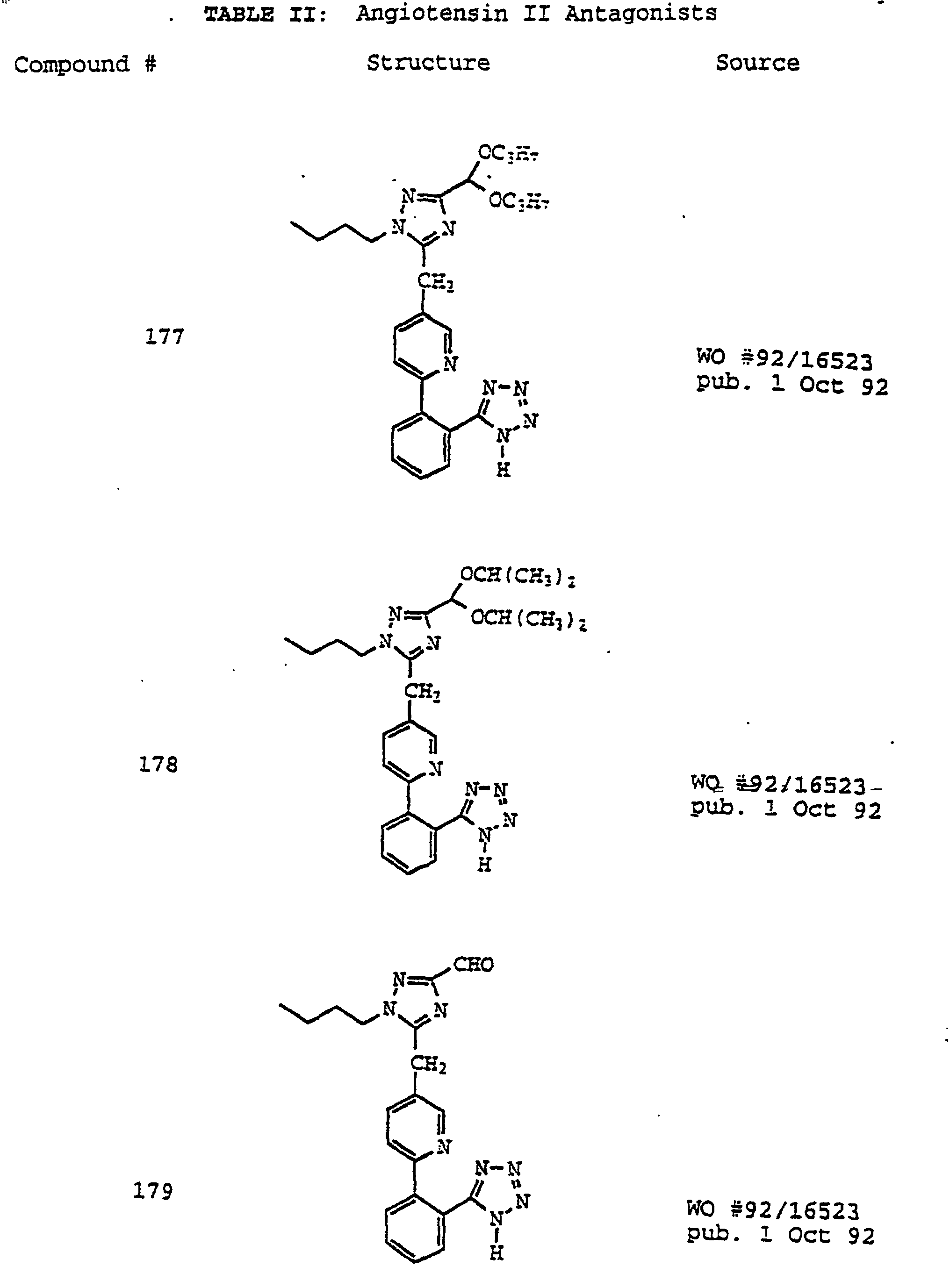

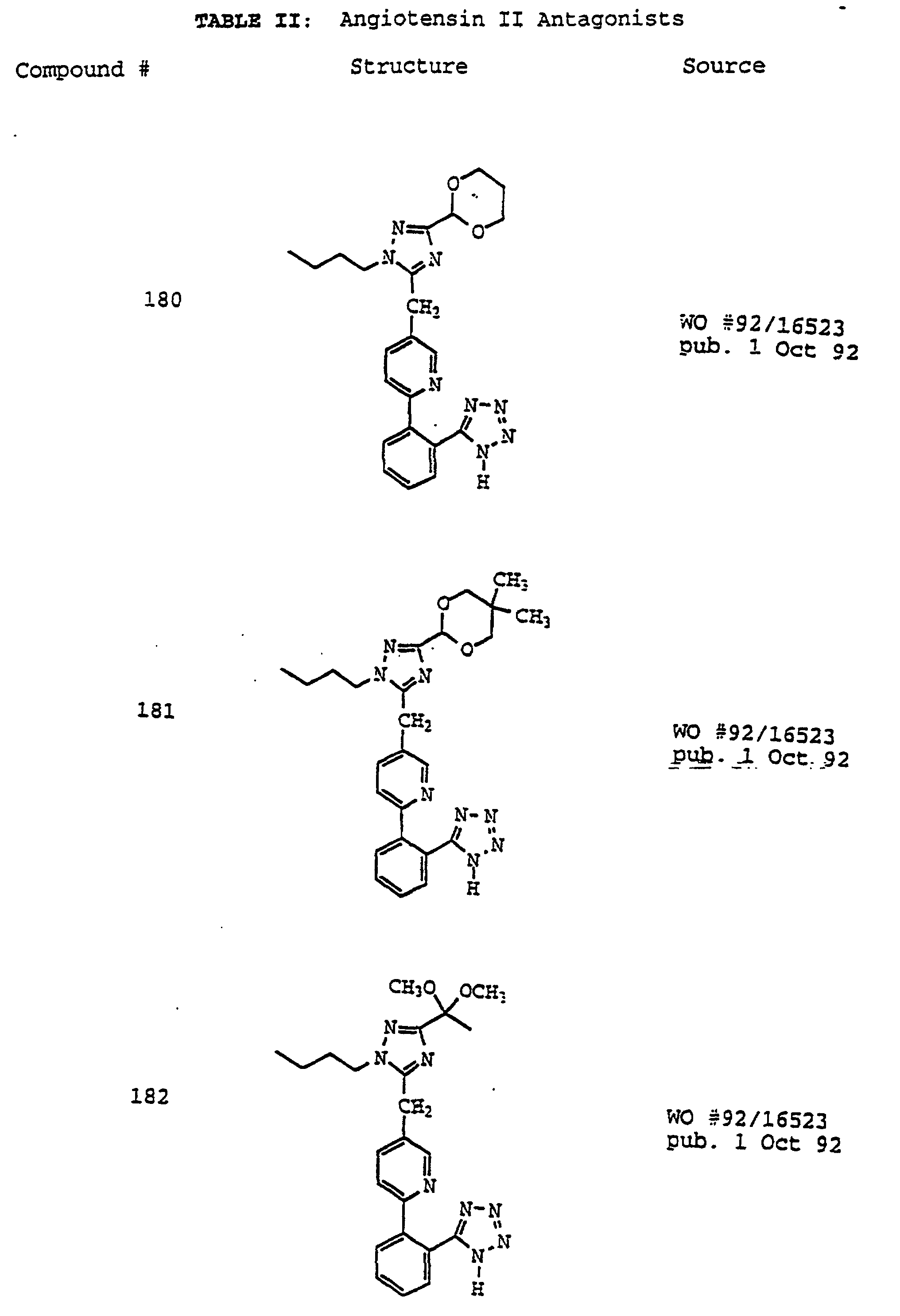

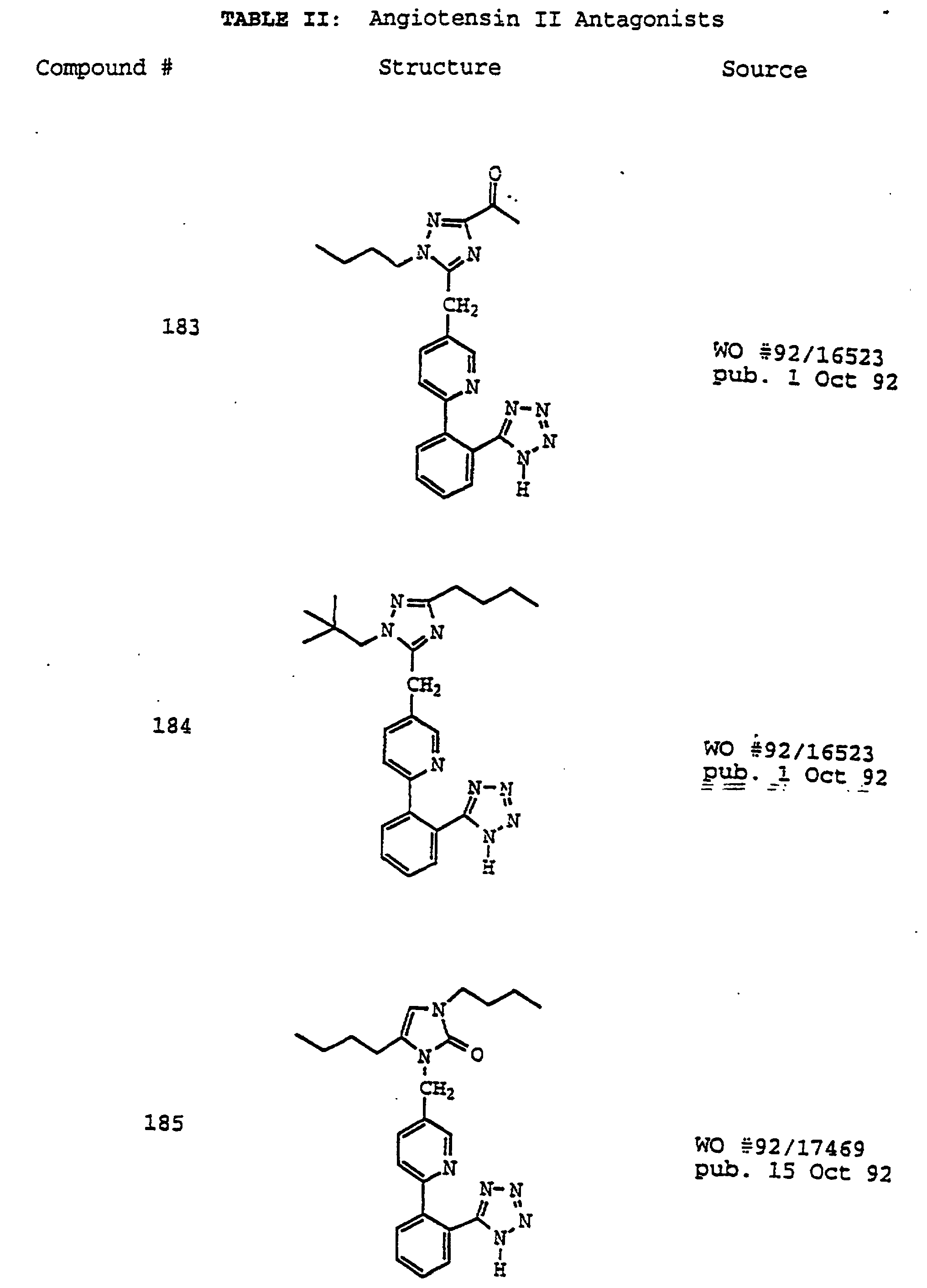

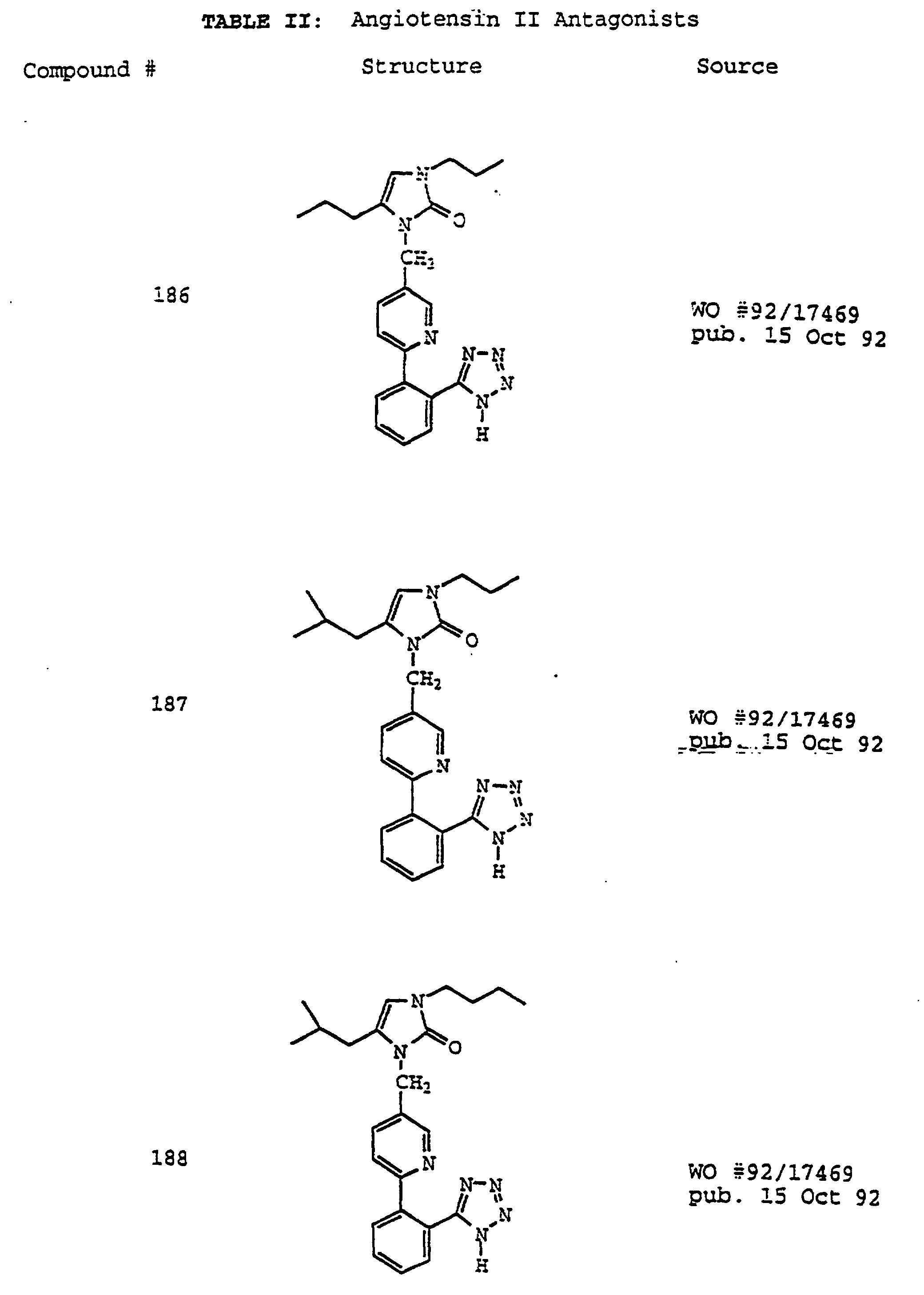

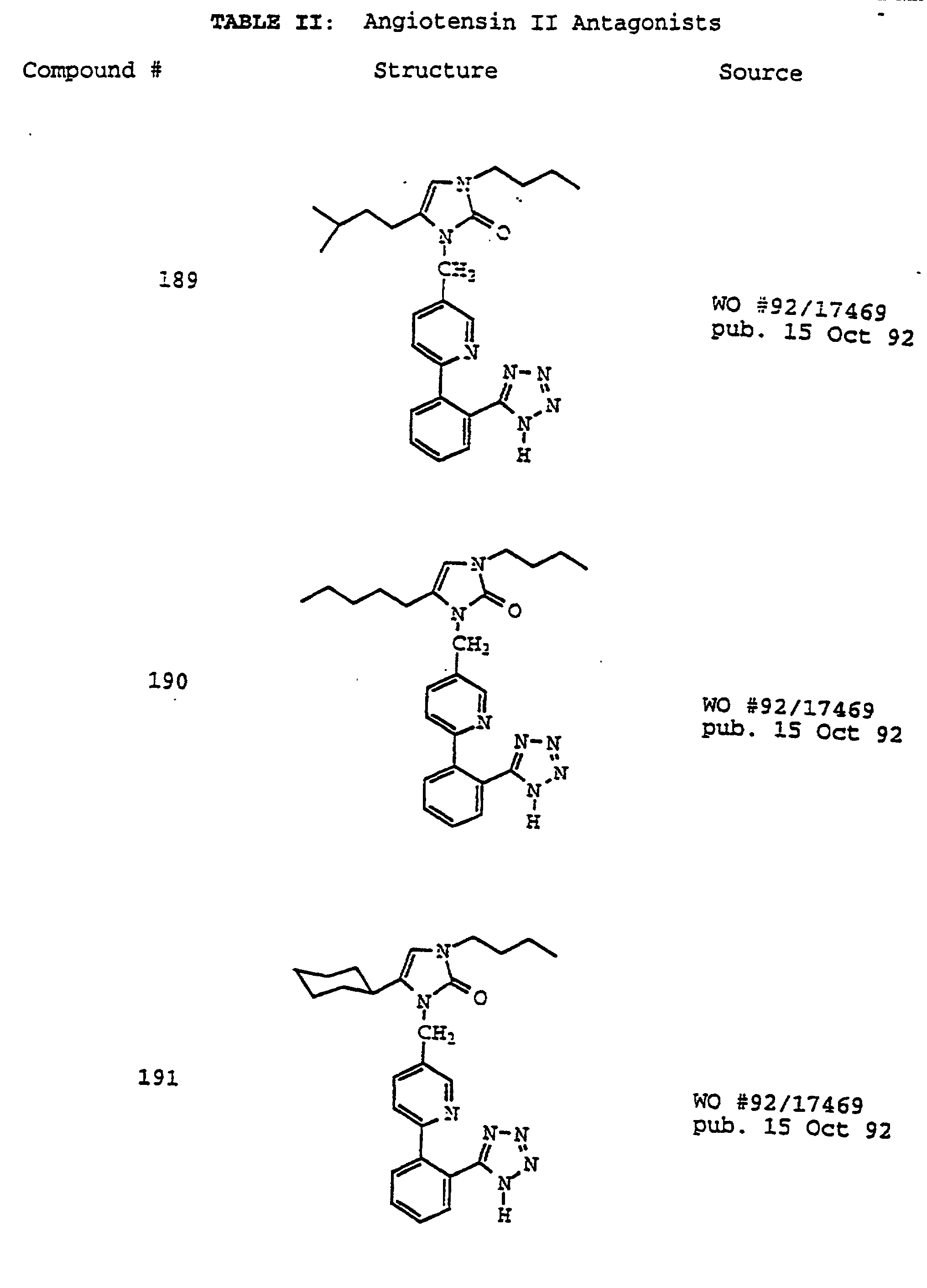

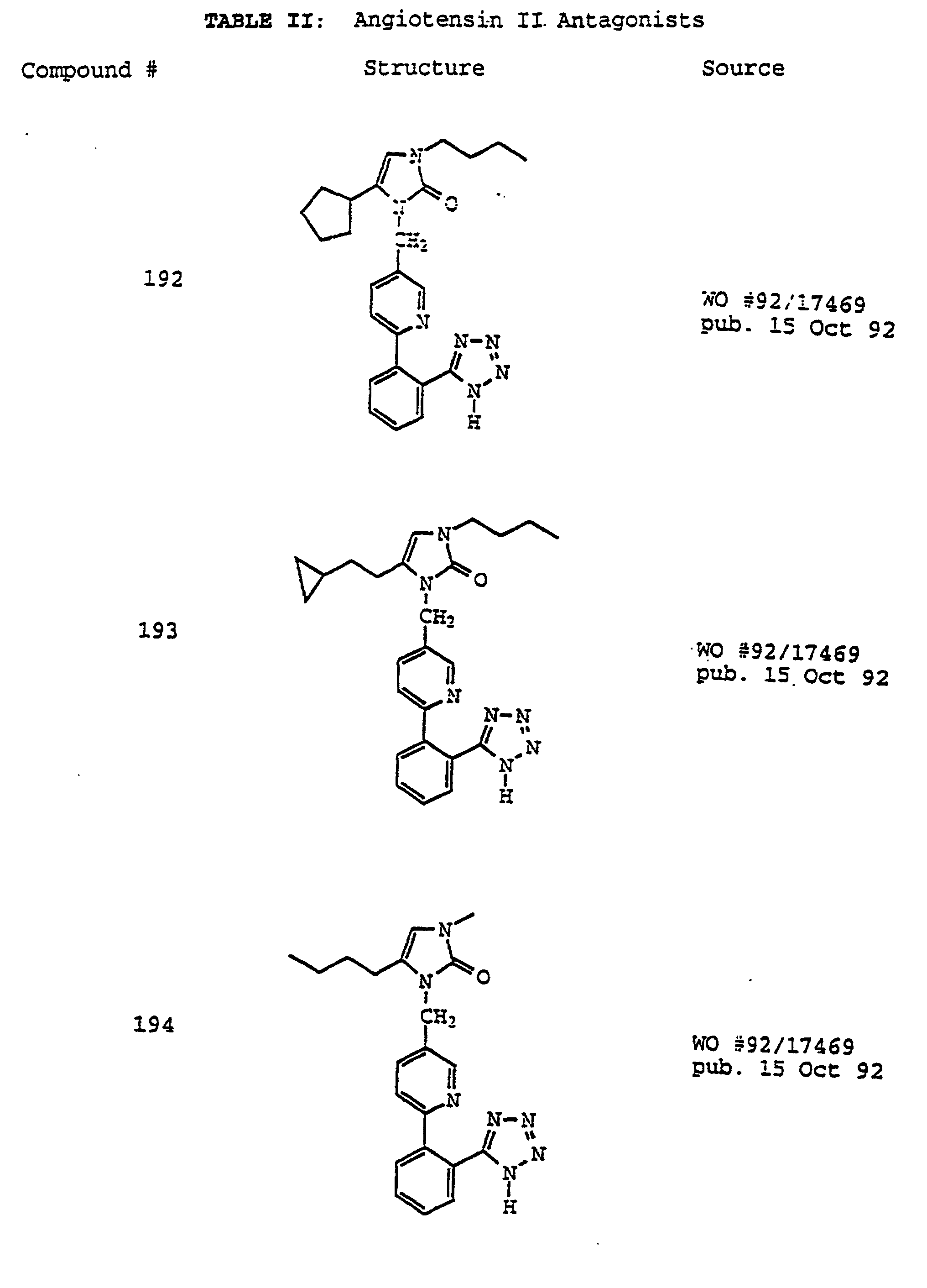

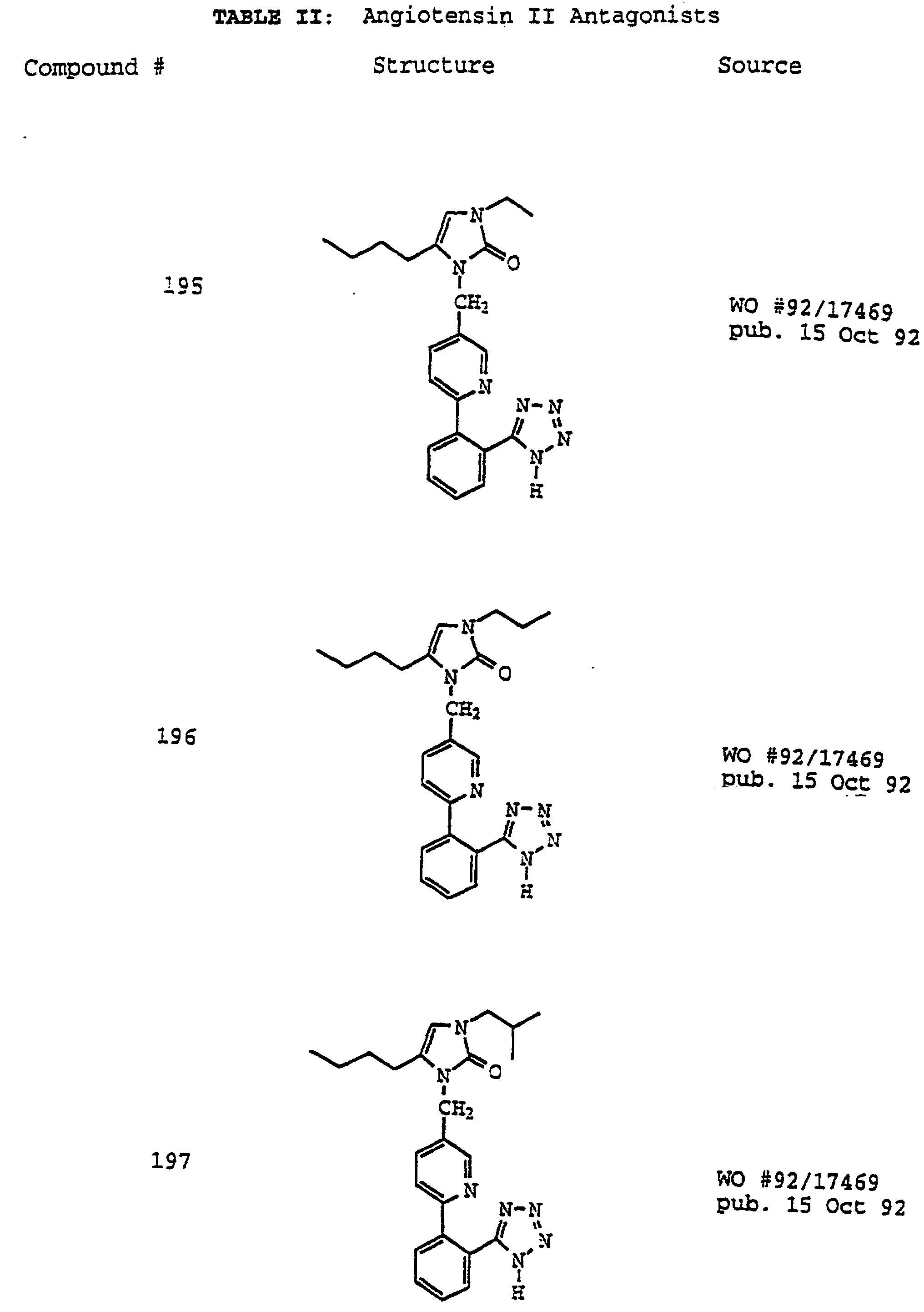

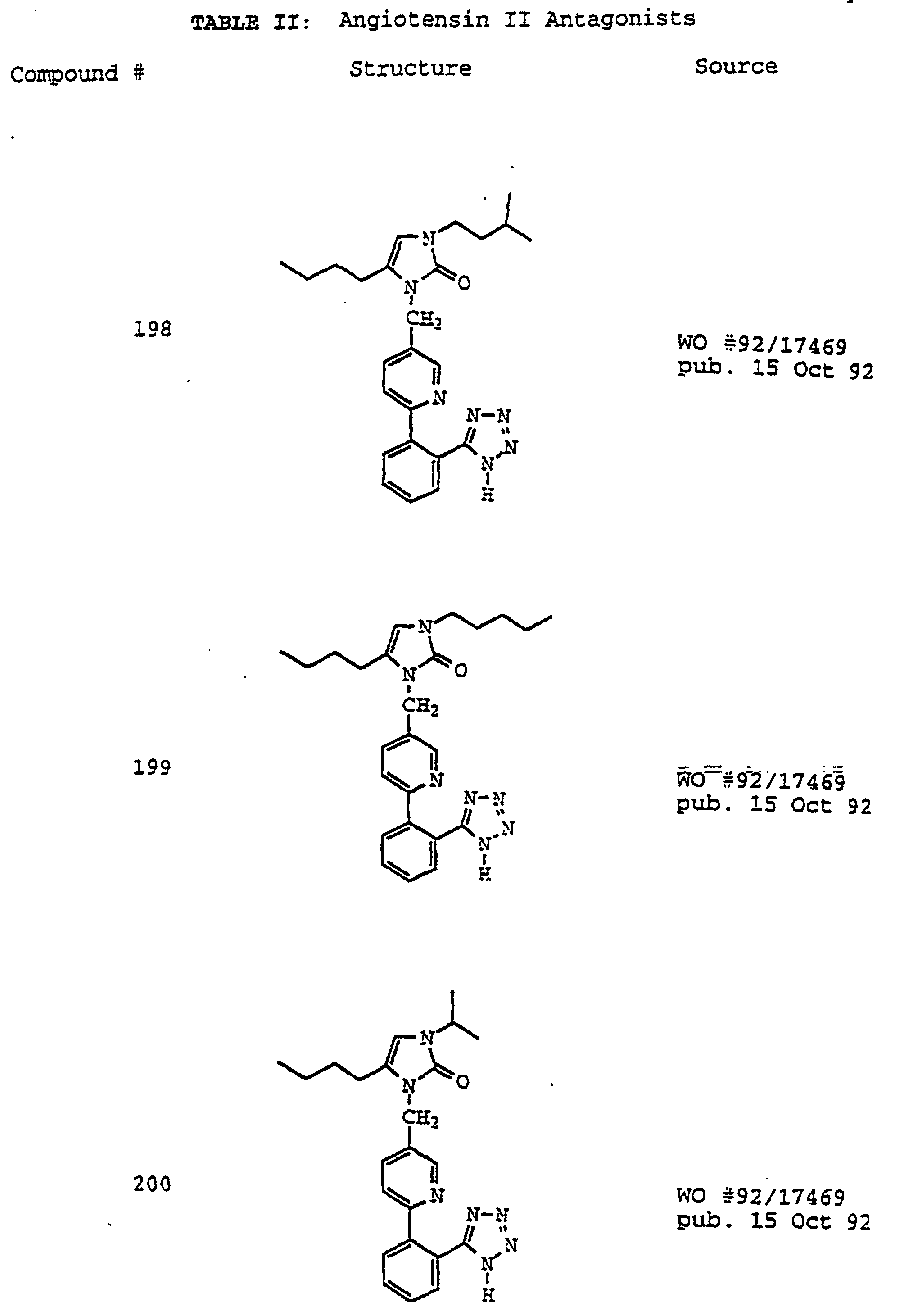

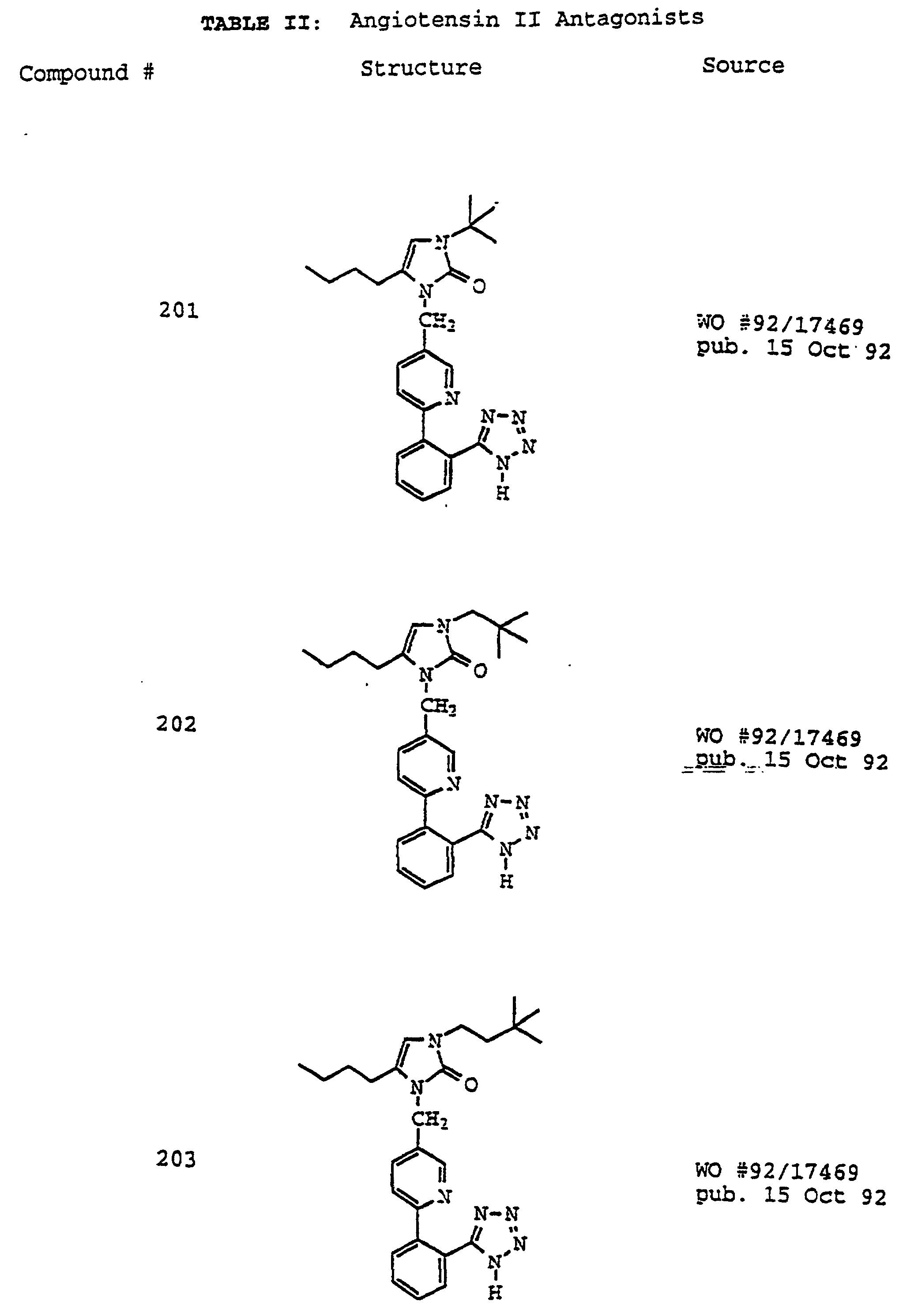

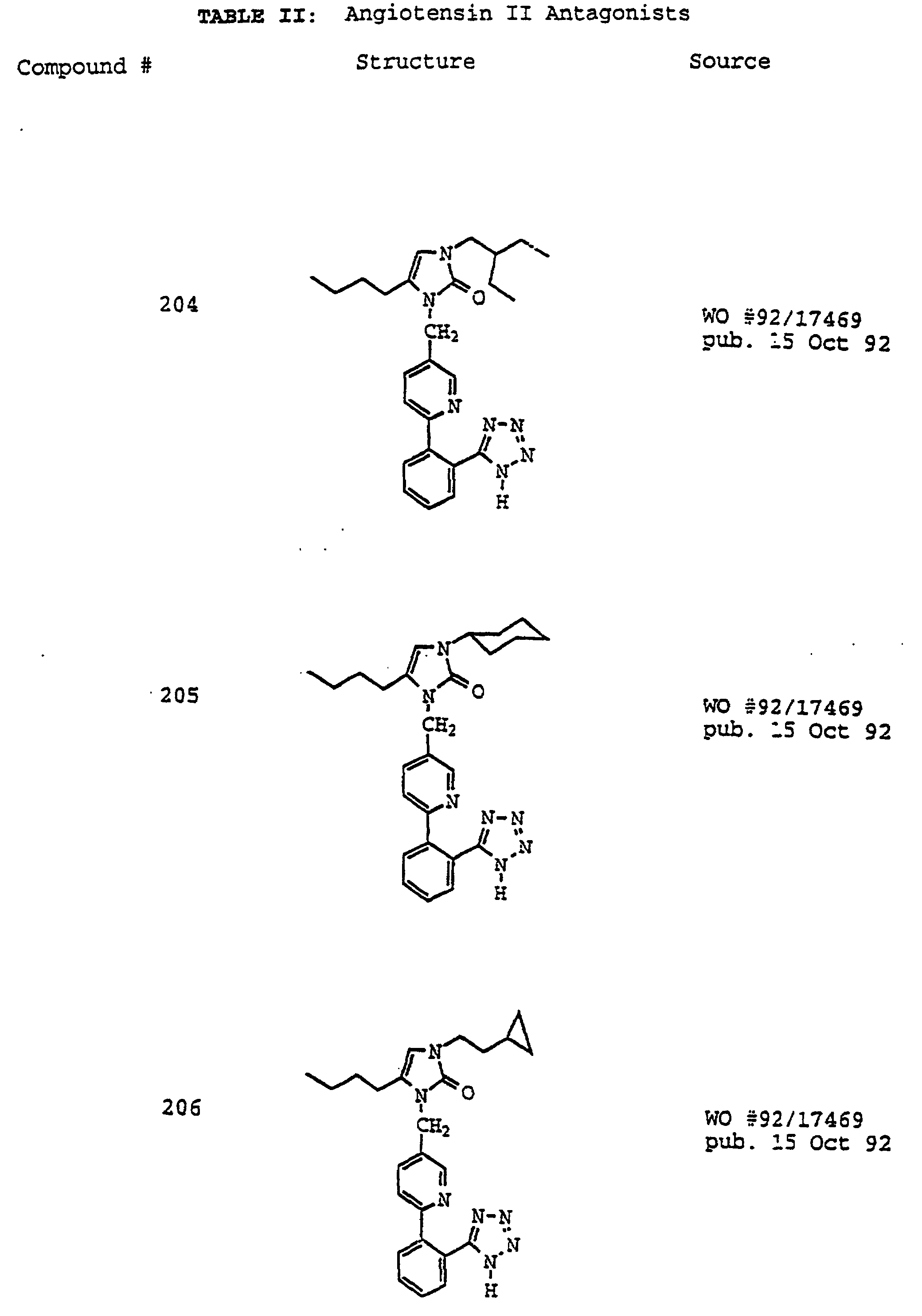

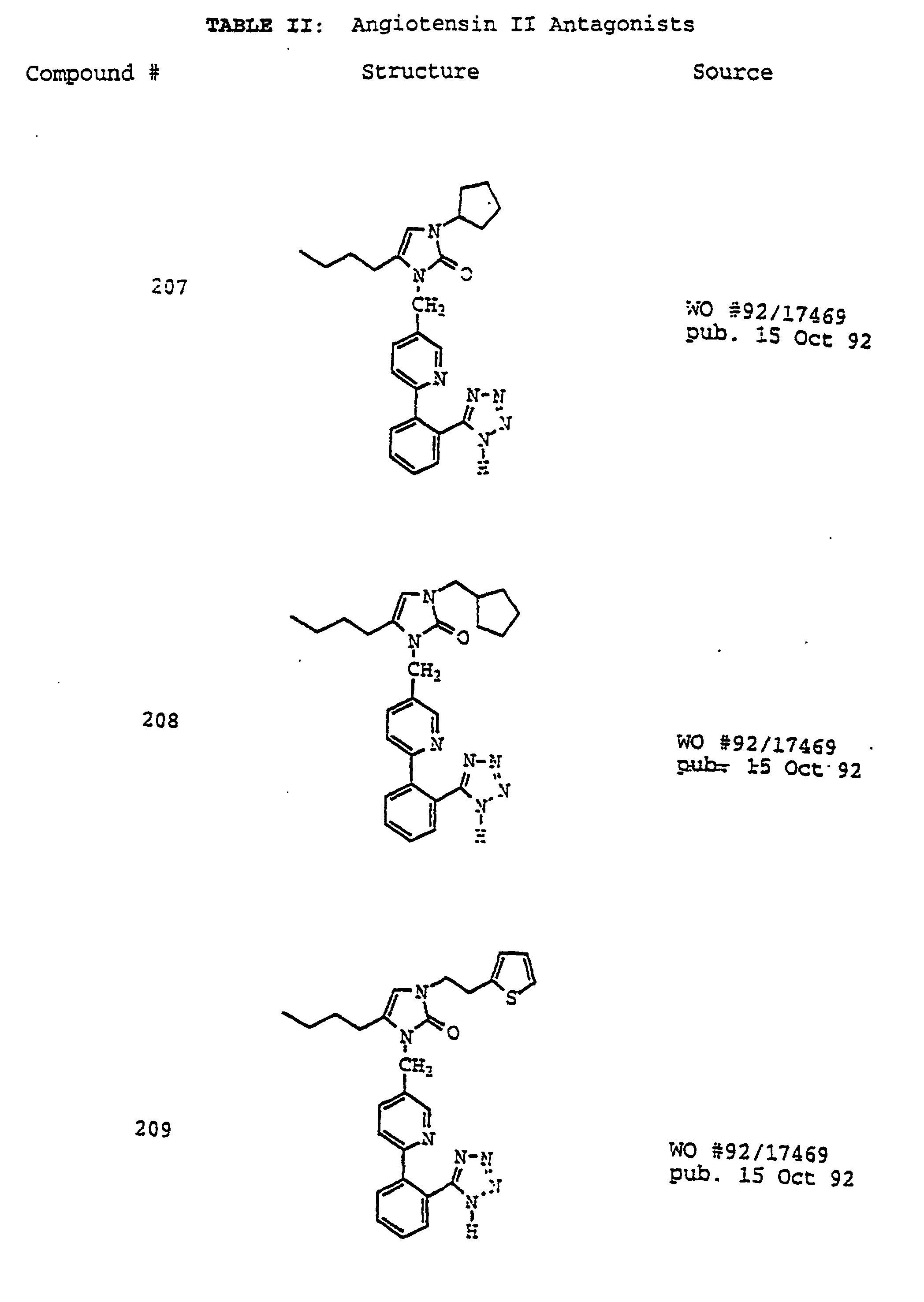

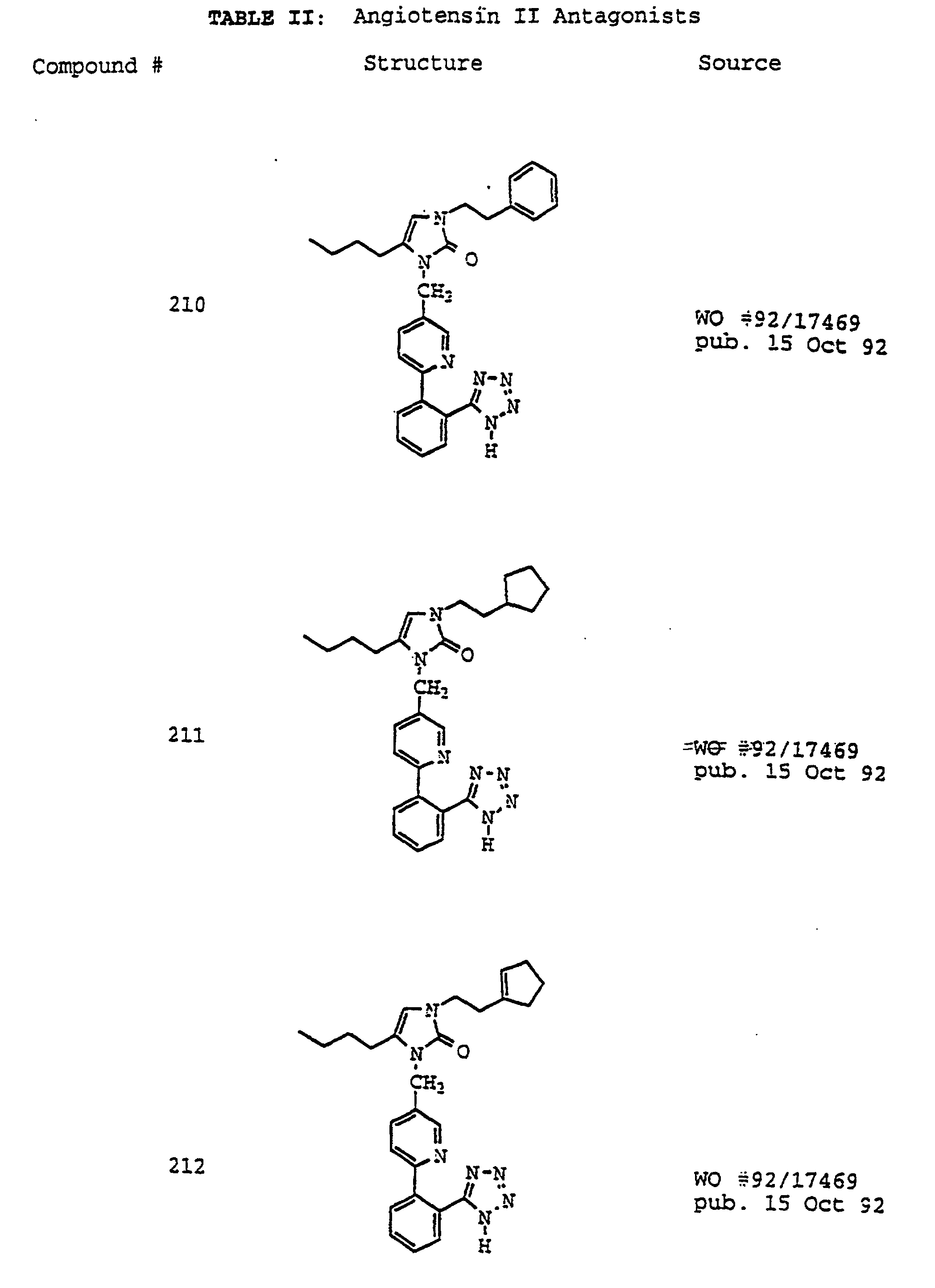

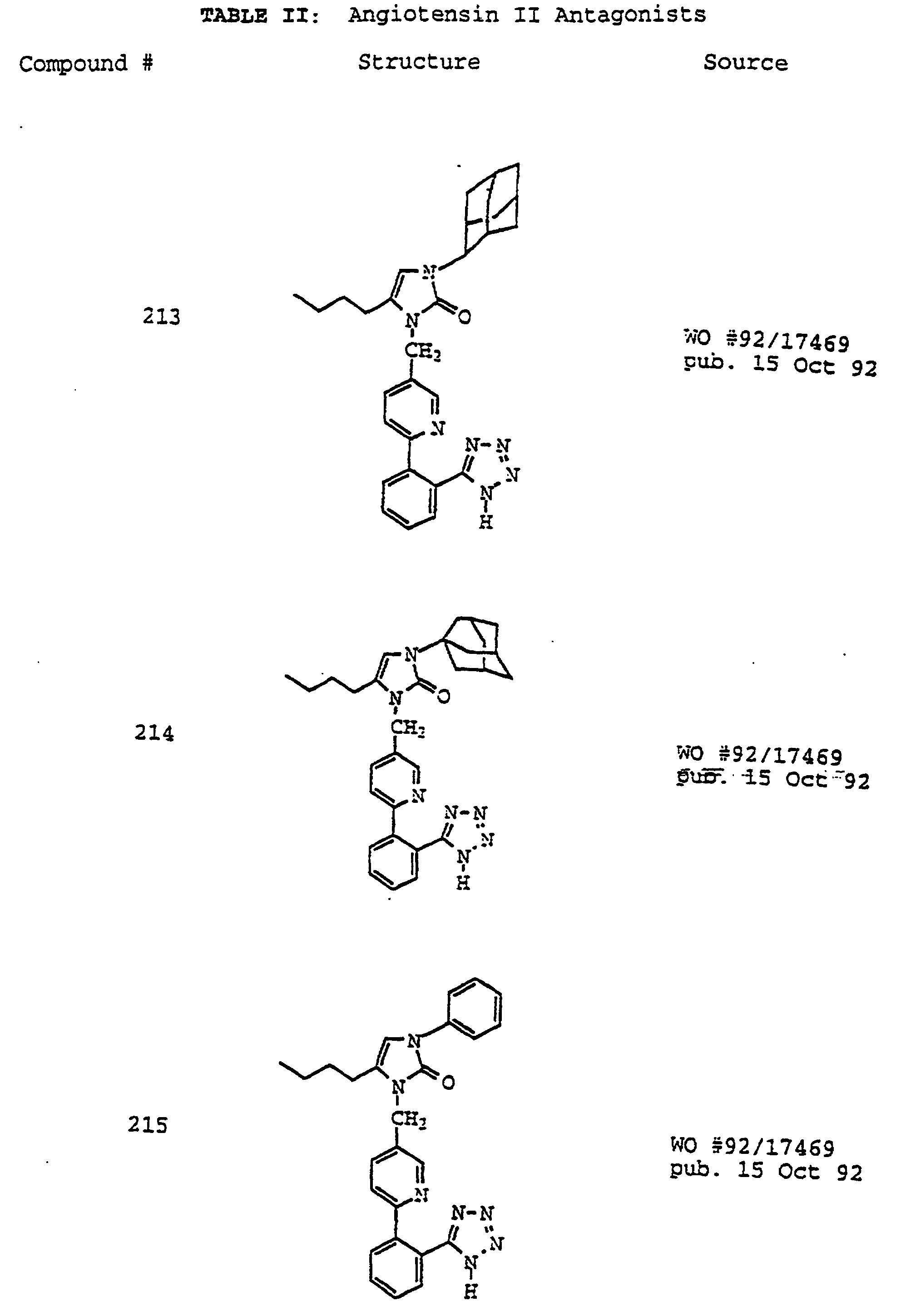

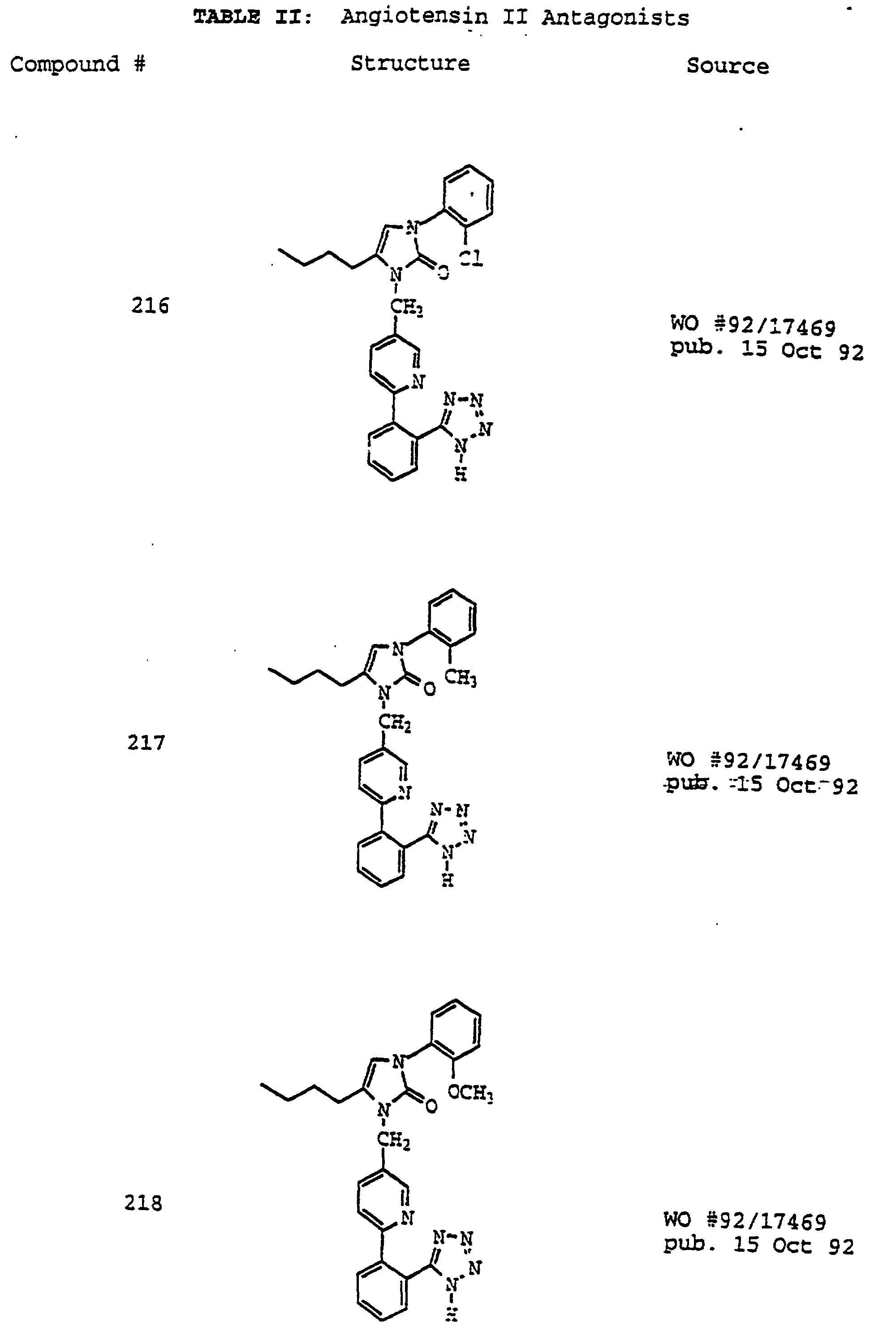

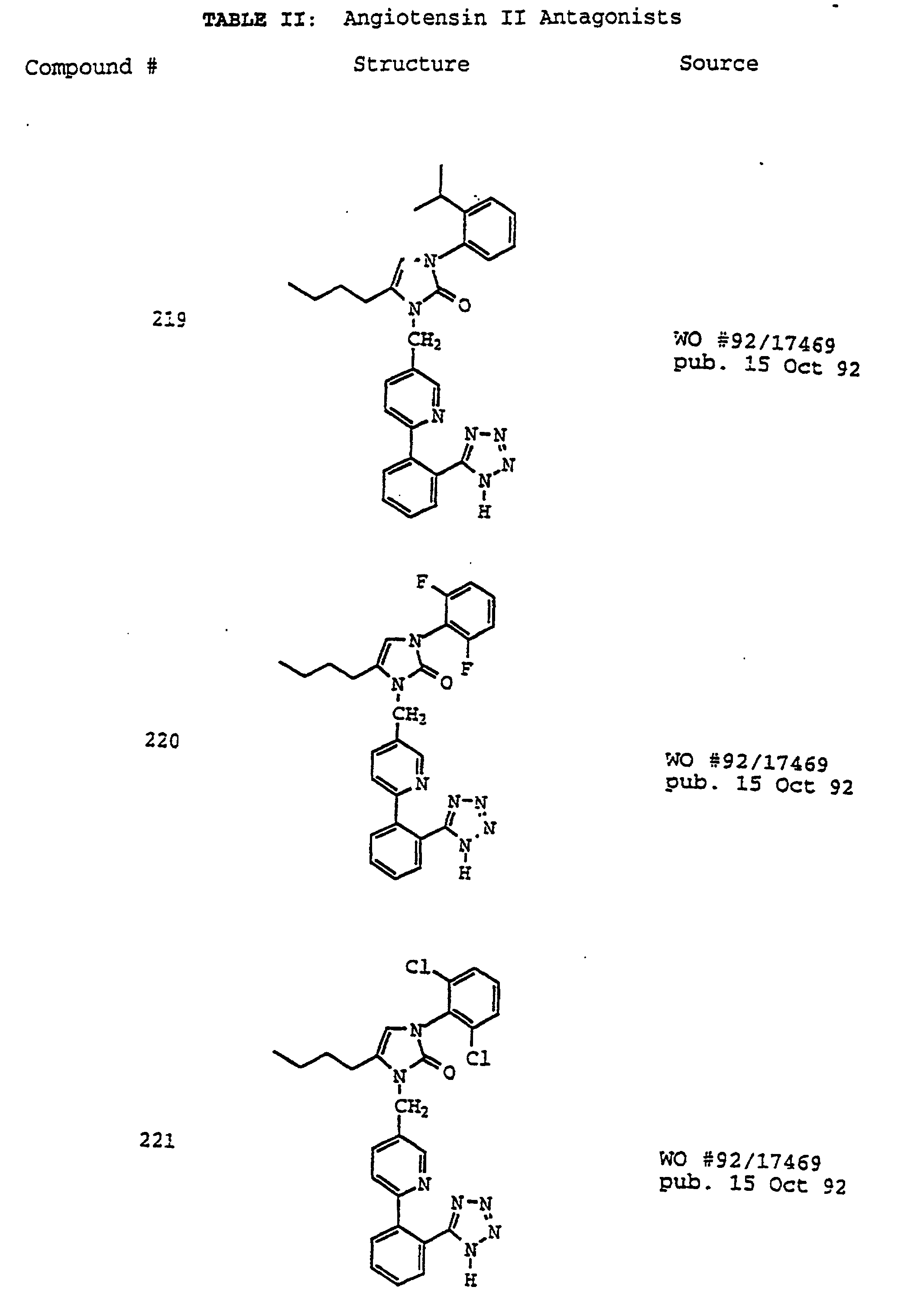

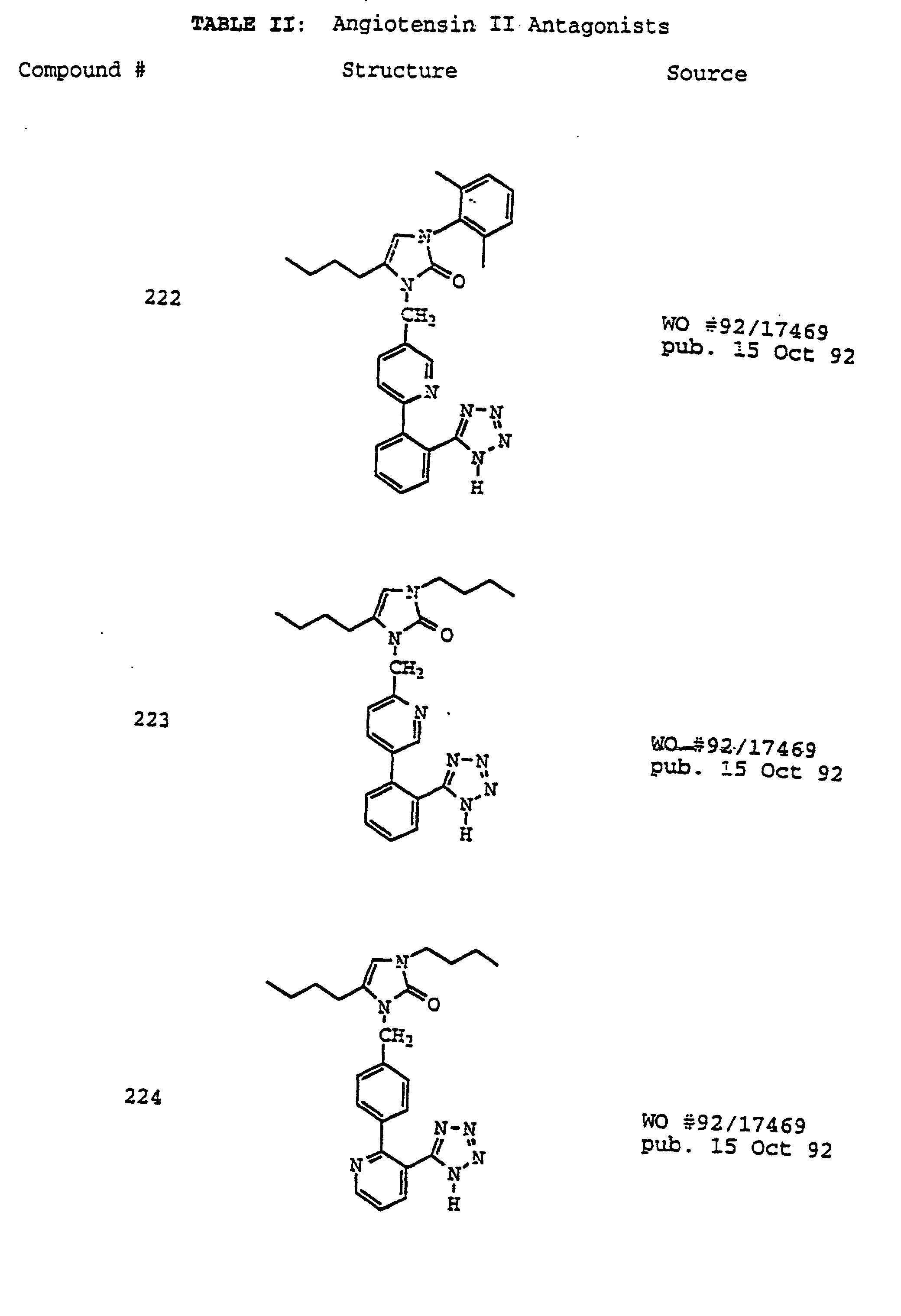

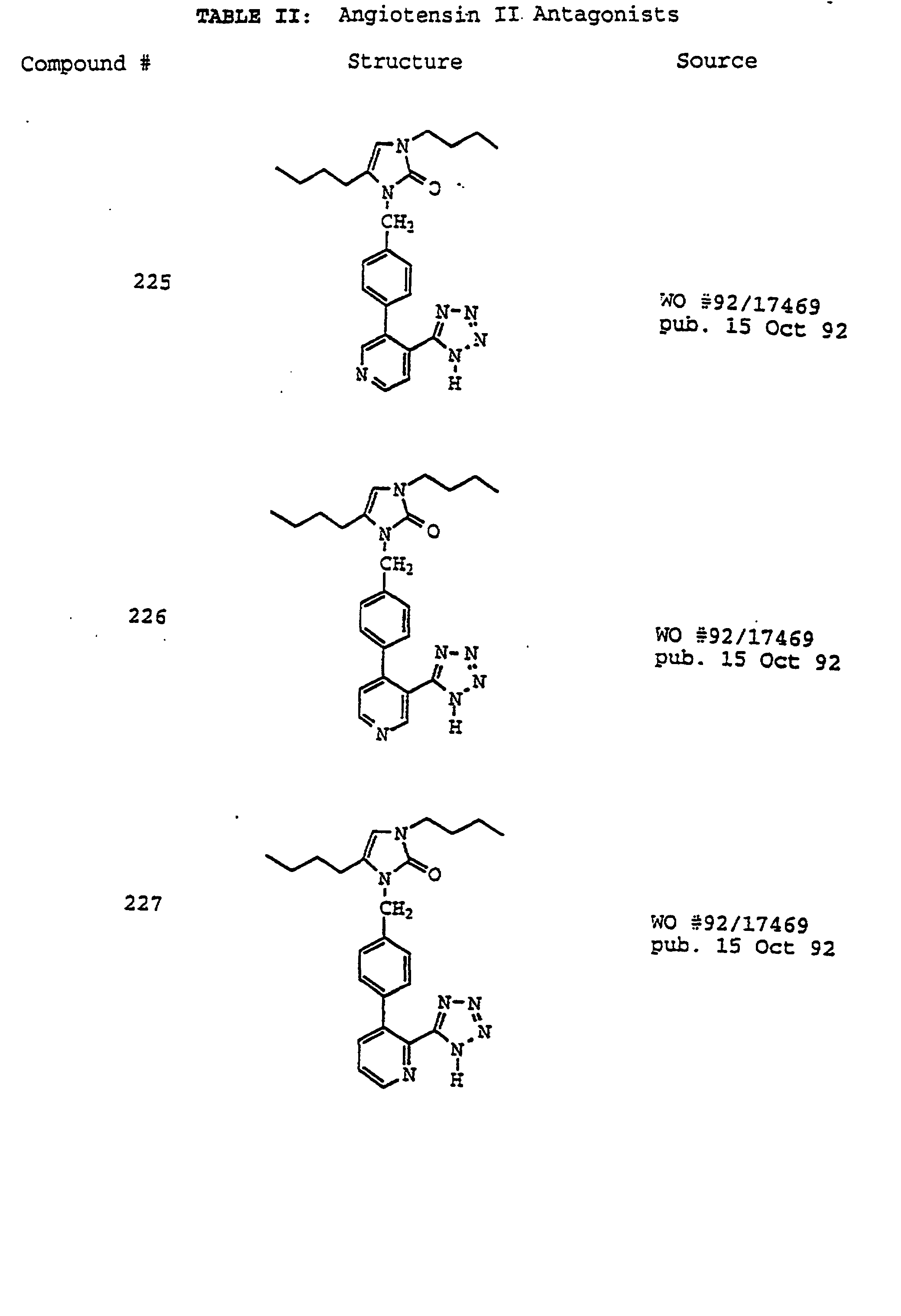

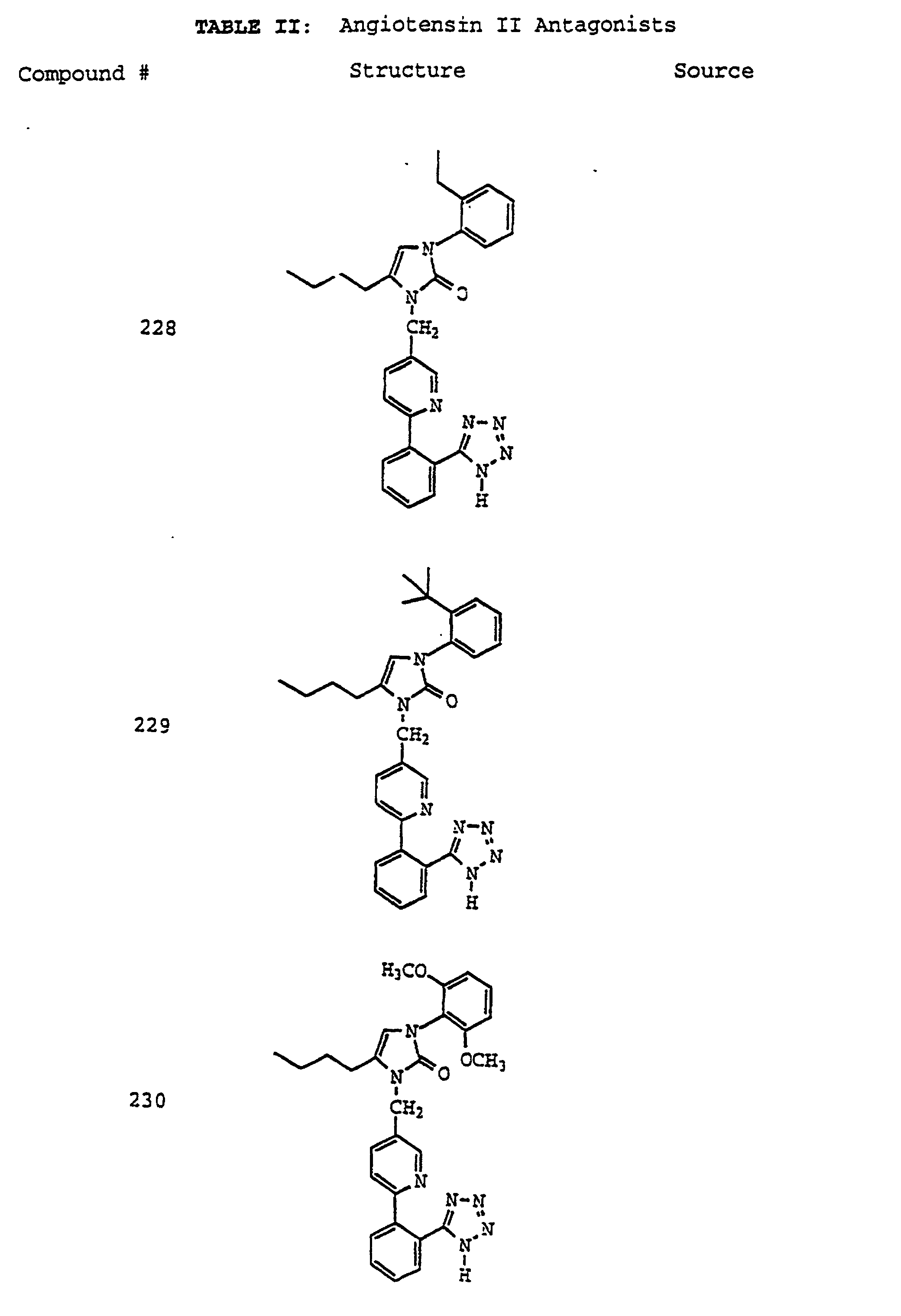

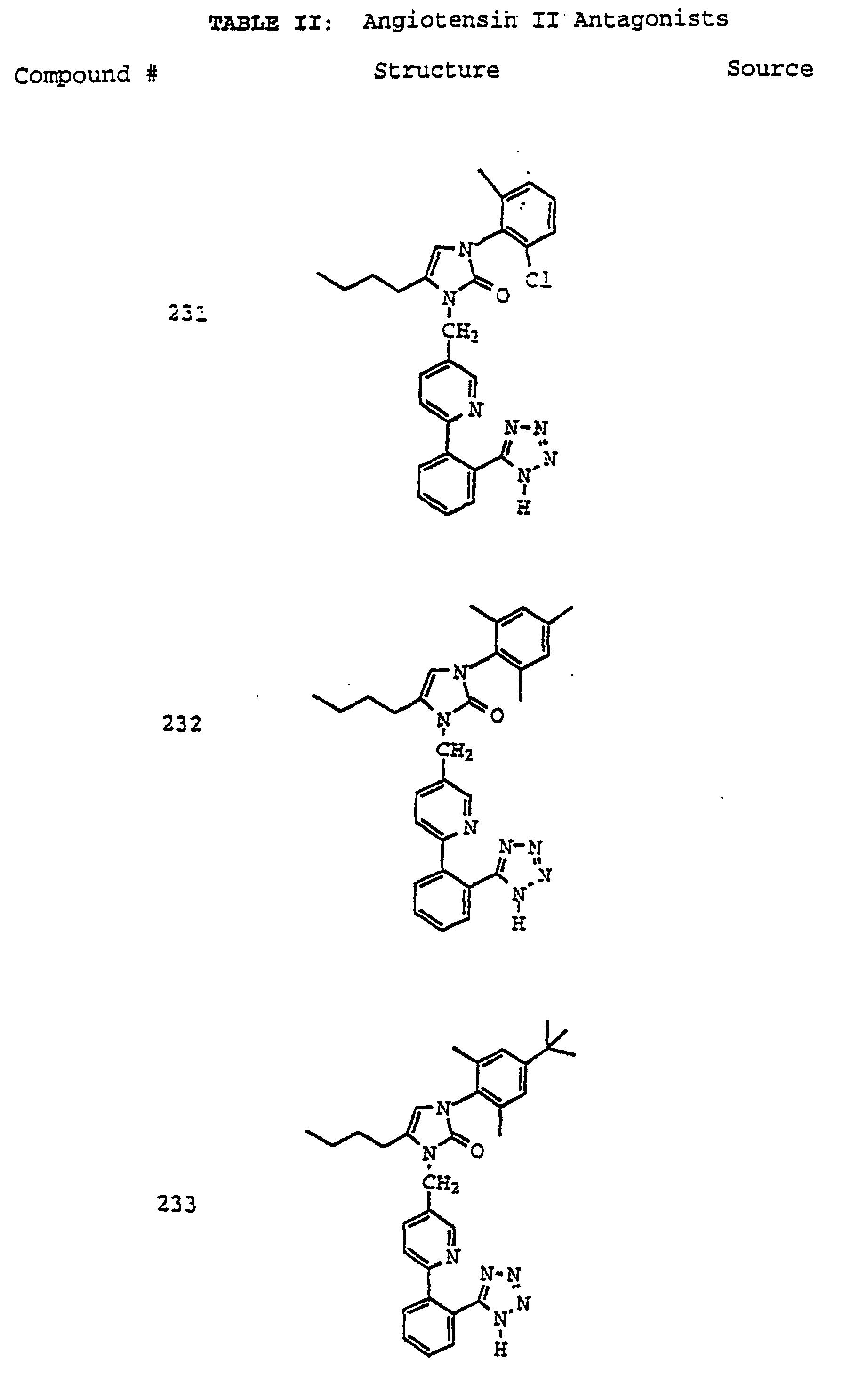

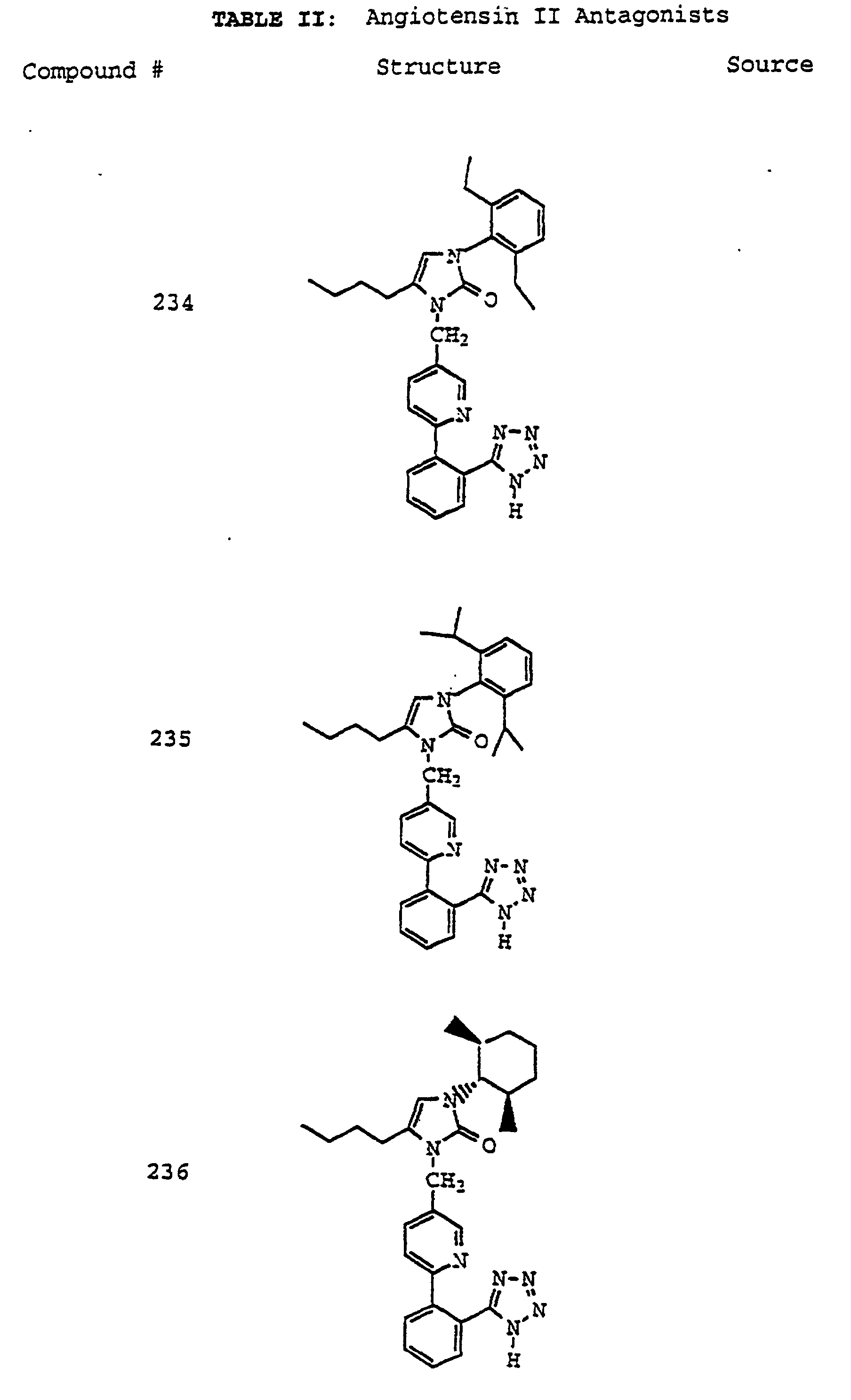

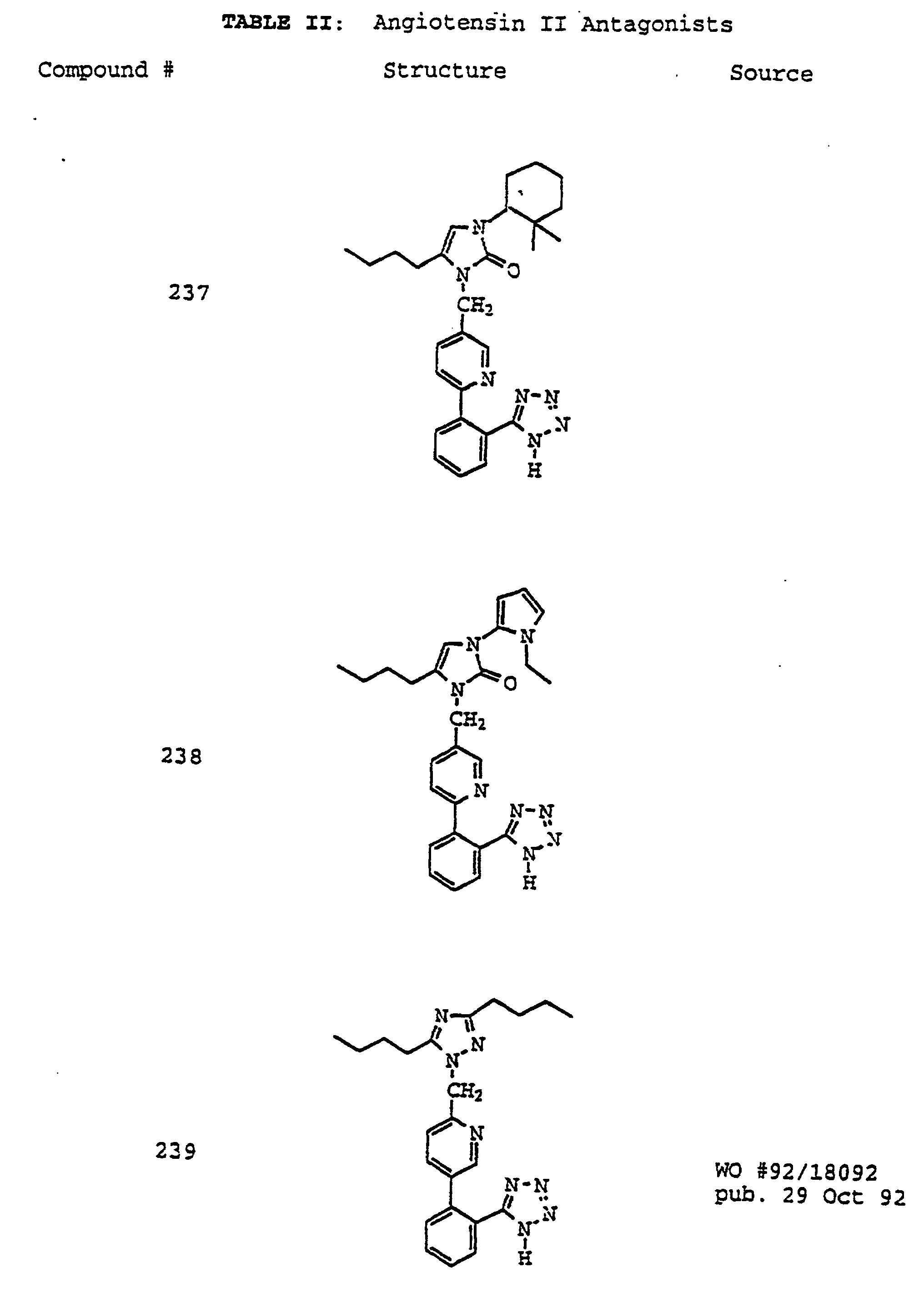

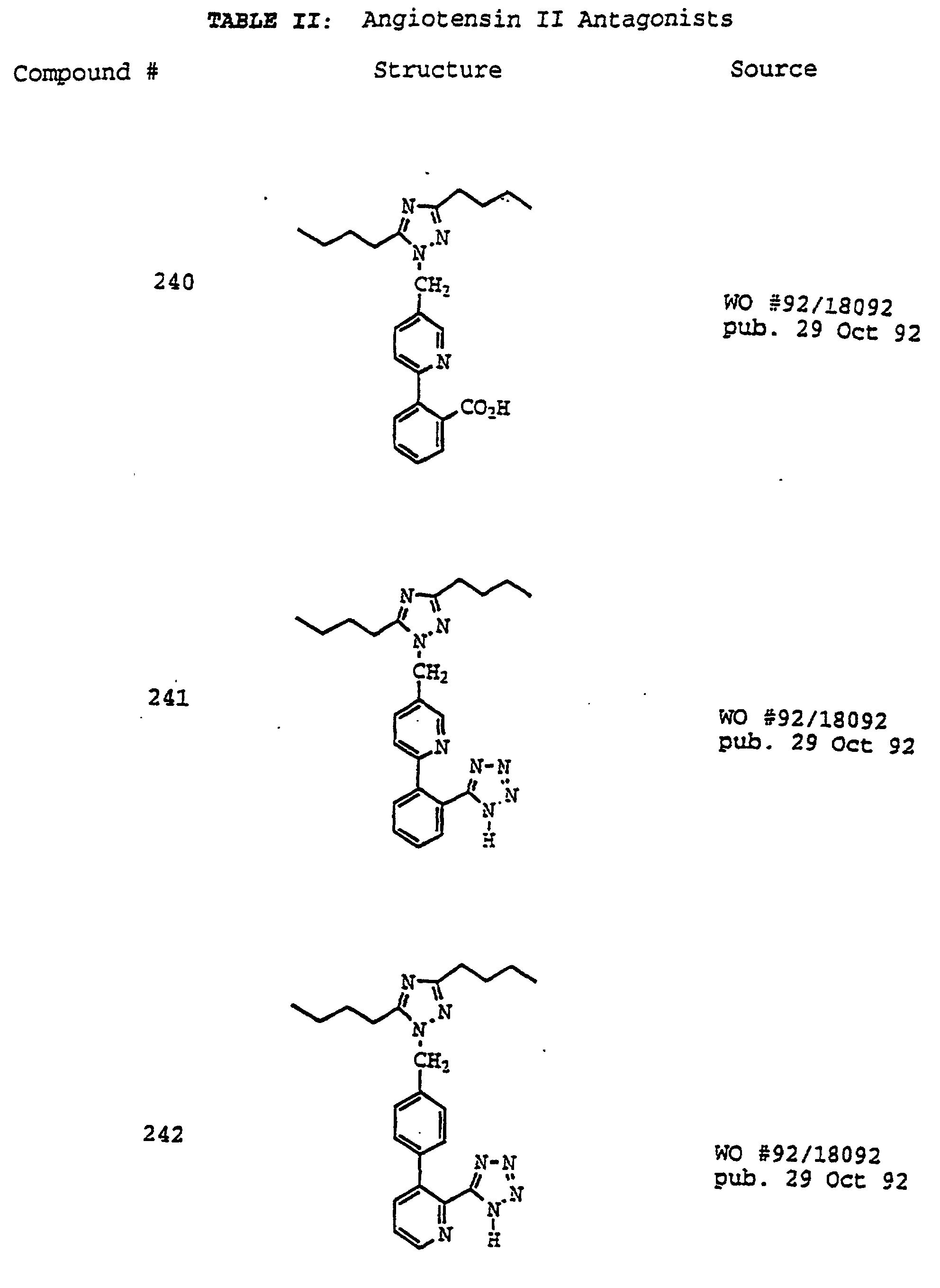

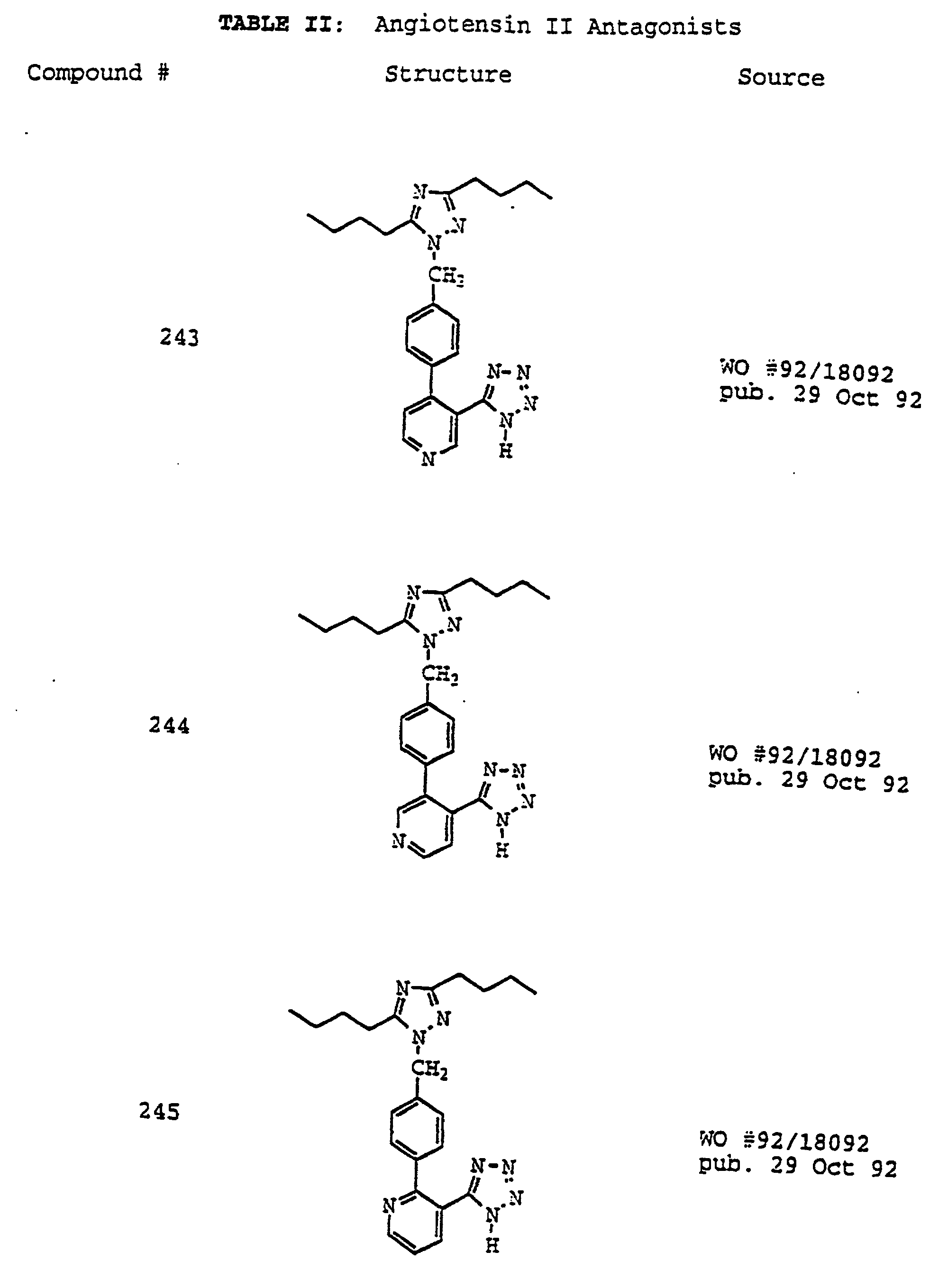

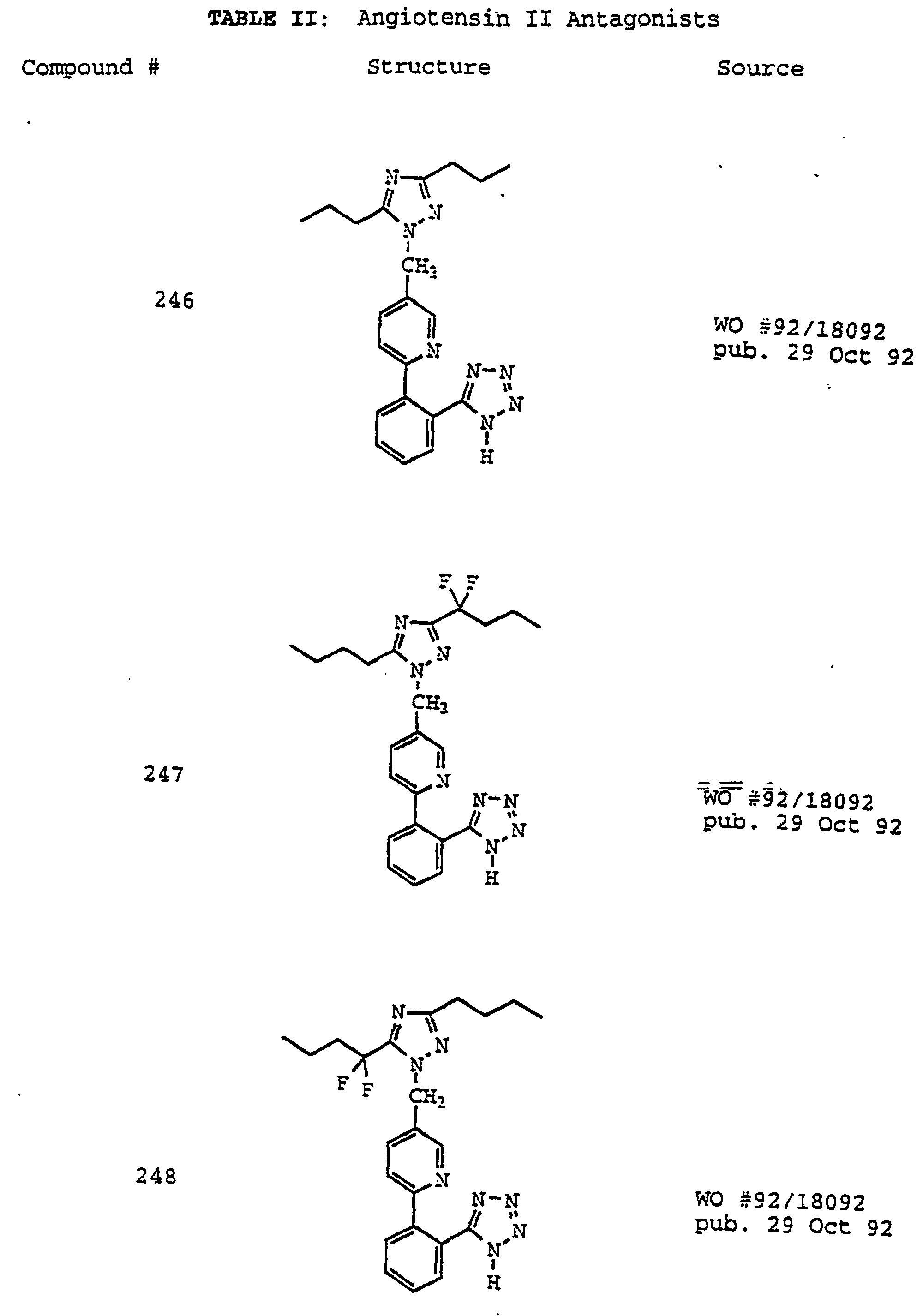

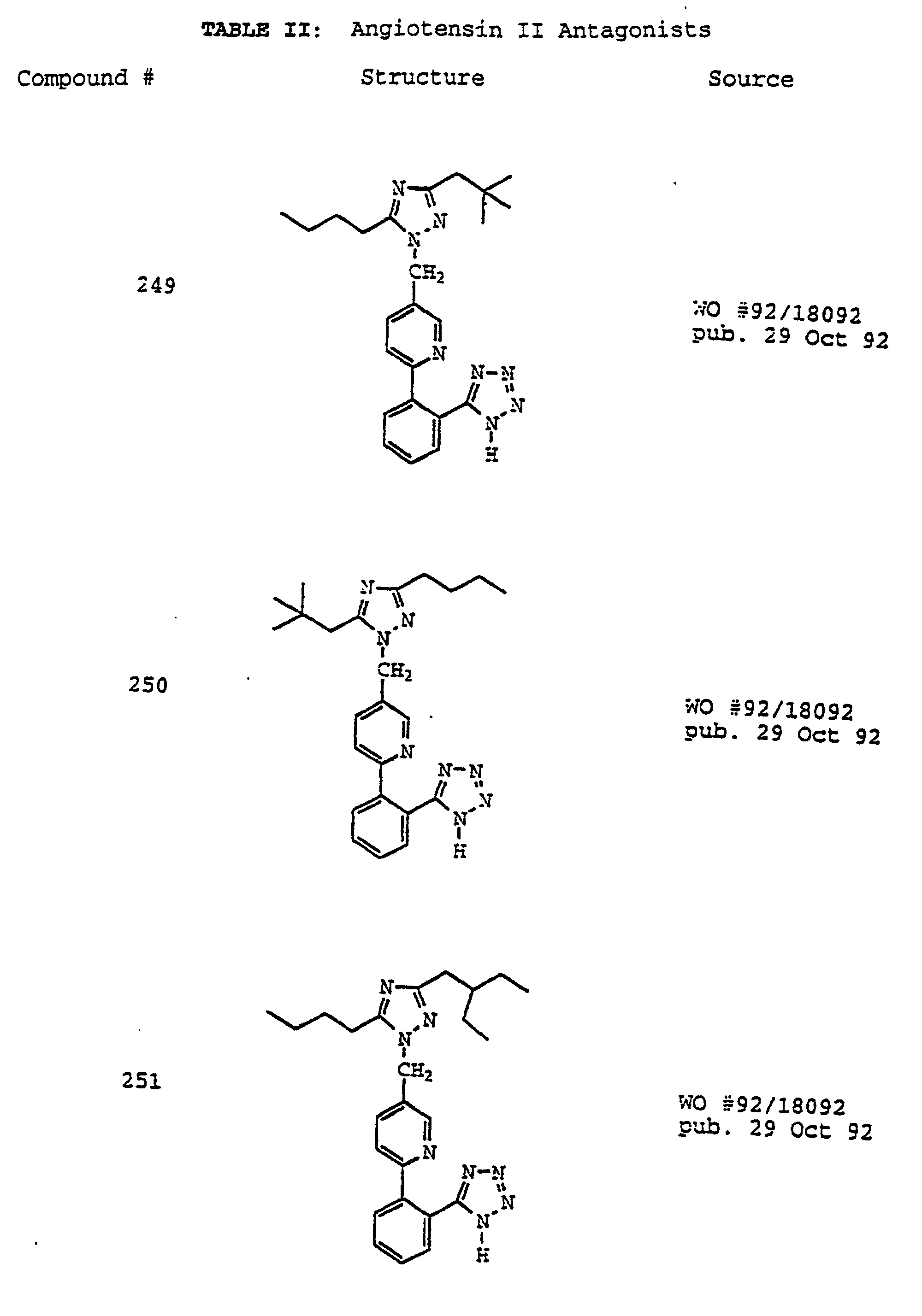

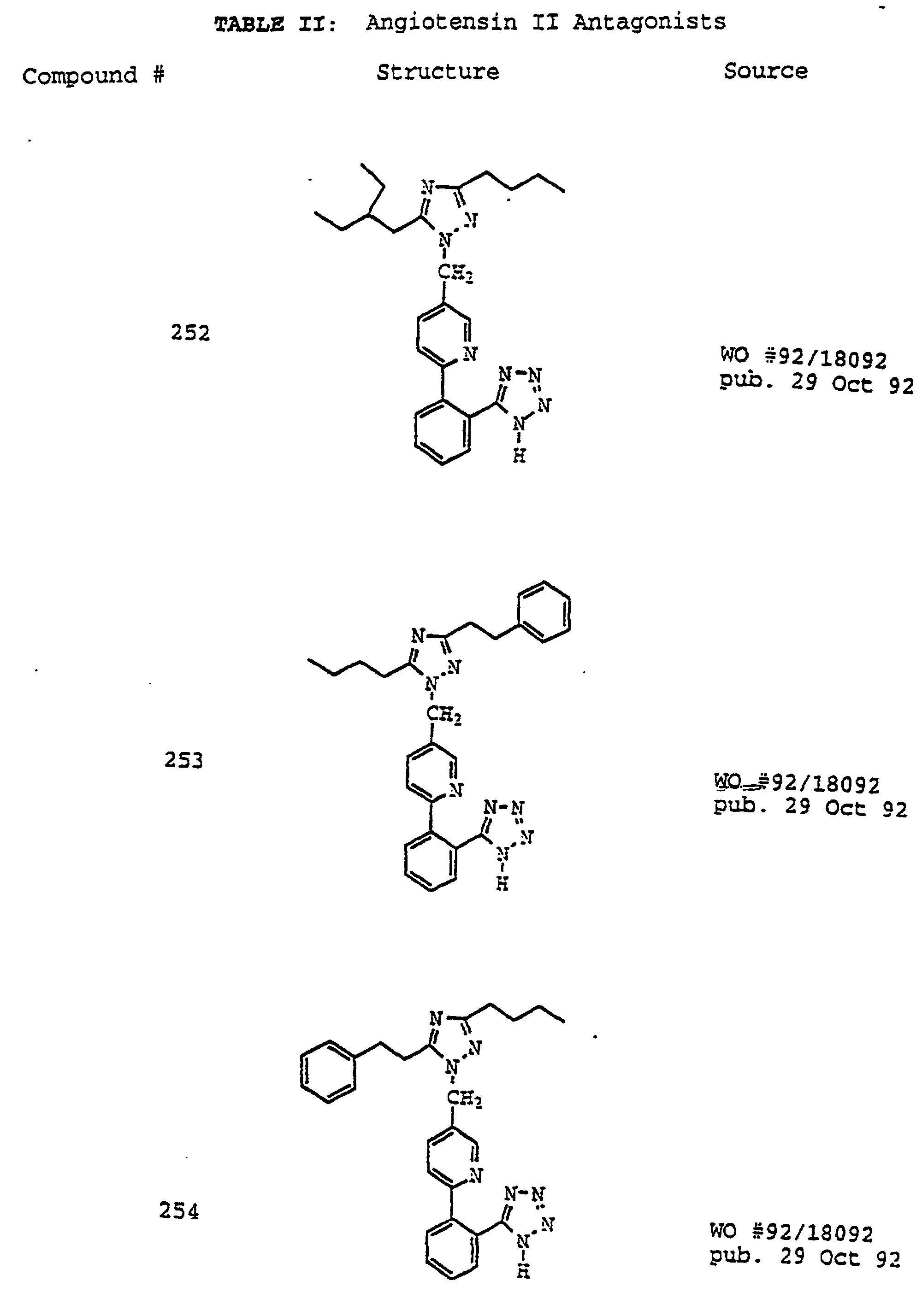

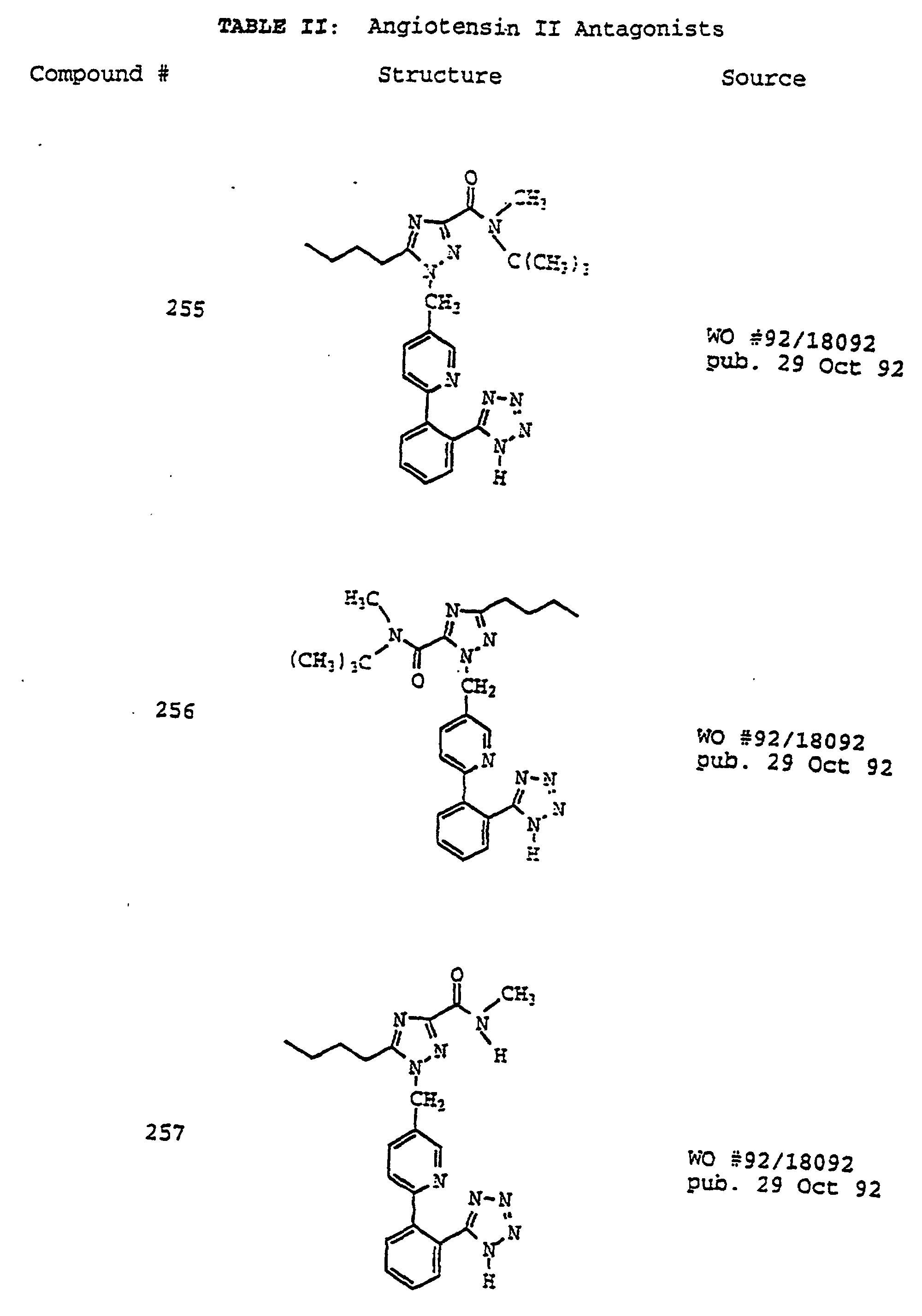

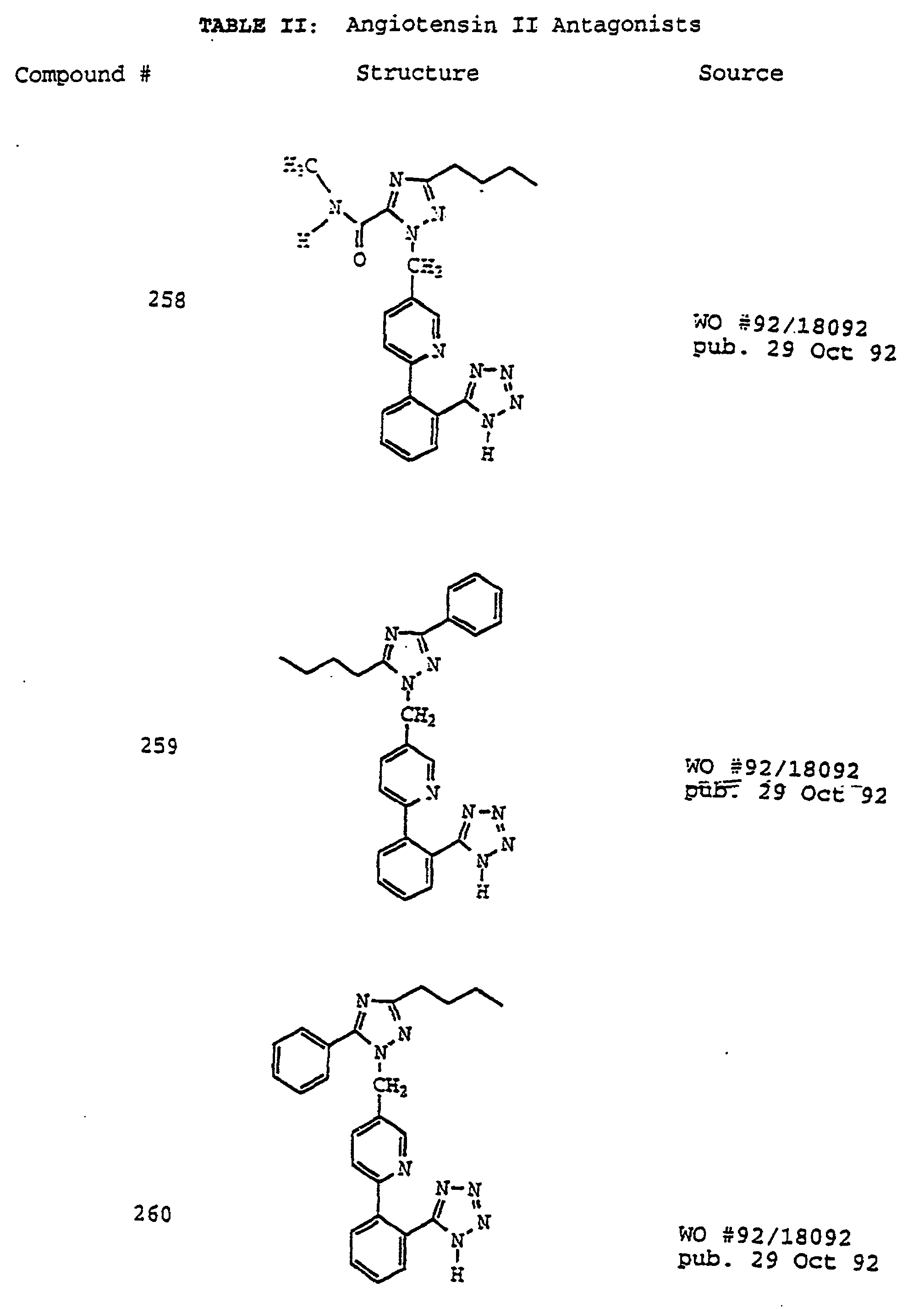

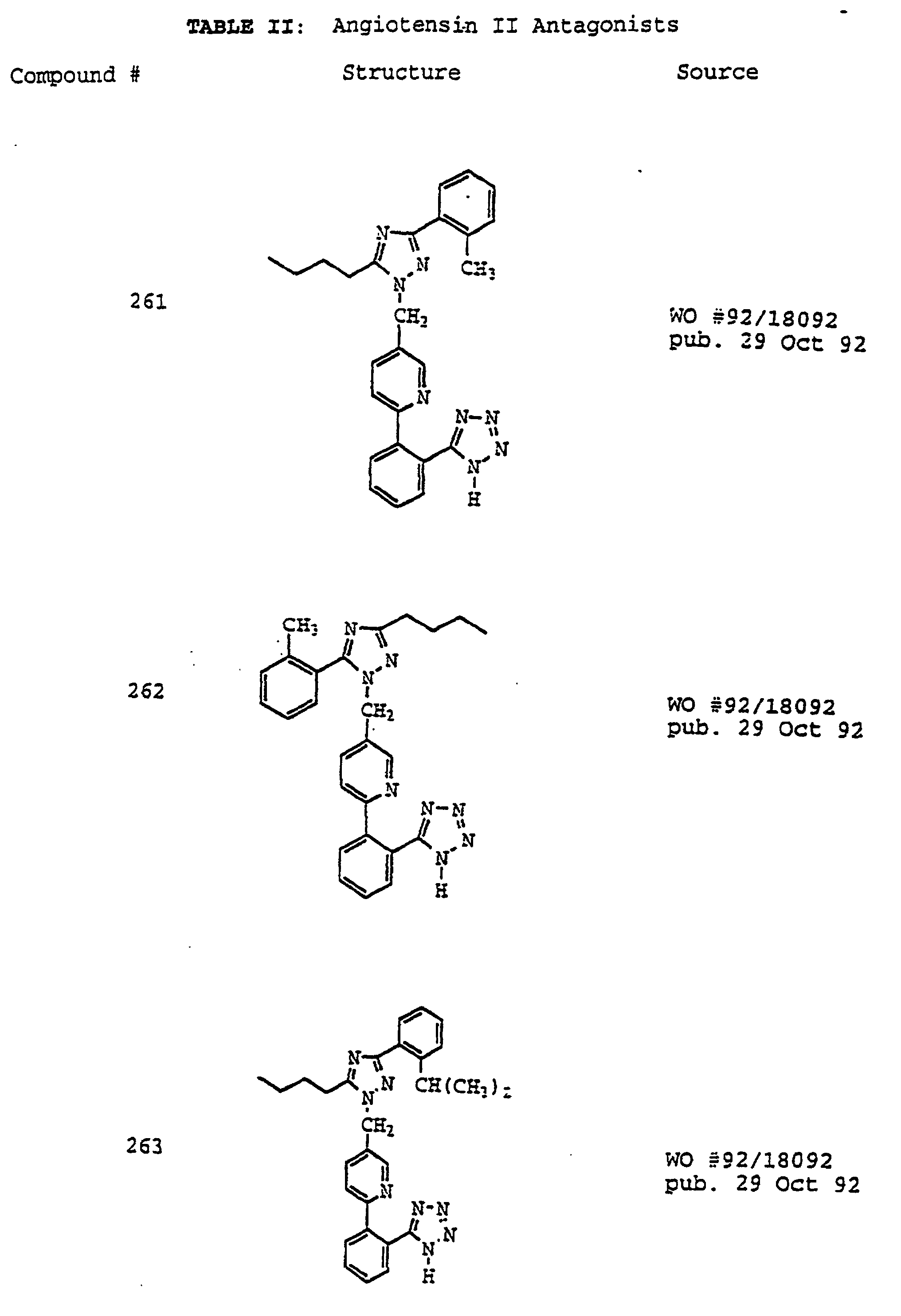

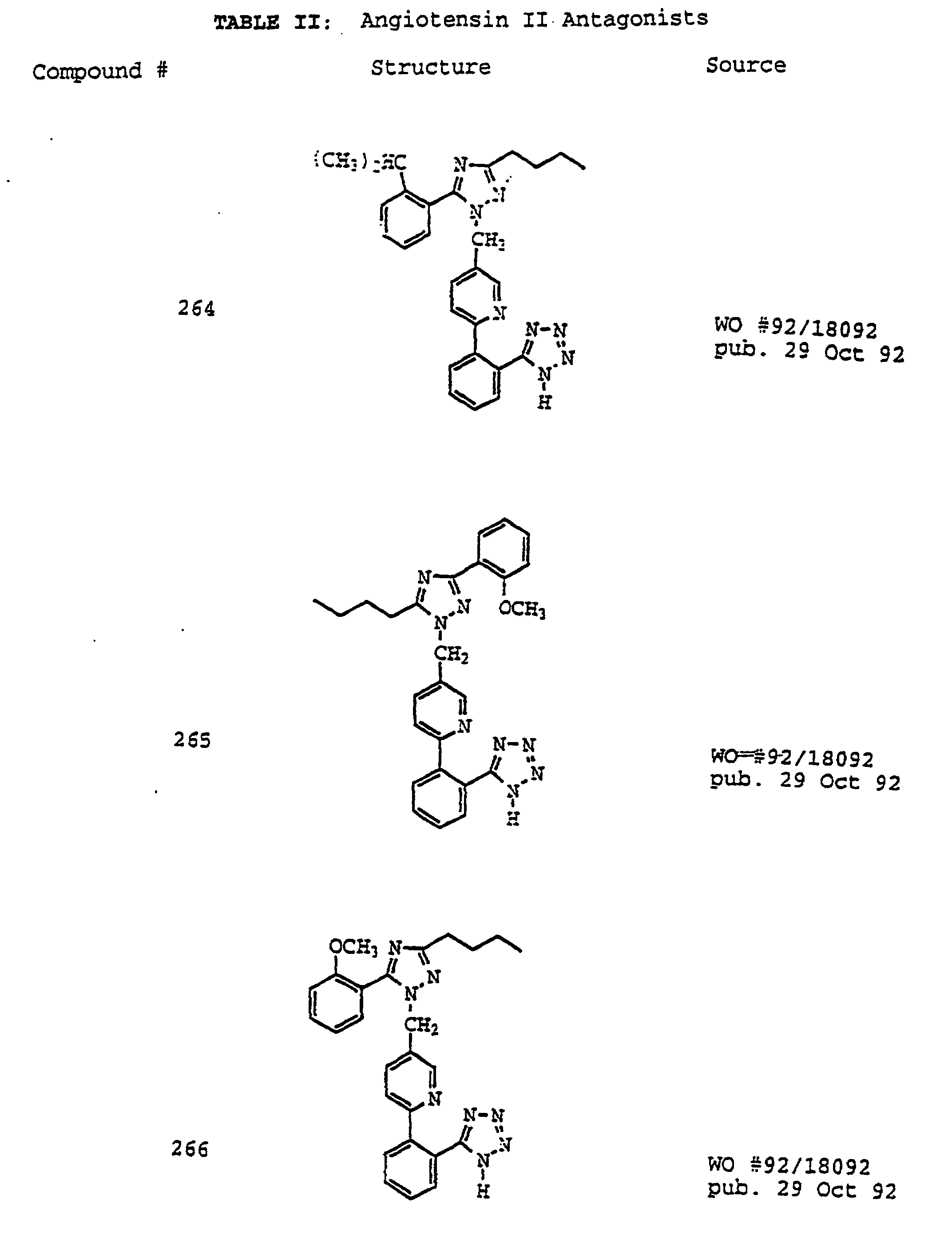

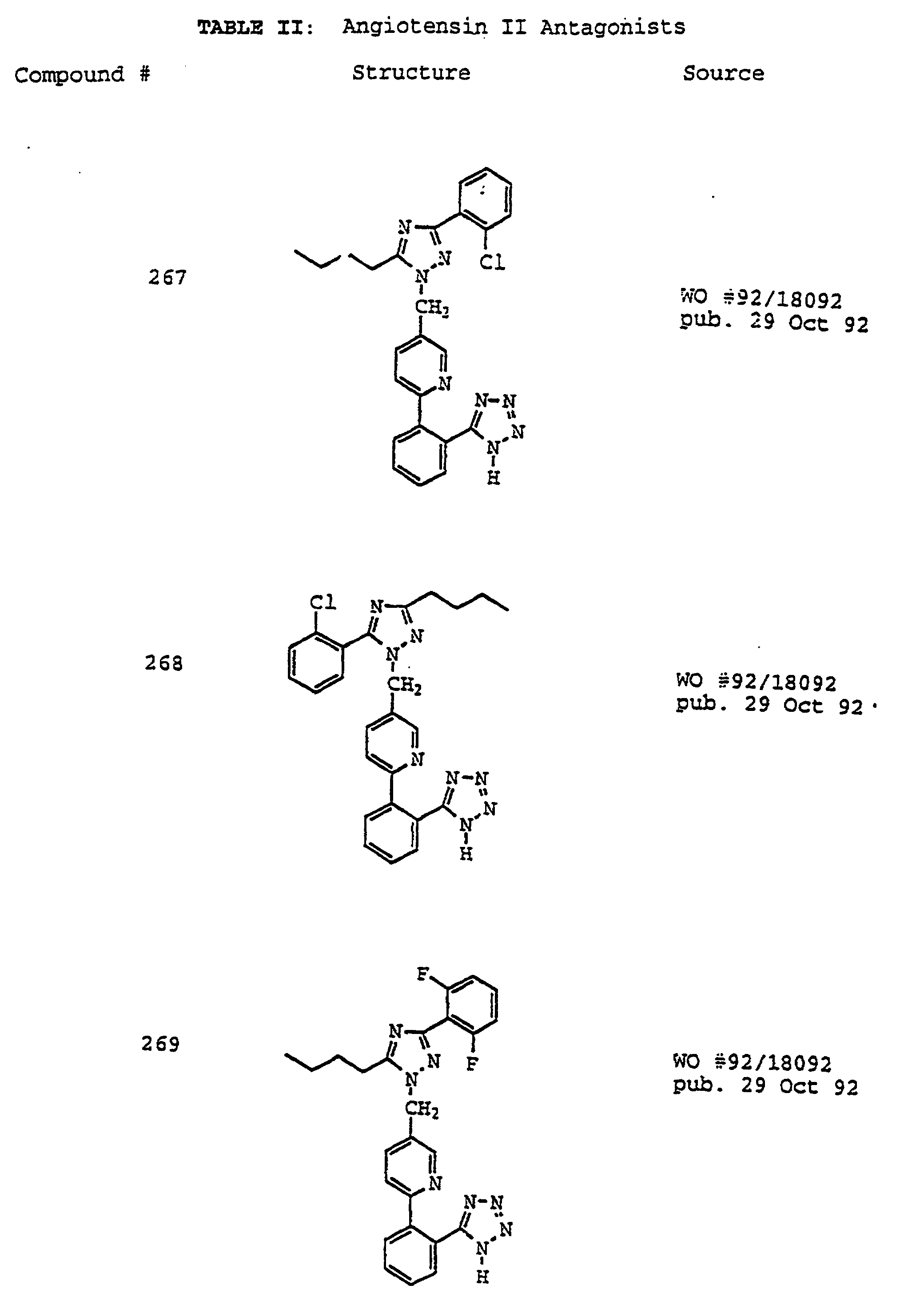

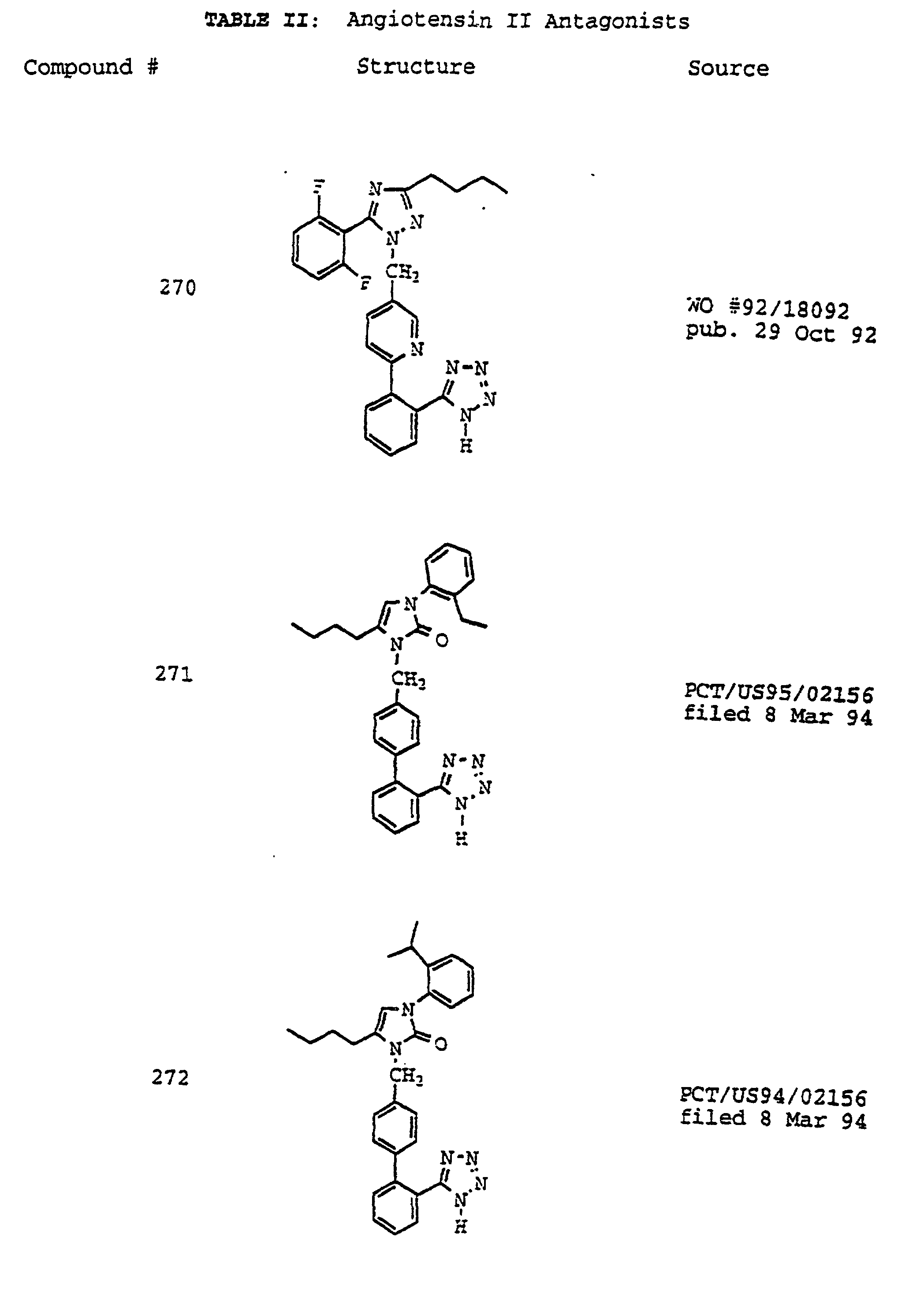

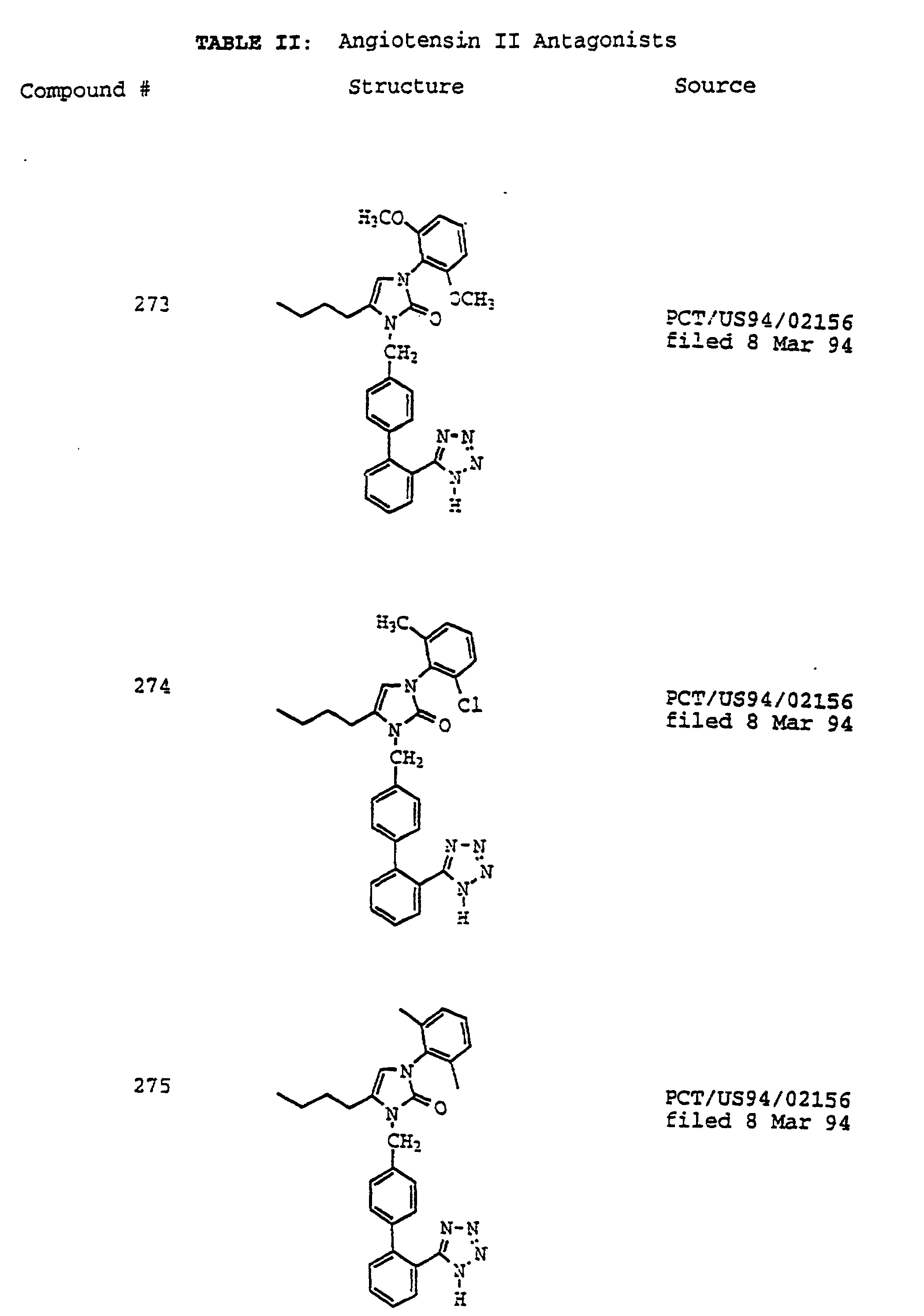

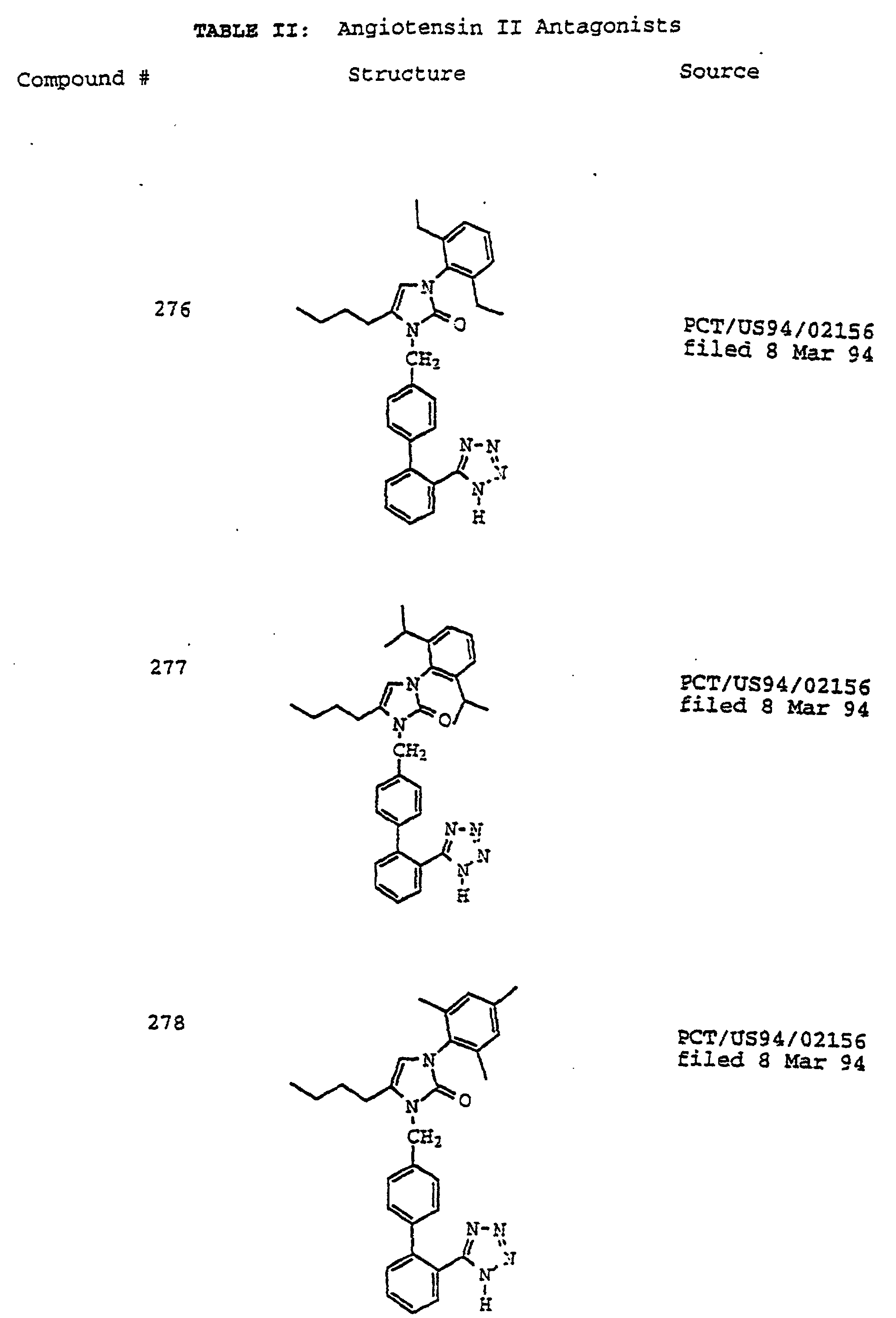

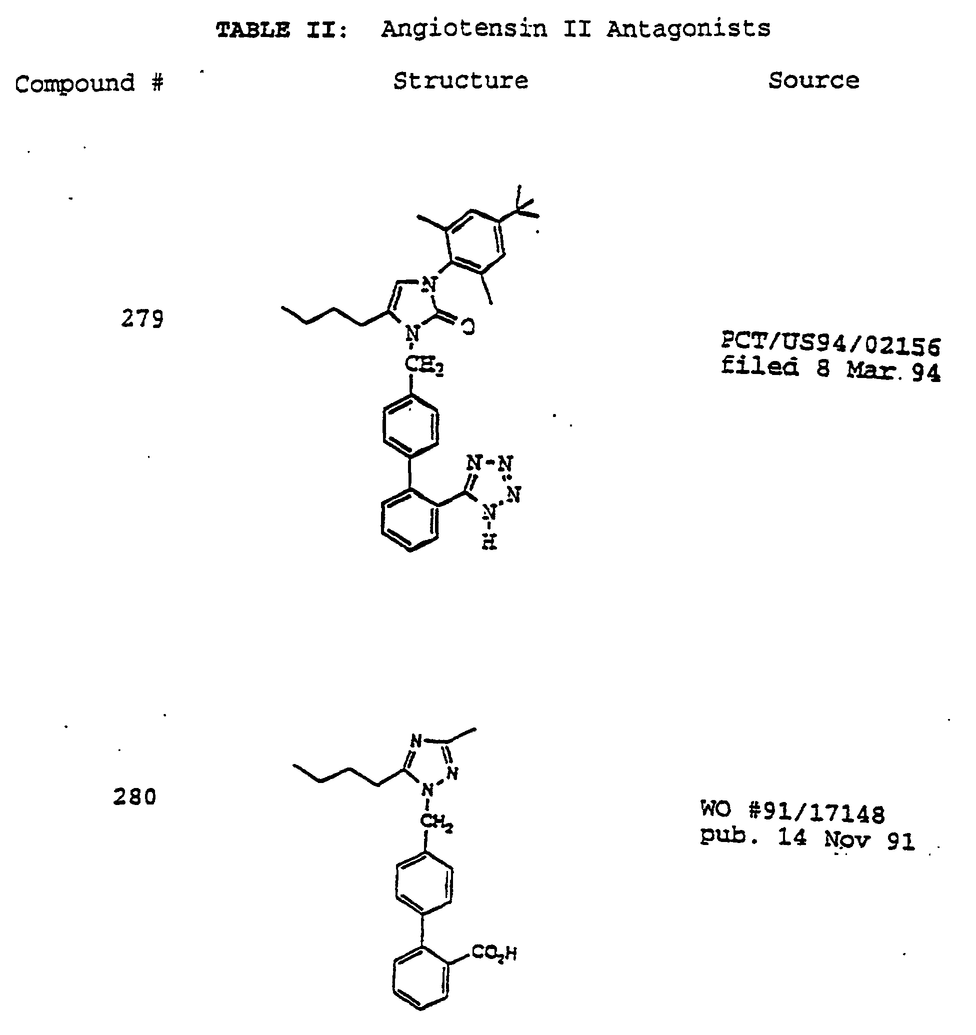

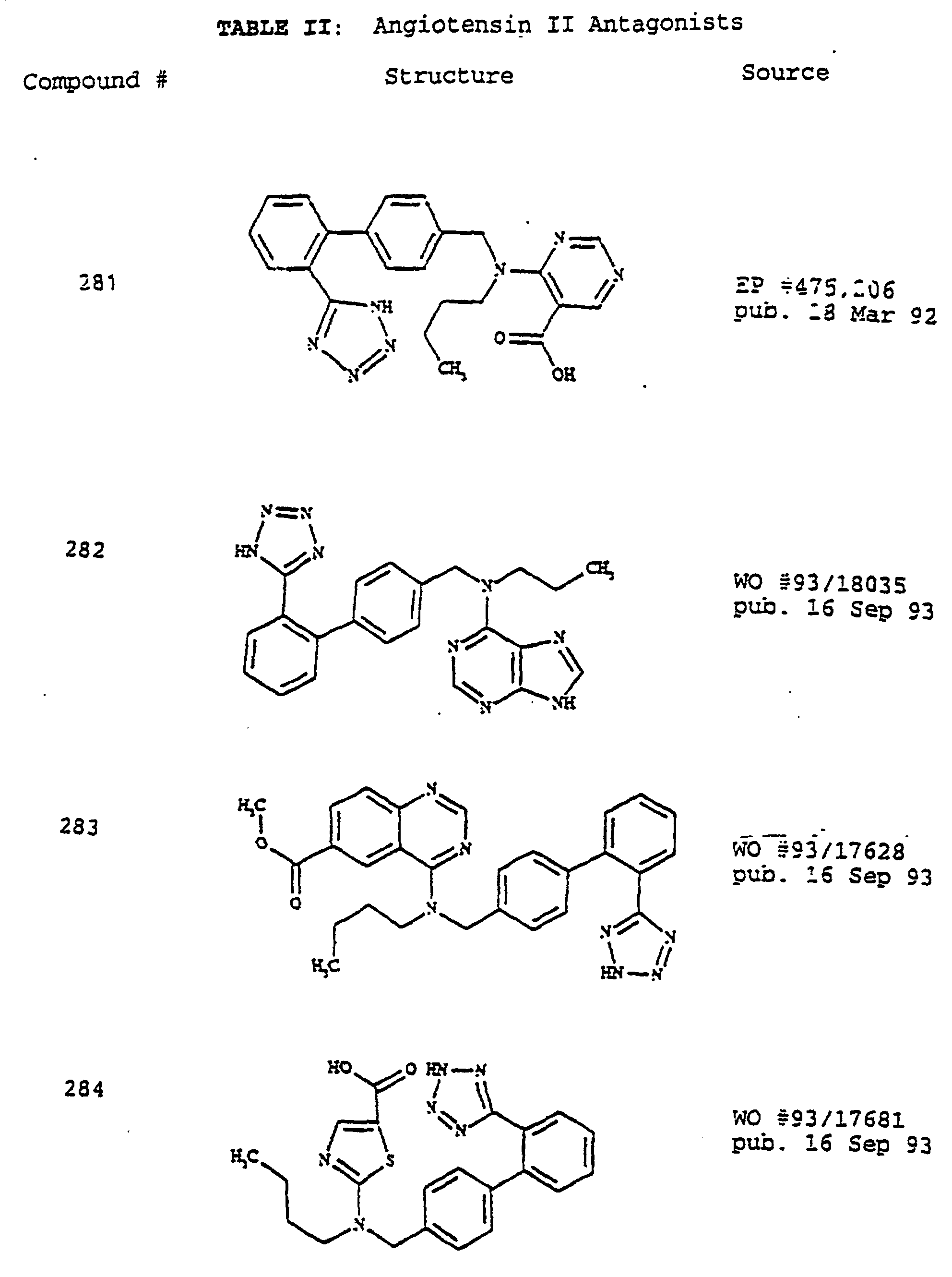

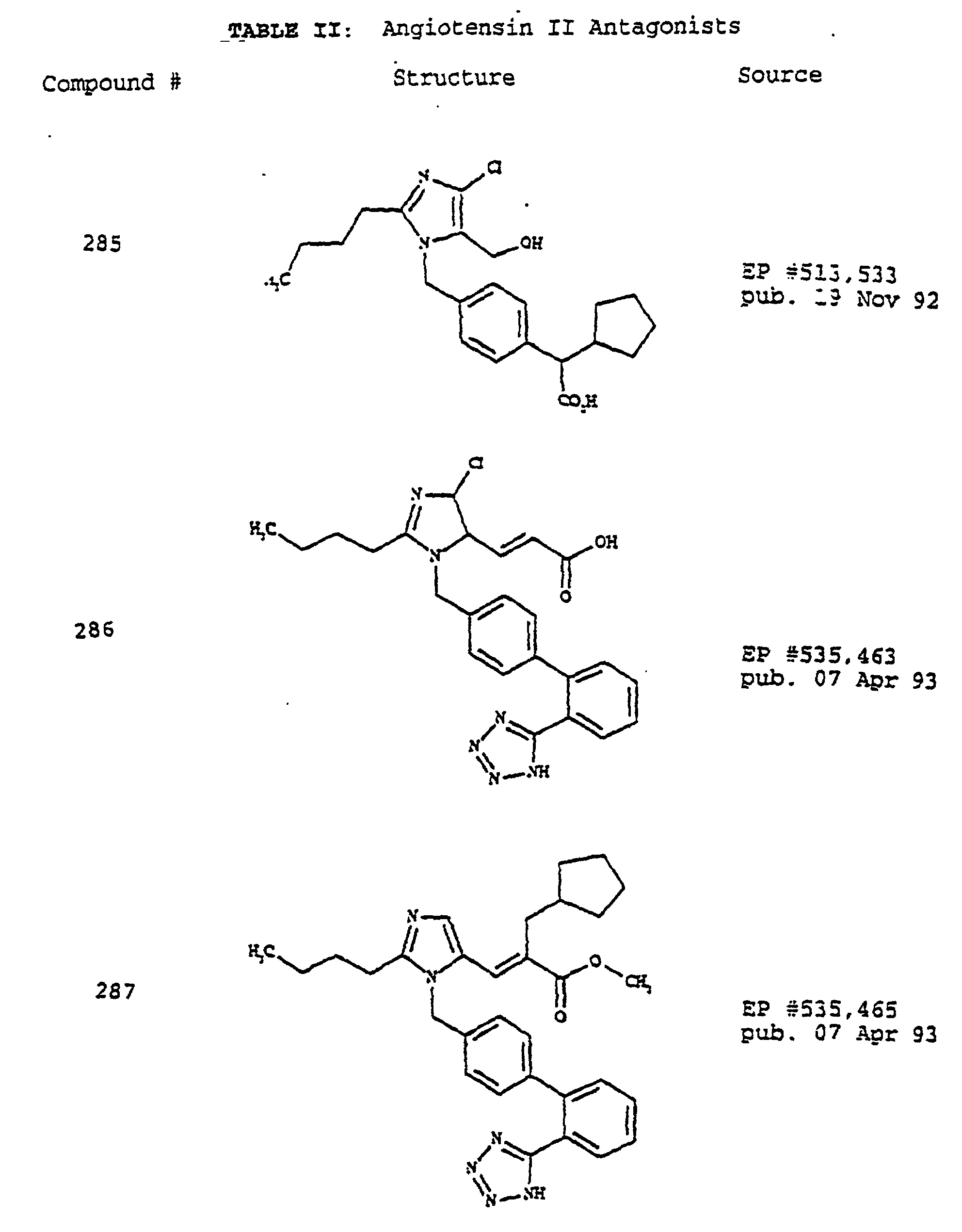

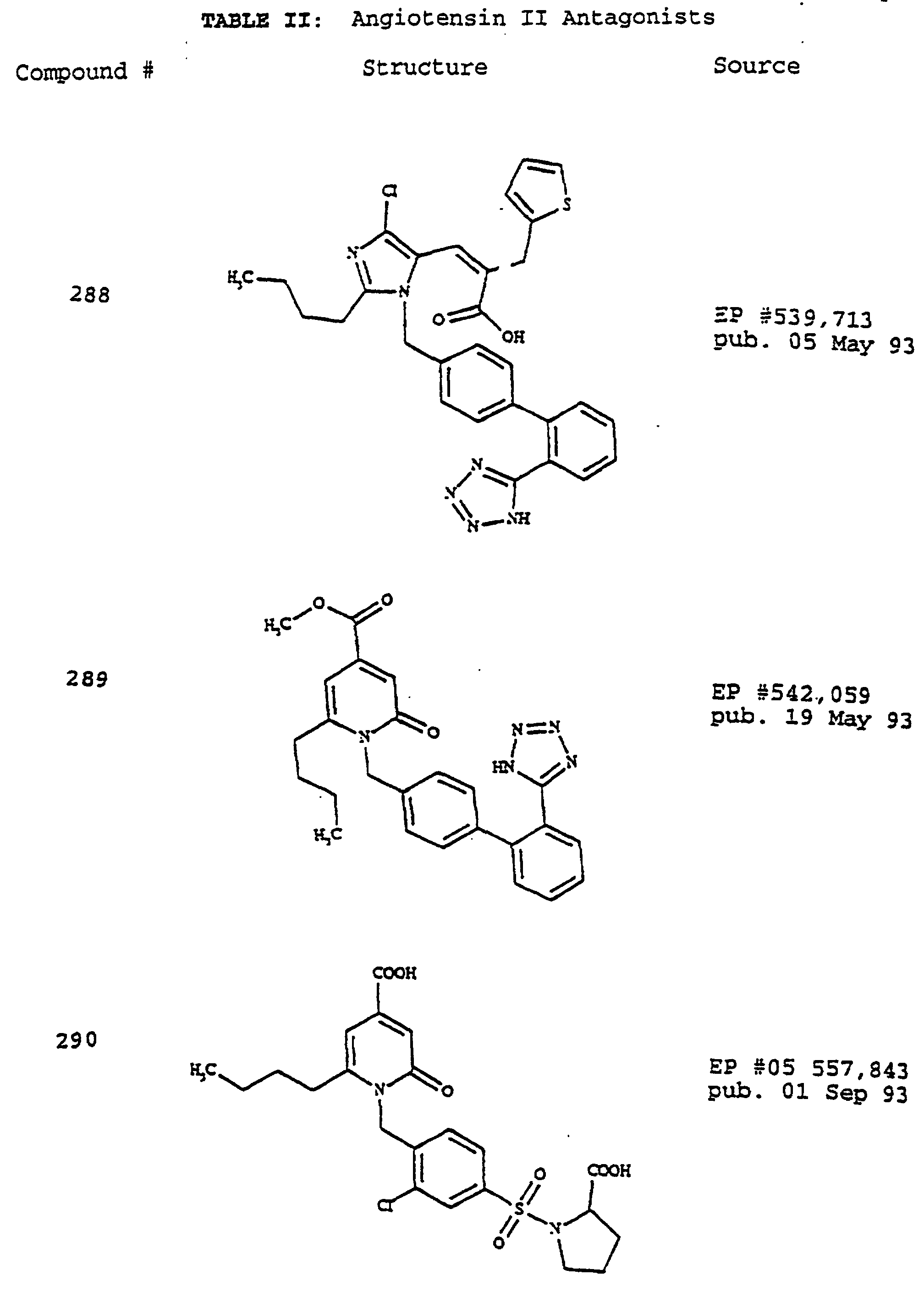

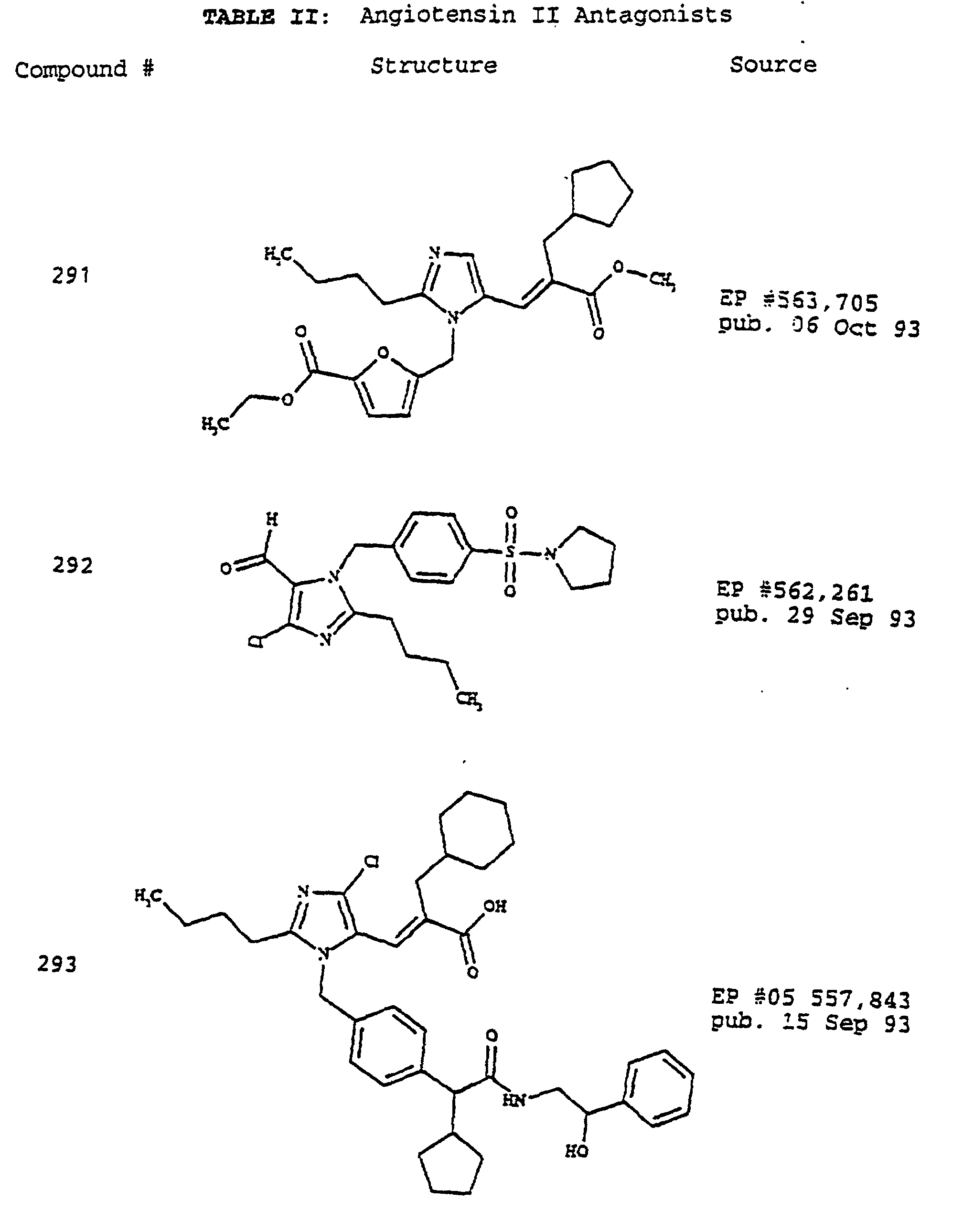

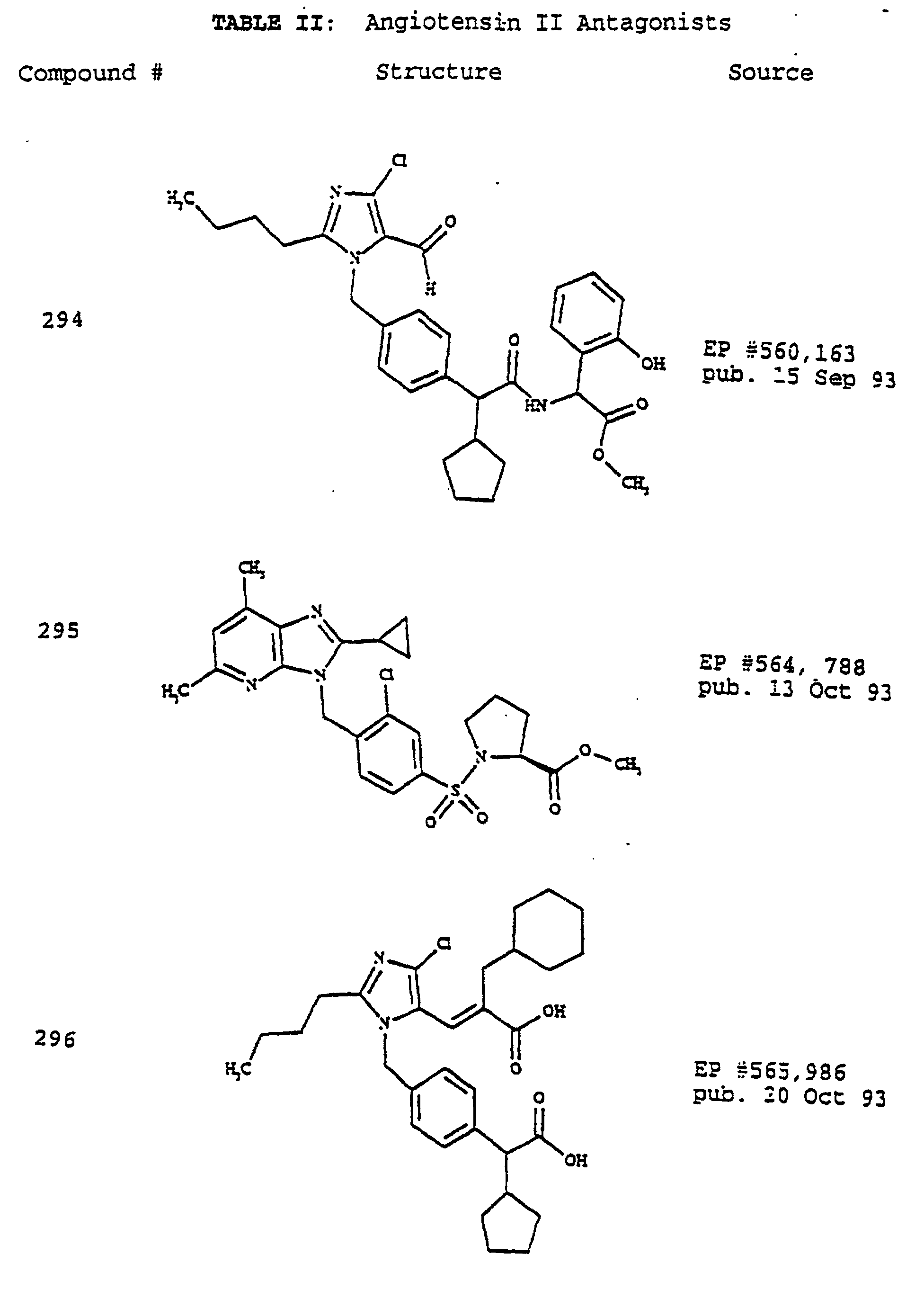

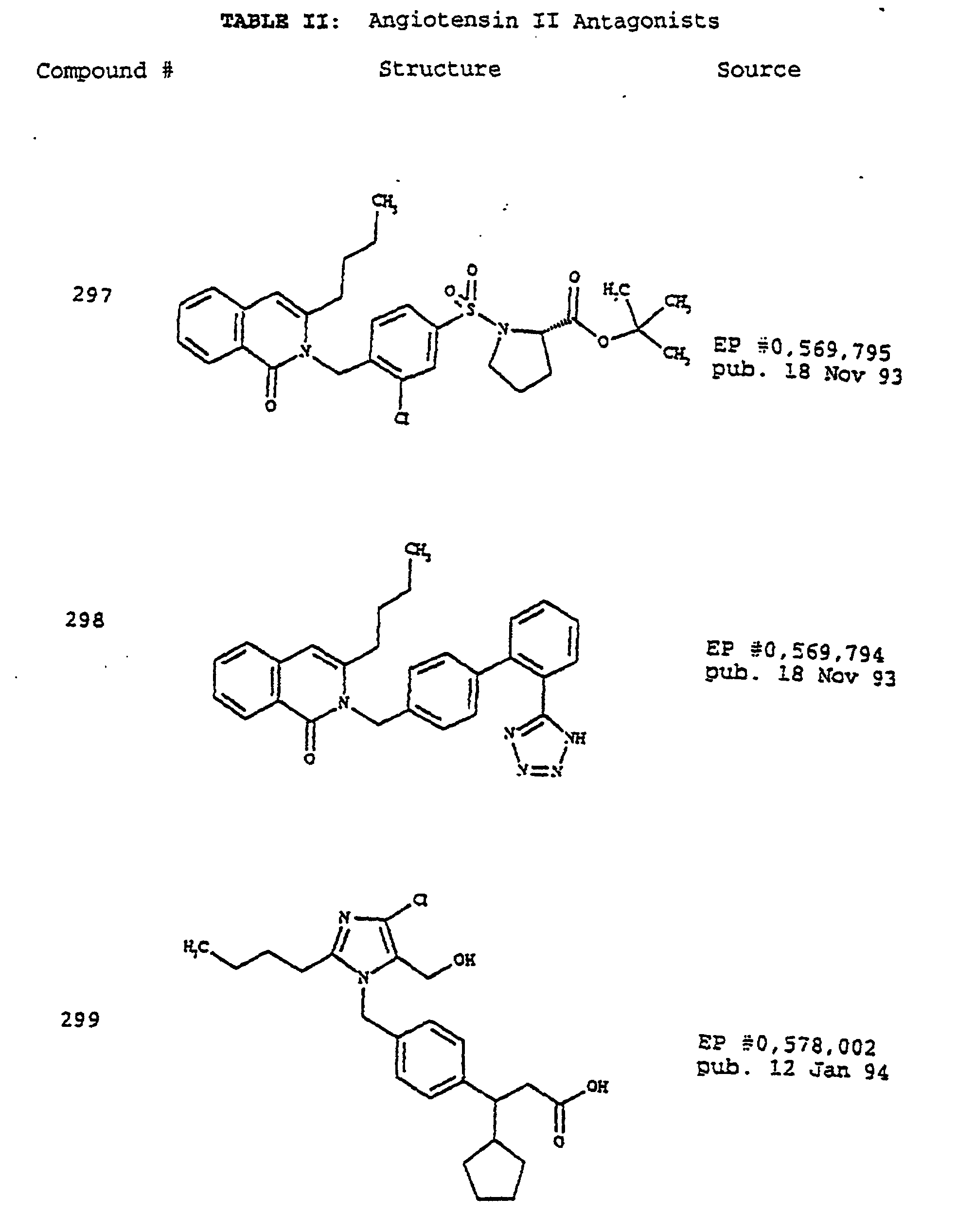

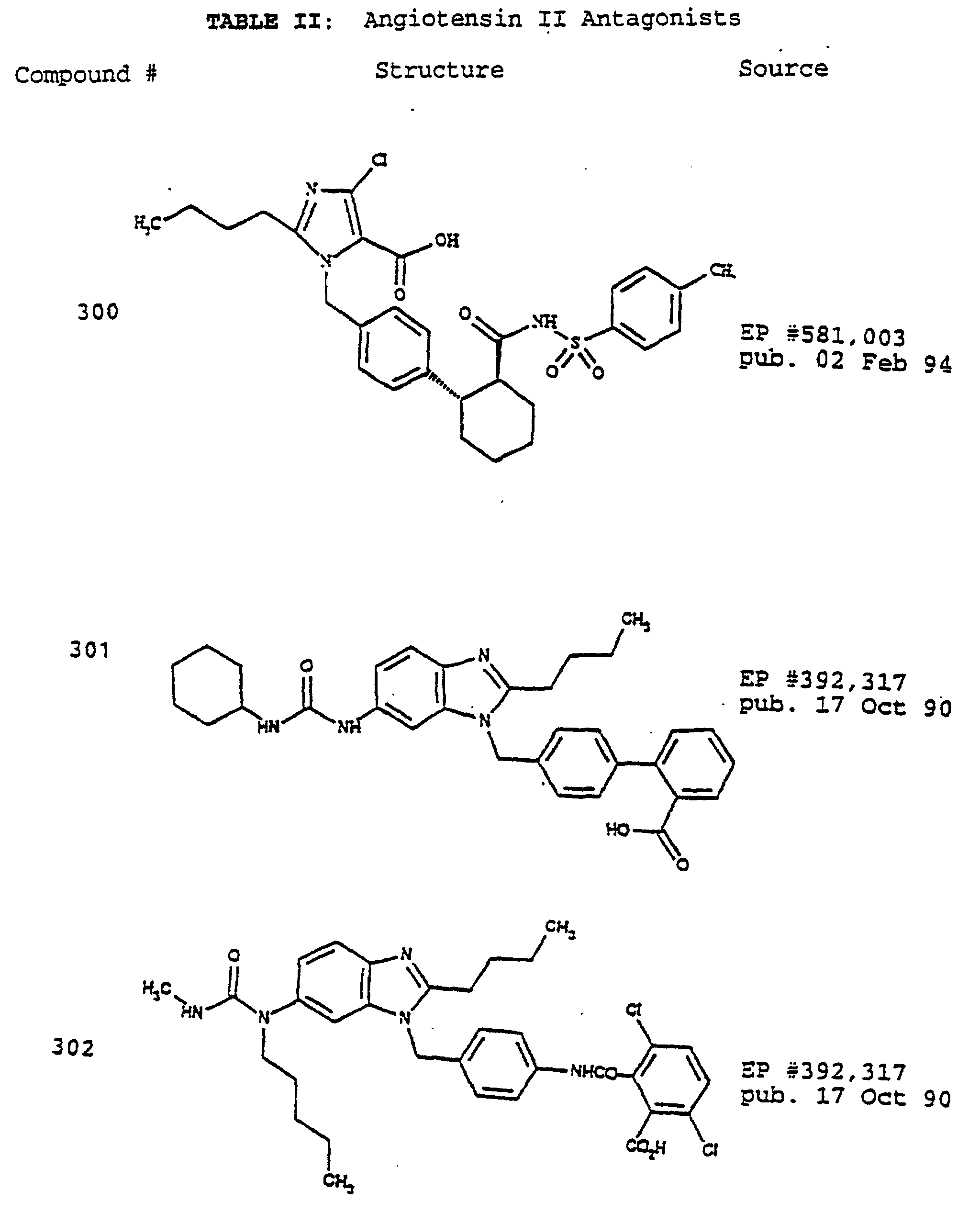

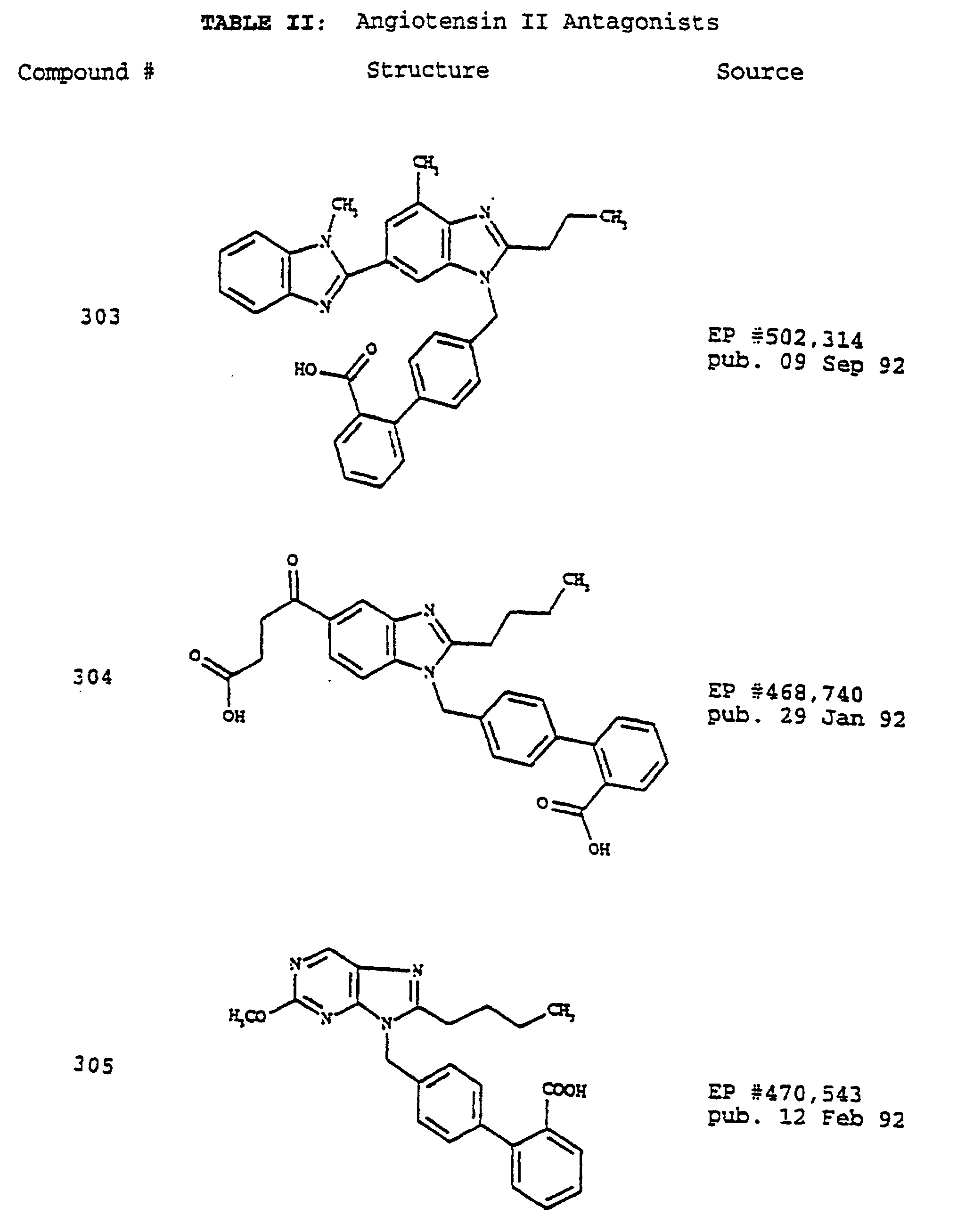

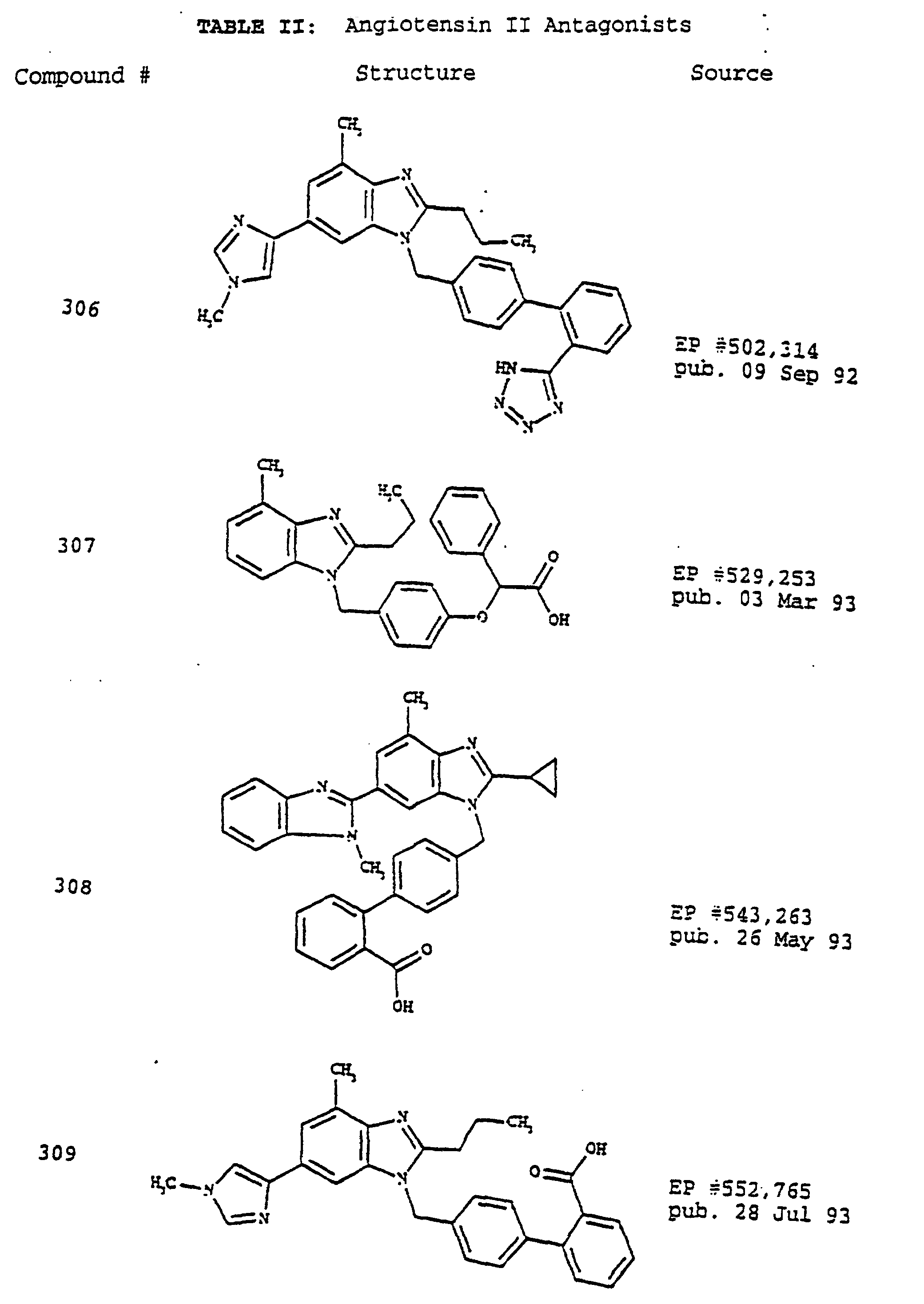

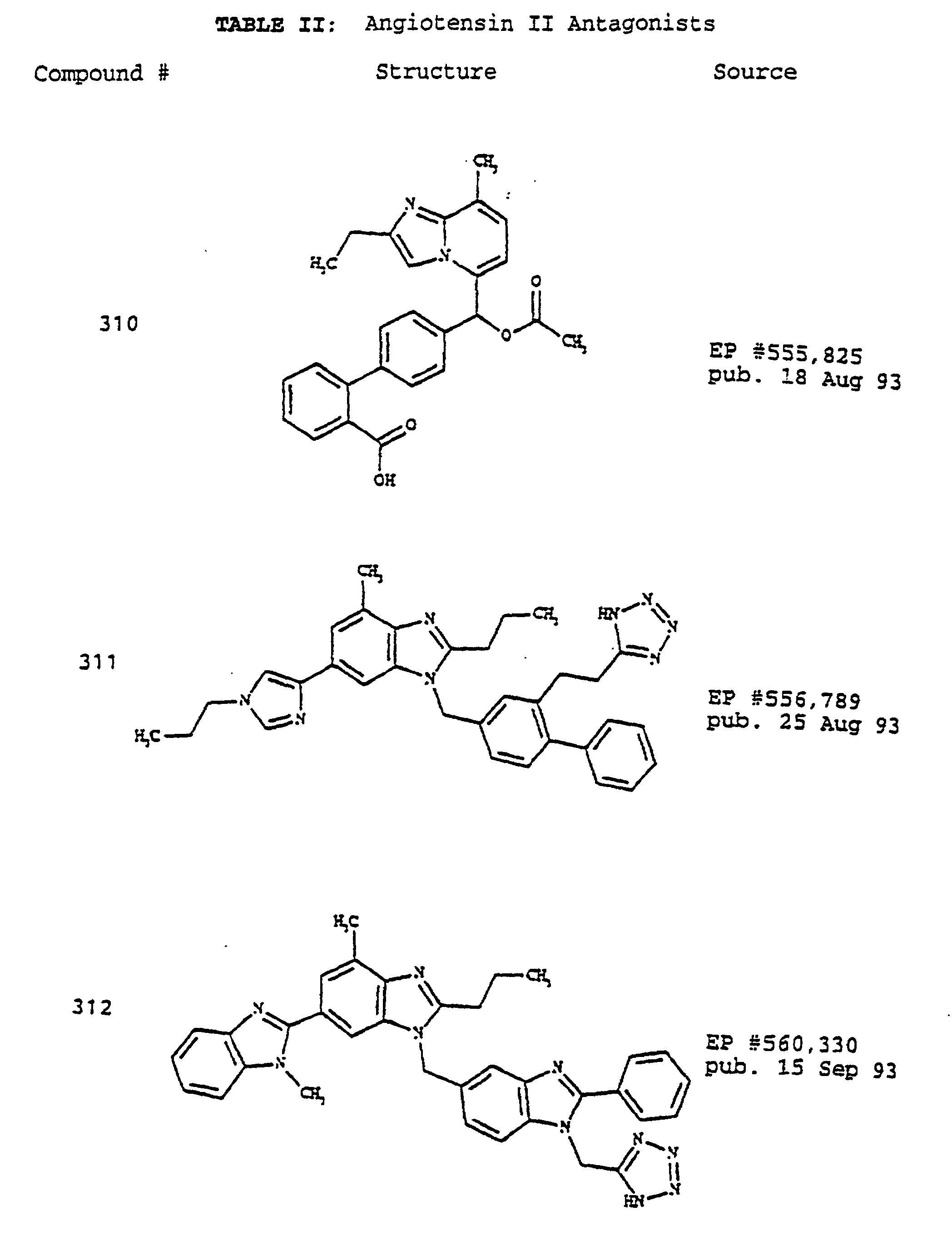

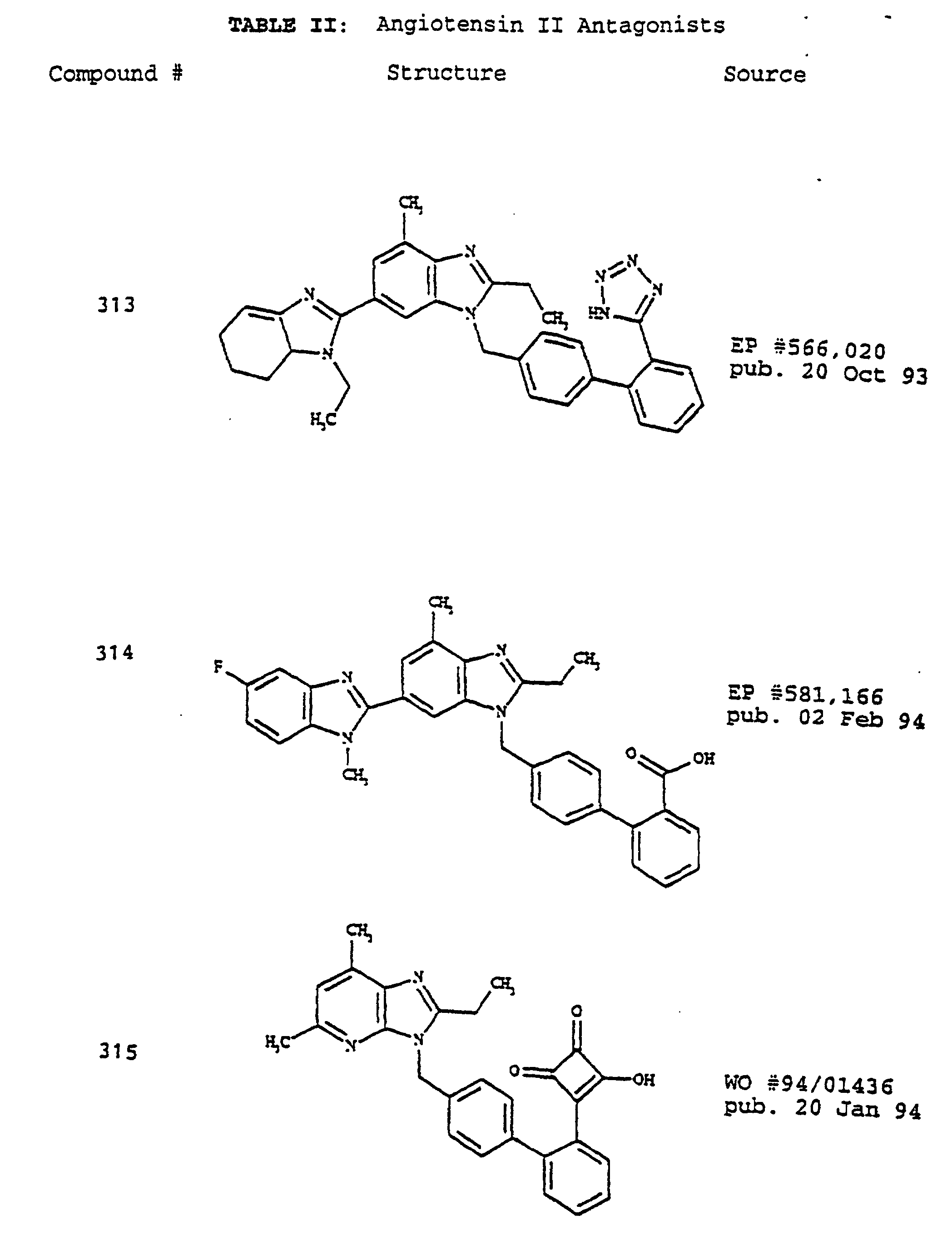

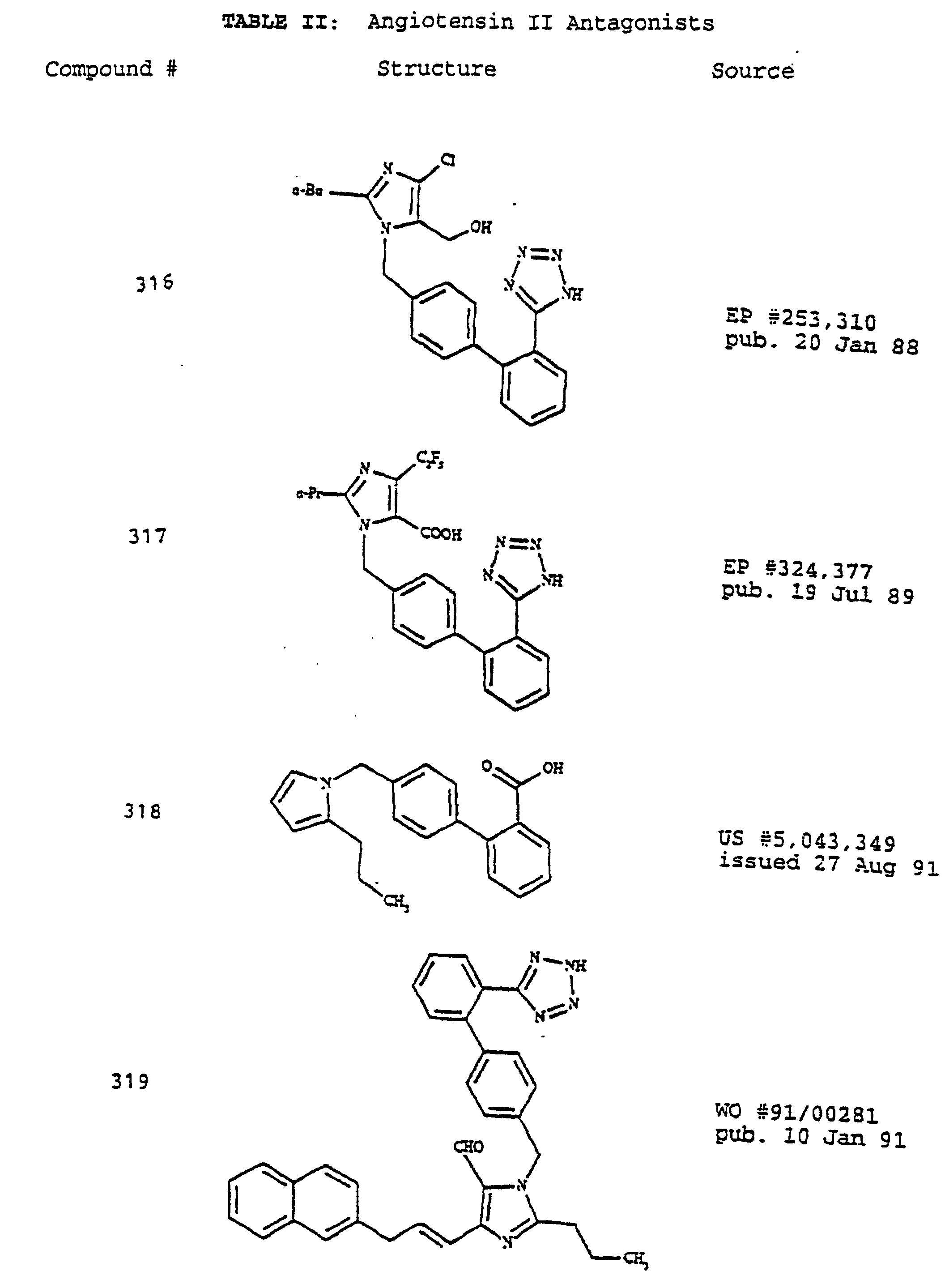

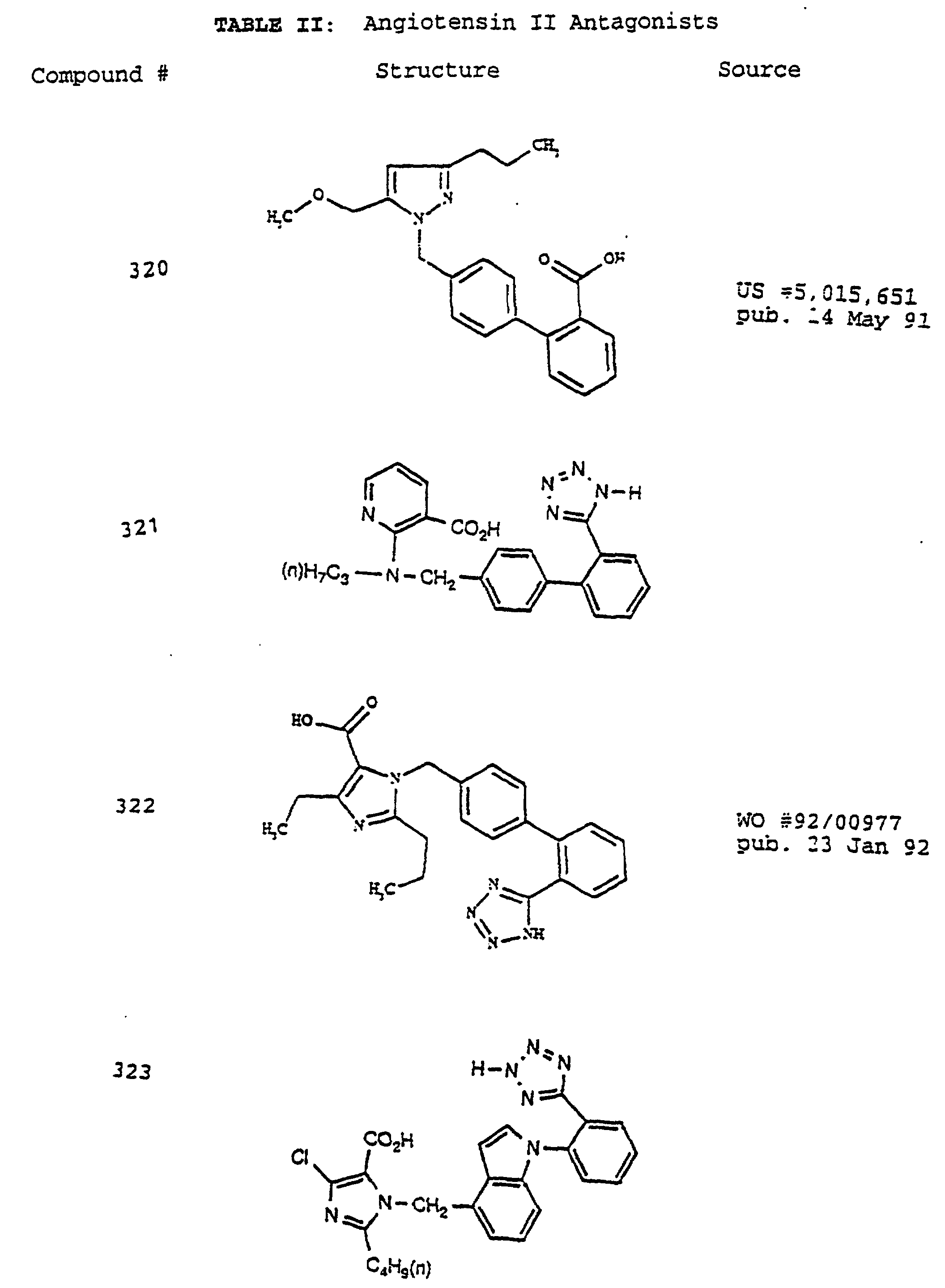

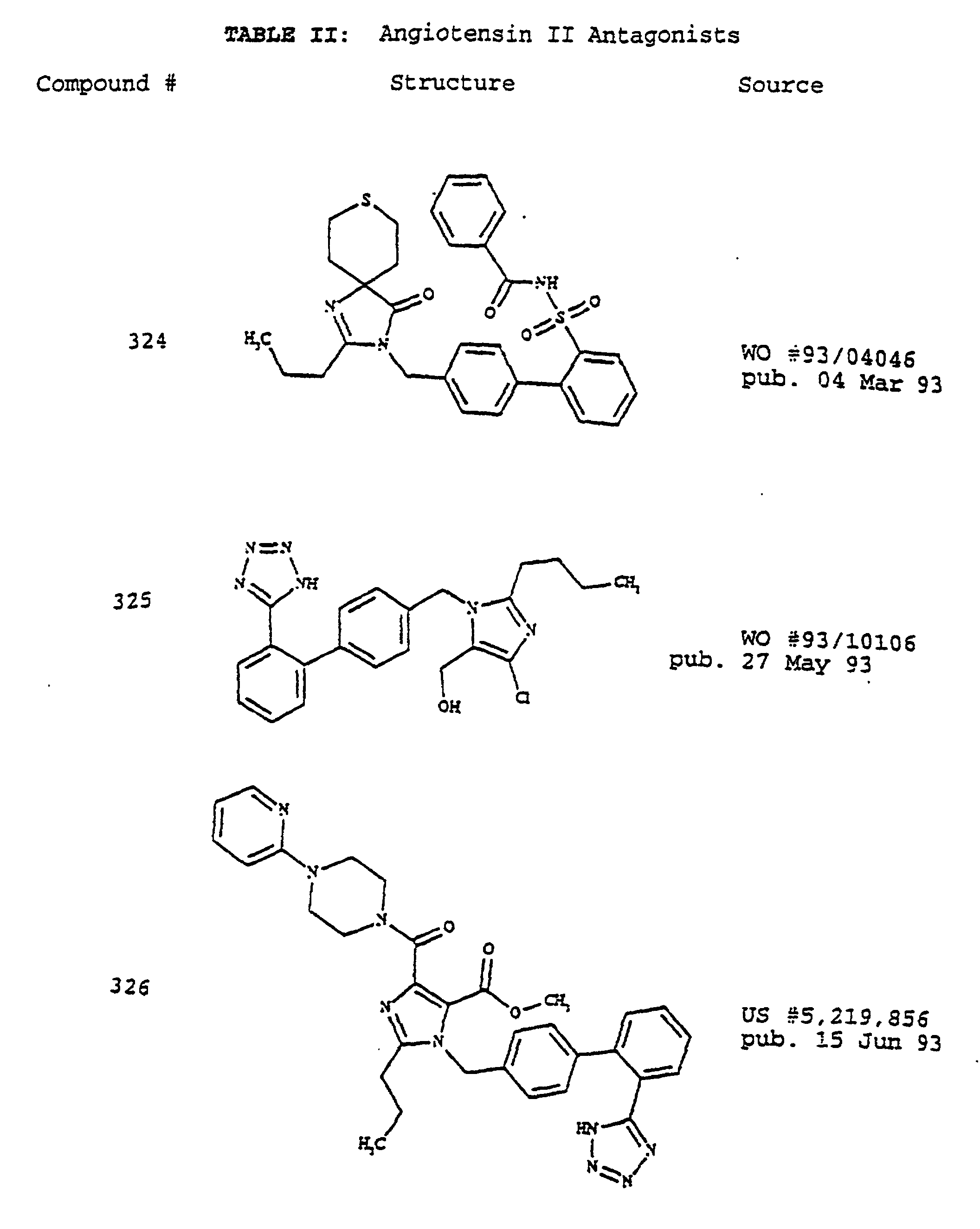

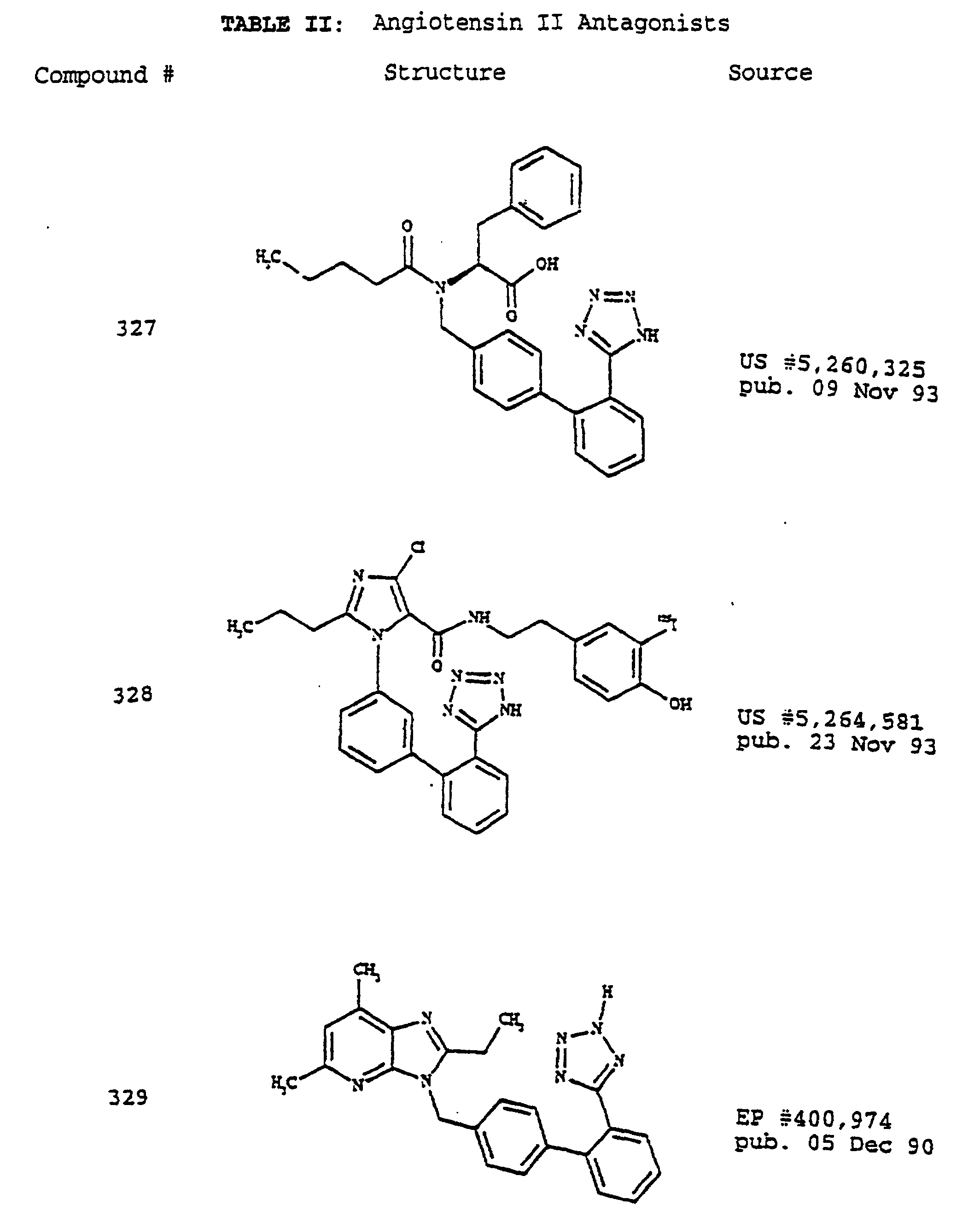

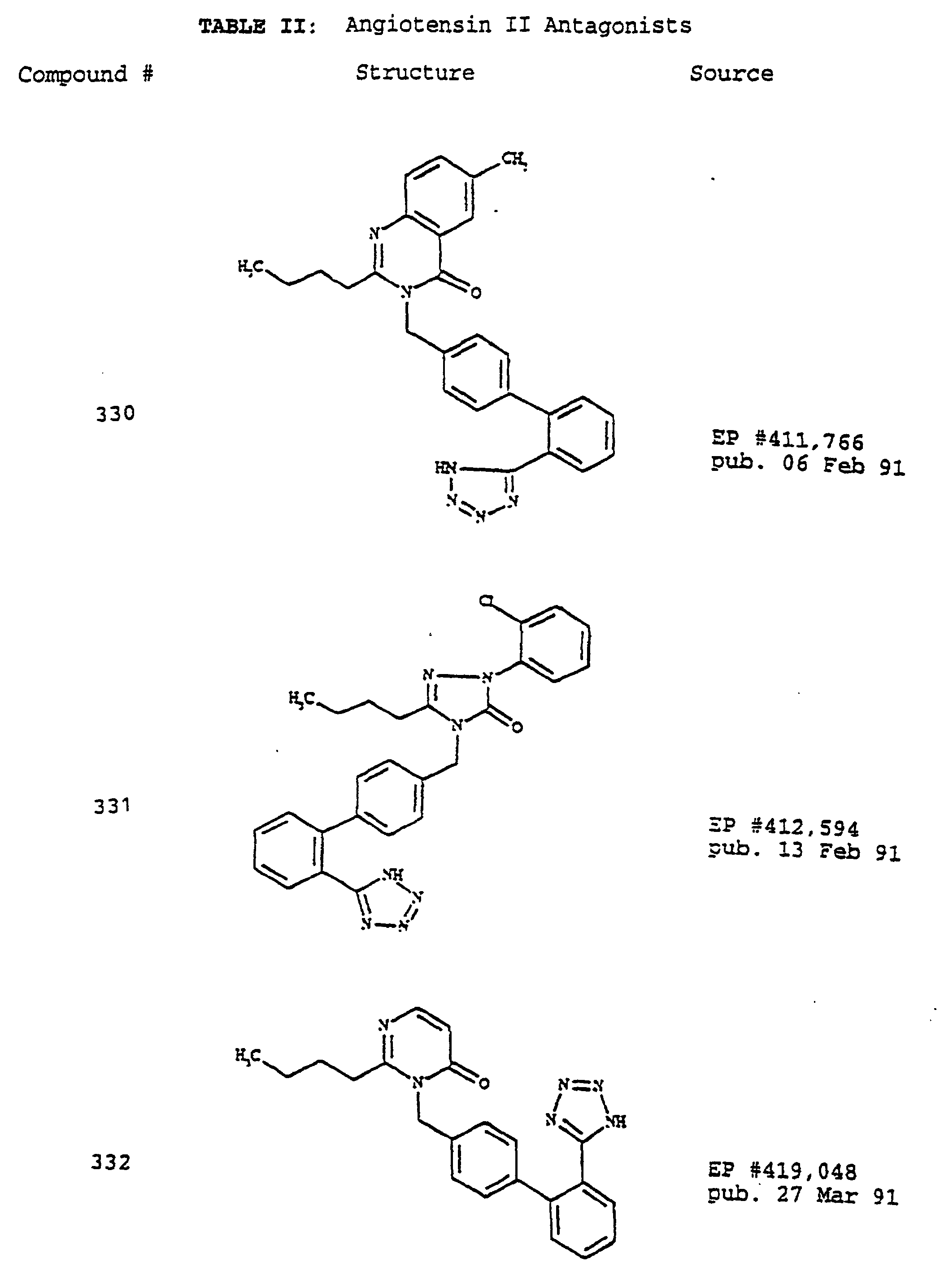

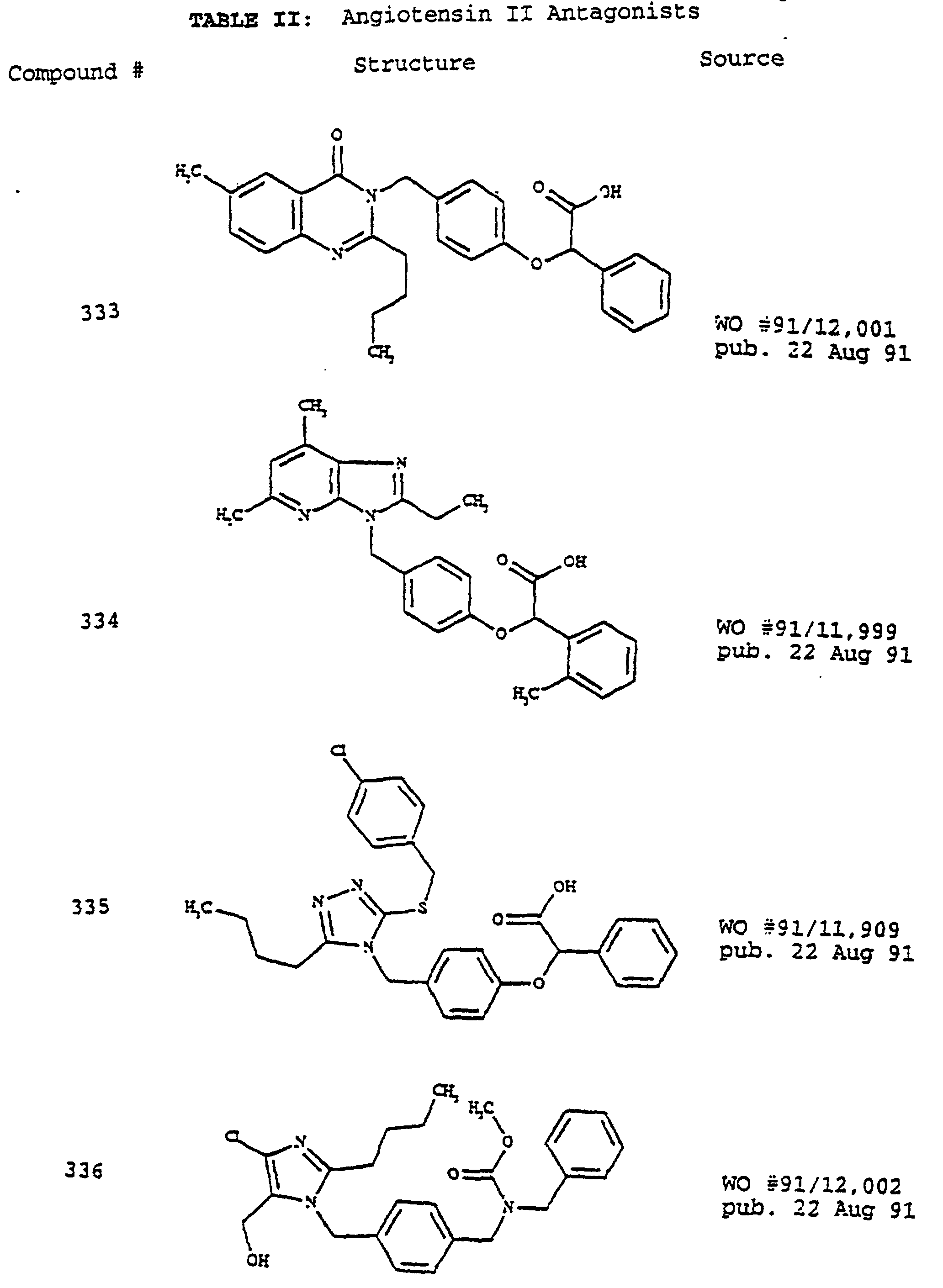

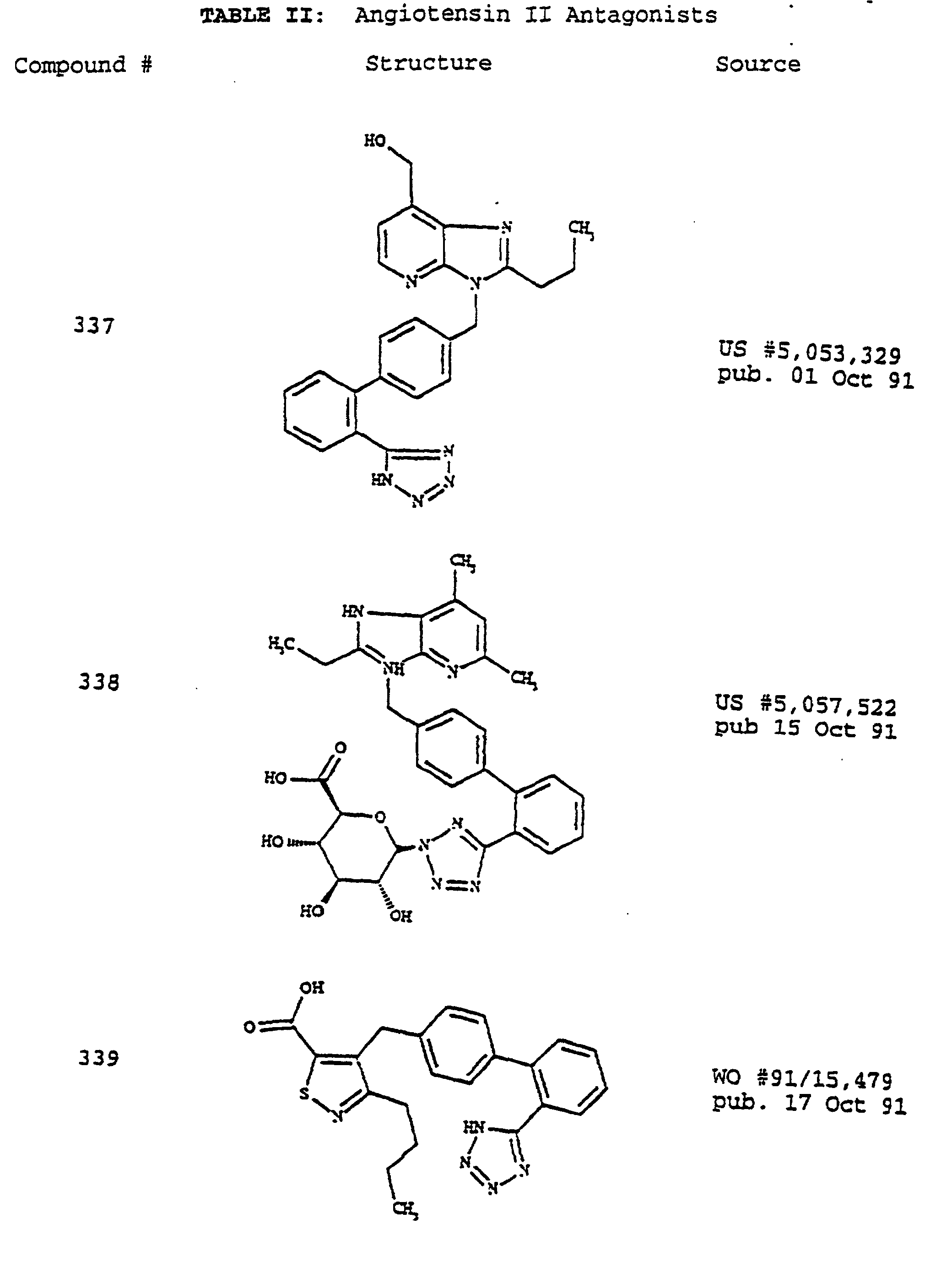

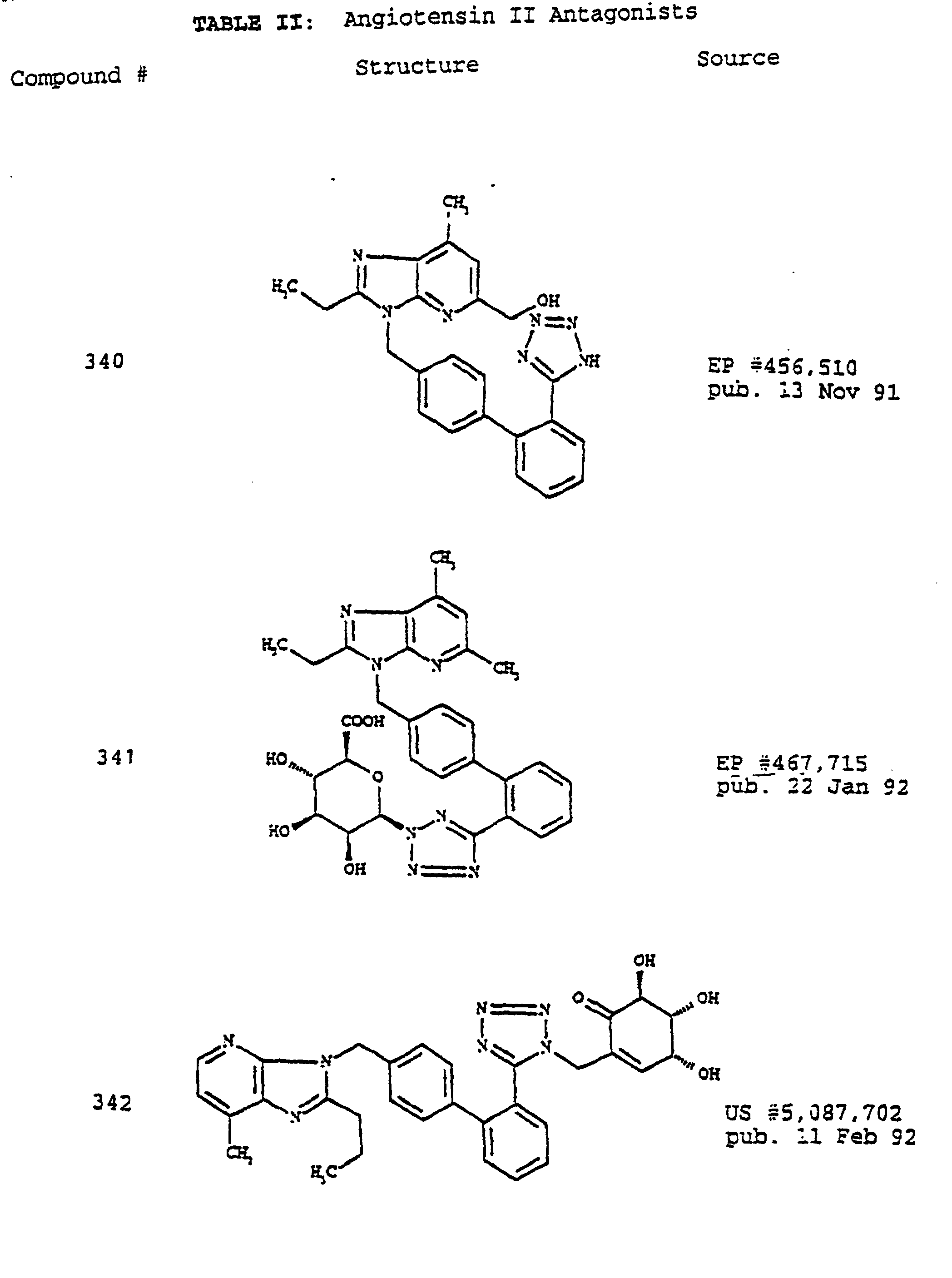

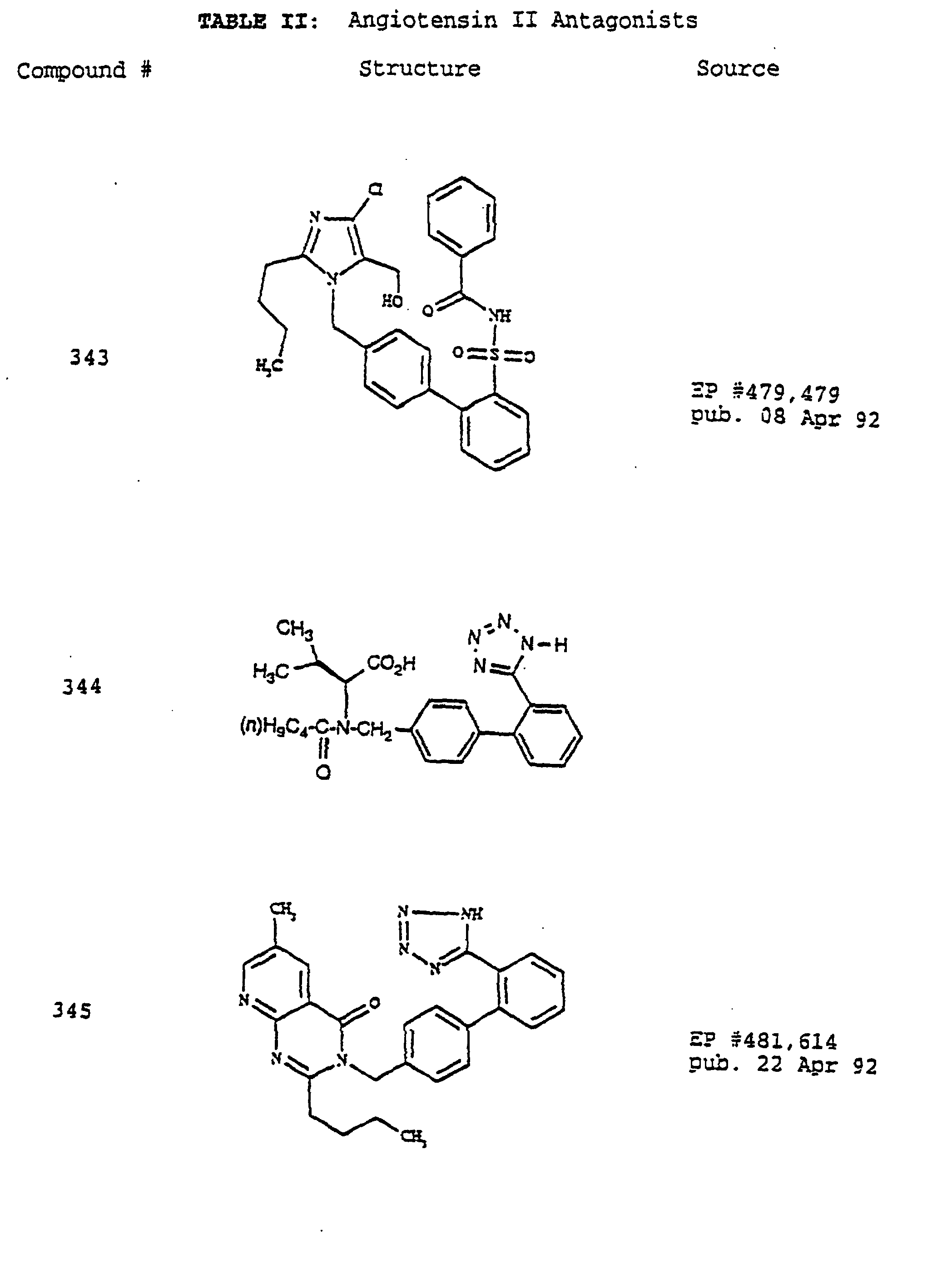

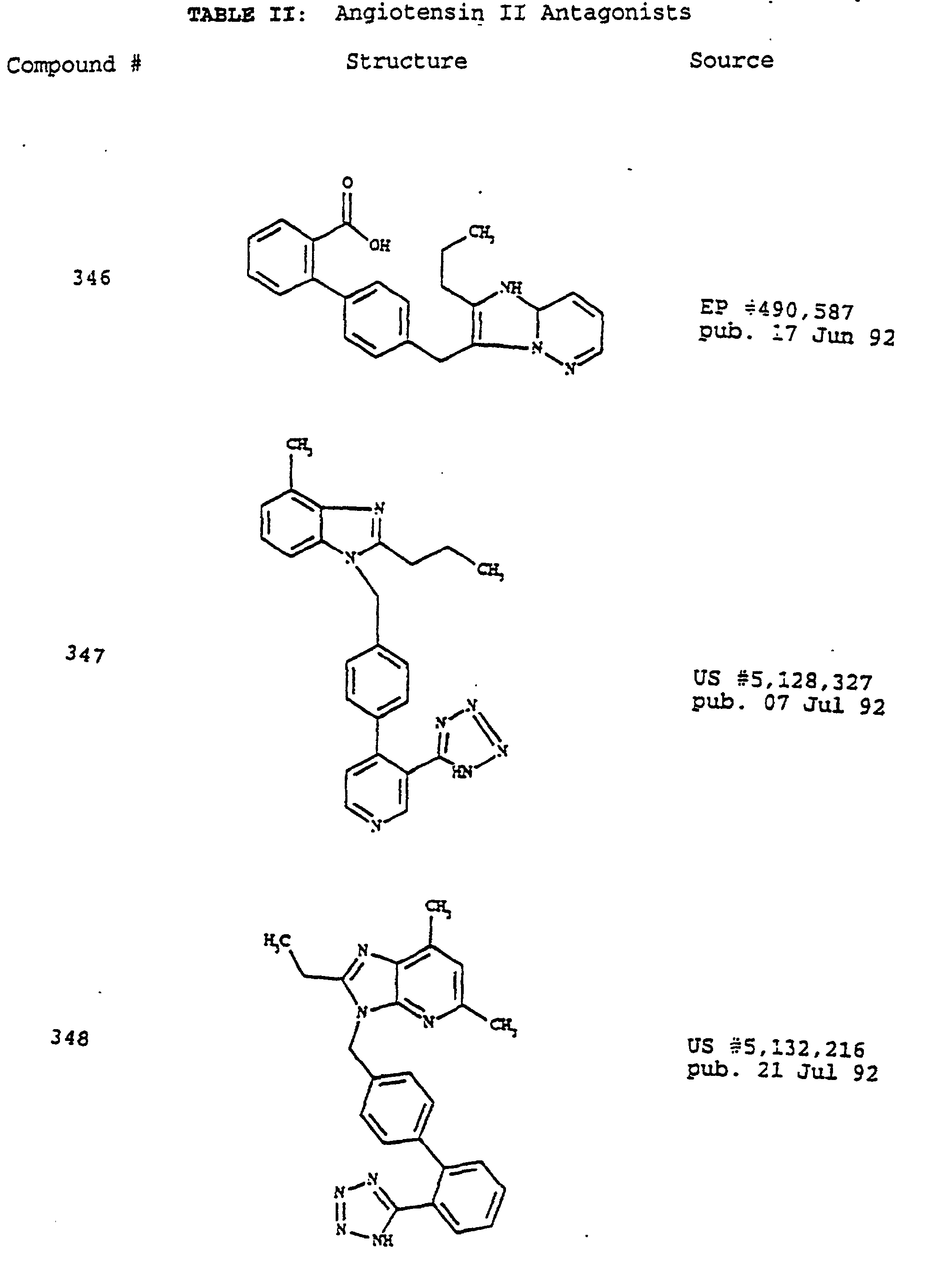

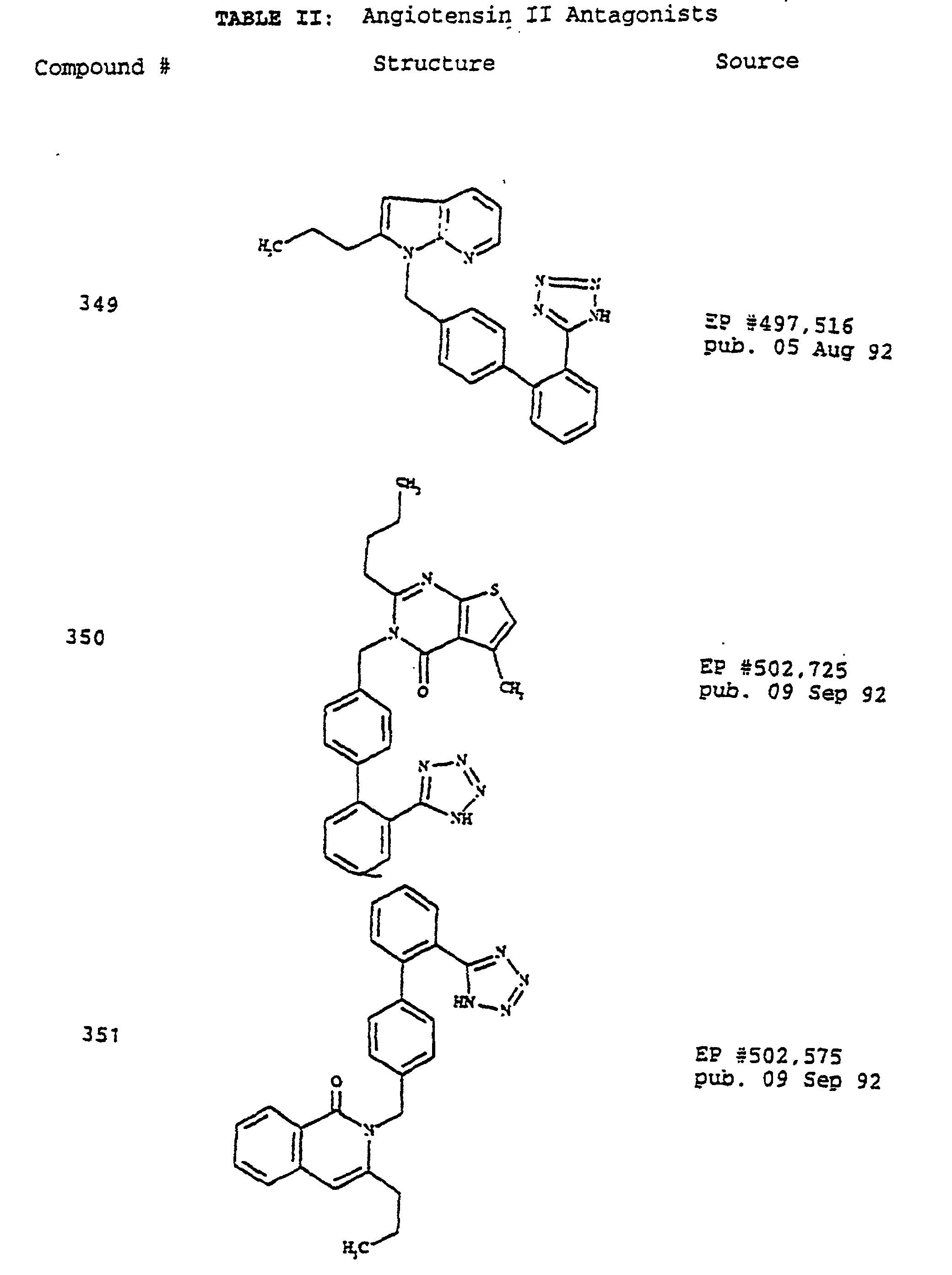

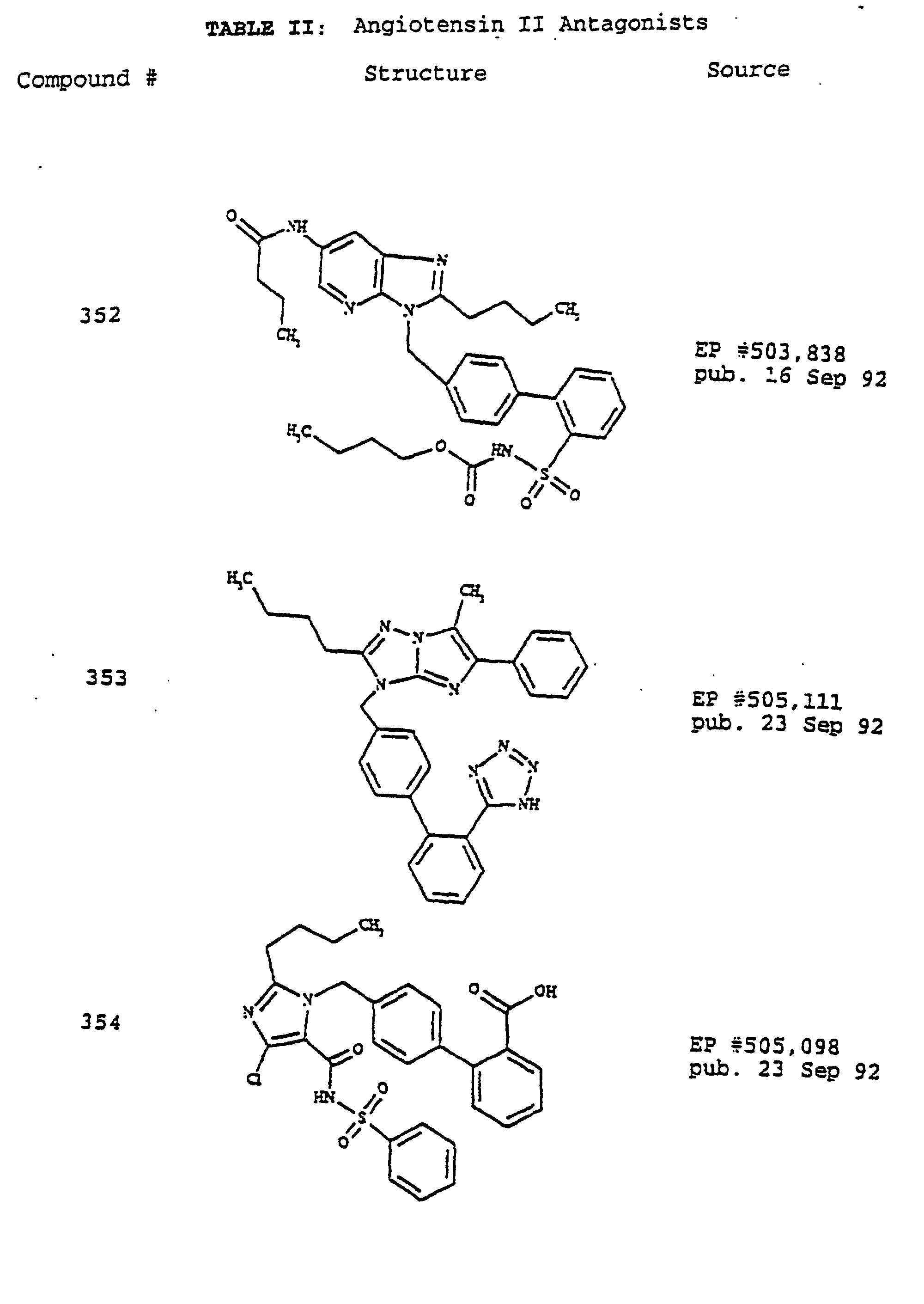

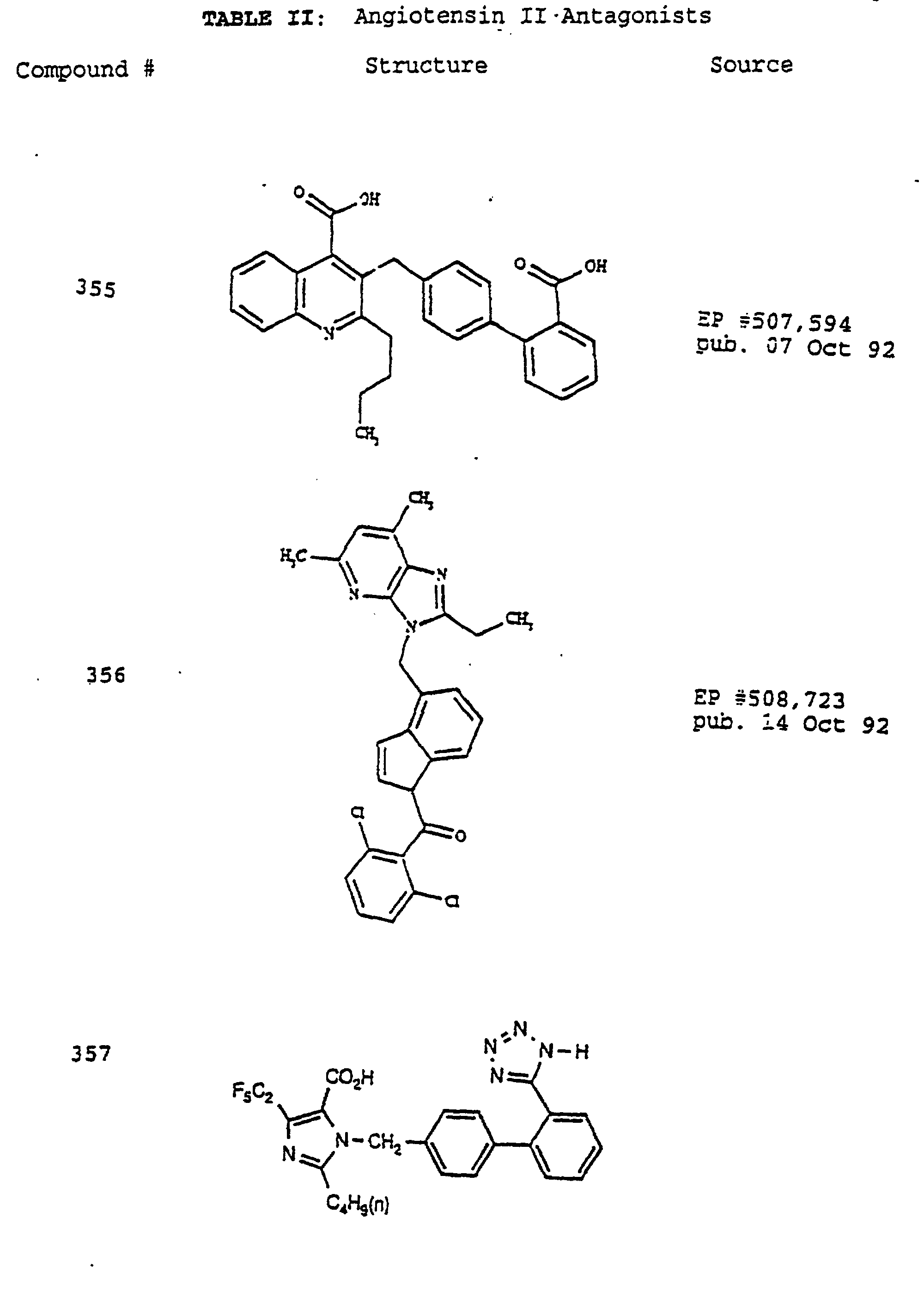

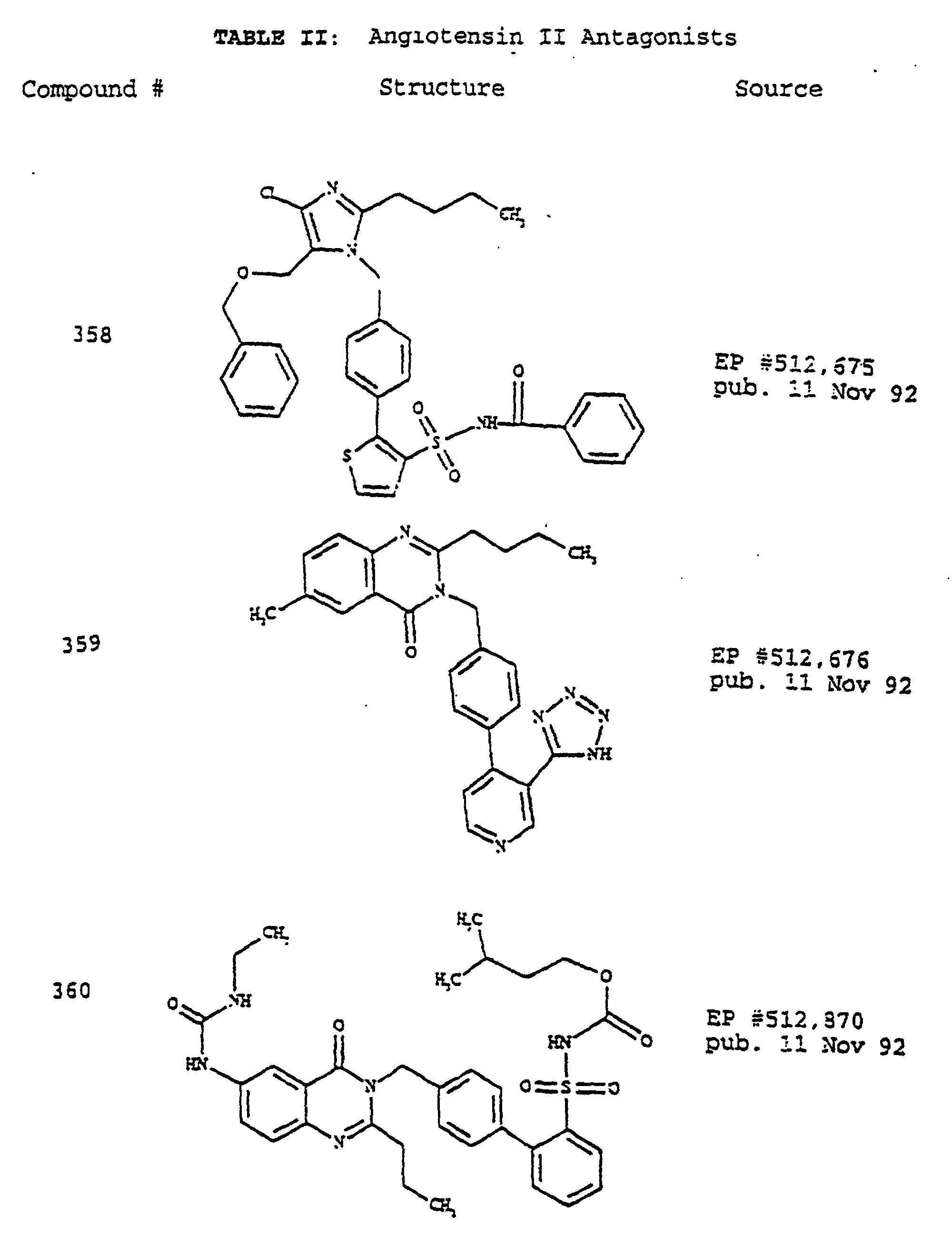

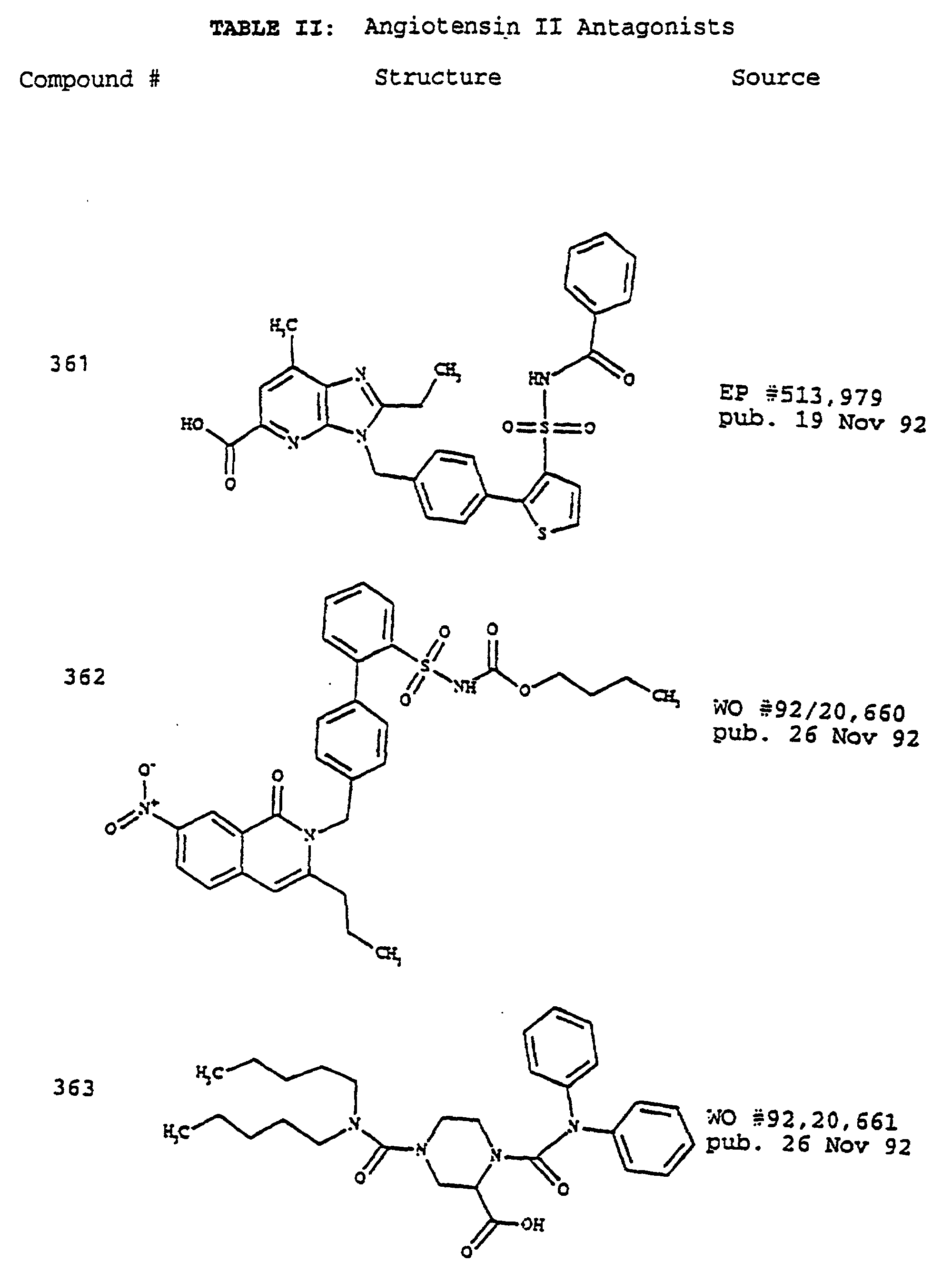

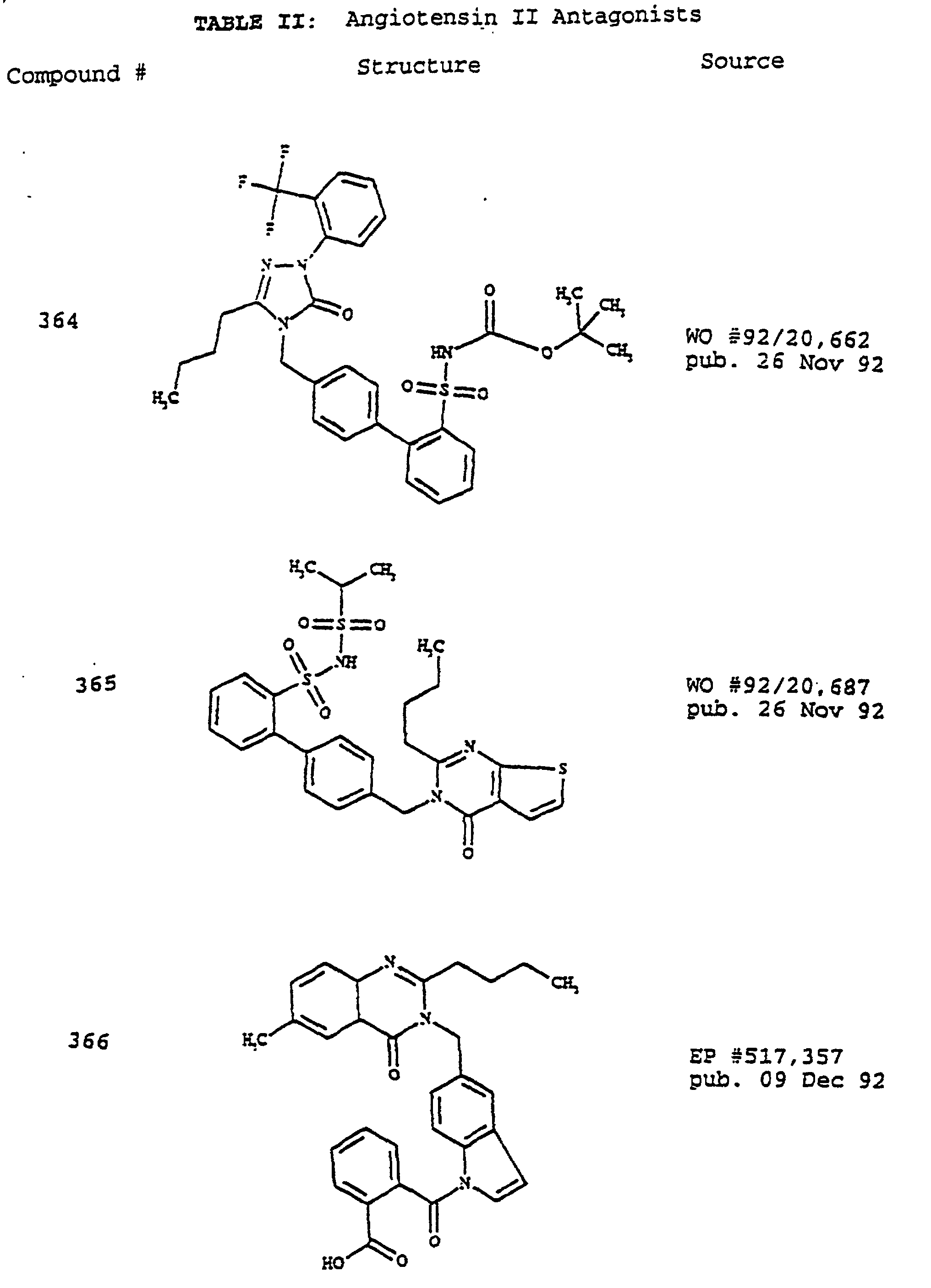

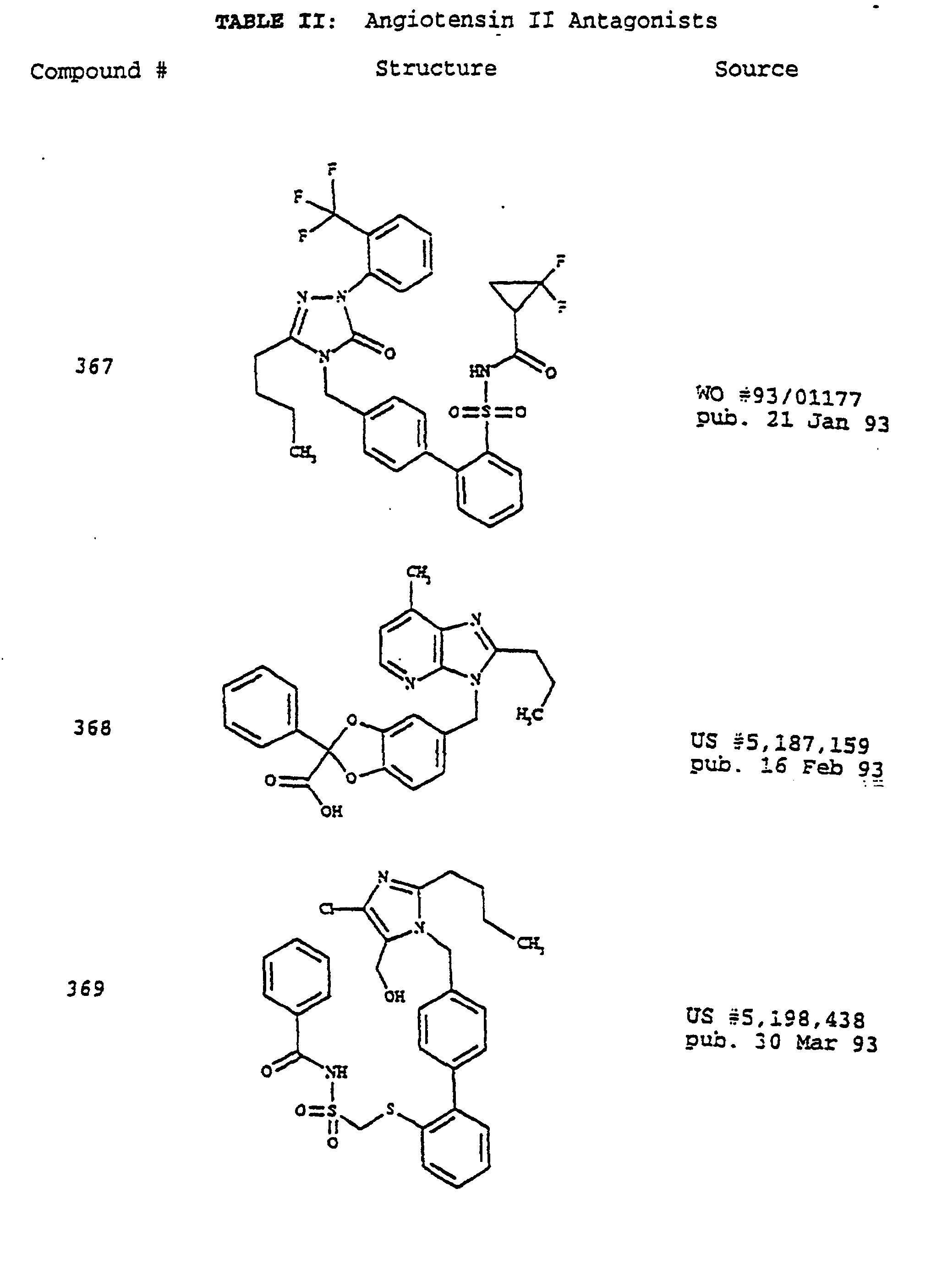

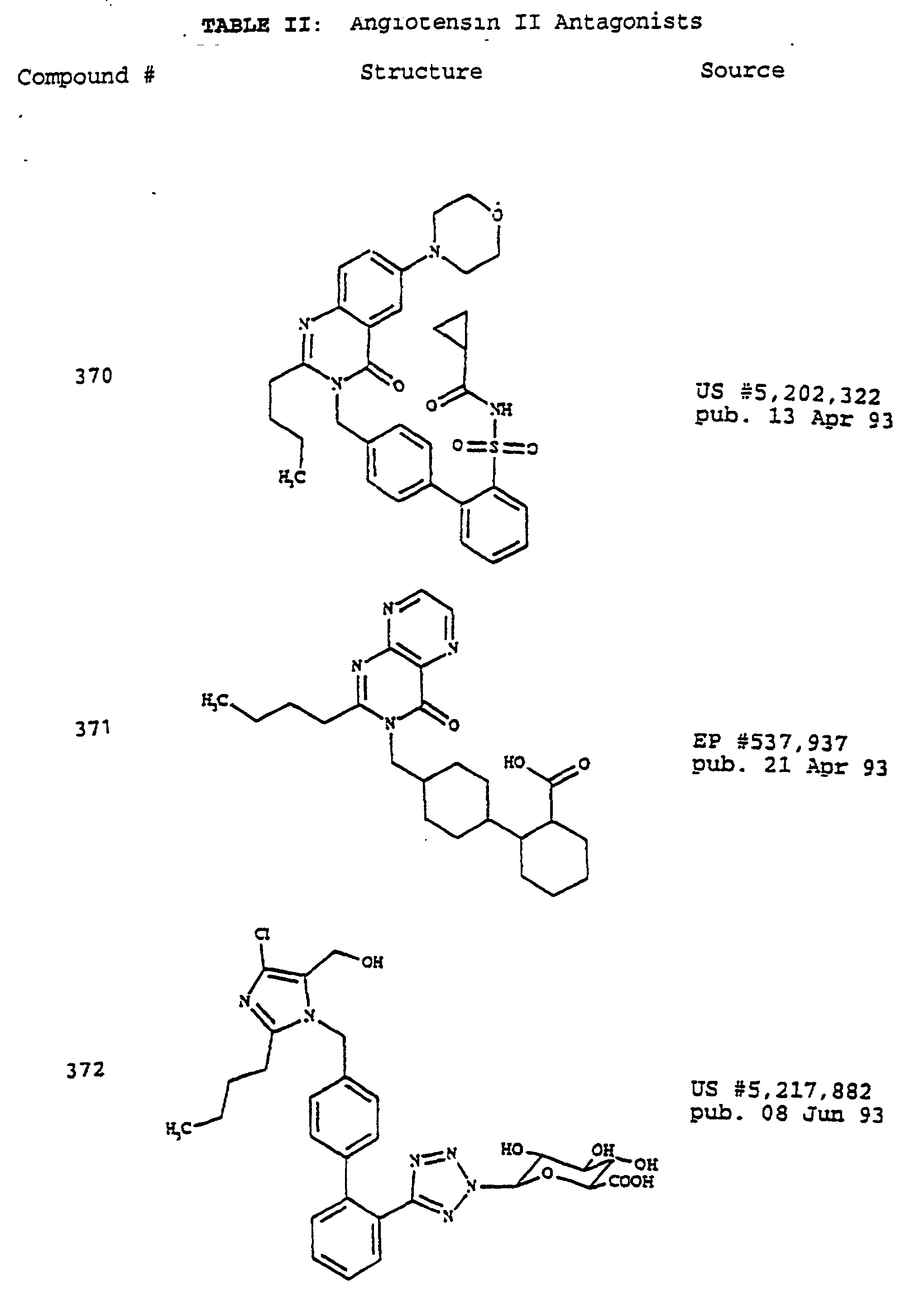

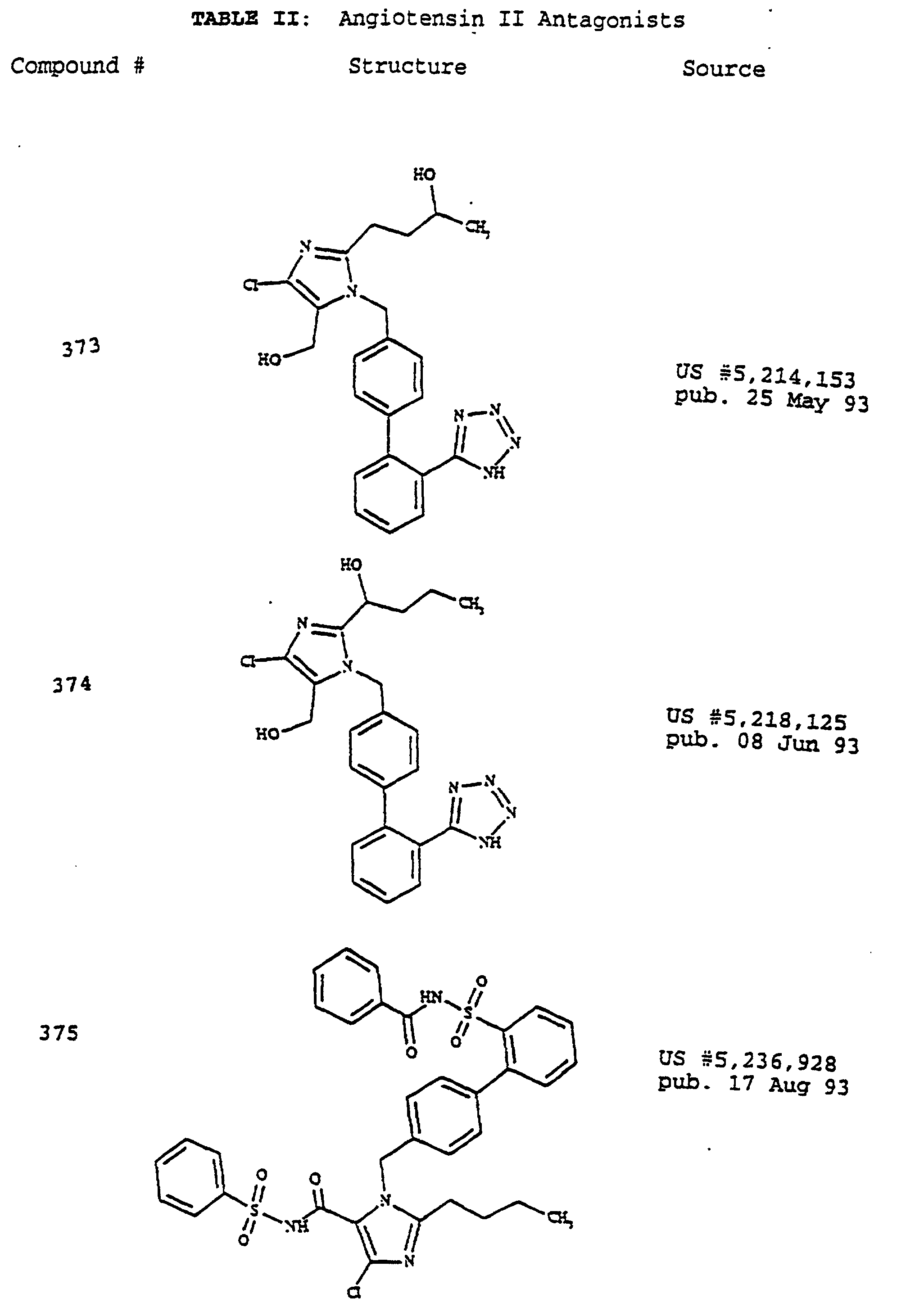

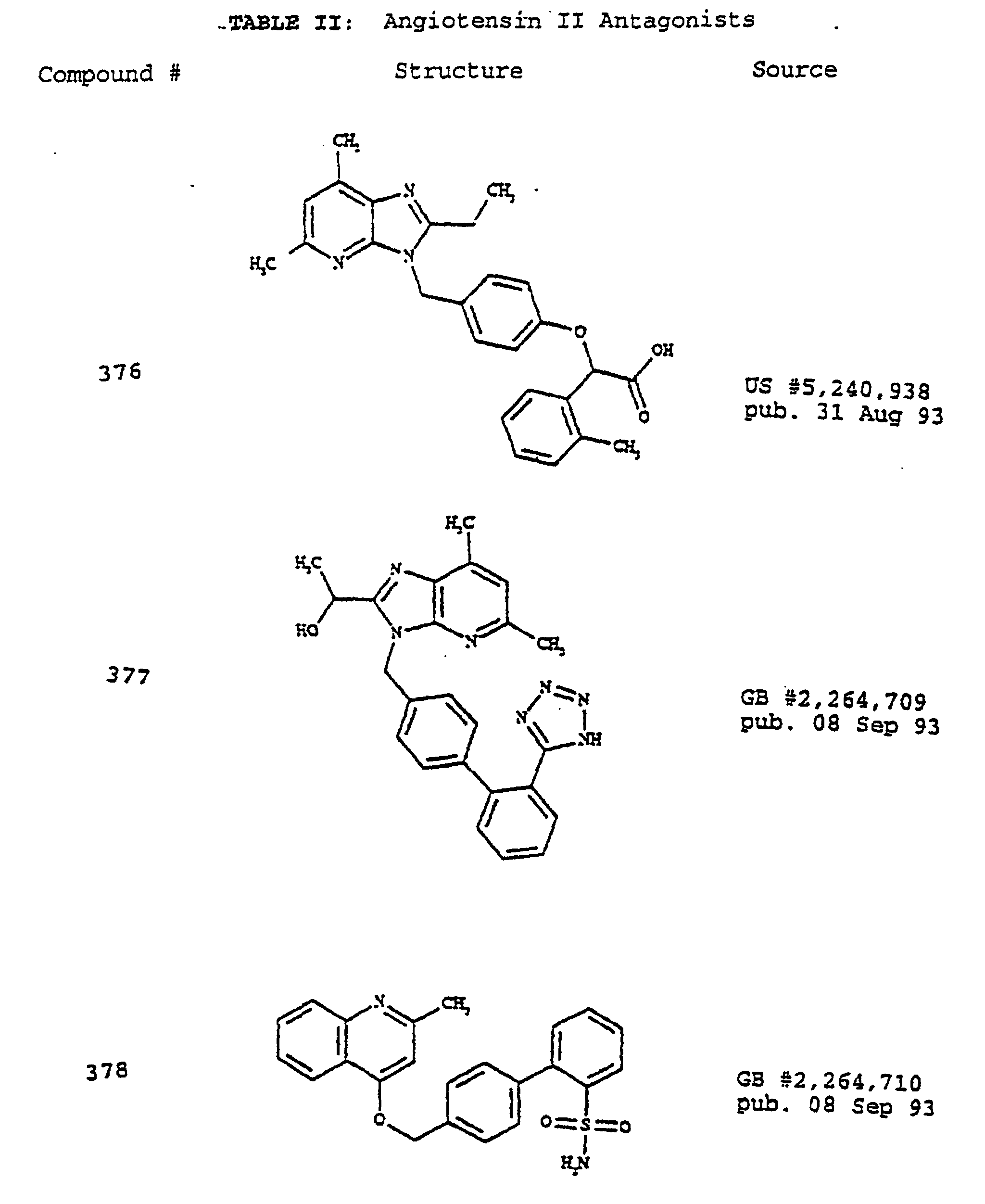

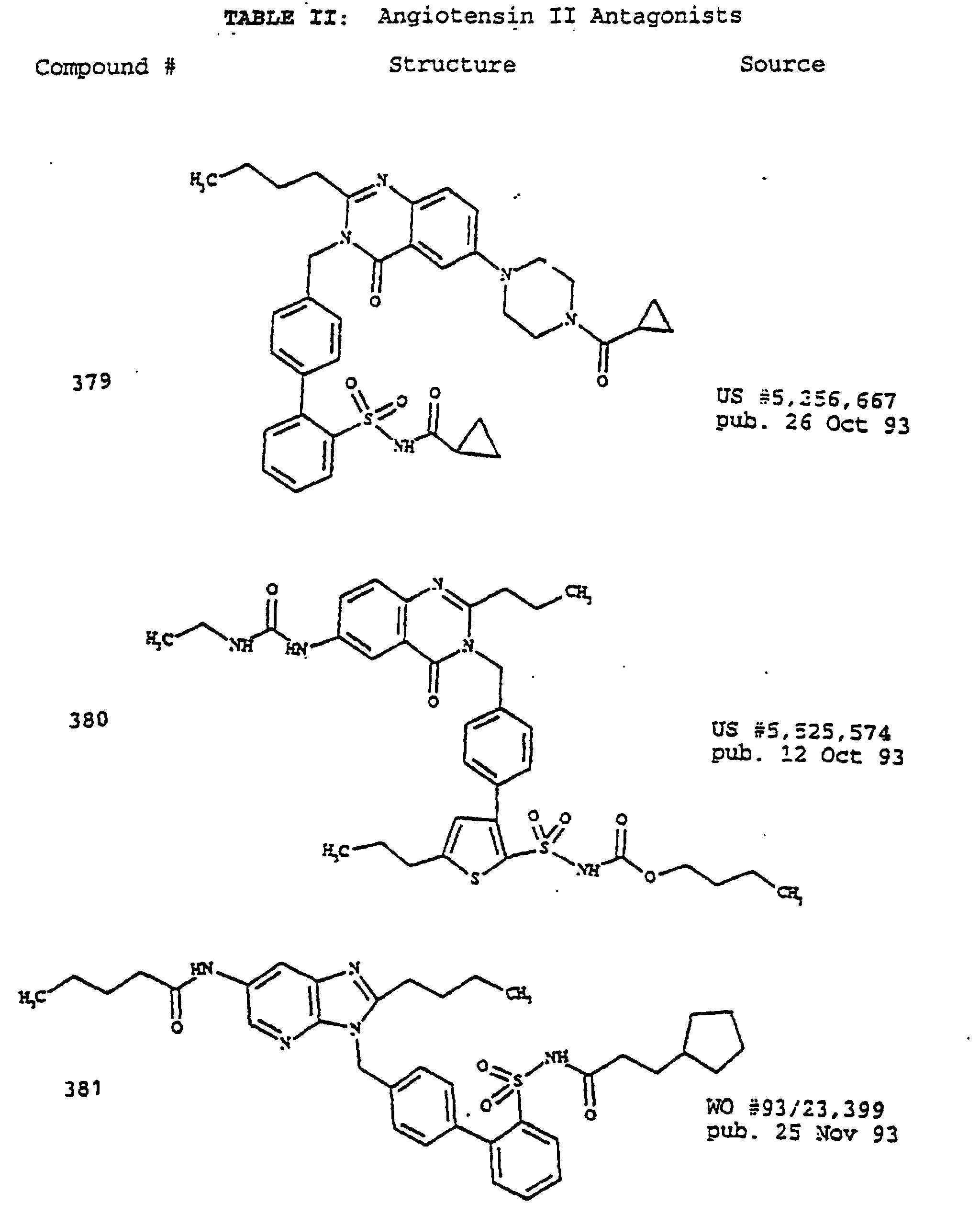

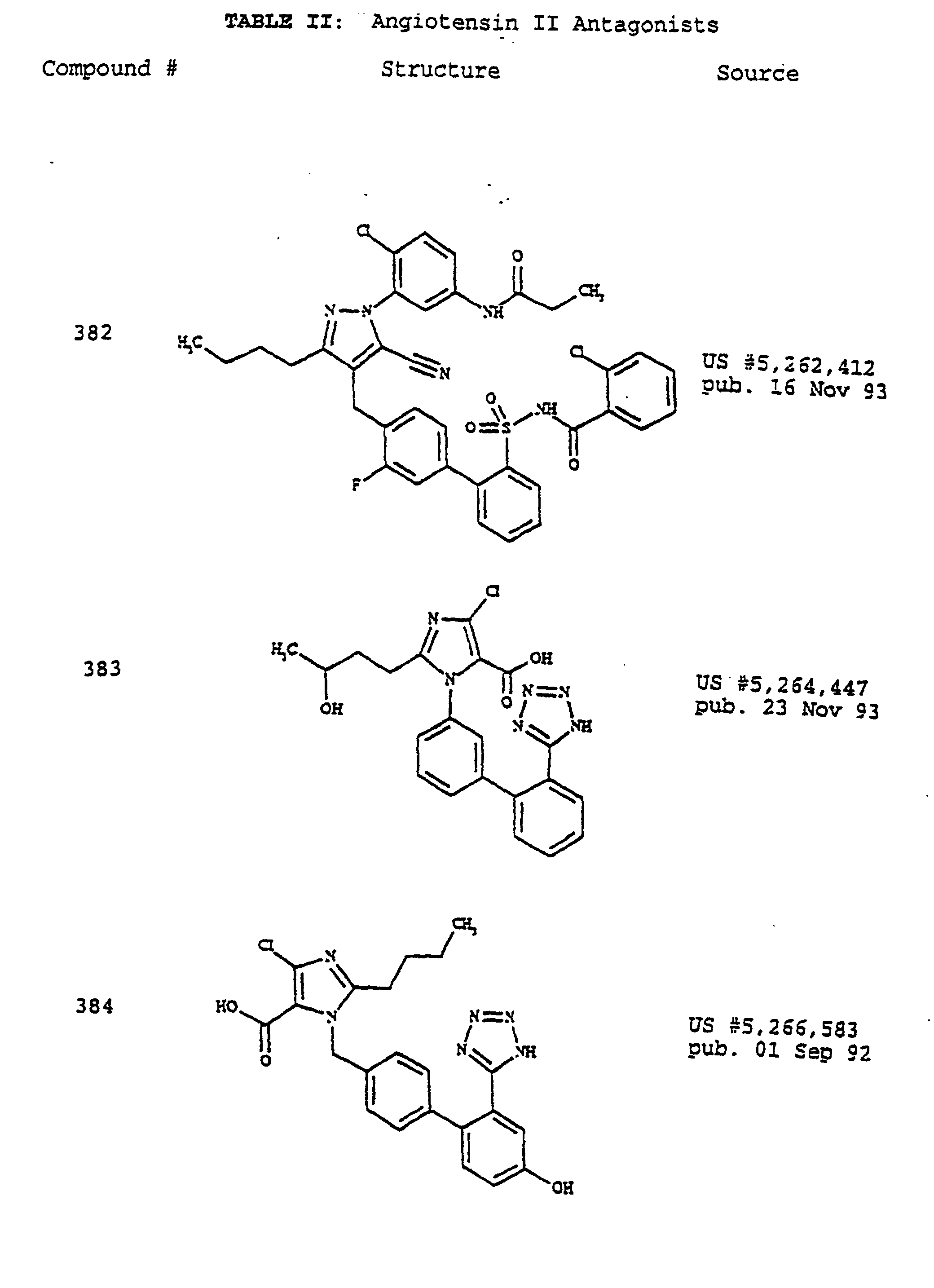

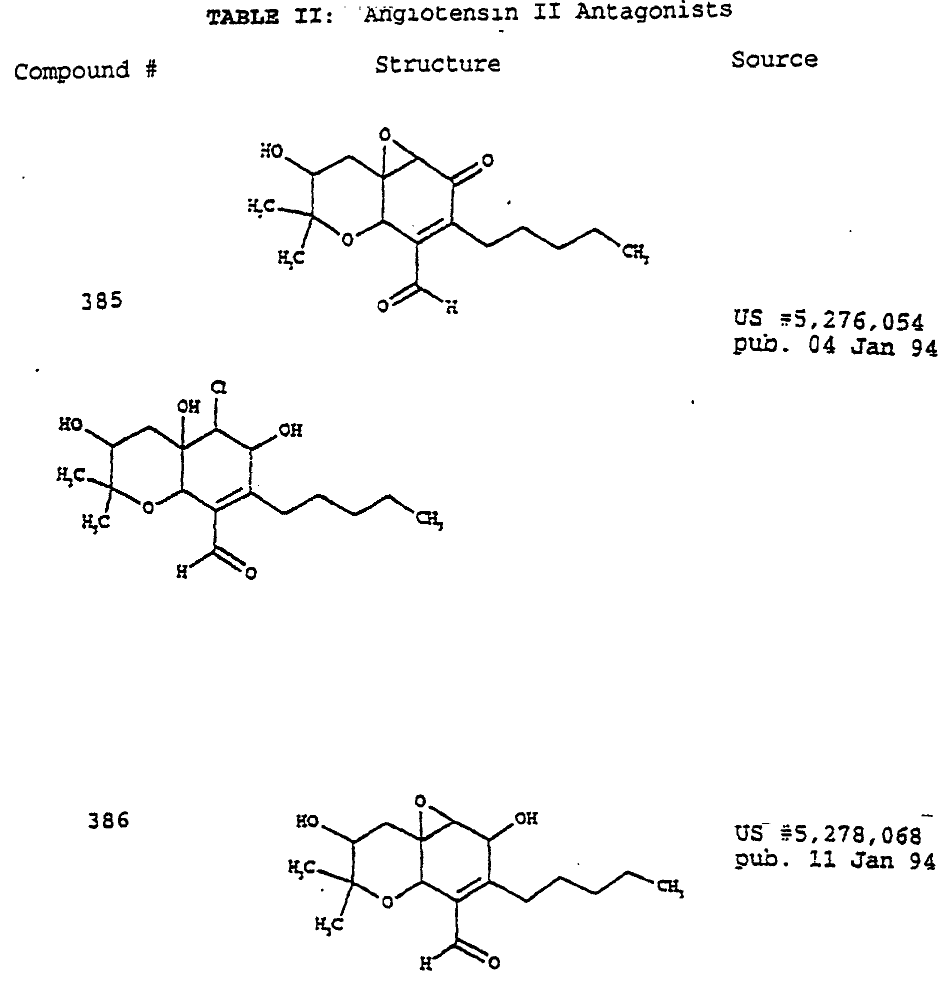

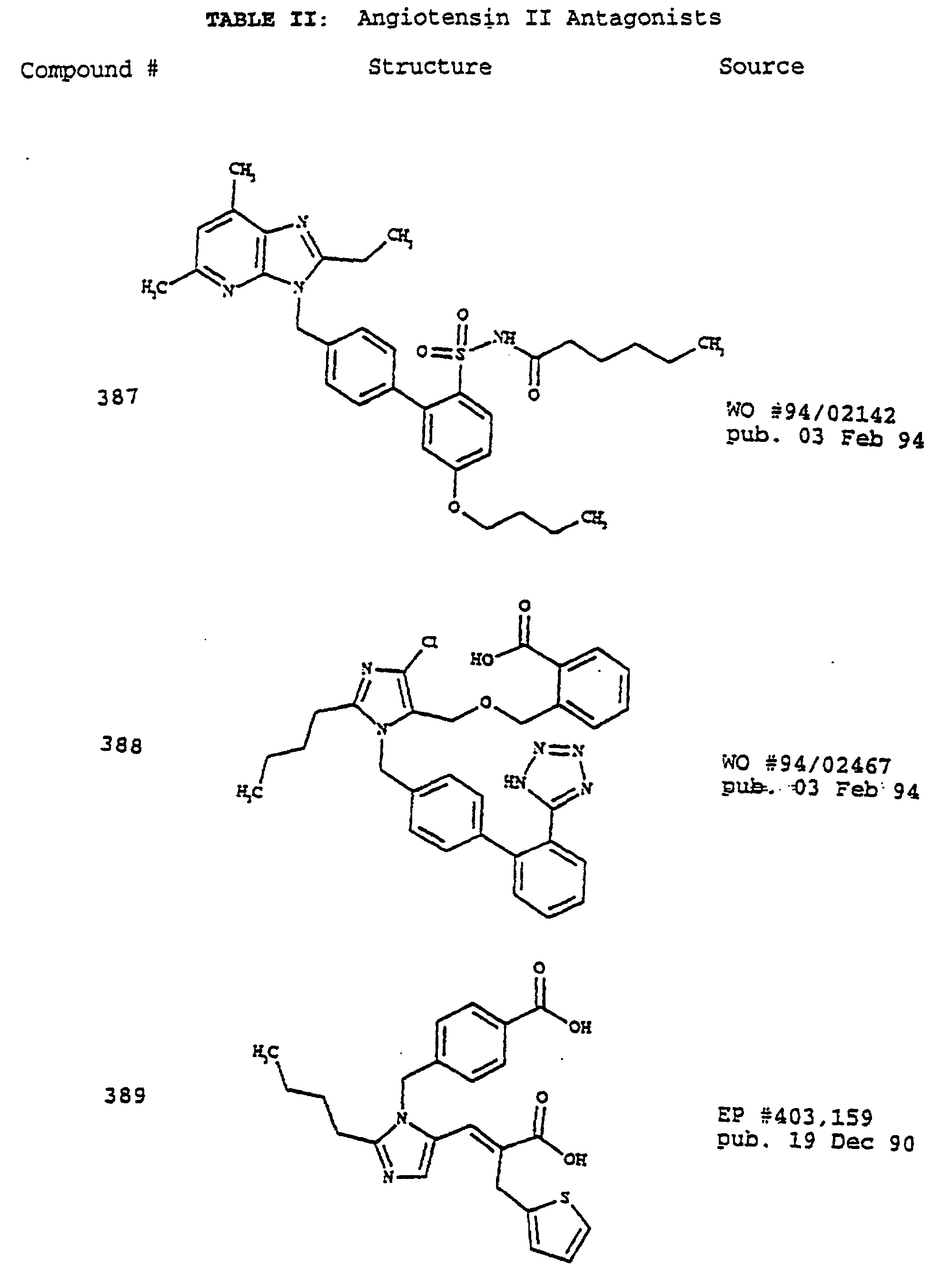

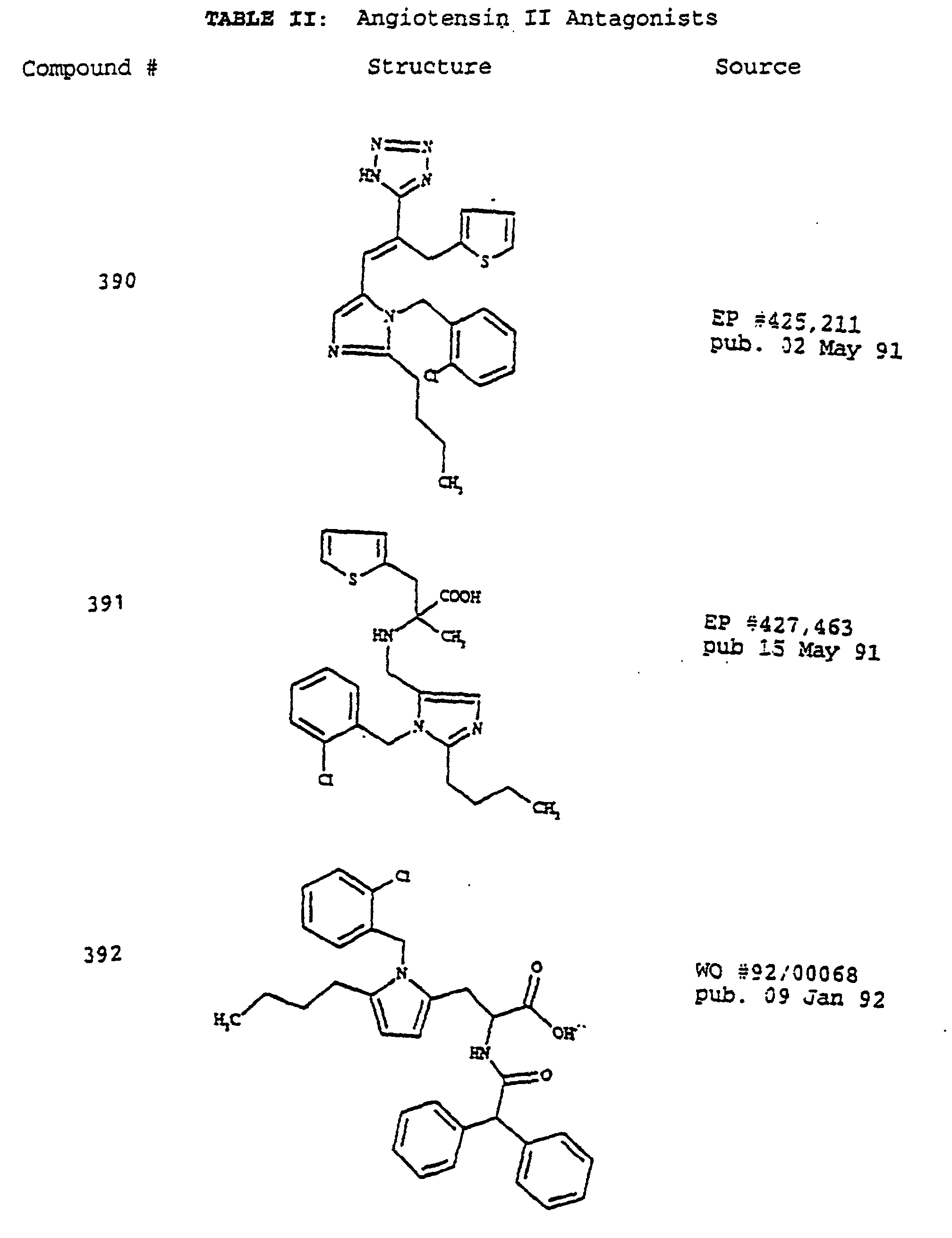

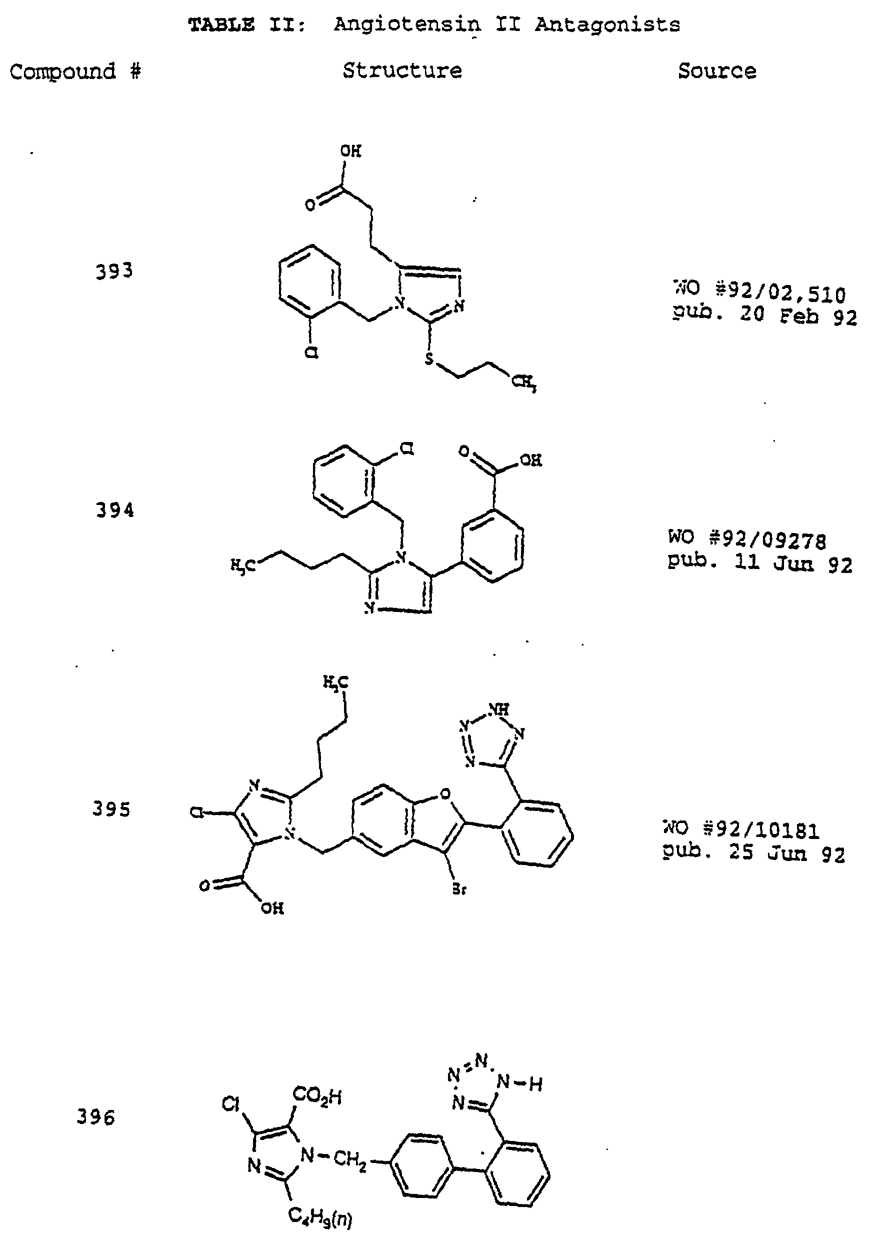

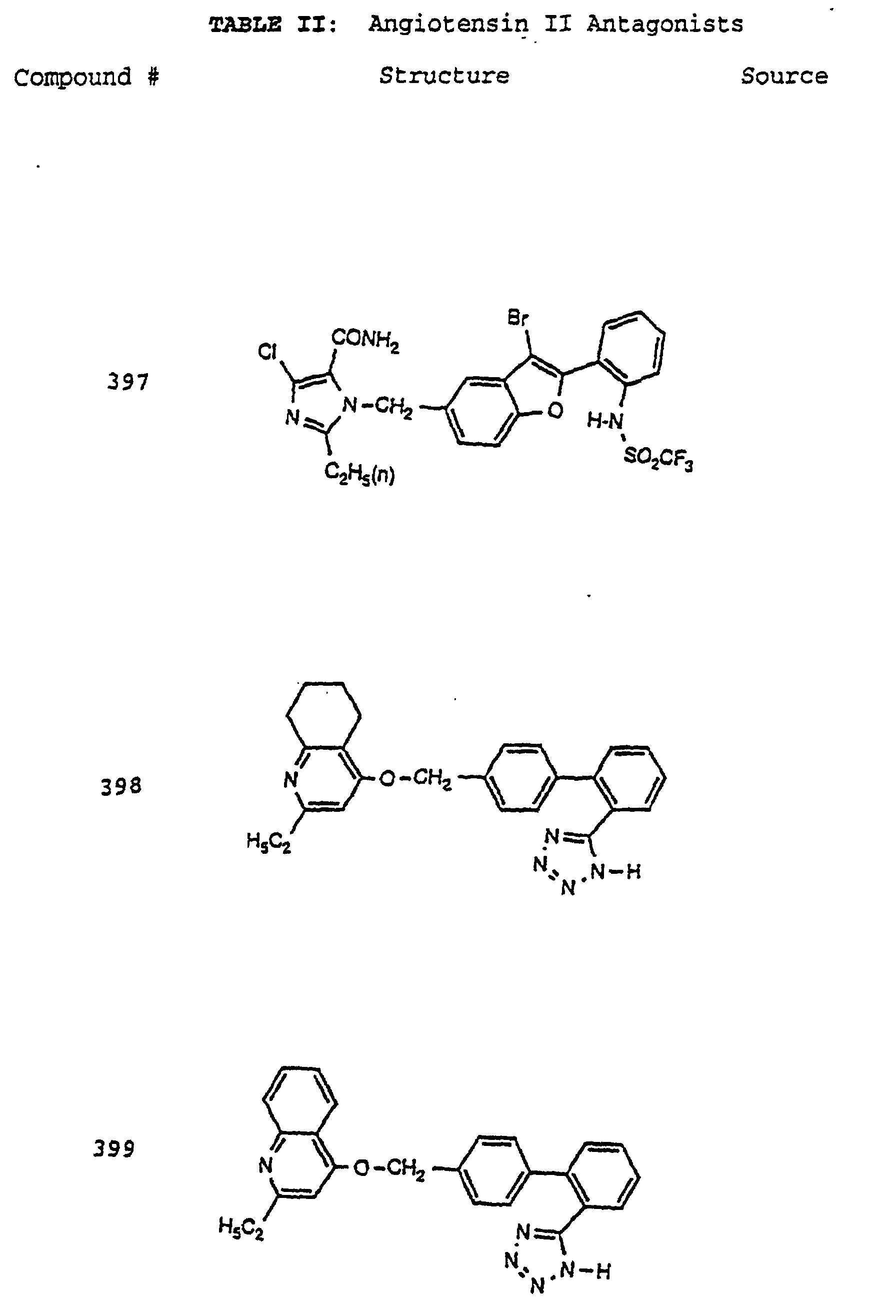

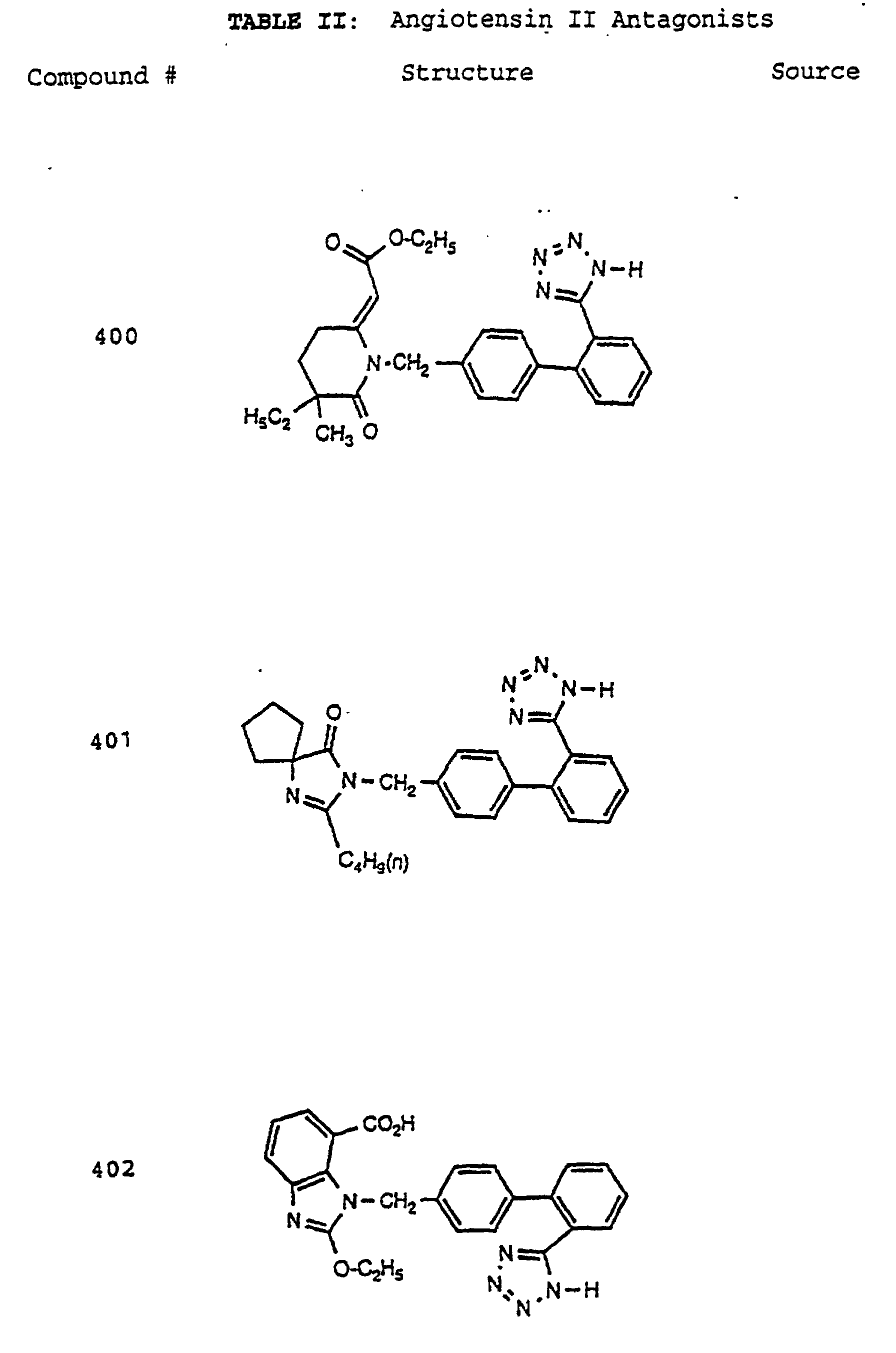

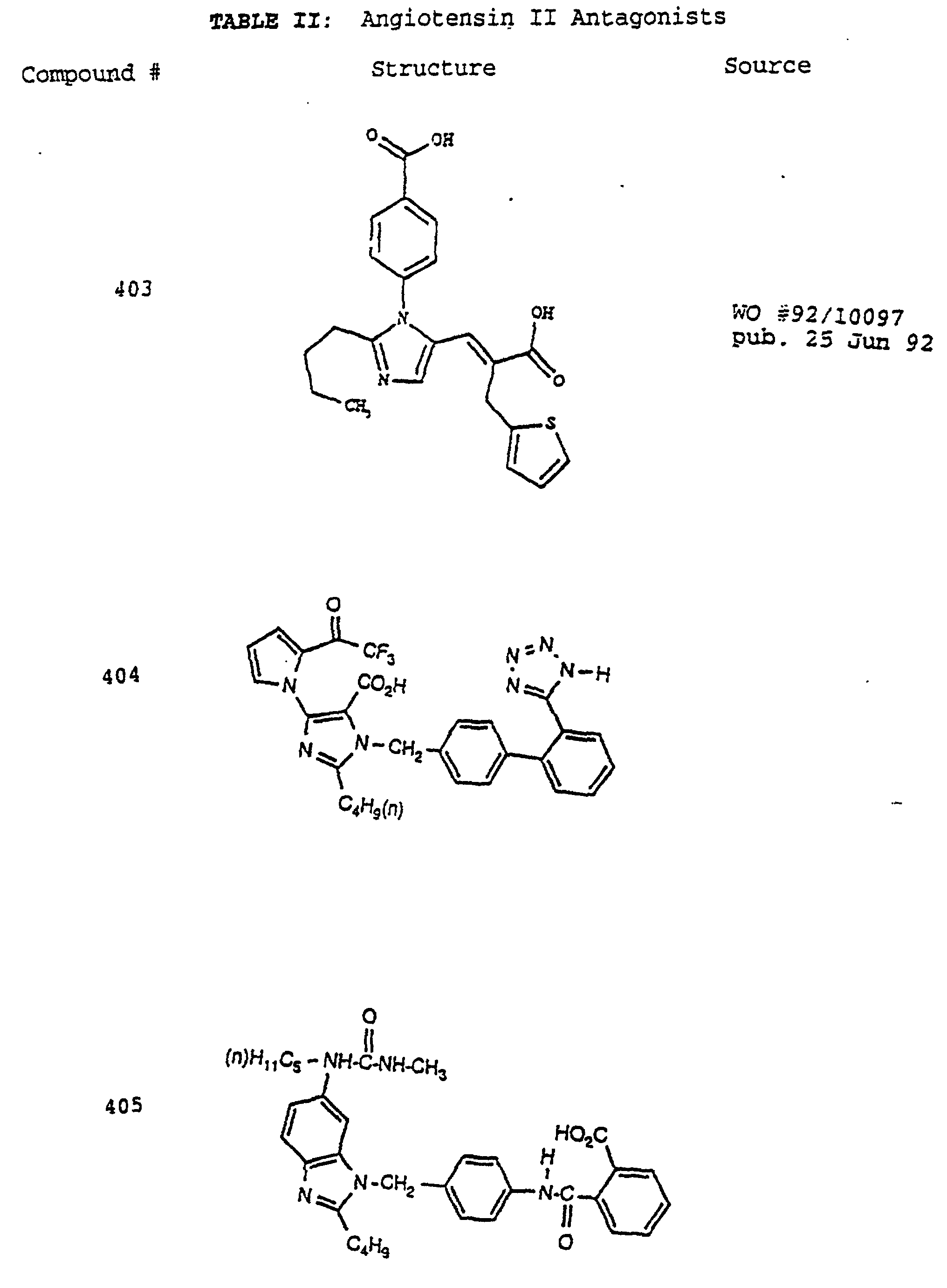

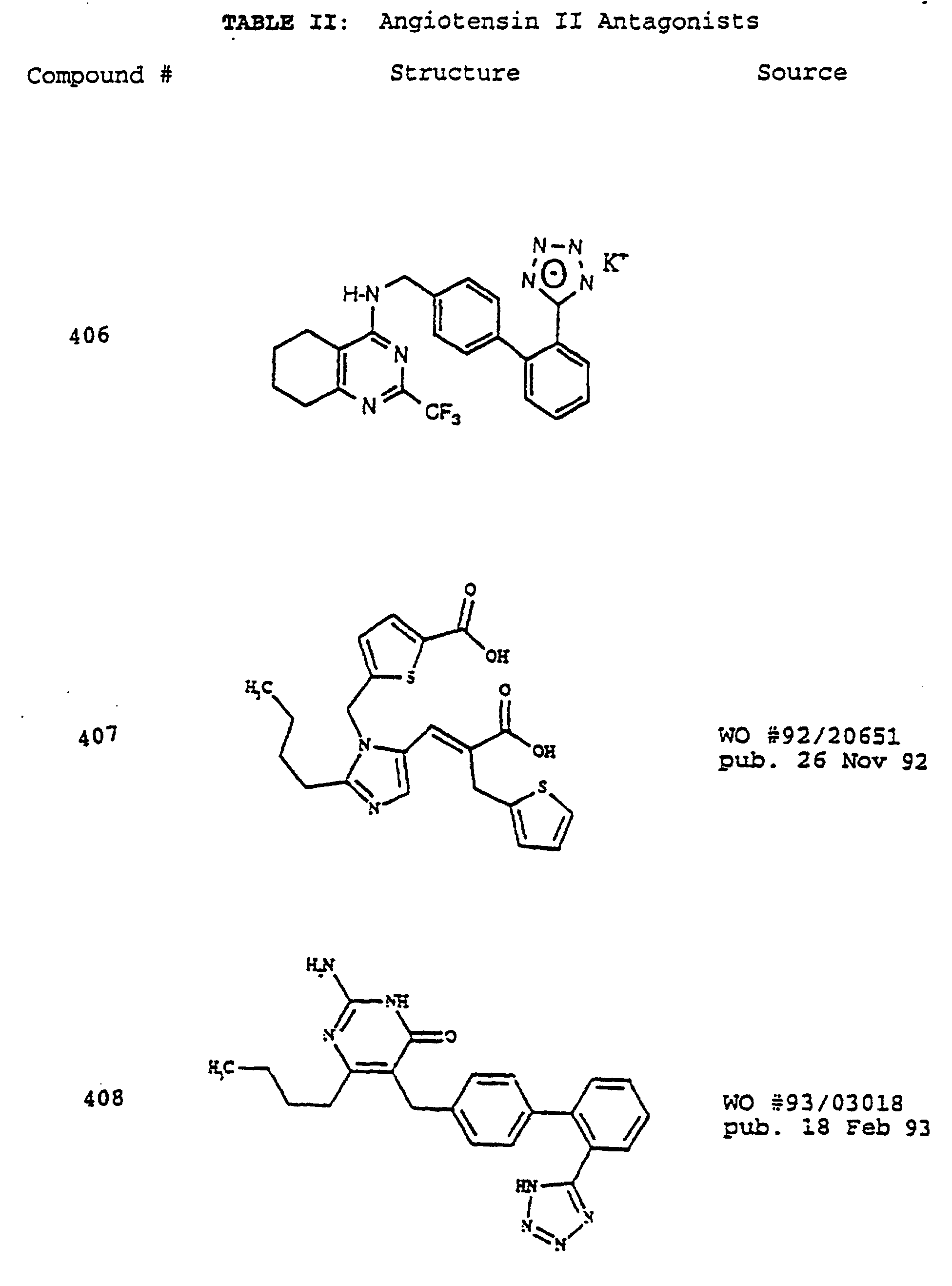

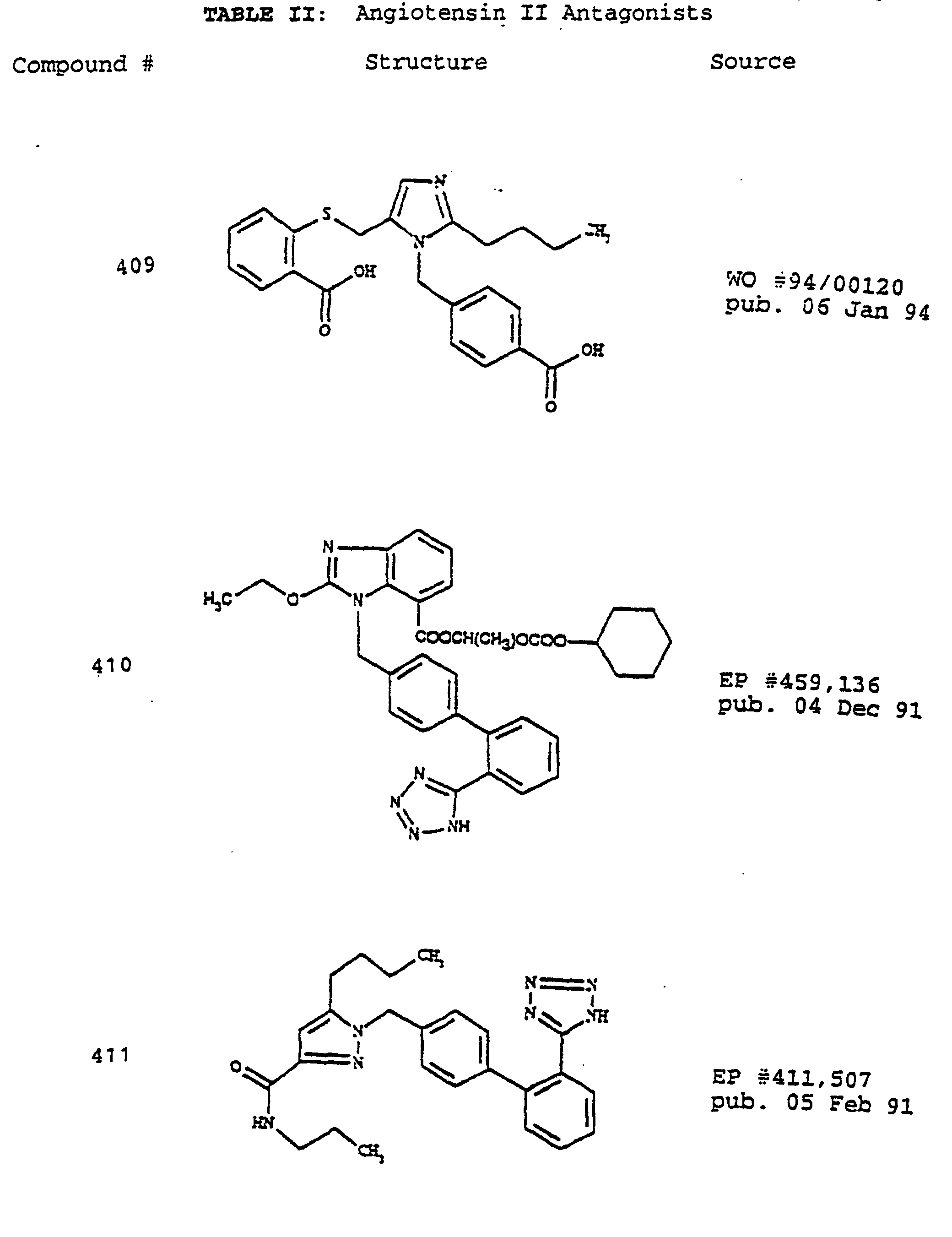

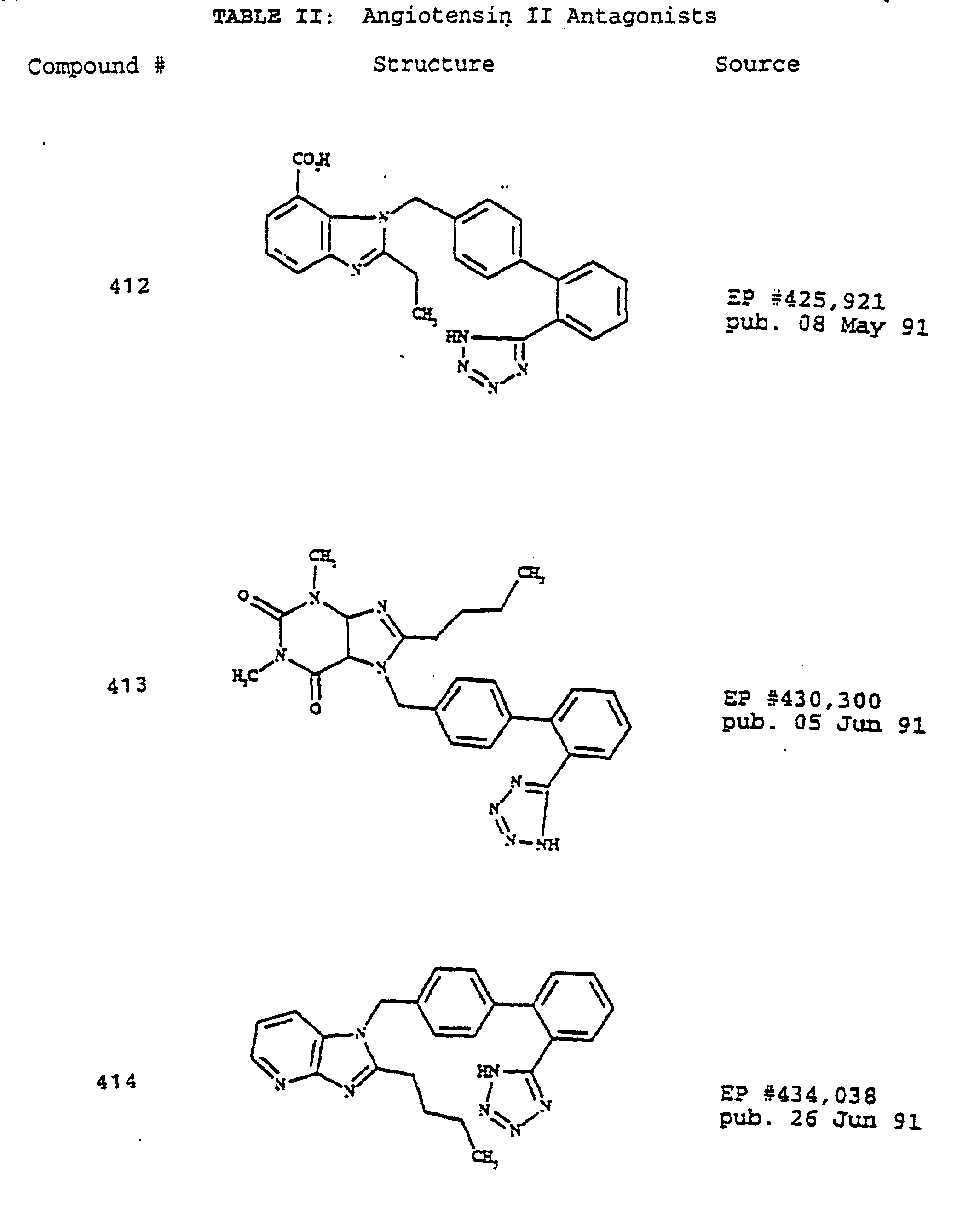

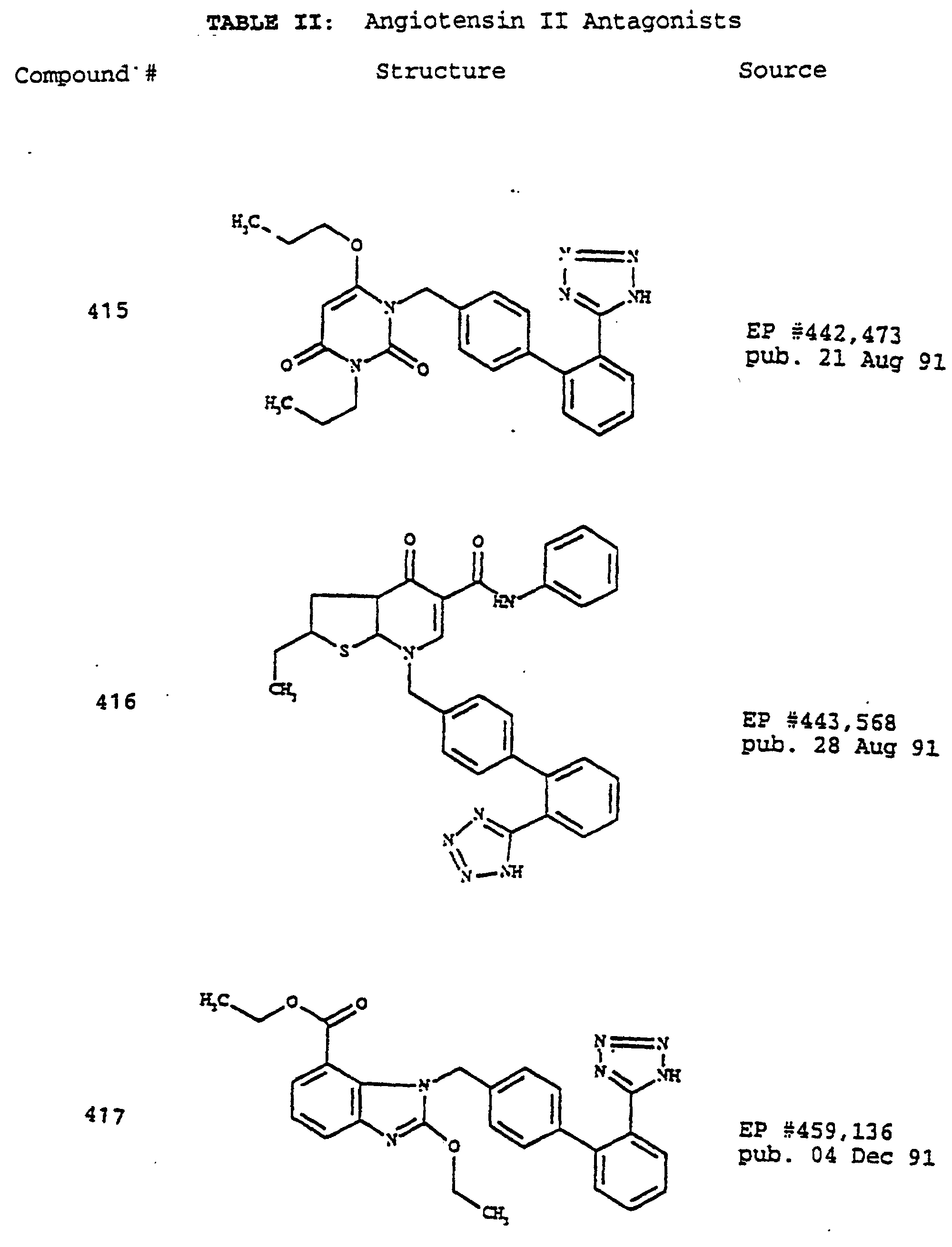

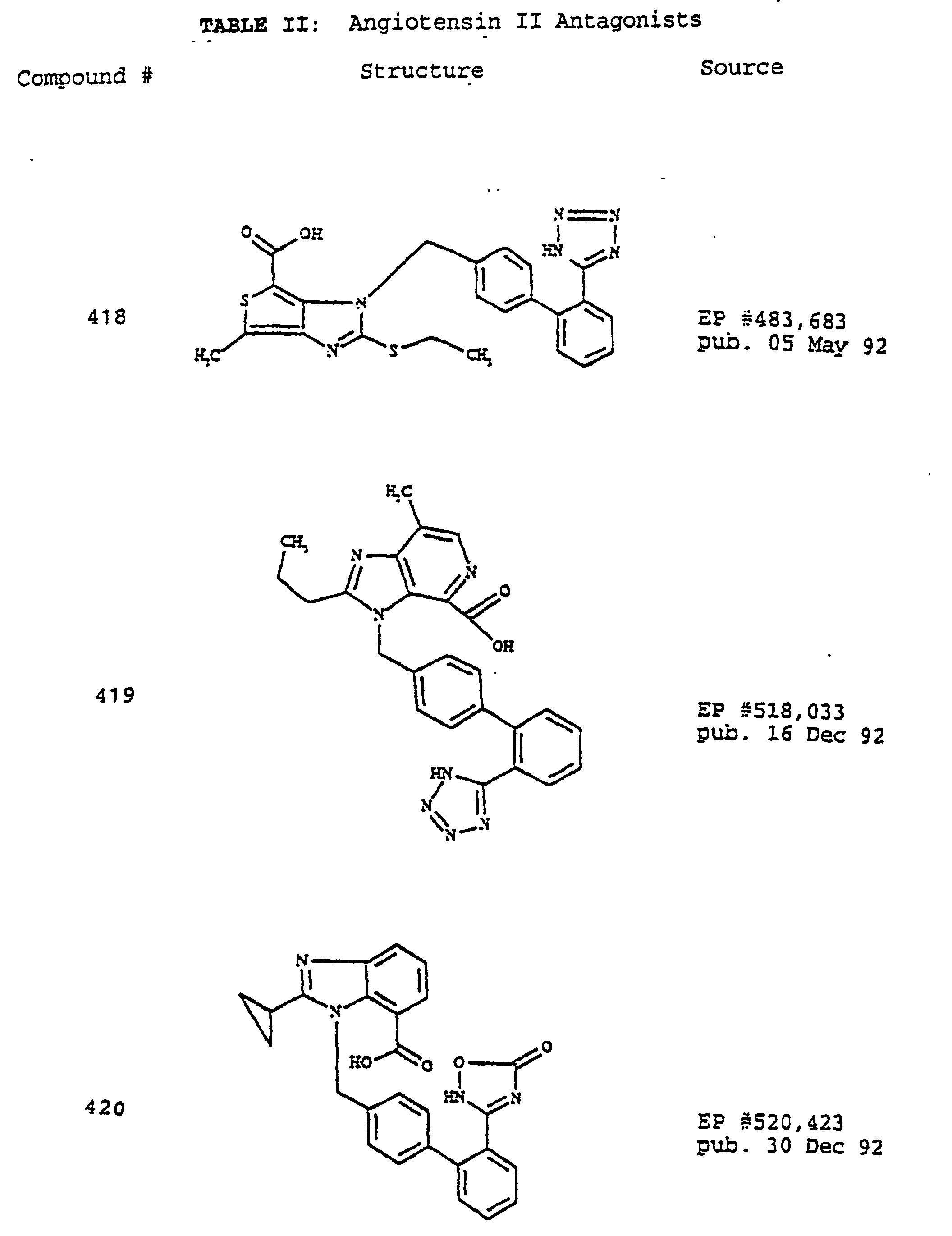

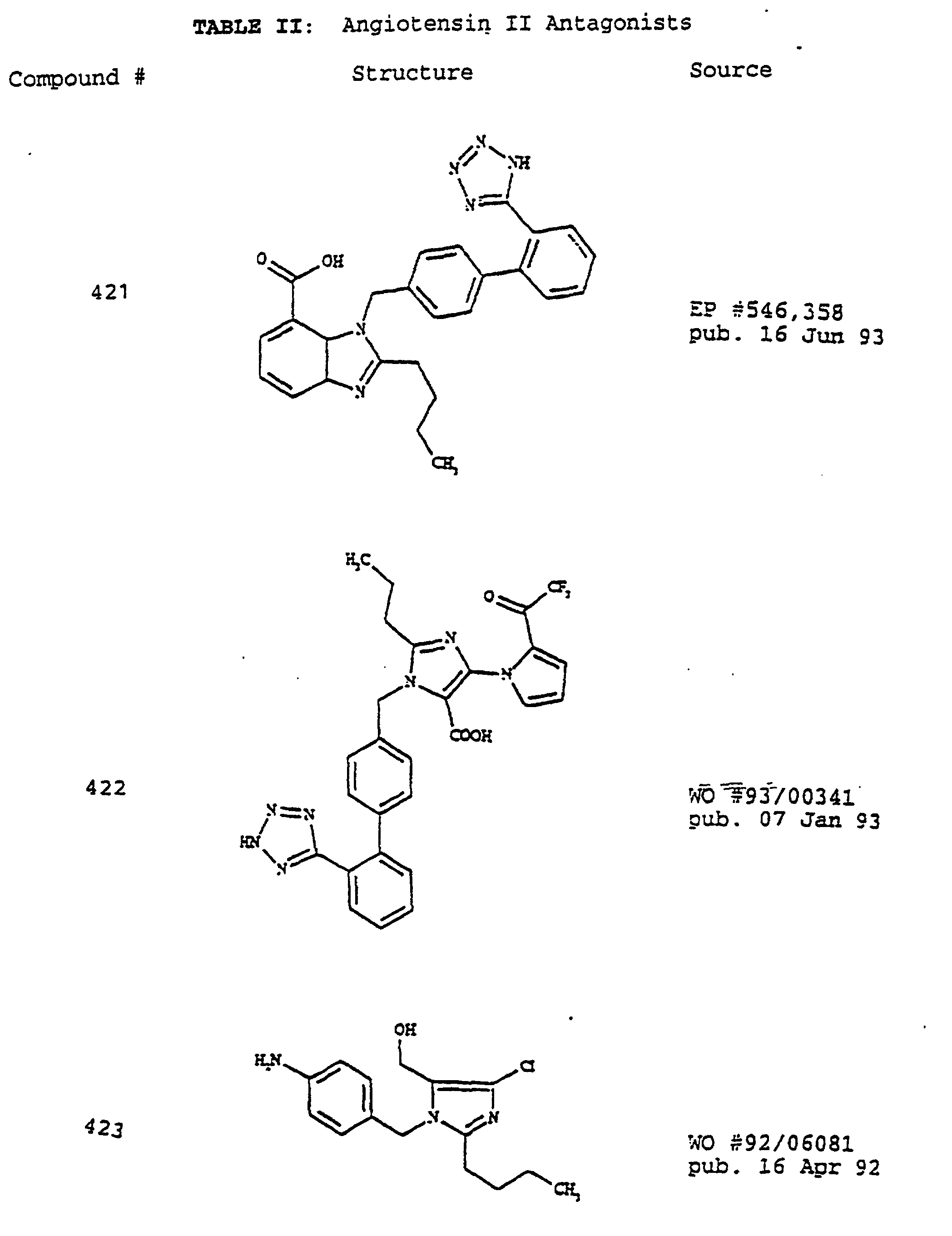

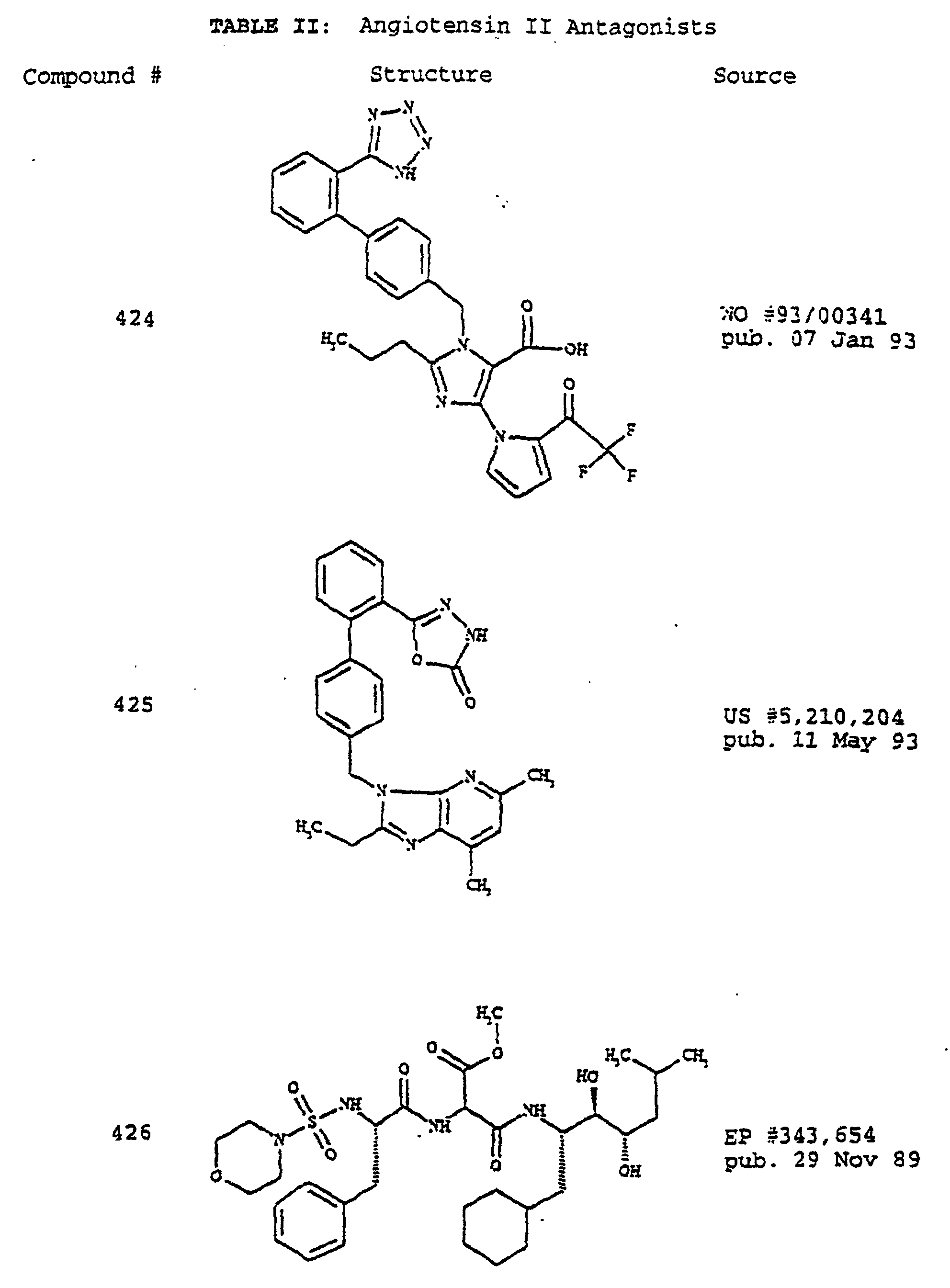

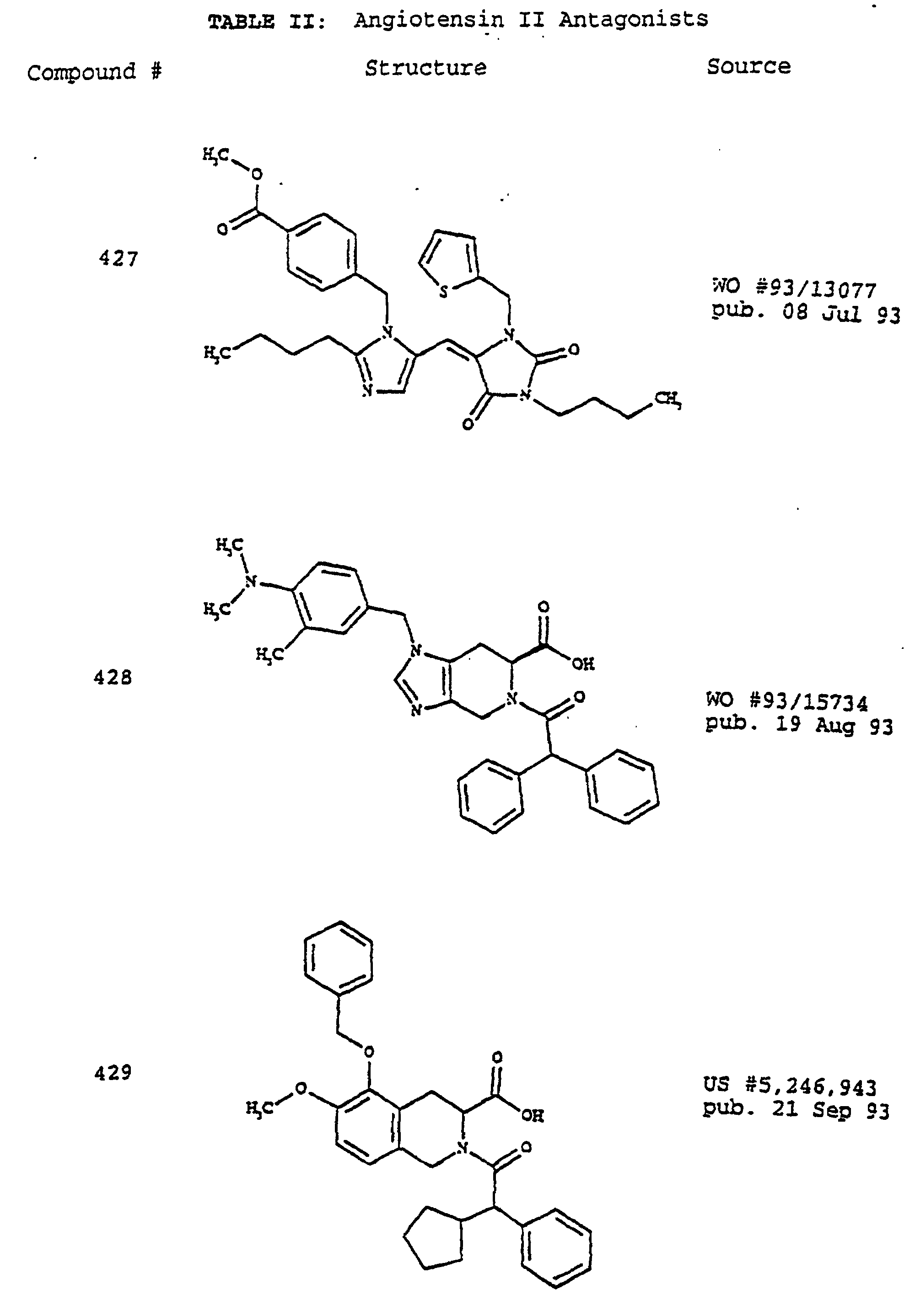

Also included in the combination of the invention are the isomeric forms of the angiotensin II receptor compounds and the spirolactone, including diastereoisomers, regioisomers and the pharmaceutically-acceptable salts thereof. The term "pharmaceutically-acceptable salts" embraces salts commonly used to form alkali metal salts and to form addition salts of free acids or free bases. The nature of the salt is not critical, provided that it is pharmaceutically-acceptable. Suitable pharmaceutically-acceptable acid addition salts may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, example of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, p-hydroxybenzoic, salicyclic, phenylacetic, mandelic, embonic (pamoic), methansulfonic, ethanesulfonic, 2-hydroxyethanesulfonic, pantothenic, benzenesulfonic, toluenesulfonic, sulfanilic, mesylic, cyclohexylaminosulfonic, stearic, algenic, β-hydroxybutyric, malonic, galactaric and galacturonic acid. Suitable pharmaceutically-acceptable base addition salts include metallic salts made from aluminium, calcium, lithium, magnesium, potassium, sodium and zinc or organic salts made from N,N'-dibenzylethylenediamine, chloroprocaine, choline, diethanolamine, ethylenediamine, meglumine (N-methylglucamine) and procaine. All of these salts may be prepared by conventional means from the corresponding compound by reacting, for example, the appropriate acid or base with such compound.

### BIOLOGICAL EVALUATION

Human congestive heart failure (CHF) is a complex condition usually initiated by vascular hypertension or a myocardial infarction (MI). In order to determine the probable effectiveness of a combination therapy for CHF, it is important to determine the potency of individual components of the combination therapy. Accordingly, in Assays "A" through "C", the angiotensin II receptor antagonist profiles were determined for many of the compounds described in Table II, herein. In Assays "D" and "E", there are described methods for evaluating a combination therapy of the invention, namely, an angiotensin II receptor antagonist of Table II and an epoxy-free spirolactone-type aldosterone receptor antagonist. The efficacy of the individual drugs, spironolactone and the angiotensin II receptor blocker, and of these drugs given together at various doses, are evaluated in rodent models of hypertension and CHF using surgical alterations to induce either hypertension or an MI. The methods and results of such assays are described below.

### Assay A: Antiotensin II Binding Activity

Compounds of the combination of the invention were tested for ability to bind to the smooth muscle angiotensin II receptor using a rat uterine membrane preparation. Angiotensin II (AII) was purchased from Peninsula Labs. ¹²⁵I-angiotensin II (specific activity of 2200 Ci/mmol) was purchased from Du Pont-New England Nuclear. Other chemicals were obtained from Sigma Chemical Co. This assay was carried out according to the method of Douglas et al [Endocrinology, 106, 120-124 (1980)]. Rat uterine membranes were prepared from fresh tissue. All procedures were carried out at 4°C. Uteri were stripped of fat and homogenized in phosphate-buffered saline at pH 7.4 containing 5 mM EDTA. The homogenate was centrifuged at 1500 x g for 20 min., and the supernatant was recentrifuged at 100,000 x g for 60 min. The pellet was resuspended in buffer consisting of 2 mM EDTA and 50 mM Tris-HCl (pH 7.5) to a final protein concentration of 4 mg/ml. Assay tubes were charged with 0.25 ml of a solution containing 5 mM MgCl₂, 2 mM EDTA, 0.5% bovine serum albumin, 50 mM Tris-HCl, pH 7.5 and ¹²⁵I-AII (approximately 10⁵ cpm) in the absence or in the presence of unlabelled ligand. The reaction was initiated by the addition of membrane protein and the mixture was incubated at 25°C for 60 min. The incubation was terminated with ice-cold 50 mM Tris-HCl (pH 7.5) and the mixture was filtered to separate membrane-bound labelled peptide from the free ligand. The incubation tube and filter were washed with ice-cold buffer. Filters were assayed for radioactivity in a Micromedic gamma counter. Nonspecific binding was defined as binding in the presence of 10 µM of unlabelled AII. Specific binding was calculated as total binding minus nonspecific binding. The receptor binding affinity of an AII antagonist compound was indicated by the concentration (IC₅₀) of the tested AII antagonist which gives 50% displacement of the total specifically bound ¹²⁵I-AII from the angiotensin II AT₁ receptor. Binding data were analyzed by a nonlinear least-squares curve fitting program. Results are reported in Table III.

### Assay B: In Vitro vascular Smooth Muscle-Response for AII

The compounds of the combination of the invention were tested for antagonist activity in rabbit aortic rings. Male New Zealand white rabbits (2-2.5 kg) were sacrificed using an overdose of pentobarbital and exsanguinated via the carotid arteries. The thoracic aorta was removed, cleaned of adherent fat and connective tissue and then cut into 3-mm ring segments. The endothelium was removed from the rings by gently sliding a rolled-up piece of filter paper into the vessel lumen. The rings were then mounted in a water-jacketed tissue bath, maintained at 37°C, between moveable and fixed ends of a stainless steel wire with the moveable end attached to an FT03 Grass transducer coupled to a Model 7D Grass Polygraph for recording isometric force responses. The bath was filled with 20 ml of oxygenated (95% oxygen/5% carbon dioxide) Krebs solution of the following composition (mM) : 130 NaCl, 15 NaHCO₃, 15 KCl, 1.2 NaH₂PO₄, 1.2 MgSO₄, 2.5 CaCl₂, and 11.4 glucose. The preparations were equilibrated for one hour before approximately one gram of passive tension was placed on the rings. Angiotensin II concentration-response curves were then recorded (3 X 10⁻¹⁰ to 1 X 10⁻⁵ M). Each concentration of AII was allowed to elicit its maximal contraction, and then AII was washed out repeatedly for 30 minutes before rechallenging with a higher concentration of AII. Aorta rings were exposed to the test antagonist at 10⁻⁵ M for 5 minutes before challenging with AII. Adjacent segments of the same aorta ring were used for all concentration-response curves in the presence or absence of the test antagonist. The effectiveness of the test compound was expressed in terms of pA₂ values and were calculated according to H.O. Schild [Br. J. Pharmacol. Chemother., 2, 189-206 (1947)]. The pA₂ value is the concentration of the antagonist which increases the EC₅₀ value for AII by a factor of two. Each test antagonist was evaluated in aorta rings from two rabbits. Results are reported in Table III.

### Assay C: In Vivo Intragastric Pressor Assay Response for All Antagonists

Male Sprague-Dawley rats weighing 225-300 grams were anesthetized with methohexital (30 mg/kg, i.p.) and catheters were implanted into the femoral artery and vein. The catheters were tunneled subcutaneously to exit dorsally, posterior to the head and between the scapulae. The catheters were filled with heparin (1000 units/ml of saline). The rats were returned to their cage and allowed regular rat chow and water ad libitum. After full recovery from surgery (3-4 days), rats were placed in Lucite holders and the arterial line was connected to a pressure transducer. Arterial pressure was recorded on a Gould polygraph (mmHg). Angiotensin II was administered as a 30 ng/kg bolus via the venous catheter delivered in a 50 µl volume with a 0.2 ml saline flush. The pressor response in mm Hg was measured by the difference from pre-injection arterial pressure to the maximum pressure achieved. The AII injection was repeated every 10 minutes until three consecutive injections yielded responses within 4 mmHg of each other. These three responses were then averaged and represented the control response to AII. The test compound was suspended in 0.5% methylcellulose in water and was administered by gavage. The volume administered was 2 ml/kg body weight. The standard dose was 3 mg/kg. Angiotensin II bolus injections were given at 30, 45, 60, 75, 120, 150, and 180 minutes after gavage. The pressor response to AII was measured at each time point. The rats were then returned to their cage for future testing. A minimum of 3 days was allowed between tests. Percent inhibition was calculated for each time point following gavage by the following formula: [(Control Response - Response at time point)/Control Response] X 100. Results are shown in Table III.

### Assay "D" : Hypertensive Rat Model

Male rats are made hypertensive by placing a silver clip with an aperture of 240 microns on the left renal artery, leaving the contralateral kidney untouched. Sham controls undergo the same procedure but without attachment of the clip. One week prior to the surgery, animals to be made hypertensive are divided into separate groups and drug treatment is begun. Groups of animals are administered vehicle, AII antagonist alone, spironolactone alone, and combinations of AII antagonist and spironolactone, at various doses, as follow:

| AII Antagonist | Spironolactone | Combination of AII Antagonist & Spironolactone | |
|---|---|---|---|
| (mg/kg/day) | (mg/kg/day) | (mg/kg/day) | (mg/kg/day) |
| 3 | 5 | 3 | 5 |
| | 20 | 3 | 20 |
| | 50 | 3 | 50 |
| | 100 | 3 | 100 |
| | 200 | 3 | 200 |
| 10 | 5 | 10 | 5 |
| | 20 | 10 | 20 |
| | 50 | 10 | 50 |
| | 100 | 10 | 100 |
| | 200 | 10 | 200 |
| 30 | 5 | 30 | 5 |
| | 20 | 30 | 20 |
| | 50 | 30 | 50 |
| | 100 | 30 | 100 |
| | 200 | 30 | 200 |

After 12 to 24 weeks, systolic and diastolic blood pressure, left ventricular end diastolic pressure, left ventricular dP/dt, and heart rate are evaluated. The hearts are removed, weighed, measured and fixed in formalin. Collagen content of heart sections are evaluated using computerized image analysis of picrosirius stained sections. It would be expected that rats treated with a combination therapy of AII antagonist and spironolactone components, as compared to rats treated with either component alone, will show improvements in cardiac performance.

### Assay "E": Myocardial Infarction Rat Model:

Male rats are anesthetized and the heart is exteriorized following a left sided thoracotomy. The left anterior descending coronary artery is ligated with a suture. The thorax is closed and the animal recovers. Sham animals have the suture passed through without ligation. One week prior to the surgery, animals to undergo infarction are divided into separate groups and drug treatment is begun. Groups of animals are administered vehicle, AII antagonist alone, spironolactone alone, and combinations of AII antagonist and spironolactone, at various doses, as follow:

| AII Antagonist | Spironolactone | Combination of AII Antagonist & Spironolactone | |
|---|---|---|---|
| (mg/kg/day) | (mg/kg/day) | (mg/kg/day) | (mg/kg/day) |
| 3 | 5 | 3 | 5 |
| | 20 | 3 | 20 |
| | 50 | 3 | 50 |
| | 100 | 3 | 100 |
| | 200 | 3 | 200 |
| 10 | 5 | 10 | 5 |
| | 20 | 10 | 20 |
| | 50 | 10 | 50 |
| | 100 | 10 | 100 |
| | 200 | 10 | 200 |
| 30 | 5 | 30 | 5 |
| | 20 | 30 | 20 |
| | 50 | 30 | 50 |
| | 100 | 30 | 100 |
| | 200 | 30 | 200 |

After six weeks, systolic and diastolic blood pressure, left ventricular end diastolic pressure, left ventricular dP/dt, and heart rate are evaluated. The hearts are removed, weighed, measured and fixed in formalin. Collagen content of heart sections are evaluated using computerized image analysis of picrosirius stained sections. It would be expected that rats treated with a combination therapy of AII antagonist and spironolactone components, as compared to rats treated with either component alone, will show improvements in cardiac performance.

**TABLE III**

| In Vivo and In Vitro Angiotensin II Activity of Compounds | | | | | |
|---|---|---|---|---|---|
| Test Compound | ¹Assay A IC₅₀ | ²Assay B pA₂ | | ³Assay C | |
| Example # | (nM) | | Dose (mg/kg) | Inhibition (%) | Duration (min.) |
| 1 | NT | NT | NT | NT | NT |
| 2 | 95 | 7.37/7.59 | 10 | 95 | 60 |
| | | | 30 | 98 | 90-120 |
| 3 | 5.4 | 8.70 ± 0.2 | 10 | 50 | >180 |
| | | | 30 | 100 | 200⁺ |
| 4 | NT | NT | NT | NT | NT |
| 5 | 200 | 7.48/6.91 | 30 | 38 | 20-30 |
| 6 | 1300 | 6.55/6.82 | 100 | 90 | 120 |
| 7 | 84 | 8.01/8.05 | 30 | 90 | 130 |
| 8 | 17,000 | NT | NT | NT | NT |
| 9 | 700 | 6.67/6.12 | 30 | 80 | 75 |
| | | | 100 | 100 | 130 |
| 10 | 4.9 | 8.19/7.59 | 3 | 86 | 100 |
| | | | 30 | 100 | 240 |
| 11 | 160 | 6.45/6.77 | NT | NT | NT |
| 12 | 6.0 | 8.66/8.59 | NT | NT | NT |
| 13 | 17 | 8.70/8.85 | NT | NT | NT |
| 14 | 7.2 | 8.84/8.71 | NT | NT | NT |
| 15 | 16 | 8.31/8.30 | NT | NT | NT |
| 16 | 6.4 | 8.95/9.24 | NT | NT | NT |
| 17 | 4.0 | 8.64/8.40 | NT | NT | NT |
| 18 | 970 | 6.14/6.09 | NT | NT | NT |
| 19 | 12,000 | 5.18/5.35 | NT | NT | NT |
| 20 | 78,000 | 5.89/5.99 | 100 | 10 | 45 |
| 21 | 87 | 7.71.7.21 | NT | NT | NT |
| 22 | 460 | 6.60/6.46 | NT | NT | NT |
| 23 | 430 | 6.48/7.15 | NT | NT | NT |
| 24 | 10 | 7.56/7.73 | NT | NT | NT |
| 25 | 480 | 6.80/6.73 | NT | NT | NT |
| 26 | 3.2 | 9.83/9.66 | 10 | 50 | >180 |
| 27 | 180 | NT | NT | NT | NT |
| 28 | 570 | 5.57/6.00 | NT | NT | NT |
| 29 | 160 | NT | NT | NT | NT |
| 30 | 22 | 7.73/7.88 | 30 | 50 | >180 |
| 31 | 14 | NT | NT | NT | NT |
| 32 | 16 | 7.68/7.29 | NT | NT | NT |
| 33 | 630 | 6.73/6.36 | NT | NT | NT |
| 34 | 640 | 5.34/5.69 | NT | NT | NT |
| 35 | 41 | 7.25/7.47 | NT | NT | NT |
| 36 | 1400 | 5.92/5.68 | NT | NT | NT |
| 37 | 340 | 6.90/6.85 | NT | NT | NT |
| 38 | 10 | 7.82/8.36 | NT | NT | NT |
| 39 | 10 | 7.88/7.84 | NT | NT | NT |
| 40 | 83 | 7.94/7.61 | NT | NT | NT |
| 41 | 3700 | 5.68/5.96 | NT | NT | NT |
| 42 | 370 | 6.56/6.26 | NT | NT | NT |
| 43 | 19 | 8.97/8.61 | NT | NT | NT |
| 44 | 16 | 8.23/7.70 | NT | NT | NT |
| 45 | 4.4 | 8.41/8.24 | NT | NT | NT |
| 46 | 110 | 6.80/6.64 | NT | NT | NT |
| 47 | 21 | 7.85/7.58 | NT | NT | NT |
| 48 | 680 | 6.27/6.75 | NT | NT | NT |
| 49 | 120 | 7.06/7.07 | NT | NT | NT |
| 50 | 54 | 7.71/7.89 | NT | NT | NT |
| 51 | 8.7 | 8.39/8.51 | NT | NT | NT |
| 52 | 100 | 8.14/8.12 | NT | NT | NT |
| 53 | 65 | 7.56/7.83 | NT | NT | NT |
| 54 | 3100 | 6.02 | NT | NT | NT |
| 55 | 80 | 6.56/7.13 | NT | NT | NT |
| 56 | 5.0 | 9.04/8.35 | NT | NT | NT |
| 57 | 2300 | 6.00 | NT | NT | NT |
| 58 | 140 | 6.45/6.57 | NT | NT | NT |
| 59 | 120 | 7.23/7.59 | NT | NT | NT |
| 60 | 2200 | 6.40/6.03 | NT | NT | NT |
| 61 | 110 | 7.29/7.70 | NT | NT | NT |
| 62 | 26 | 8.69/8.61 | NT | NT | NT |
| 63 | 61 | 7.77/7.67 | NT | NT | NT |
| 64 | 54 | 7.00/6.77 | NT | NT | NT |
| 65 | 23 | 7.85/7.75 | NT | NT | NT |
| 66 | 12 | 9.34/8.58 | NT | NT | NT |
| 67 | 3100 | 5.88/5.78 | NT | NT | NT |
| 68 | 8.6 | 8.19/8.65 | NT | NT | NT |
| 69 | 15 | 7.80/8.28 | NT | NT | NT |
| 70 | 44 | 7.71/8.05 | NT | NT | NT |
| 71 | 12,000 | * | NT | NT | NT |
| 72 | 83 | 6.11/6.10 | NT | NT | NT |
| 73 | 790 | 7.65/7.46 | NT | NT | NT |
| 74 | 6.5 | 8.56/8.39 | NT | NT | NT |
| 75 | 570 | 6.00/5.45 | NT | NT | NT |
| 76 | 5400 | 5.52/5.78 | NT | NT | NT |
| 77 | 15,000 | 5.77 | NT | NT | NT |
| 78 | 101 | 7.0 | | 93 | 60-100 |
| 79 | 4.9 | 9.2 | | 100 | >200 |
| | | | | 50 | >180 |
| 80 | 25 | 8.1 | | NT | NT |
| 81 | 18 | 8.0 | | 40 | 180 |
| 82 | 7.9 | 8.5 | | 20 | 180 |
| 83 | 3.6 | 8.3 | | 15 | >180 |
| 84 | 16 | 7.1 | | 20 | 30 |
| 85 | 8.7 | 8.9 | | NT | NT |
| 86 | 9 | 7.8 | | NT | NT |
| 87 | 91 | 7.8 | | NT | NT |
| 88 | 50 | 7.7 | | NT | NT |
| 89 | 18 | 7.9 | | NT | NT |
| 90 | 5.6 | 9.0 | | NT | NT |
| 91 | 30 | 8.6 | | 40 | >180 |
| 92 | 35 | 7.9 | | NT | NT |
| 93 | 480 | NT | | NT | NT |
| 94 | 5,800 | NT | | NT | NT |
| 95 | 66 | 8.2 | | NT | NT |
| 96 | 21 | 8.0 | | NT | NT |
| 97 | 280 | 7.7 | | NT | NT |
| 98 | 22 | 8.1 | | NT | NT |
| 99 | 280 | 6.5 | | NT | NT |
| 100 | 4.4 | 9.4 | | NT | NT |
| 101 | 36 | 7.8 | | NT | NT |
| 102 | 43 | 7.7 | | NT | NT |
| 103 | 12 | 8.0 | | NT | NT |
| 104 | 15 | 8.0 | | NT | NT |
| 105 | 290 | 6.6 | | NT | NT |
| 106 | 48 | 7.7 | | NT | NT |
| 107 | 180 | 8.3 | | NT | NT |
| 108 | 720 | 5.3 | 100 | 45 | 90 |
| 109 | 250 | 7.3 | 30 | 50 | 30 |
| 110 | 590 | 6.4 | | NT | NT |
| 111 | 45 | 9.0 | 30 | 87 | 160 |
| 112 | 2000 | 5.2 | | NT | NT |
| 113 | 12 | 8.4 | 10 | 60 | 180 |
| 114 | 400 | 6.4 | | NT | |
| 115 | 11 | 8.2 | 3 | 40 | >240 |
| 116 | 230 | 6.5 | | NT | |
| 117 | 170 | 6.5 | | NT | |
| 118 | 37 | 9.21/9.17 | 10 | 70 | 120 |
| 119 | 16 | 9.21/9.00 | 3 | 20 | 60 |
| 120 | 25 | 9.05/8.77 | 10 | 80 | 240 |
| 121 | 46 | NT | | NT | |
| 122 | 46 | NT | | NT | |
| 123 | 50 | NT | | NT | |
| 124 | 40 | 9.42/9.12 | 3 | 45 | >180 |
| 125 | 40 | 9.25/8.80 | 3 | 35 | >240 |
| 126 | 240 | 7.20/7.05 | | NT | |
| 127 | 12,000 | 4.96 | | NT | |
| 128 | 15 | 8.63/3.40 | | NT | |
| 129 | 6,700 | 5.30 | | NT | |
| 130 | 40 | 8.10/7.94 | | NT | |
| 131 | 9.5 | 7.53/8.25 | | | |
| 132 | 12 | 8.6 | | NT | |
| 133 | 10 | 8.7 | 3 | 20 | 180 |
| | | | | | 90-120 |
| 134 | 22 | 9.3 | 3 | 35 | 180 |
| 135 | 16 | 8.5 | 3 | 35 | >180 |
| 136 | NT | NT | | NT | |
| 137 | 220 | 8.3 | | NT | |
| 138 | 130 | 8.2 | | NT | |
| 139 | 0.270 | 6.3 | | NT | |
| 140 | 0.031 | 8.1 | | 100 | 160 |
| 141 | 0.110 | 8.02 | | NT | NT |
| 142 | 2.000 | NA | | NT | NT |
| 143 | 0.052 | 7.7 | | 85 | 75 |
| 144 | 0.088 | 7.7 | | 50 | 125 |
| 145 | 0.480 | 6.7 | | NT | NT |
| 146 | 0.072 | 6.4 | | NT | NT |
| 147 | 5.8 | 5.6 | 3 | 74 | 5-10 |
| 148 | 0.87 | 5.8 | 3 | 92 | 20-30 |
| 149 | 1.1 | 6.1 | 3 | NT | NT |
| 150 | 14 | 8.03/7.80 | 3 | 25 | >180 |
| 151 | 17 | 7.76/7.97 | 3 | 15 | 180 |
| 152 | 150 | 7.46/7.23 | 3 | 10 | 140 |
| 153 | 13 | 8.30/7.69 | 3 | 25 | >180 |
| 154 | 97 | 8.19/8.38 | | NA | |
| 155 | 86 | 7.60/7.14 | | NA | |
| 156 | 78 | 8.03/7.56 | | NA | |
| 157 | 530 | - /6.22 | | NA | |
| 158 | 54 | 8.23/8.14 | 3 | 30 | >180 |
| 159 | 21 | 7.92/7.56 | 3 | 10 | 150 |
| 160 | 64 | 7.87/7.71 | | | |
| 161 | 28 | | | NA | |
| 162 | 380 | 6.21/6.55 | | NA | |
| 163 | 420 | 7.42/6.75 | | NA | |
| 164 | 1700 | | | NA | |
| 165 | 410 | 6.90/7.18 | | NA | |
| 166 | 160 | 7.57/7.74 | | NA | |
| 167 | 370 | 7.08/7.11 | | NA | |
| 168 | 420 | 7.69/7.58 | | NA | |
| 169 | 150 | 7.78/7.58 | 3 | 15 | 180 |
| 170 | 26 | 7.08/7.77 | 3 | 40 | >180 |
| 171 | 28 | 7.52/7.11 | 3 | 0 | 0 |
| 172 | 70 | 7.15/7.04 | | NA | |
| 173 | 90 | 7.49/6.92 | | NA | |
| 174 | 180 | 7.29/7.02 | | NA | |
| 175 | 27 | NA | 3 | 0 | 0 |
| 176 | 9.8 | 7.69/7.55 | 3 | 10 | 150 |
| 177 | 26 | 7.41/7.85 | 3 | 15 | 180 |
| 178 | 88 | 7.54/7.47 | | NA | |
| 179 | 310 | 6.67/ - | | NA | |
| 180 | 20 | 7.56/7.15 | 3 | 25 | 180 |
| 181 | 21 | 7.70/7.12 | 3 | 20 | 180 |
| 182 | 59 | NA | | NA | |
| 183 | 390 | NA | | NA | |
| 184 | 1100 | 6.78/ - | | NA | |
| 185 | 6.5 | 8.82/8.53 | 3 | 50 | > 180 |
| 186 | 38 | 8.13/7.40 | 3 | 25 | 180 |
| 187 | 770 | 7.46/6.95 | | NA | |
| 188 | 140 | 7.72/7.09 | | NA | |
| 189 | 29 | 8.64/8.23 | | NA | |
| 190 | 10 | 7.87/7.89 | 3 | 10 | 180 |
| 191 | 81 | 7.75/7.76 | 3 | 10 | 180 |
| 192 | 140 | | | NA | |
| 193 | 11 | 9.27/8.87 | 3 | 10 | 180 |
| 194 | 47 | 7.64/7.35 | | NA | |
| 195 | 34 | 8.44/8.03 | | NA | |
| 196 | 31 | 7.68/8.26 | | NA | |
| 197 | 14 | 8.03/8.60 | | NA | |
| 198 | 7.6 | 8.76/8.64 | 3 | 35 | > 180 |
| 199 | 10 | 8.79/8.85 | 3 | 60 | > 180 |
| 200 | 20 | 8.42/8.77 | 3 | 45 | > 180 |
| 201 | 17 | 8.78/8.63 | 3 | 10 | 180 |
| 202 | 12 | 8.79/8.64 | 3 | 65 | > 180 |
| 203 | 9.2 | 8.43/8.36 | 3 | 50 | > 180 |
| 204 | 16 | 9.17/8.86 | 3 | 75 | > 180 |
| 205 | 20 | 9.14/9.15 | 3 | 40 | > 180 |
| 206 | 5.4 | 8.75/8.89 | 3 | 30 | > 180 |
| 207 | 99 | 9.04/8.60 | | NA | |
| 208 | 22 | 9.19/8.69 | 3 | 50 | > 180 |
| 209 | 5.0 | 9.41/9.16 | 3 | 25 | > 180 |
| 210 | 3.6 | 8.36/8.44 | 3 | 15 | 180 |
| 211 | 18 | 8.74/8.67 | 3 | 35 | > 180 |
| 212 | 23 | 8.85/8.25 | 3 | 15 | 180 |
| 213 | 51 | NA | | NA | |
| 214 | 65 | NA | | NA | |
| 215 | 45 | NA | | NA | |
| 216 | 5.4 | 8.80/9.04 | 3 | 50 | > 180 |
| 217 | 9.4 | NA | 3 | 65 | > 180 |
| 218 | 9.0 | NA | | NA | |
| 219 | 14 | NA | | NA | |
| 220 | 7.0 | NA | 3 | 75 | 120 |
| 221 | 4.8 | NA | 3 | 25 | > 180 |
| 222 | 5.0 | NA | | NA | |
| 223 | 14 | 7.45/7.87 | 3 | 20 | > 180 |
| 224 | 91 | NA | | NA | |
| 225 | 160 | NA | | NA | |
| 226 | 93 | NA | | NA | |
| 227 | 89 | 7.55/7.67 | | NA | |
| 228 | 4.5 | 9.17/8.25 | 3 | 80 | >180 |
| 229 | 19 | NT | 3 | 40 | >180 |
| 230 | 2.6 | 8.23/8.69 | 3 | 25 | >180 |
| 231 | 3.6 | NT | 3 | 75 | >180 |
| 232 | 4.4 | 8.59/8.89 | 3 | 70 | >180 |
| 233 | 84 | 8.51/8.78 | | NT | |
| 234 | 5.0 | 8.49/9.00 | 3 | 20 | - |
| 235 | 34 | 7.14/7.07 | | NT | |
| 236 | 4.9 | NC | 3 | 70 | >180 |
| 237 | 3.6 | NT | | NT | |
| 238 | 1.7 | NT | 3 | 15 | >180 |
| 239 | 6.8 | 7.88/8.01 | 3 | 20 | >180 |
| 240 | 120 | NA | | NA | |
| 241 | 6.9 | 8.57/8.24 | 3 | 40 | >180 |
| 242 | 110 | 7.11/6.60 | | NA | |
| 243 | 250 | NA | | NA | |
| 244 | 150 | 7.17/7.17 | | NA | |
| 245 | 98 | 6.64/7.04 | | NA | |
| 246 | 72 | 7.46/7.59 | | NA | |
| 247 | 9.4 | 8.26/8.41 | 3 | 20 | 180 |
| 248 | 20 | 7.68/7.50 | 3 | 10 | -- |
| 249 | 4.4 | NA | 3 | 20 | >180 |
| 250 | 43 | NA | 3 | 0 | -- |
| 251 | 25 | NA | | NA | |
| 252 | 13 | NA | | NA | |
| 253 | 2.6 | NA | | NA | |
| 254 | 72 | NA | | NA | |
| 255 | 12 | 7.61/7.46 | 3 | 20 | >180 |
| 256 | 4.1 | 8.43/7.78 | 3 | 30 | >180 |
| 257 | 160 | 6.63/6.68 | | NA | |
| 258 | 350 | 6.84/6.84 | | NA | |
| 259 | 54 | NA | | NA | |
| 260 | 220 | NA | | NA | |
| 261 | 18 | NA | | NA | |
| 262 | 530 | -/6.22 | | NA | |
| 263 | 57 | NA | | NA | |
| 264 | 11 | NA | | NA | |
| 265 | 110 | NA | | NA | |
| 266 | 290 | NA | | NA | |
| 267 | 25 | NA | 3 | 25 | >180 |
| 268 | 520 | NA | 3 | 0 | -- |
| 269 | 9.7 | NA | | NA | |
| 270 | 21 | NA | | NA | |
| 271 | 14 | NC | 3 | 20% | -- |
| 272 | 97 | NC | 3 | 70% | >180 min. |
| 273 | 9.8 | 8.53/8.61 | 3 | 25% | >180 min. |
| 274 | 13 | 9.06/8.85 | 3 | 35% | >180 min. |
| 275 | 6.3 | 9.07/ -- | 3 | 40% | >180 min. |
| 276 | 33 | 8.71/8.64 | 3 | <20% | |
| 277 | 190 | -- /6.54 | | NT | |
| 278 | 30 | 8.49/8.51 | 3 | 50% | >180 min. |
| 279 | 270 | 8.06/8.25 | | NT | |
| 280 | 480 | 6.41/6.35 | NT | NT | NT |

| | | | | | |
|---|---|---|---|---|---|
| NT = NOT TESTED NC = Non-Competitive antagonist *Antagonist Activity not observed up to 10 µM of test compound. | | | | | |
| 1Assay A: Angiotensin II Binding Activity | | | | | |
| 2Assay B: In Vitro Vascular Smooth Muscle Response | | | | | |
| 3Assay C: In Vivo Pressor Response | | | | | |

Test Compounds administered intragastrically, except for compounds of examples #1-#2, #4-#25, #27-#29, #30-#79, #108-#109, #111, #118 and #139-#149 which were given intraduodenally.

Administration of the angiotensin II receptor antagonist and the aldosterone receptor antagonist may take place sequentially in separate formulations, or may be accomplished by simultaneous administration in a single formulation or separate formulations. Administration may be accomplished by oral route, or by intravenous, intramuscular or subcutaneous injections. The formulation may be in the form of a bolus, or in the form of aqueous or non-aqueous isotonic sterile injection solutions or suspensions. These solutions and suspensions may be prepared from sterile powders or granules having one or more pharmaceutically-acceptable carriers or diluents, or a binder such as gelatin or hydroxypropyl-methyl cellulose, together with one or more of a lubricant, preservative, surface-active or dispersing agent.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. These may with advantage contain an amount of each active ingredient from about 1 to 250 mg, preferably from about 25 to 150 mg. A suitable daily dose for a mammal may vary widely depending on the condition of the patient and other factors. However, a dose of from about 0.01 to 30 mg/kg body weight, particularly from about 1 to 15 mg/kg body weight, may be appropriate.

The active ingredients may also be administered by injection as a composition wherein, for example, saline, dextrose or water may be used as a suitable carrier. A suitable daily dose of each active component is from about 0.01 to 15 mg/kg body weight injected per day in multiple doses depending on the disease being treated. A preferred daily dose would be from about 1 to 10 mg/kg body weight. Compounds indicated for prophylactic therapy will preferably be administered in a daily dose generally in a range from about 0.1 mg to about 15 mg per kilogram of body weight per day. A more preferred dosage will be a range from about 1 mg to about 15 mg per kilogram of body weight. Most preferred is a dosage in a range from about 1 to about 10 mg per kilogram of body weight per day. A suitable dose can be administered, in multiple sub-doses per day. These sub-doses may be administered in unit dosage forms. Typically, a dose or sub-dose may contain from about 1 mg to about 100 mg of active compound per unit dosage form. A more preferred dosage will contain from about 2 mg to about 50 mg of active compound per unit dosage form. Most preferred is a dosage form containing from about 3 mg to about 25 mg of active compound per unit dose.

In combination therapy, the aldosterone receptor antagonist may be present in an amount in a range from about 5 mg to about 400 mg, and the AII antagonist may be present in an amount in a range from about 1 mg to about 800 mg, which represents aldosterone antagonist-to-All antagonist ratios ranging from about 400:1 to about 1:160.

In a preferred combination therapy, the aldosterone receptor antagonist may be present in an amount in a range from about 10 mg to about 200 mg, and the AII antagonist may be present in an amount in a range from about 5 mg to about 600 mg, which represents aldosterone antagonist-to-AII antagonist ratios ranging from about 40:1 to about 1:60.

In a more preferred combination therapy, the aldosterone receptor antagonist may be present in an amount in a range from about 20 mg to about 100 mg, and the AII antagonist may be present in an amount in a range from about 10 mg to about 400 mg, which represents aldosterone antagonist-to-AII antagonist ratios ranging from about 10:1 to about 1:20.

The dosage regimen for treating a disease condition with the combination therapy of this invention is selected in accordance with a variety of factors, including the type, age, weight, sex and medical condition of the patient, the severity of the disease, the route of administration, and the particular compound employed, and thus may vary widely.

For therapeutic purposes, the active components of this combination therapy invention are ordinarily combined with one or more adjuvants appropriate to the indicated route of administration. If administered per os, the components may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The components may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

## Claims

1. A Combination comprising a therapeutically- effective substantially non- diuretic-effective amount of the aldosterone receptor antagonist spironolactone and a therapeutically-effective amount of an angiotensin II receptor antagonist selected from compounds of the group consisting of saralasin acetate, CGP-63170, EMD-66397, KT3-671, LR-B/081, valsartan, A-81282, BIBR-363, BIBS-222, BMS-184698, KW-3433, L-161177, LR-B/057, LY-235656, PD-150304, U-96849, U-97018, UP-275-22, WAY-126227, WK-1492.2K, YM-31472, E-4177, EMD-73495, HN-65021(elisartan), L-159282 ME-3221, SL-91.0102, tasosartan, telmisartan, UP-269-6(ripisartan), YM-358, CGP-49870, GA-0056, L-159689, L-162234, L-162441, L-163007, PD-123177, A-81988, BMS-180560, CGP-38560, CGP-48369, DA-2079, DE-3489, DuP-167, EXP-063, EXP-6155, EXP-6803, EXP-7711, EXP-9270, FK-739, HR-720, ICI-D7155, isoteoline, KRI-1177, L-158978, L-159874, LR-B087, LY-285434, LY-302289, LY-315995, RG-13647, RWJ-38970, RWJ-46458, S-8307, S-8308, saprisartan, saralasin, sarmesin, WK-1360, X-6803, ZD-7155, Cl-996, DMP-811, EXP-929, L-163017, LY-301875, XH-148, XR-510, PD 123319, and 5-[(3,5-dibutyl-1H-1,2,4-triazol-1-yl)methyl]-2-[2-(1H-tetrazol-5-yl)phenyl]pyridine.

2. A combination of claim 1 wherein said angiotensin II receptor antagonist is selected from compounds of the group consisting of valsartan, tasosartan, telmisartan, saprisartan, elisartan, ripisartan and 5-[(3,5-dibutyl-1H-1,2,4-triazol-1-yl)methyl]-2-[2-(1H-tetrazol-5-yl)phenyl] pyridine.

3. A combination of claim 2 comprising said angiotensin II receptor antagonist and 5 to 20 mg of spironolactone in the same unit dosage form or in two different associated dosage forms of the combination.

4. A combination comprising 5 to 20 mg of spironolactone and a therapeutically-effective amount of an angiotensin II receptor antagonist selected from compounds of the group consisting of candesartan, losartan, eprosartan, irbesartan, zolasartan, valsartan, tasosartan, telmisartan, saprisartan, elisartan, ripisartan and 5-[(3,5-dibutyl-1H-1,2,4-triazol-1-yl)methyl]-2-[2-(1H-tetrazol-5-yl) phenyl]pyridine in the same unit dosage form or in two different associated dosage forms of the combination.

5. The combination of Claim 4 wherein said angiotensin II receptor antagonist is candesartan.

6. The combination of Claim 4 wherein said angiotensin II receptor antagonist is losartan.

7. The combination of Claim 4 wherein said angiotensin II receptor antagonist is eprosartan.

8. The combination of Claim 4 wherein said angiotensin II receptor antagonist is irbesartan.

9. The combination of Claim 4 wherein said angiotensin II receptor antagonist is zolasartan.

10. The combination of Claim 4 wherein said angiotensin II receptor antagonist is valsartan

11. The combination of Claim 4 wherein said angiotensin II receptor antagonist is tasosartan.

12. The combination of Claim 4 wherein said angiotensin II receptor antagonist is telmisartan.

13. The combination of Claim 4 wherein said angiotensin II receptor antagonist is saprisartan.

14. The combination of Claim 4 wherein said angiotensin II receptor antagonist is elisartan.

15. The combination of Claim 4 wherein said angiotensin II receptor antagonist is ripisartan.

16. The combination of Claim 4 wherein said angiotensin II receptor antagonist is 5-[(3,5-dibutyl 1H-1,2,4-triazol-1-yl)methyl]-2-[2-(1H-tetrazol-5-yl)phenyl]pyridine.

17. The combination of Claim 4 in which spironolactone is present at not less than 10 mg in the unit dosage form.

## Patentansprüche

1. Eine Kombination, enthaltend eine therapeutisch wirksame im wesentlichen nicht diuretisch wirksame Menge des Aldosteron-Rezeptorantagonisten Spironolakton und eine therapeutisch wirksame Menge eines Angiotensin II Rezeptorantagonisten, ausgewählt aus Verbindungen der Gruppe bestehend aus Saralasinacetat, CGP-63170, EMD-66397, KT3-671, LR-B/081, Valsartan, A-81282, BIBR-363, BIBS-222, BMS-184698, KW-3433, L-161177, LR-B/057, LY-235656, PD-150304, U-96849, U-97018, UP-275-22, WAY-126227, WK-1492.2K, YM-31472, E-4177, EMD-73495, HN-65021(Elisartan), L-159282 ME-3221, SL-91.0102, Tasosartan, Telmisartan, UP-269-6 (Ripisartan), YM-358, CGP-49870, GA-0056, L-159689, L-162234, L-162441, L-1639007, PD-123177, A-81988, BMS-180560, CGP-38560, CGP-48369, DA-2079, DE-3489, DuP-167, EXP-063, EXP-6155, EXP-6803, EXP-7711, EXP-9270, FK-739, HR-720, ICI-D7155, Isoteolin, KRI-1177, L-158978, LR-B087, LY-285434, LY-302289, LY-315995, RG-13647, RWJ-38970, RWJ-46458, S-8307, S-8308, Saprisartan, Saralasin, Sarmesin, WK-1360, X-6803, ZD-7155, Cl-996, DMP-811, EXP-929, L-163017, LY-301875, XH-148, XR-510, PD 123319 und 5-[(3,5-Dibutyl-1H-1,2,4-triazol-1 yl)methyl]-2-[2-(1H-tetrazol-5-yl)phenyl]pyridin.

2. Eine Kombination des Anspruchs 1, worin dieser Angiotensin II Rezeptorantagonist ausgewählt ist aus Verbindungen der Gruppe bestehend aus Valsartan, Tasosartan, Telmisartan, Saprisartan, Elisartan, Ripisartan und 5-[(3,5-Dibutyl-1H-1,2,4-triazol-1yl)methyl]-2-[2-(1H-tetrazol-5-yl)phenyl]pyridin.

3. Eine Kombination des Anspruchs 2, enthaltend diesen Angiotensin II Rezeptorantagonisten und 5 bis 20 mg Spironolakton in der gleichen Dosierungseinheitsform oder in zwei unterschiedlichen zusammengehörigen Dosierungsformen der Kombination.

4. Eine Kombination, enthaltend 5 bis 20 mg Spironolakton und eine therapeutisch wirksame Menge eines Angiotensin II Rezeptorantagonisten, ausgewählt aus Verbindungen der Gruppe bestehend aus Candesartan, Losartan, Eprosartan Irbesartan, Zolasartan, Valsartan, Tasosartan, Telmisartan, Saprisartan, Elisartan, Ripisartan und 5-[(3,5-Dibutyl-1H-1,2,4-triazol-1yl)methyl]-2-[2-(1H-tetrazol-5-yl)phenyl]pyridin in der gleichen Dosierungseinheitsform oder in zwei unterschiedlichen zusammengehörigen Dosierungsformen der Kombination.

5. Die Kombination des Anspruchs 4, worin dieser Angiotensin II Rezeptorantagonist Candesartan ist.

6. Die Kombination des Anspruchs 4, worin dieser Angiotensin II Rezeptorantagonist Losartan ist.

7. Die Kombination des Anspruchs 4, worin dieser Angiotensin II Rezeptorantagonist Eprosartan ist.

8. Die Kombination des Anspruchs 4, worin dieser Angiotensin II Rezeptorantagonist Irbesartan ist.

9. Die Kombination des Anspruchs 4, worin dieser Angiotensin II Rezeptorantagonist Zolasartan ist.

10. Die Kombination des Anspruchs 4, worin dieser Angiotensin II Rezeptorantagonist Valsartan ist.

11. Die Kombination des Anspruchs 4, worin dieser Angiotensin II Rezeptorantagonist Tasosartan ist.

12. Die Kombination des Anspruchs 4, worin dieser Angiotensin II Rezeptorantagonist Telmisartan ist.

13. Die Kombination des Anspruchs 4, worin dieser Angiotensin II Rezeptorantagonist Saprisartan ist.

14. Die Kombination des Anspruchs 4, worin dieser Angiotensin II Rezeptorantagonist Elisartan ist.

15. Die Kombination des Anspruchs 4, worin dieser Angiotensin II Rezeptorantagonist Ripisartan ist.

16. Die Kombination des Anspruchs 4, worin dieser Angiotensin II Rezeptorantagonist 5-[(3,5-Dibutyl-1H-1,2,4-triazol-1yl)methyl]-2-[2-(1H-tetrazol-5-yl)phenyl]pyridin ist.

17. Die Kombination von Anspruch 4, worin Spironolakton in einer Menge von nicht weniger als 10 mg in der Dosierungseinheitsform vorliegt.

## Revendications

1. Combinaison comprenant une quantité essentiellement non diurétique, efficace du point de vue thérapeutique de spironolactone, à titre d'antagoniste du récepteur d'aldostérone, et une quantité efficace du point de vue thérapeutique d'un antagoniste du récepteur d'angiotensine II choisi parmi les composés du groupe comprenant l'acétate de saralasine, CGP-63170, EMD-66397, KT3-671, LR-B/081, le Valsartan, A-81282, BIBR-363, BIBS-222, BMS-184698, KW-3433, L-161177, LR-B/057, LY-235656, PD-150304, U-96849, U-97018, UP-275-22, WAY-126227, WK-1492.2K, YM-31472, E-4177, EMD-73495, HN-65021 (Elisartan), L-159282, ME-3221, SL-91.0102, le Tasosartan, Telmisartan, UP-269-6 (Ripisartan), YM-358, CGP-49870, GA-0056, L-159689, L-162234, L-162441, L-163007, PD-123177, A-81988, BMS-180560, CGP-38560, CGP-48369, DA-2079, DE-3489, DuP-167, EXP-063, EXP-6155, EXP-6803, EXP-7711, EXP-9270, FK-739, HR-720, ICI-D7155, l'Isotéoline, KRI-1177, L-158978, L-159874, LR-B087, LY-285434, LY-302289, LY-315995, RG-13647, RWJ-38970, RWJ-46458, S-8307, S-8308, le Saprisartan, la Saralasine, Sarmésine, WK-1360, X-6803, ZD-7155, CI-996, DMP-811, EXP-929, L-163017, LY-301875, XH-148, XR-510, PD 123319, et la 5[(3,5-dibutyl-1H-1,2,4-triazol-1-yl)méthyl]-2-[2-(lH-tétrazol-5-yl)phényl]pyridine.

2. Combinaison selon la revendication 1, dans laquelle ledit antagoniste du récepteur d'angiotensine II est choisi parmi les composés du groupe comprenant le Valsartan, Tasosartan, Telmisartan, Saprisartan, Elisartan, Ripisartan et la 5[(3,5-dibutyl-1H-1,2,4-triazol-1-yl)méthyl]-2-[2-(1H-tétrazol-5-yl)phényl]-pyridine.

3. Combinaison selon la revendication 2 comprenant ledit antagoniste du récepteur d'angiotensine II et 5 à 20 mg de spironolactone dans la même forme pharmaceutique unitaire ou deux formes pharmaceutiques différentes, associées, de la combinaison.

4. Combinaison comprenant 5 à 20 mg de spironolactone et une quantité efficace du point de vue thérapeutique d'un antagoniste du récepteur d'angiotensine II choisi parmi les composés du groupe comprenant le Candesartan, le Losartan, l'Eprosartan, L'Irbesartan, le Zolasartan, le Valsartan, le Tasosartan, le Telmisartan, le Saprisartan, l'Elisartan, le Ripisartan et la 5[(3,5-dibutyl-1H-1,2,4-triazol-1-yl)-méthyl]-2-[2-(1H-tétrazol-5-yl)phényl]pyridine dans la même forme pharmaceutique ou deux formes pharmaceutiques différentes, associées, de la combinaison.

5. Combinaison selon la revendication 4, dans laquelle ledit antagoniste du récepteur d'angiotensine II est le Candesartan.

6. Combinaison selon la revendication . 4, dans laquelle ledit antagoniste du récepteur d'angiotensine II est le Losartan.

7. Combinaison selon la revendication 4, dans laquelle ledit antagoniste du récepteur d'angiotensine II est l'Eprosartan.

8. Combinaison selon la revendication 4, dans laquelle ledit antagoniste du récepteur d'angiotensine II est L'Irbesartan.

9. Combinaison selon la revendication 4, dans laquelle ledit antagoniste du récepteur d'angiotensine II est le Zolasartan.

10. Combinaison selon la revendication 4, dans laquelle ledit antagoniste du récepteur d'angiotensine II est le Valsartan.

11. Combinaison selon la revendication 4, dans laquelle ledit antagoniste du récepteur d'angiotensine II est le Tasosartan.

12. Combinaison selon la revendication 4, dans laquelle ledit antagoniste du récepteur d'angiotensine II est le Telmisartan.

13. Combinaison selon la revendication 4, dans laquelle ledit antagoniste du récepteur d'angiotensine II est le Saprisartan.

14. Combinaison selon la revendication 4, dans laquelle ledit antagoniste du récepteur d'angiotensine II est l'Elisartan.

15. Combinaison selon la revendication 4, dans laquelle ledit antagoniste du récepteur d'angiotensine II est le Ripisartan.

16. Combinaison selon la revendication 4, dans laquelle ledit antagoniste du récepteur d'angiotensine II est la 5[(3,5-dibutyl-1H-1,2,4-triazol-1-yl)méthyl]-2-[2-(1H-tétrazol-5-yl)phényl]pyridine.

17. Combinaison selon la revendication 4, dans laquelle la spironolactone est présente en une quantité non inférieure à 10 mg dans la forme pharmaceutique unitaire.
